# EUROPEAN PATENT SPECIFICATION

(11) **EP 2 316 956 B1**
(45) Date of publication and mention of the grant of the patent: **22.04.2015**
(21) Application number: 10181988.6
(22) Date of filing: 02.05.2008
(51) Int. Cl.: C12N 15/82, C07K 14/415

(54) **Plants having enhanced yield-related traits and a method for making the same**
Pflanzen mit verbesserten Eigenschaften bezüglich des Ertrags und Verfahren zu ihrer Herstellung
Installations dotées de caractéristiques de rendement améliorées et procédé de fabrication de celles-ci

(30) Priority: 03.05.2007 EP 07107448; 08.05.2007 US 916575 P; 29.05.2007 EP 07109052; 29.05.2007 EP 07109068; 06.06.2007 US 942214 P; 11.06.2007 EP 07109961; 19.06.2007 EP 07110548; 19.06.2007 EP 07110557; 29.06.2007 US 937989 P; 05.07.2007 US 948036 P
(43) Date of publication of application: 04.05.2011
(62) Divisional of application: 08767542.7
(73) Proprietor: BASF Plant Science GmbH, 67056 Ludwigshafen (DE)
(72) Inventor: Sanz Molinero, Ana Isabel, 9050 Gentbrugge (BE)

(56) References cited:
- WO-A2-2004/093530
- US-B1- 7 071 380
- NOH BOSL ET AL: "Divergent roles of a pair of homologous jumonji/zinc- finger-class transcription factor proteins in the regulation of Arabidopsis flowering time", PLANT CELL, AMERICAN SOCIETY OF PLANT PHYSIOLOGISTS, ROCKVILLE, MD, US, vol. 16, no. 10, 1 October 2004 (2004-10-01), pages 2601-2613, XP002459710, ISSN: 1040-4651, DOI: DOI:10.1105/TPC.104.025353
- KLOSE ROBERT J ET AL: "JmjC-domain-containing proteins and histone demethylation", NATURE REVIEWS GENETICS, MACMILLAN MAGAZINES, GB, vol. 7, no. 9, 1 September 2006 (2006-09-01), pages 715-727, XP002459709,
- TAKEUCHI TAKASHI ET AL: "Roles of jumonji and jumonji family genes in chromatin regulation and development", DEVELOPMENTAL DYNAMICS, WILEY-LISS, INC., NEW YORK, NY, US, vol. 235, no. 9, 1 September 2006 (2006-09-01), pages 2449-2459, XP002440809, ISSN: 1058-8388, DOI: DOI:10.1002/DVDY.20851

## Description

The present invention relates generally to the field of molecular biology and concerns a method for improving various plant growth characteristics by increasing expression in a plant of a nucleic acid encoding a JMJC (JUMONJI-C) polypeptide. The present invention also concerns plants having increased expression of a nucleic acid encoding JMJC polypeptide, which plants have enhanced yield traits relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defence mechanisms.

Surprisingly, it has now been found that increasing expression of a nucleic acid encoding a JUMONJI-C or JMJC polypeptide gives plants having enhanced yield-related traits, in particular various increased yield traits relative to control plants.

According to one embodiment, there is provided a method for improving yield related traits of a plant relative to control plants, comprising increasing expression of a nucleic acid encoding a JMJC polypeptide in a plant. The improved yield related traits comprised increased total seed weight per plant, thousand-kernel (1000-seed) weight, seed filling rate, harvest index, early vigour, and root/shoot index, both under optimal growth conditions and suboptimal, mild drought conditions.

### Background

The first reported JUMONJI (which means cruciform in Japanese) gene was identified by gene trapping in mice where it plays an essential role in the development of multiple tissues. To date JUMONJI polypeptides constitute a distinct class of proteins found in prokaryotes as well as in eukaryotes including bacteria, fungi and plants.

Most members of the JUMONJI polypeptide family are characterized by the presence of a JmjC or JUMONJI-C domain. It is hypothesized that during evolution ancient proteins comprising only on JmjC domain acquired additional domains broadening the spectrum of JMJC polypeptides found in nature. Of particular interest are those JMJC polypeptides that have acquired conserved domains involved in DNA, RNA, and protein binding such as zinc fingers, FY-rich, RING finger protein and F-box domains, suggesting that polypeptides of the JUMONJI family may regulate transcription, chromatin function and/or protein turnover. Accordingly many JUMONJI proteins in animals have been reported to affect development by controlling gene expression and chromatin activity. For example a mouse JUMONJI gene acts to repress cyclin D1 in embryos and this activity is required for normal cardiogenesis (Toyoda et al. 2003 Dev Cell. 5(1):85-97.).

Much progress has been made on the understanding of the mode of action of jmjC domain containing proteins. Crucial to their biological function may be some of the recently revealed enzymatic activities in JMJC polypeptides, for example JHDM1, a JMJC polypeptide of human origin (Tsukada, Nature. 2006; 439(7078):811-6) has histone dimethylase activity, and asparaginyl hydroxylase activity has been reported in FIH (Factor Inhibiting HIF-1alpha), a transcription factor involved in cellular response to hypoxia, (Linke et al. (2004) J. Biol. Chem., Vol. 279, 14391-14397).

JmjC domains typically have structures resembling those found in some metalloenzymes. Recent structural analysis of the JmjC domain present in the JUMONJI protein FIH revealed the amino acid residues involved in binding to the cofactors 2-oxoglutarate and iron Fe (II) (Dann et al.; Proc Natl Acad Sci U S A. 2002; 99(24): 15351-15356). FIH has the HXD/E iron-binding motif characteristic of most of the 2-oxoglutarate oxygenases. In addition and consistent with the hydrolase activity, FIH secondary structure is composed of a beta-strand jellyroll core that surrounds the Fe(II)-binding site. These features are conserved amongst JmjC domain containing proteins (Trewick et al. EMBO Rep. 2005 (4):315-20).

In plants, two JMJC polypeptides are reportedly involved in the control of flowering time. They both act as repressors of flowering pathways in Arabidopsis thaliana (Noh et al. Plant Cell. 2004. 16(10):2601-13). Enzymatic activity for the plant proteins has not yet been experimentally determined.

Surprisingly, it has now been found that increasing expression in a plant of a nucleic acid encoding a JUMONJI-C or JMJC polypeptide gives plants having enhanced yield-related traits, in particular increased yield relative to control plants.

According to one embodiment, there is provided a method for improving yield-related traits of a plant relative to control plants, comprising increasing expression of a nucleic acid encoding a JMJC polypeptide in a plant. The improved yield related traits comprise one or more of increased total seed weight per plant, thousand-kernel (1000-seed) weight, seed filling rate, harvest index, Plant emergence vigour, and root/shoot index, both under optimal growth conditions and suboptimal, mild drought conditions.

### Definitions

### Polypeptide(s)/Protein(s)

The terms "polypeptide" and "protein" are used interchangeably herein and refer to amino acids in a polymeric form of any length, linked together by peptide bonds.

### Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

### Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

### Homologue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag-100 epitope, c-myc epitope, FLAG^{®}-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break α-helical structures or β-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 1 below).

**Table 1: Examples of conserved amino acid substitutions**

| **Residue** | **Conservative Substitutions** | **Residue** | **Conservative Substitutions** |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

### Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

### Orthologue(s)/Paralogue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

### Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

### Motif/Consensus sequence/Signature

The term "motif" or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

### Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitro-cellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30°C lower than the thermal melting point (Tₘ) for the specific sequence at a defined ionic strength and pH. Medium stringency conditions are when the temperature is 20°C below Tₘ, and high stringency conditions are when the temperature is 10°C below Tₘ. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The Tₘ is dependent upon the solution conditions and the base composition and length of the probe. For example, longer sequences hybridise specifically at higher temperatures. The maximum rate of hybridisation is obtained from about 16°C up to 32°C below Tₘ. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7°C for each percent formamide, and addition of 50% formamide allows hybridisation to be performed at 30 to 45°C, though the rate of hybridisation will be lowered. Base pair mismatches reduce the hybridisation rate and the thermal stability of the duplexes. On average and for large probes, the Tm decreases about 1°C per % base mismatch. The Tm may be calculated using the following equations, depending on the types of hybrids:
1) DNA-DNA hybrids (Meinkoth and Wahl, Anal. Biochem., 138: 267-284, 1984): Tₘ= 81.5°C + 16.6xlog₁₀[Na⁺]^{a} + 0.41 x%[G/C^{b}] - 500x[L^{c}]⁻¹ - 0.61 x% formamide
2) DNA-RNA or RNA-RNA hybrids: Tm= 79.8 + 18.5 (log₁₀[Na⁺]^{a}) + 0.58 (%G/C^{b}) + 11.8 (%G/C^{b})² - 820/L^{c}
3) oligo-DNA or oligo-RNA^{d} hybrids:
   For <20 nucleotides: Tₘ= 2 (Iₙ)
   For 20-35 nucleotides: Tₘ= 22 + 1.46 (Iₙ)
^{a} or for other monovalent cation, but only accurate in the 0.01-0.4 M range.
^{b} only accurate for %GC in the 30% to 75% range.
^{c} L = length of duplex in base pairs.
^{d} oligo, oligonucleotide; Iₙ, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68°C to 42°C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 µg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

### Splice variant

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

### Allelic variant

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

### Gene shuffling/Directed evolution

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

### Regulatory element/Control sequence/Promoter

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell. Generally, by "medium strength promoter" is intended a promoter that drives expression of a coding sequence at a lower level than a strong promoter, in particular at a level that is in all instances below that obtained when under the control of a 35S CaMV promoter.

### Operably linked

The term "operably linked" as used herein refers to a functional linkage between the promoter sequence and the gene of interest, such that the promoter sequence is able to initiate transcription of the gene of interest.

### Constitutive promoter

A "constitutive promoter" refers to a promoter that is transcriptionally active during most, but not necessarily all, phases of growth and development and under most environmental conditions, in at least one cell, tissue or organ. Table 2a below gives examples of constitutive promoters.

**Table 2a: Examples of constitutive promoters**

| **Gene Source** | **Reference** |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |
| 34S FMV | Sanger et al., Plant. Mol. Biol., 14, 1990: 433-443 |
| Rubisco small subunit | US 4,962,028 |
| ocs | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

### Ubiquitous promoter

A ubiquitous promoter is active in substantially all tissues or cells of an organism.

### Developmentally-regulated promoter

A developmentally-regulated promoter is active during certain developmental stages or in parts of the plant that undergo developmental changes.

### Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

### Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

Examples of root-specific promoters are listed in Table 2b below:

**Table 2b: Examples of root-specific promoters**

| **Gene Source** | **Reference** |
|---|---|
| RCc3 | Plant Mol Biol. 1995 Jan;27(2):237-48 |
| Arabidopsis PHT1 | Kovama et al., 2005; Mudge et al. (2002, Plant J. 31:341) |
| Medicago phosphate transporter | Xiao et al., 2006 |
| Arabidopsis Pyk10 | Nitz et al. (2001) Plant Sci 161(2): 337-346 |
| root-expressible genes | Tingey et al., EMBO J. 6: 1, 1987. |
| tobacco auxin-inducible gene | Van der Zaal et al., Plant Mol. Biol. 16, 983, 1991. |
| β-tubulin | Oppenheimer, et al., Gene 63: 87, 1988. |
| tobacco root-specific genes | Conkling, et al., Plant Physiol. 93: 1203, 1990. |
| B. napus G1-3b gene | United States Patent No. 5, 401, 836 |
| SbPRP1 | Suzuki et al., Plant Mol. Biol. 21: 109-119, 1993. |
| LRX1 | Baumberger et al. 2001, Genes & Dev. 15:1128 |
| BTG-26 Brassica napus | US 20050044585 |
| LeAMT1 (tomato) | Lauter et al. (1996, PNAS 3:8139) |
| The LeNRT1-1 (tomato) | Lauter et al. (1996, PNAS 3:8139) |
| class I patatin gene (potato) | Liu et al., Plant Mol. Biol. 153:386-395, 1991. |
| KDC1 (Daucus carota) | Downey et al. (2000, J. Biol. Chem. 275:39420) |
| TobRB7 gene | W Song (1997) PhD Thesis, North Carolina State University, Raleigh, NC USA |
| OsRAB5a (rice) | Wang et al. 2002, Plant Sci. 163:273 |
| ALF5 (Arabidopsis) | Diener et al. (2001, Plant Cell 13:1625) |
| NRT2;1Np (N. plumbaginifolia) | Quesada et al. (1997, Plant Mol. Biol. 34:265) |

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters (endosperm/aleurone/embryo specific) are shown in Table 2d, 2e, 2f. Further examples of seed-specific promoters are given in Qing Qu and Takaiwa (Plant Biotechnol. J. 2, 113-125, 2004).

**Table 2c: Examples of seed-specific promoters**

| **Gene source** | **Reference** |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice α-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophosphorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO0136, rice alanine aminotransferase | unpublished |
| PRO0147, trypsin inhibitor ITR1 (barley) | unpublished |
| PRO0151, rice WSI18 | WO 2004/070039 |
| PRO0175, rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 2d: examples of endosperm-specific promoters**

| **Gene source** | **Reference** |
|---|---|
| glutelin (rice) | Takaiwa et al. (1986) Mol Gen Genet 208:15-22; |
| | Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley ltr1 promoter | Diaz et al. (1995) Mol Gen Genet 248(5):592-8 |
| barley B1, C, D, hordein | Cho et al. (1999) Theor Appl Genet 98:1253-62; |
| | Muller et al. (1993) Plant J 4:343-55; |
| | Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 2e: Examples of embryo specific promoters:**

| **Gene source** | **Reference** |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 2f: Examples of aleurone-specific promoters:**

| **Gene source** | **Reference** |
|---|---|
| α-amylase (Amy32b) | Lanahan et al, Plant Cell 4:203-211, 1992; |
| | Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Examples of green tissue-specific promoters which may be used to perform the methods of the invention are shown in Table 2g.

**Table 2g: Examples of green tissue-specific promoters**

| **Gene** | **Expression** | **Reference** |
|---|---|---|
| Maize Orthophosphate dikinase | Leaf specific | Fukavama et al., 2001 |
| Maize Phosphoenolpyruvate carboxylase | Leaf specific | Kausch et al., 2001 |
| Rice Phosphoenolpyruvate carboxylase | Leaf specific | Liu et al., 2003 |
| Rice small subunit Rubisco | Leaf specific | Nomura et al., 2000 |
| rice beta expansin EXBP9 | Shoot specific | WO 2004/070039 |
| Pigeonpea small subunit Rubisco | Leaf specific | Panguluri et al., 2005 |
| Pea RBCS3A | Leaf specific | |

Another example of a tissue-specific promoter is a meristem-specific promoter, which is transcriptionally active predominantly in meristematic tissue, substantially to the exclusion of any other parts of a plant, whilst still allowing for any leaky expression in these other plant parts. Examples of green meristem-specific promoters which may be used to perform the methods of the invention are shown in Table 2h below.

**Table 2h: Examples of meristem-specific promoters**

| **Gene source** | **Expression pattern** | **Reference** |
|---|---|---|
| rice OSH1 | Shoot apical meristem, from embryo globular stage to seedling stage | Sato et al. (1996) Proc. Natl. Acad. Sci. USA, 93: 8117-8122 |
| Rice metallothionein | Meristem specific | BAD87835.1 |
| WAK1 & WAK 2 | Shoot and root apical meristems, and in expanding leaves and sepals | Wagner & Kohorn (2001) Plant Cell 13(2): 303-318 |

### Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

### Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

### Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

### Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

### Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

### Decreased expression

Reference herein to "decreased expression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an α-anomeric nucleic acid sequence. An α-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation(s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

### Selectable marker (gene)/Reporter gene

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta^{®}; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

### Transgenic/Transgene/Recombinant

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either
(a) the nucleic acid sequences encoding proteins useful in the methods of the invention, or
(b) genetic control sequence(s) which is operably linked with the nucleic acid sequence according to the invention, for example a promoter, or
(c) a) and b)
are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

### Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via Agrobacterium-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994). In the case of corn transformation, the preferred method is as described in either Ishida et al. (Nat. Biotechnol 14(6): 745-50, 1996) or Frame et al. (Plant Physiol 129(1): 13-22, 2002). Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis (Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

### TILLING

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product. Methods for TILLING are well known in the art (McCallum et al., (2000) Nat Biotechnol 18: 455-457; reviewed by Stemple (2004) Nat Rev Genet 5(2): 145-50).

### Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; lida and Terada (2004) Curr Opin Biotech 15(2): 132-8), and approaches exist that are generally applicable regardless of the target organism (Miller et al, Nature Biotechnol. 25, 778-785, 2007).

### Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

### Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

### Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

### Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

### Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

### Plant

The term "plant" as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., *Cocos* spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Eragrostis tef, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Tripsacum dactyloides, Triticosecale rimpaui, Triticum* spp. *(e.g. Triticum aestivum, Triticum durum, Triticum turgidum, Triticum hybernum, Triticum macha, Triticum sativum, Triticum monococcum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

### Detailed description of the invention

Surprisingly, it has now been found that increasing expression in a plant of a nucleic acid encoding a JMJC polypeptide gives plants having enhanced yield-related traits relative to control plants. According to a first embodiment, the present invention provides a method for enhancing yield-related traits in plants relative to control plants, comprising increasing expression in a plant of a nucleic acid encoding a JMJC polypeptide.

A preferred method for increasing expression of a nucleic acid encoding a JMJC polypeptide is by introducing and expressing in a plant a nucleic acid encoding a JMJC polypeptide.

Any reference hereinafter to a "protein useful in the methods of the invention" is taken to mean a JMJC polypeptide as defined herein. Any reference hereinafter to a "nucleic acid useful in the methods of the invention" is taken to mean a nucleic acid capable of encoding such a JMJC polypeptide. The nucleic acid to be introduced into a plant (and therefore useful in performing the methods of the invention) is any nucleic acid encoding the type of protein, which will now be described, hereafter also named "JMJC nucleic acid" or "JMJC gene".

A JMJC polypeptide as defined herein refers to a polypeptide comprising a JmjC domain represented by a sequence having at least 60% or more sequence identity to:
(i) SEQ ID NO: 6; and/or
(ii) one of the JmjC domains comprised in the JMJC polypeptides represented by SEQ ID NO: 12; SEQ ID NO: 14; SEQ ID NO: 24; SEQ ID NO: 26; SEQ ID NO: 32; SEQ ID NO: 34, SEQ ID NO: 36; SEQ ID NO: 38; SEQ ID NO: 40; SEQ ID NO: 42; SEQ ID NO: 44; SEQ ID NO: 46; SEQ ID NO: 48; SEQ ID NO: 50; SEQ ID NO: 52; SEQ ID NO: 56; SEQ ID NO: 58; SEQ ID NO: 60; and SEQ ID NO: 62, whose amino acid coordinates are given in Table A4; and
(iii) wherein said JMJC polypeptides comprise a motif having at least 60% or more sequence identity to any one or more of:
   (a) SEQ ID NO: 7,
   (b) SEQ ID NO: 8,
   (c) SEQ ID NO: 9,
   (d) SEQ ID NO: 10.

The JmjC domain is a conserved sequence found in proteins of prokaryotic and eukaryotic organisms. JmjC domains are on average 111 amino acids long. Typically the length of a JmjC domain can range between 25 to 200 amino acids though shorter and longer versions maybe possible. JmjC domains are predicted to be metalloenzymes that adopt the cupin fold, and are candidates for enzymes that regulate chromatin remodelling (Clissold et al. Trends Biochem Sci. 2001 Jan;26(1):7-9).

JMJC polypeptides can be found in specialized databases such as Pfam, (Finn et al. Nucleic Acids Research (2006) Database Issue 34:D247-D251). Pfam compiles a large collection of multiple sequence alignments and hidden Markov models (HMM) covering many common protein domains and families and is available through the Sanger Institute in the United Kingdom.

The gathering cutoff threshold of the JmjC domain in the Pfam HMM_fs model is of 16,0 and of -8,0 in the HMM_ls model. Trusted matches as considered in the Pfam database are those scoring higher than the gathering cut-off threshold. However potential matches, comprising true JmjC domains, may still fall under the gathering cut-off. Preferably a JMJC polypeptide is a protein having one or more domains in their sequence that exceed the gathering cutoff of the Pfam protein domain family PF02373, jumonji, jmjC,

Alternatively, a JmjC domain in a polypeptide may be identified by performing a sequence comparison with known polypeptides comprising a JmjC domain and establishing the similarity in the region of the JmjC domain. The sequences may be aligned using any of the methods well known in the art such as Blast algorithms and the probability for the alignment to occur with a given sequence is taken as basis for identifying similar polypeptides. A parameter that is typically used to represent such probability in pair wise comparisons is called e-value. The e-value describes how often a given score is expected to occur random; The e-value cut-off may be as high as 1.0. Typically alignments with high likelihood to occur have an e-value lower than 0.1, 0.01, 0.001, 1.e-05, 1.e-10, 1.e-15, 1.e-20, 1.e-25, 1.e-50, 1.e-75, 1.e-100 and 1.e-200. Preferably JMJC polypeptides of the invention comprise a sequence having in increasing order of preference an e-value lower than 0.1, 0.01, 0.001, 1.e-05, 1.e-10, 1.e-15, 1.e-20, 1.e-25, 1.e-50, 1.e-75, 1.e-100 and 1.e-200 for an alignment with JmjC domain found in a known JMJC polypeptide.

Examples of JMJC polypeptides are given in Table A1. The amino acid coordinates of the JmjC domains as present in representative JMJC polypeptides of dicotyledonous origin is given in Table A4. An example of JmjC domain is given in SEQ ID NO: 6.

The sequence identity between JmjC domains is typically low, in average 23% and maybe as low as 10%. Preferred JMJC polypeptides of the inventions are those having in increasing order of preference at least 60%, 65%, 70%, 75%, 80%, 85%, 90%, 95%, or more sequence identity to SEQ ID NO: 6 or to any of the JmjC domains comprised in the JMJC polypeptides represented by SEQ ID NO: 12; SEQ ID NO: 14; SEQ ID NO: 24; SEQ ID NO: 26; SEQ ID NO: 32; SEQ ID NO: 36; SEQ ID NO: 38; SEQ ID NO: 112; SEQ ID NO: 42; SEQ ID NO: 44; SEQ ID NO: 46; SEQ ID NO: 48; SEQ ID NO: 50; SEQ ID NO: 52; SEQ ID NO: 56; SEQ ID NO: 58; SEQ ID NO: 60; and SEQ ID NO: 62, whose amino acid coordinates are given in Table A4.

In addition to the JmjC domain, JMJC polypeptides may optionally comprise other highly conserved sequence motifs, such as those represented in SEQ ID NO: 7, SEQ ID NO: 8, SEQ ID NO: 9, which are found in SEQ ID NO: 2. Further, the polypeptides of the invention may comprise a conserved sequence found in oxygenases which has been involved in the coordination of iron cations, herein represented by HXD(V) or EXnH (SEQ ID NO: 10), wherein "X" represents any amino acid and "n" represents the number of X-residues ranging between 1-5, both included.

A JMJC polypeptide of the invention refers to any polypeptide comprising a JmjC domain and having in increasing order of preference at least 60%, 65%, 70%, 75%, 80%, 85%, 90%, 95% or more sequence identity to any one or more of the conserved sequence motifs as represented by SEQ ID NO: 7, SEQ ID NO: 8, SEQ ID NO: 9 and SEQ ID NO: 10.

Evidence of domain swapping in the JUMONJI family of proteins has been reported, (Balciunas and Ronne. Trends Biochem Sci 2000;25:274-276). Accordingly, in addition to the JmjC domain, JMJC polypeptides typically contain one or more different kinds of other known conserved domains. Relevant to the polypeptides of the invention are JMJC polypeptides that in addition to the JmjC domain comprise other conserved domains, which are presumably involved in DNA binding and transcription activities such as zinc fingers and/or in protein interaction and protein turnover, such as F-box and zinc finger-RING-type domains.

Therefore the JMJC polypeptide useful in the methods of the invention comprises a JmjC domain and optionally one or more of the following conserved domains: JmJN (pfam accession number PF02375); C5HC2 zinc finger (pfam accession number: PF02928), FY-rich domain, N-terminal (InterPro accession number: IPR003888) and FY-rich domain, C-terminal (InterPro accession number: IPR003889), a Zinc finger FYVE/PHD-Zn type (InterPro accession number: IPR011011), a Zinc finger C2H2 type (pfam accession number: PF00096), a Zinc finger - RING type (zf-C3HC4) (pfam accession number PF00097) and an F-box domain (pfam accession number: PF00646).

The JMJC polypeptide of the invention comprises a sequence having in increasing order of preference of 60%, 70%, 75%, 60%, 85%, 90%, 92%, 94%, 96%, 98% or more sequence identity to SEQ ID NO: 2, SEQ ID NO: 14, SEQ ID NO: 22, SEQ ID NO: 32, SEQ ID NO: 50, SEQ ID NO: 62, SEQ ID NO: 64, SEQ ID NO: 68, SEQ ID NO: 70 and SEQ ID NO: 76.

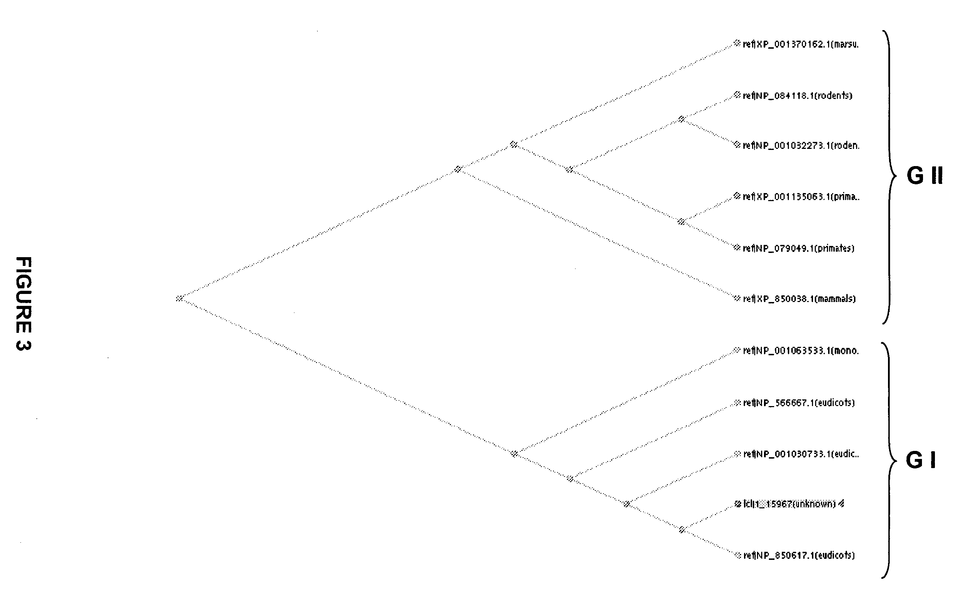

Preferably, the polypeptide sequence which when used in the construction of a phylogenetic tree, such as the one depicted in Figure 3, clusters with the group of JMJC polypeptides (Takeuchi et al. Dev Dyn. 2006 235(9): 2449-59) comprising the amino acid sequence represented by SEQ ID NO: 2 rather than with any other group.

The term "domain" and "motif" is defined in the "definitions" section herein. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244, InterPro (Mulder et al., (2003) Nucl. Acids. Res. 31, 315-318, Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004), or Pfam (Bateman et al., Nucleic Acids Research 30(1): 276-280 (2002). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31:3784-3788(2003)). Domains may also be identified using routine techniques, such as by sequence alignment.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters.

Furthermore, JMJC polypeptides may typically have protein hydroxylases activity, in particular peptide-aspartate beta-dioxygenase (PABD) activity (EC 1.14.11.16). The reaction catalyzed is: peptide L-aspartate + 2-oxoglutarate + O(2) <=> peptide 3-hydroxy-L-aspartate + succinate + CO(2). The cofactors in the reaction are iron and 2-Oxogluatarate. Tools and techniques for measuring PABD activity are well known in the art (see for example Lavaissiere et al. J Clin Invest. 1996;98(6):1313-23; Linke et al. J Biol Chem. 2004;279(14):14391-7; Lee, et al. J. Biol. Chem. 278:7558-7563; 2003; Lando et al. Genes Dev. 16:1466-1471; 2002). Iron (II)/2-oxoglutarate (2-OG)-dependent oxygenases catalyse oxidative reactions in various metabolic reactions. Recently it has been proposed that JMJC polypeptides can also have histone demethylase activity (Trewick et al. 2005).

The present invention is illustrated by transforming plants with the nucleic acid sequence represented by SEQ ID NO: 1, encoding the polypeptide sequence of SEQ ID NO: 2. However, performance of the invention is not restricted to these sequences; the methods of the invention may advantageously be performed using any JMJC-encoding nucleic acid or JMJC polypeptide as defined herein.

Examples of nucleic acids encoding JMJC polypeptides are given in Table A1 of Example 1 herein. Such nucleic acids are useful in performing the methods of the invention. The amino acid sequences given in Table A1 of Example 1 are example sequences of orthologues and paralogues of the JMJC polypeptide represented by SEQ ID NO: 2, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table A1 of Example 1) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against Arabidopsis sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acid variants may also be useful in practising the methods of the invention. Examples of such variants include nucleic acids encoding homologues and derivatives of any one of the amino acid sequences given in Table A1 of Example 1, the terms "homologue" and "derivative" being as defined herein. Also useful in the methods of the invention are nucleic acids encoding homologues and derivatives of orthologues or paralogues of any one of the amino acid sequences given in Table A1 of Example 1. Homologues and derivatives useful in the methods of the present invention have substantially the same biological and functional activity as the unmodified protein from which they are derived.

Further nucleic acid variants useful in practising the methods of the invention include portions of nucleic acids encoding JMJC polypeptides, nucleic acids hybridising to nucleic acids encoding JMJC polypeptides, splice variants of nucleic acids encoding JMJC polypeptides, allelic variants of nucleic acids encoding JMJC polypeptides and variants of nucleic acids encoding JMJC polypeptides obtained by gene shuffling. The terms hybridising sequence, splice variant, allelic variant and gene shuffling are as described herein.

Nucleic acids encoding JMJC polypeptides need not be full-length nucleic acids, since performance of the methods of the invention does not rely on the use of full-length nucleic acid sequences. According to the present invention, there is provided a method for enhancing yield-related traits in plants, comprising introducing and expressing in a plant a portion of any one of the nucleic acid sequences given in Table A1 of Example 1, or a portion of a nucleic acid encoding an orthologue, paralogue or homologue of any of the amino acid sequences given in Table A1 of Example 1.

A portion of a nucleic acid may be prepared, for example, by making one or more deletions to the nucleic acid. The portions may be used in isolated form or they may be fused to other coding (or non-coding) sequences in order to, for example, produce a protein that combines several activities. When fused to other coding sequences, the resultant polypeptide produced upon translation may be bigger than that predicted for the protein portion.

Portions useful in the methods of the invention, encode a JMJC polypeptide as defined herein, and have substantially the same biological activity as the amino acid sequences given in Table A1 of Example 1. Preferably, the portion is a portion of any one of the nucleic acids given in Table A1 of Example 1, or is a portion of a nucleic acid encoding an orthologue or paralogue of any one of the amino acid sequences given in Table A1 of Example 1. Preferably the portion is at least 100, 200, 500, 550, 600, 650, 700, 750, 800, 850, 900, 950, 1000, 1500, 2000, 2500, 3000, 3500, 4000, 4500, 5000 consecutive nucleotides in length, the consecutive nucleotides being of any one of the nucleic acid sequences given in Table A1 of Example 1, or of a nucleic acid encoding an orthologue or paralogue of any one of the amino acid sequences given in Table A1 of Example 1. Most preferably the portion is a portion of the nucleic acid of SEQ ID NO: 1. Preferably, the portion encodes an amino acid sequence which when used in the construction of a phylogenetic tree, such as the one depicted in Figure 3, clusters with the group of JMJC polypeptides (Takeuchi et al. Dev Dyn. 2006 235(9):2449-59) comprising the amino acid sequence represented by SEQ ID NO: 2 rather than with any other group.

Another nucleic acid variant useful in the methods of the invention is a nucleic acid capable of hybridising, under reduced stringency conditions, preferably under stringent conditions, with a nucleic acid encoding a JMJC polypeptide as defined herein, or with a portion as defined herein.

According to the present invention, there is provided a method for enhancing yield-related traits in plants, comprising introducing and expressing in a plant a nucleic acid capable of hybridizing to any one of the nucleic acids given in Table A1 of Example 1, or comprising introducing and expressing in a plant a nucleic acid capable of hybridising to a nucleic acid encoding an orthologue, paralogue or homologue of any of the nucleic acid sequences given in Table A1 of Example 1.

Hybridising sequences useful in the methods of the invention encode a JMJC polypeptide as defined herein, and have substantially the same biological activity as the amino acid sequences given in Table A1 of Example 1. Preferably, the hybridising sequence is capable of hybridising to any one of the nucleic acids given in Table A1 of Example 1, or to a portion of any of these sequences, a portion being as defined above, or wherein the hybridising sequence is capable of hybridising to a nucleic acid encoding an orthologue or paralogue of any one of the amino acid sequences given in Table A1 of Example 1. Most preferably, the hybridising sequence is capable of hybridising to a nucleic acid as represented by SEQ ID NO: 1 or to a portion thereof.

Preferably, the hybridising sequence encodes an amino acid sequence which when used in the construction of a phylogenetic tree, such as the one depicted in Figure 3, clusters with the group of JMJC polypeptides (Takeuchi et al. Dev Dyn. 2006 235(9):2449-59) comprising the amino acid sequence represented by SEQ ID NO: 2 rather than with any other group.

Another nucleic acid variant useful in the methods of the invention is a splice variant encoding a JMJC polypeptide as defined hereinabove, a splice variant being as defined herein.

According to the present invention, there is provided a method for enhancing yield-related traits in plants, comprising introducing and expressing in a plant a splice variant of any one of the nucleic acid sequences given in Table A1 of Example 1, or a splice variant of a nucleic acid encoding an orthologue, paralogue or homologue of any of the amino acid sequences given in Table A1 of Example 1. An example of a spliced variant of the gene encoding SEQ ID NO: 2 is represented by SEQ ID NO: 1 and SEQ ID NO: 83 (encoding SEQ ID NO: 12).

Preferred splice variants are splice variants of a nucleic acid represented by SEQ ID NO: 1, or a splice variant of a nucleic acid encoding an orthologue or paralogue of SEQ ID NO: 2. Preferably, the amino acid sequence encoded by the splice variant, when used in the construction of a phylogenetic tree, such as the one depicted in Figure 3, clusters with the group of JMJC polypeptides (Takeuchi et al. Dev Dyn. 2006 235(9):2449-59) comprising the amino acid sequence represented by SEQ ID NO: 2 any other group.

Another nucleic acid variant useful in performing the methods of the invention is an allelic variant of a nucleic acid encoding a JMJC polypeptide as defined hereinabove, an allelic variant being as defined herein.

According to the present invention, there is provided a method for enhancing yield-related traits in plants, comprising introducing and expressing in a plant an allelic variant of any one of the nucleic acids given in Table A1 of Example 1, or comprising introducing and expressing in a plant an allelic variant of a nucleic acid encoding an orthologue, paralogue or homologue of any of the amino acid sequences given in Table A1 of Example 1.

The allelic variants useful in the methods of the present invention have substantially the same biological activity as the JMJC polypeptide of SEQ ID NO: 2 and any of the amino acids depicted in Table A1 of Example 1. Allelic variants exist in nature, and encompassed within the methods of the present invention is the use of these natural alleles. Preferably, the allelic variant is an allelic variant of SEQ ID NO: 1 or an allelic variant of a nucleic acid encoding an orthologue or paralogue of SEQ ID NO: 2. Preferably, the amino acid sequence encoded by the allelic variant, when used in the construction of a phylogenetic tree, such as the one depicted in Figure 3, clusters with the JMJC polypeptides (Takeuchi et al. Dev Dyn. 2006 235(9):2449-59) comprising the amino acid sequence represented by SEQ ID NO: 2 rather than with any other group.

Gene shuffling or directed evolution may also be used to generate variants of nucleic acids encoding JMJC polypeptides as defined above; the term "gene shuffling" being as defined herein.

According to the present invention, there is provided a method for enhancing yield-related traits in plants, comprising introducing and expressing in a plant a variant of any one of the nucleic acid sequences given in Table A1 of Example 1, or comprising introducing and expressing in a plant a variant of a nucleic acid encoding an orthologue, paralogue or homologue of any of the amino acid sequences given in Table A1 of Example 1, which variant nucleic acid is obtained by gene shuffling.

Advantageously, the present invention provides hitherto unknown JMJ nucleic acid and polypeptide sequences.

According to a further embodiment of the present application, there is provided an isolated nucleic acid molecule comprising:
(i) a nucleic acid represented by SEQ ID NO: 25;
(ii) a nucleic acid or fragment thereof that is complementary to SEQ ID NO: 25;
(iii) a nucleic acid encoding an JMJC polypeptide having, in increasing order of preference, at least 70%, 75%, 80%, 85%, 90%, 95%, 96%, 97%, 98%, 99% or more sequence identity to the amino acid sequence given in SEQ ID NO: 26;
(iv) a nucleic acid capable of hybridizing under stringent conditions to any one of the nucleic acids given in (i), (ii) or (iii) above.

According to a further embodiment of the present application, there is therefore provided an isolated polypeptide comprising:
(i) an amino acid sequence having, in increasing order of preference, at least 70%, 75%, 80%, 85%, 90%, 95%, 96%, 97%, 98%, 99% or 100% or more sequence identity to the amino acid sequence given in SEQ ID NO: 26;
(ii) derivatives of any of the amino acid sequences given in (i).

Preferably, the amino acid sequence encoded by the variant nucleic acid obtained by gene shuffling, when used in the construction of a phylogenetic tree such as the one depicted in Figure 3, clusters with the group of JMJC polypeptides (Takeuchi et al. Dev Dyn. 2006 235(9):2449-59) comprising the amino acid sequence represented by SEQ ID NO: 2 rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding JMJC polypeptides may be derived from any natural or artificial source. The nucleic acid may be modified from its native form in composition and/or genomic environment through deliberate human manipulation. Preferably the JMJC polypeptide-encoding nucleic acid is from a plant, further preferably from a dicotyledonous plant, more preferably from the family Brassicaceae, most preferably the nucleic acid is from *Arabidopsis thaliana.*

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground. In particular, such harvestable parts are seeds, and performance of the methods of the invention results in plants having increased yield relative to the yield of control plants.

Taking com as an example, a yield increase may be manifested as one or more of the following: increase in the number of plants established per hectare or acre, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per hectare or acre, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

The present invention provides a method for increasing at least one yield-related trait selected from plant emergence vigour, root/shoot index, seed yield, seed filling rate, harvest index and thousand kernel weight in plants, relative to control plants, which method comprises increasing expression in a plant of a nucleic acid encoding a JMJC polypeptide as defined herein.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per acre (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises increasing expression in a plant of a nucleic acid encoding a JMJC polypeptide as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location. Plants with optimal growth conditions, (grown under non-stress conditions) typically yield in increasing order of preference at least 90%, 87%, 85%, 83%, 80%, 77% or 75% of the average production of such plant in a given environment. Average production may be calculated on harvest and/or season basis. Persons skilled in the art are aware of average yield productions of a crop.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises increasing expression in a plant of a nucleic acid encoding a JMJC polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a JMJC polypeptide. Nutrient deficiency may result from a lack or excess of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding a JMJC polypeptide as defined above.

The invention also provides genetic constructs and vectors to facilitate introduction and/or expression in plants of nucleic acids encoding JMJC polypeptides. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:
(a) a nucleic acid encoding a JMJC polypeptide as defined above;
(b) one or more control sequences capable of driving expression of the nucleic acid sequence of (a) comprising a GOS2 promoter; and optionally
(c) a transcription termination sequence.

Preferably, the nucleic acid encoding a JMJC polypeptide is as defined above. The term "control sequence" and "termination sequence" are as defined herein.

Plants are transformed with a vector comprising any of the nucleic acids described above. The skilled artisan is well aware of the genetic elements that must be present on the vector in order to successfully transform, select and propagate host cells containing the sequence of interest. The sequence of interest is operably linked to one or more control sequences (at least to a promoter).

Advantageously, any type of promoter, whether natural or synthetic, may be used to drive expression of the nucleic acid sequence. A constitutive promoter is particularly useful in the methods. See the "Definitions" section herein for definitions of the various promoter types.

It should be clear that the applicability of the method of the present invention is not restricted to the JMJC polypeptide-encoding nucleic acid represented by SEQ ID NO: 1, nor is the applicability of the method of the invention restricted to expression of a JMJC polypeptide-encoding nucleic acid when driven by a constitutive promoter.

The constitutive promoter is preferably a GOS2 promoter, preferably a GOS2 promoter from rice. Further preferably the constitutive promoter is represented by a nucleic acid sequence substantially similar to SEQ ID NO: 3, most preferably the constitutive promoter is as represented by SEQ ID NO: 3. See the "Definitions" section herein for further examples of constitutive promoters.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die). The marker genes may be removed or excised from the transgenic cell once they are no longer needed. Techniques for marker gene removal are known in the art, useful techniques are described above in the definitions section.

The invention also provides a method for the production of transgenic plants having enhanced yield-related traits relative to control plants, comprising introduction and expression in a plant of any nucleic acid encoding a JMJC polypeptide as defined hereinabove.

More specifically, the present invention provides a method for the production of transgenic plants having increased enhanced yield-related traits, particularly increased (seed) yield, which method comprises:
(i) introducing and expressing in a plant or plant cell a JMJC polypeptide-encoding nucleic acid operably linked to a GOS2 promoter; and
(ii) cultivating the plant cell under conditions promoting plant growth and development.

The nucleic acid of (i) may be any of the nucleic acids capable of encoding a JMJC polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques. The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a JMJC polypeptide as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, roots, rhizomes, tubers and bulbs. The invention furthermore relates to products derived, preferably directly derived, from a harvestable part of such a plant, such as dry pellets or powders, oil, fat and fatty acids, starch or proteins.

According to the invention, the geneexpression is increased expression. Methods for increasing expression of nucleic acids or genes, or gene products, are well documented in the art and examples are provided in the definitions section.

As mentioned above, a preferred method for increasing expression of a nucleic acid encoding a JMJC polypeptide is by introducing and expressing in a plant a nucleic acid encoding a JMJC polypeptide; however the effects of performing the method, i.e. enhancing yield-related traits may also be achieved using other well known techniques, including but not limited to T-DNA activation tagging, TILLING, homologous recombination. A description of these techniques is provided in the definitions section.

The present invention also encompasses use of nucleic acids encoding JMJC polypeptides as described herein and use of these JMJC polypeptides in enhancing any of the aforementioned yield-related traits in plants.

Nucleic acids encoding JMJC polypeptides described herein, or the JMJC polypeptides themselves, may find use in breeding programmes in which a DNA marker is identified which may be genetically linked to a JMJC polypeptide-encoding gene. The nucleic acids/genes, or the JMJC polypeptides themselves may be used to define a molecular marker. This DNA or protein marker may then be used in breeding programmes to select plants having enhanced yield-related traits as defined hereinabove in the methods of the invention.

Allelic variants of a JMJC polypeptide-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding JMJC polypeptides may also be used as probes for genetically and physically mapping the genes that they are a part of, and as markers for traits linked to those genes. Such information may be useful in plant breeding in order to develop lines with desired phenotypes. Such use of JMJC polypeptide-encoding nucleic acids requires only a nucleic acid sequence of at least 15 nucleotides in length. The JMJC polypeptide-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the JMJC-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the JMJC polypeptide-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

### Description of figures

The present invention will now be described with reference to the following figures in which:

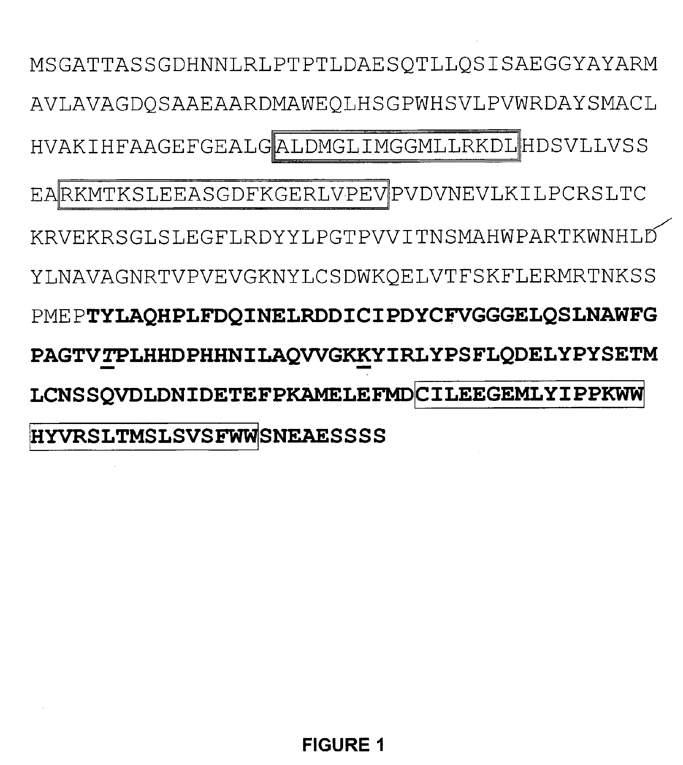

**Figure 1** represents the amino acid sequence and domain structure of SEQ ID NO: 2. Conserved motifs and domains are indicated. JmjC domain is indicated in bold. Conserved motifs SEQ ID NO: 7, SEQ ID NO: 8 and SEQ ID NO: 9 are indicated by single, double and triple lined rectangles respectively. Underlined are the T (Theonine) and K (Lysine) amino acid residues that typically participate in 2-Oxogutarate coordination. Capital H indicates the Histidine amino acid residue coordinating the iron ion.
**Figure 2** represents a multiple alignment of JMJC proteins. The origin of the protein is indicated by the first two alphanumeric digits in the name, At: Arabidopsis thaliana, Pp: Populus trichocarpa, Os: Oryza sativa, Ot: streococcus tauri, Ce: Caenorhabditis elegans, Hs: Homo sapiens. Position of the conserved amino acid residues and domains corresponding to those described in Figure 1 is indicated. A consensus sequence is given. Highly conserved amino acids in the consensus sequences are given; empty blank spaces represent any amino acid.
**Figure 3** shows a phylogenetic tree of JMJC polypeptides. The arrow shows SEQ ID NO: 2. Other JMJC polypeptides are named using the Genbank accession number. Group I (G I) comprises proteins of plant origin; Group II (GII) comprises proteins of non-plant origin.
**Figure 4** represents the binary vector for increased expression in Oryza sativa of SEQ ID NO: 1 under the control of a rice GOS2 promoter (pGOS2).

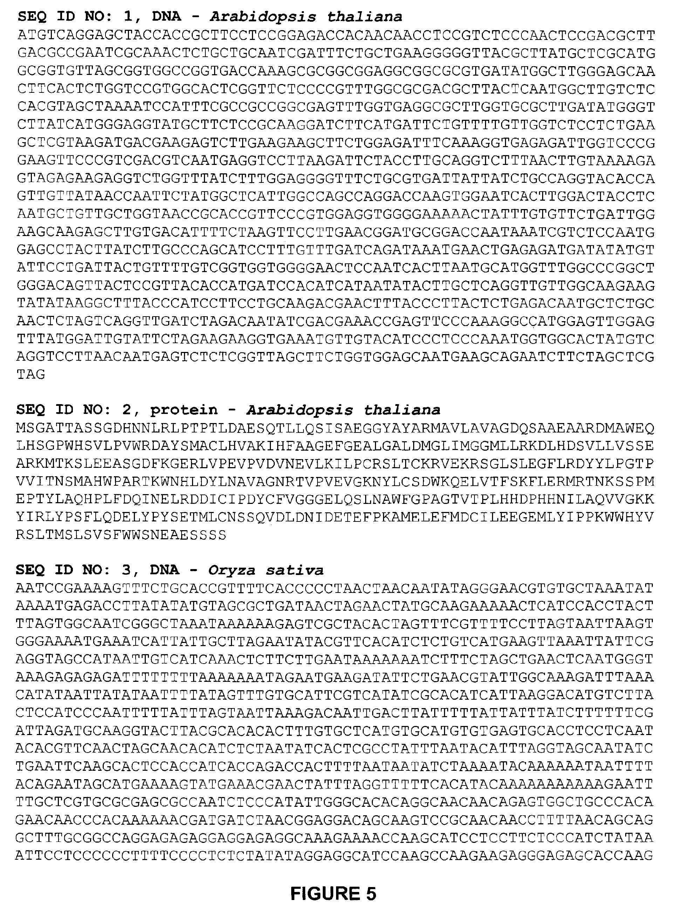

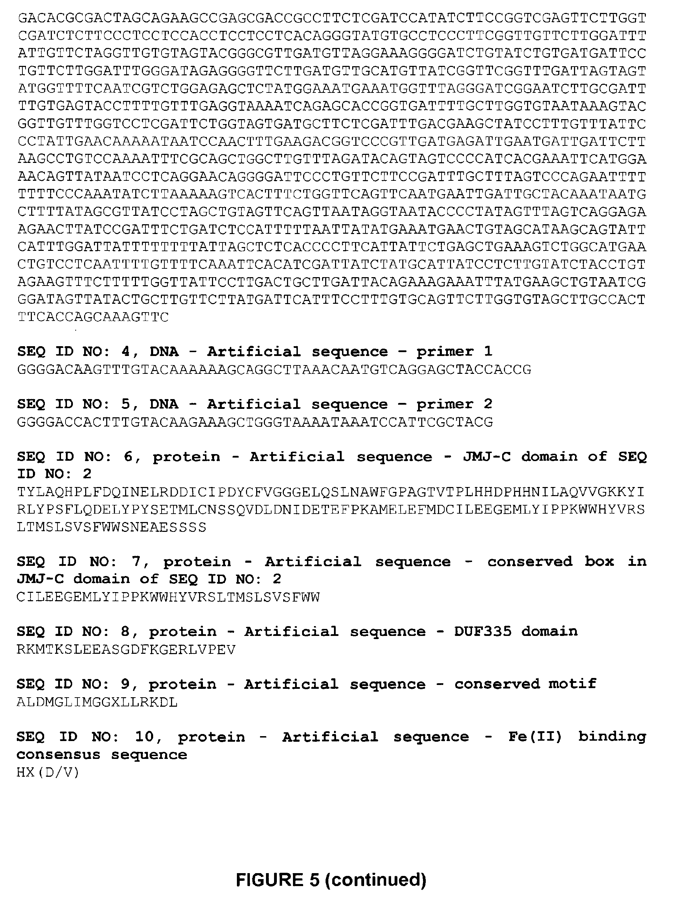

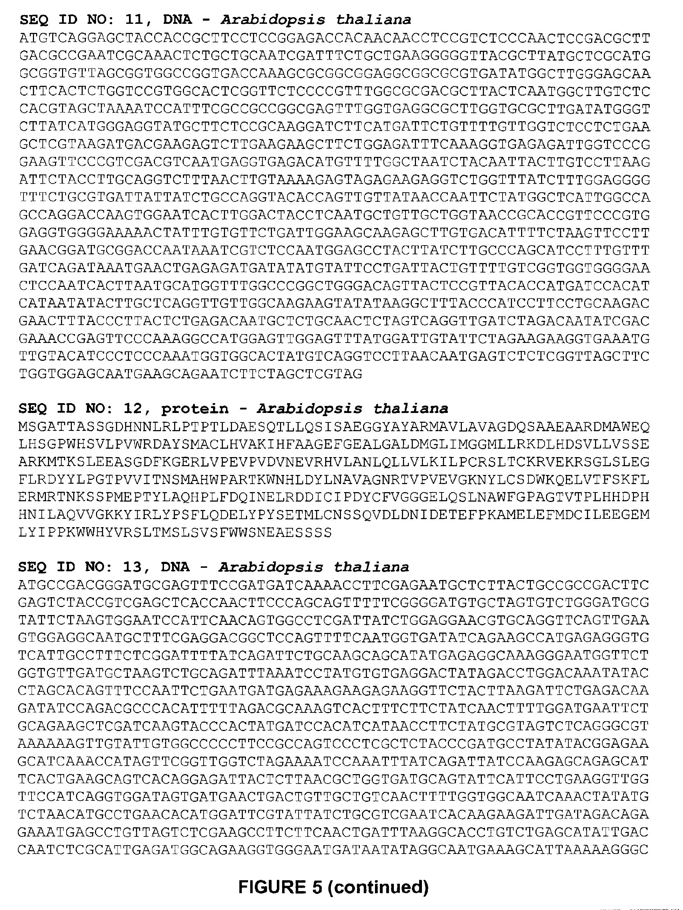

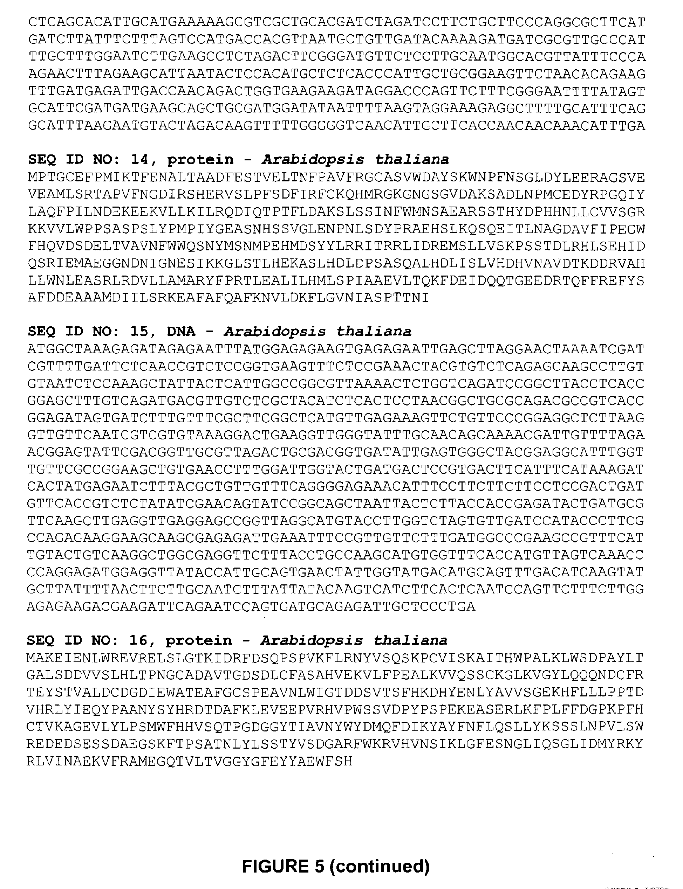

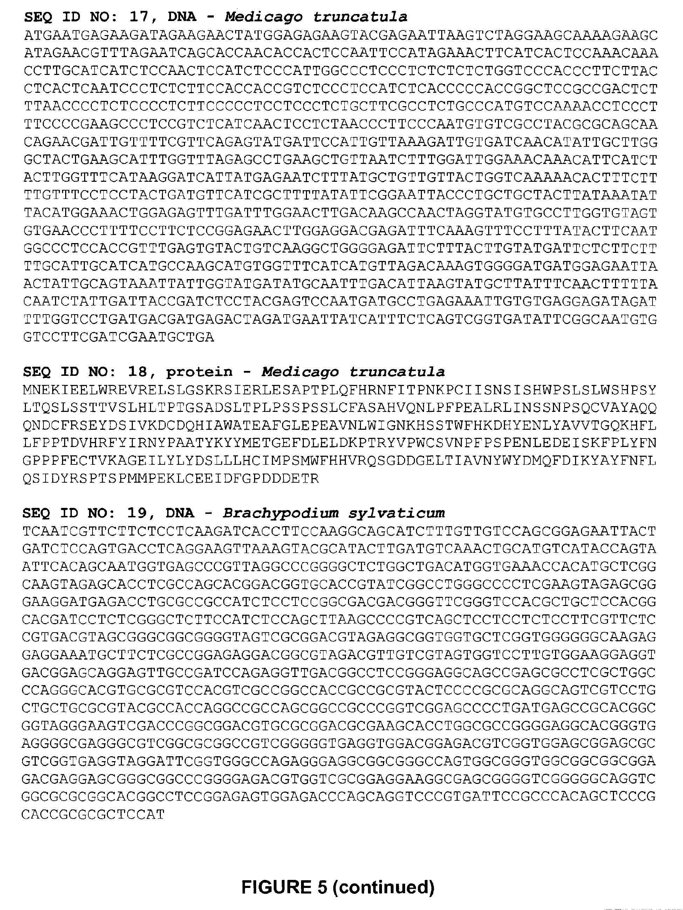

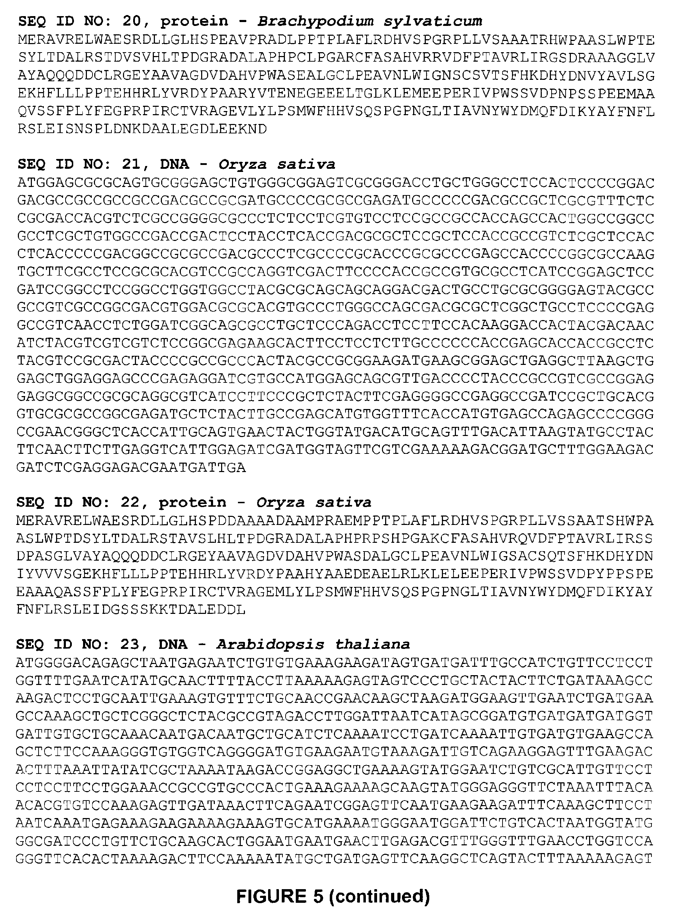

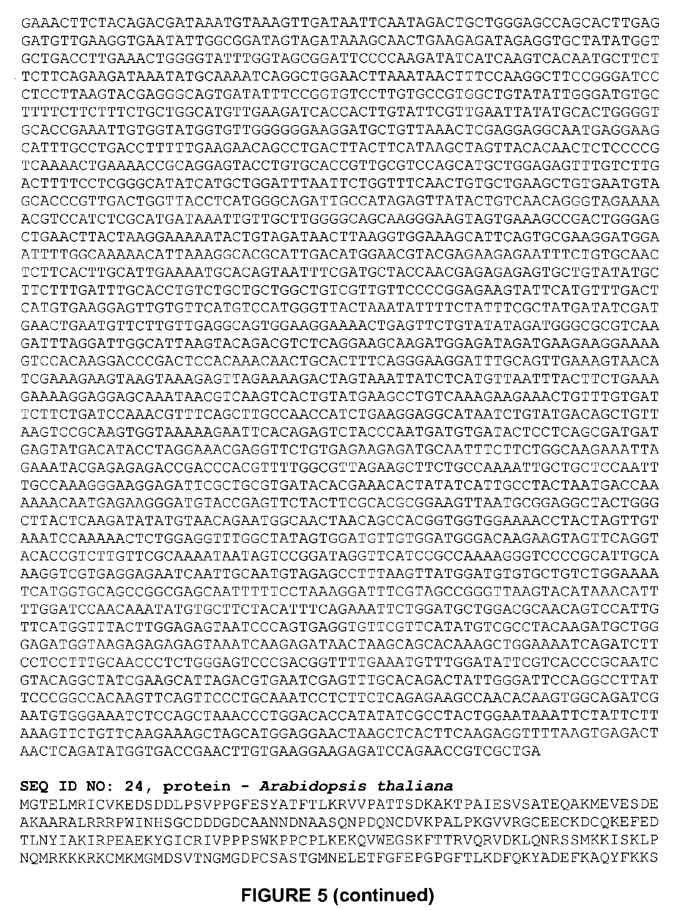

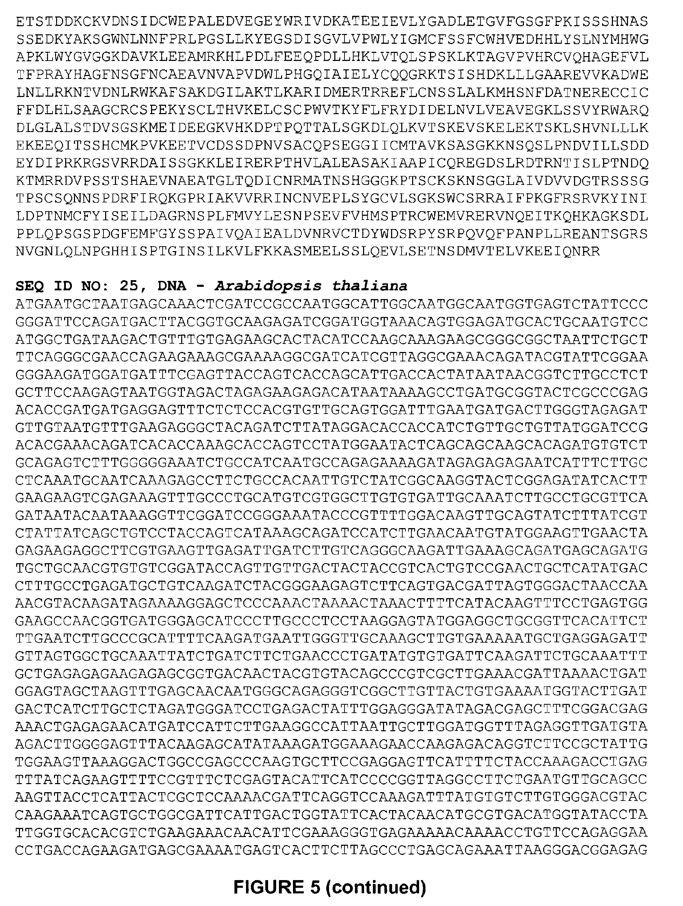

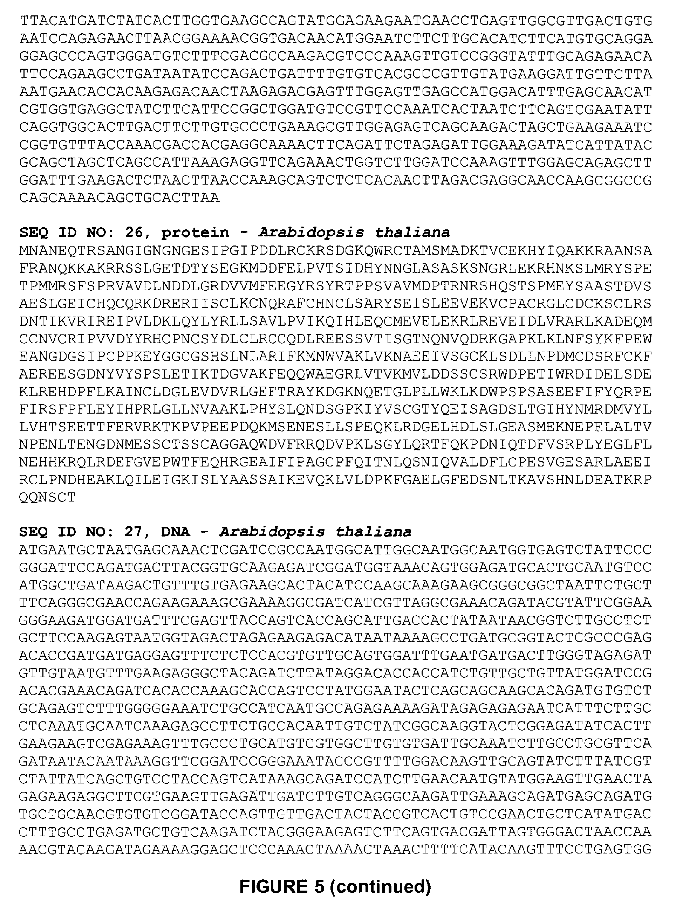

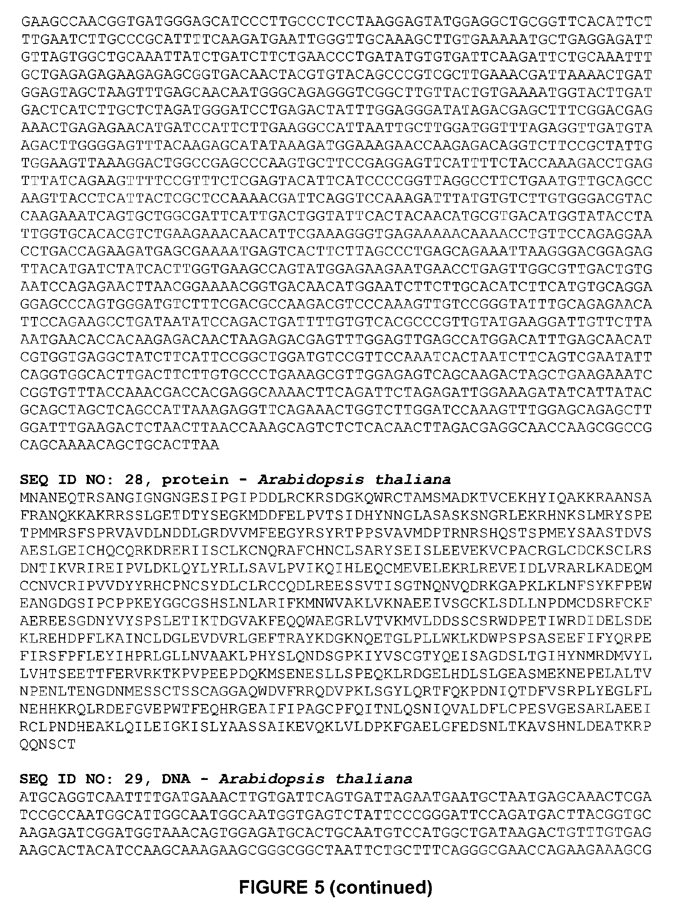

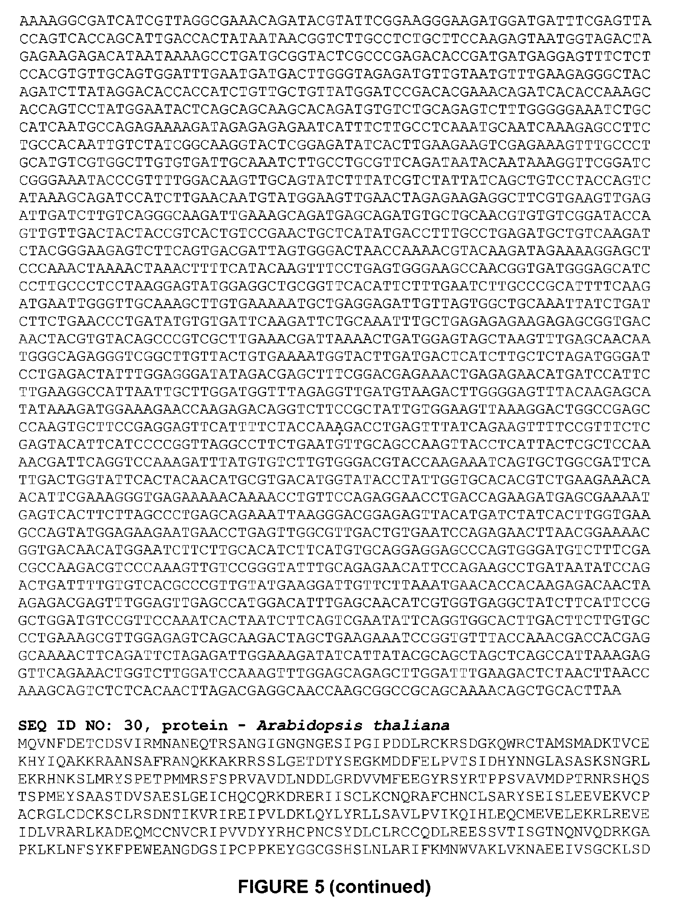

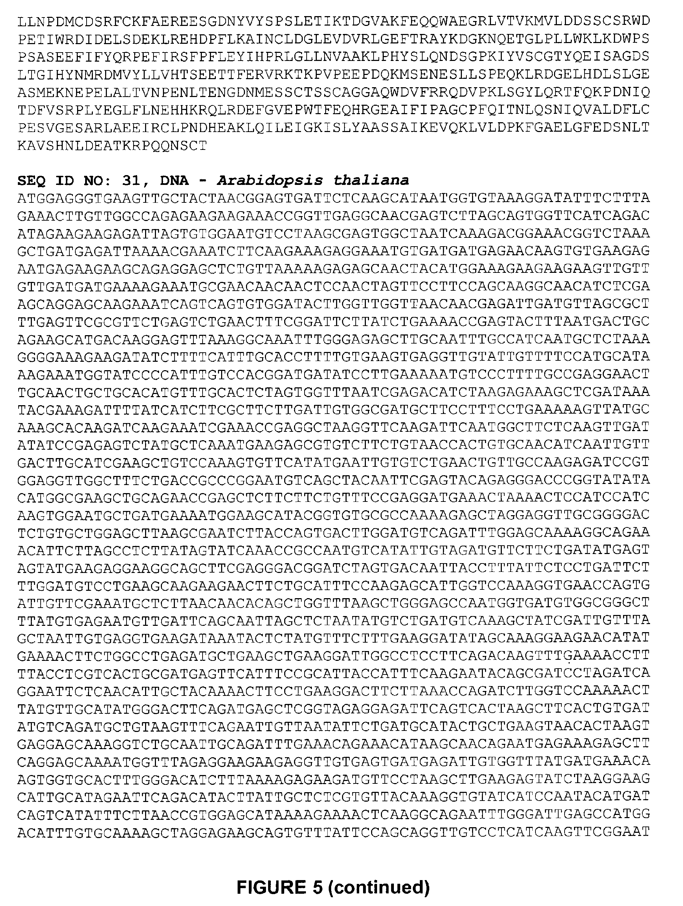

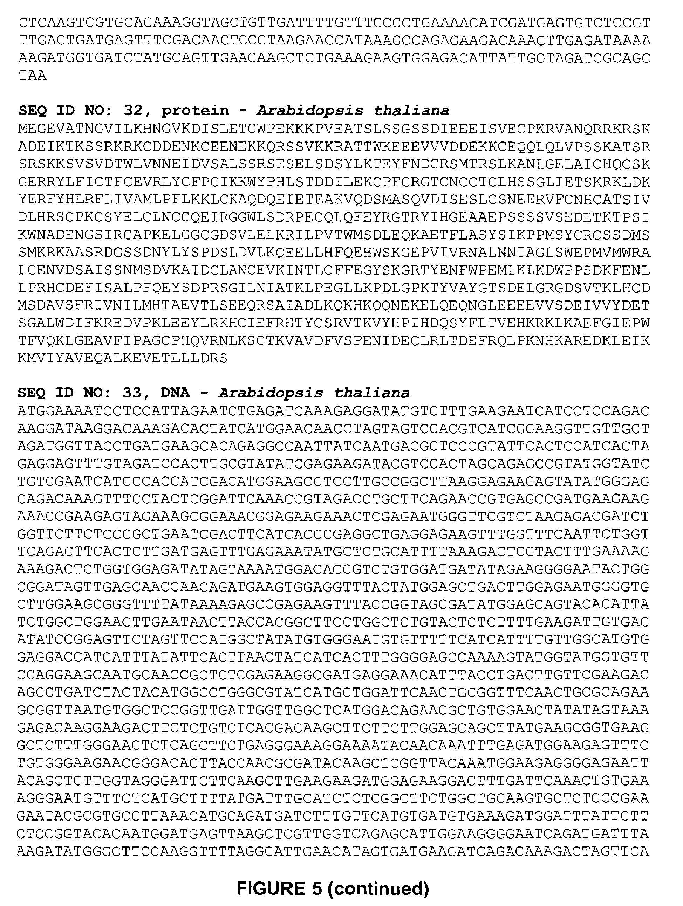

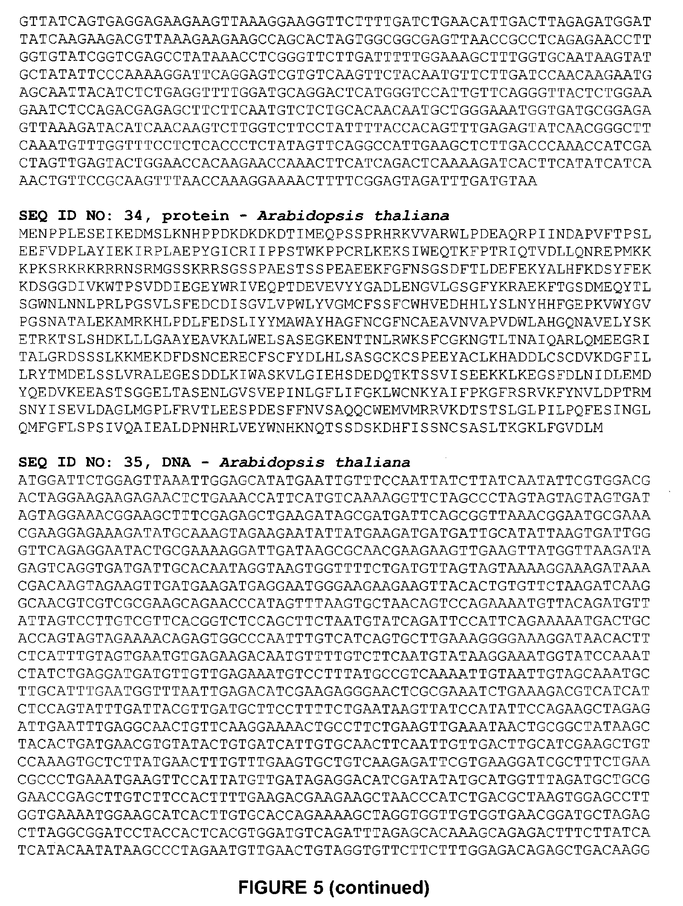

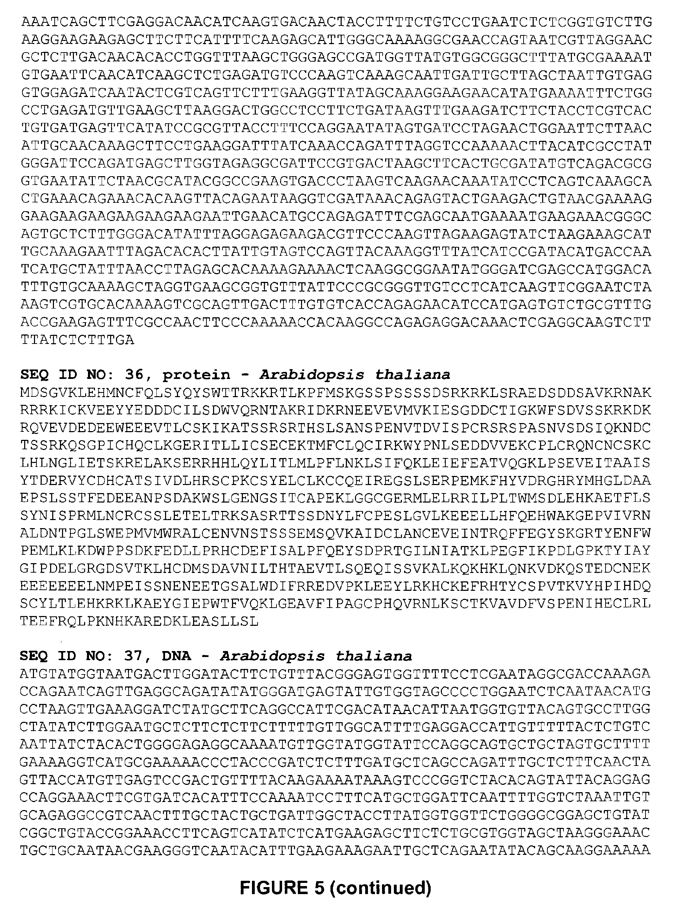

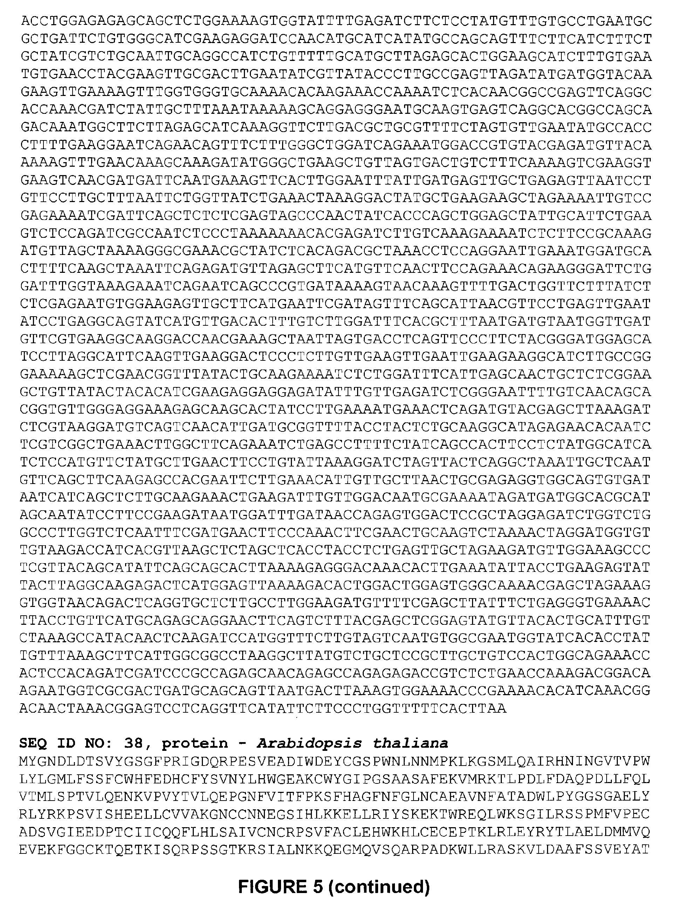

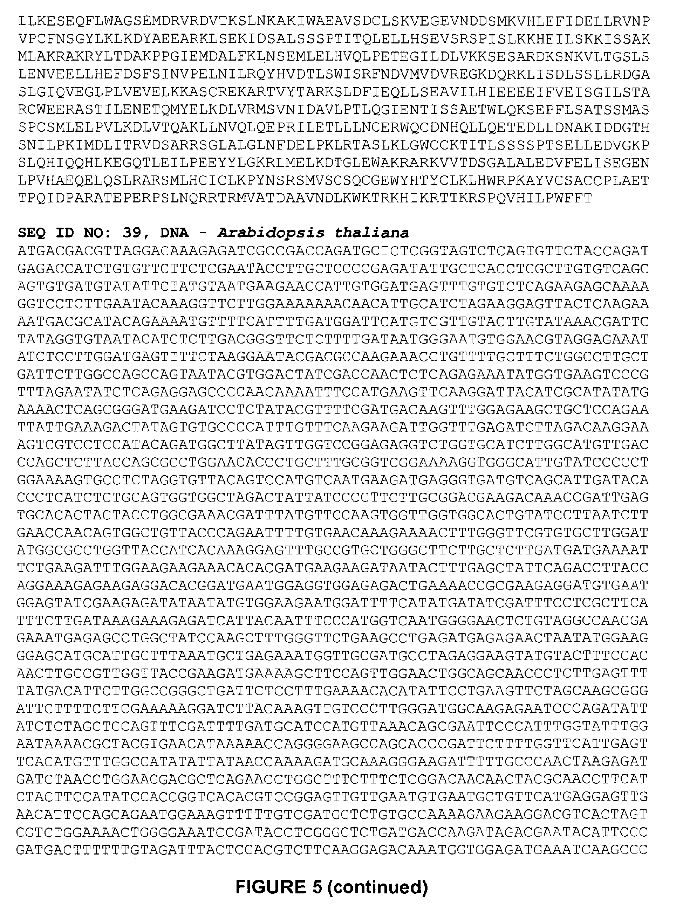

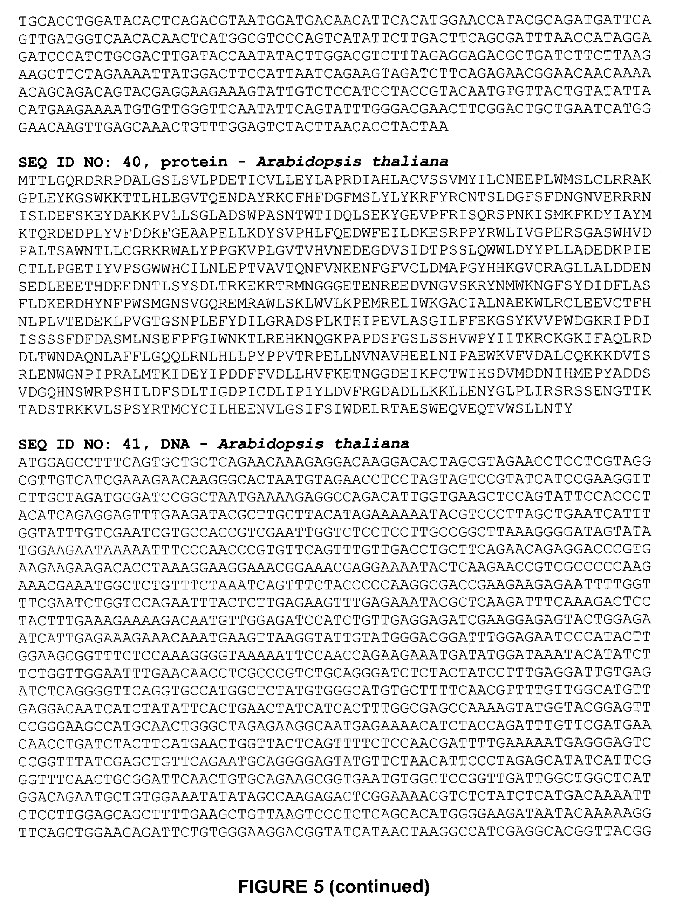

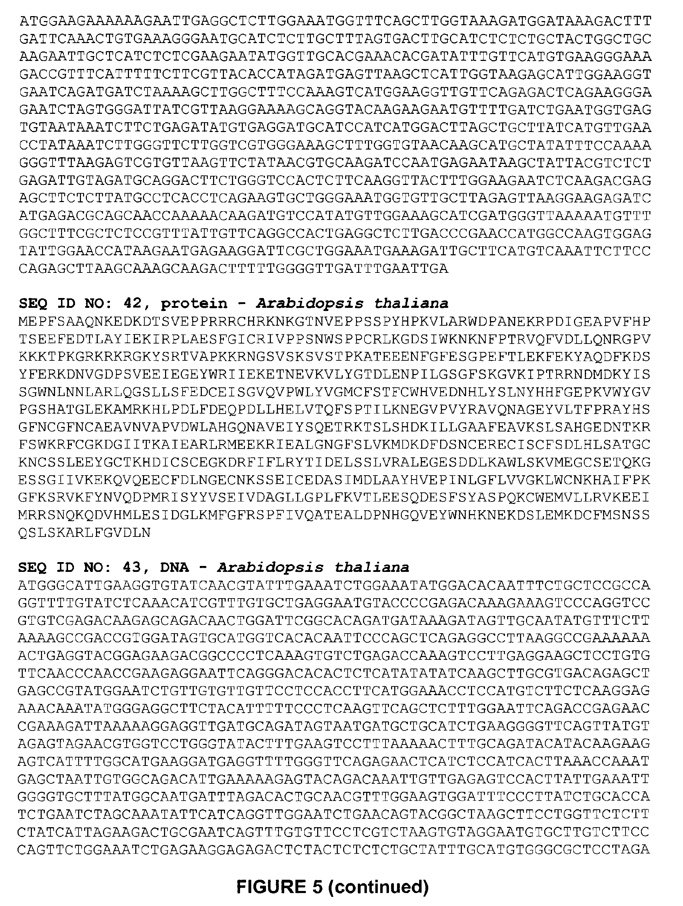

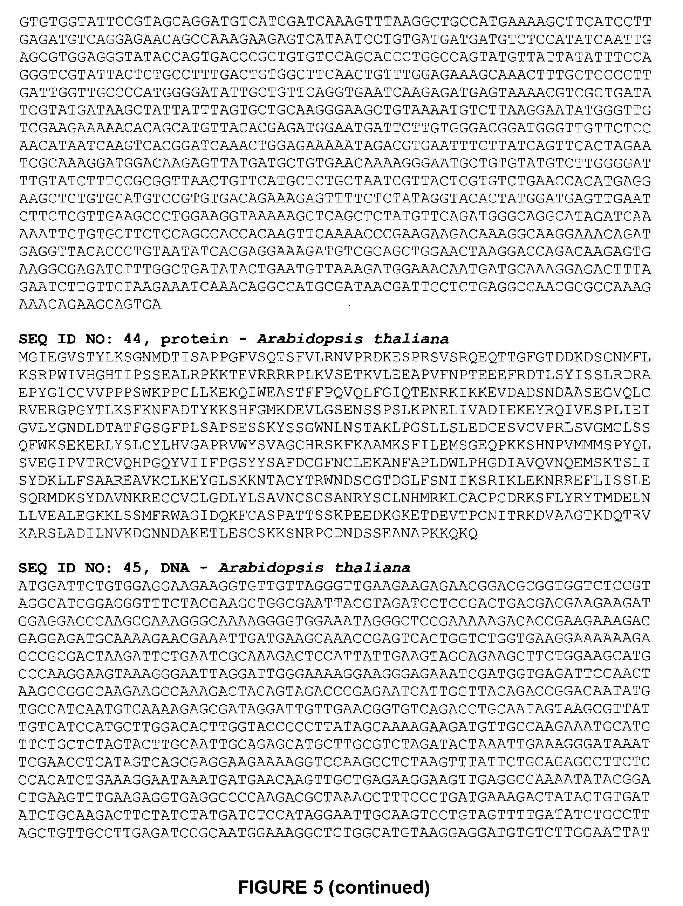

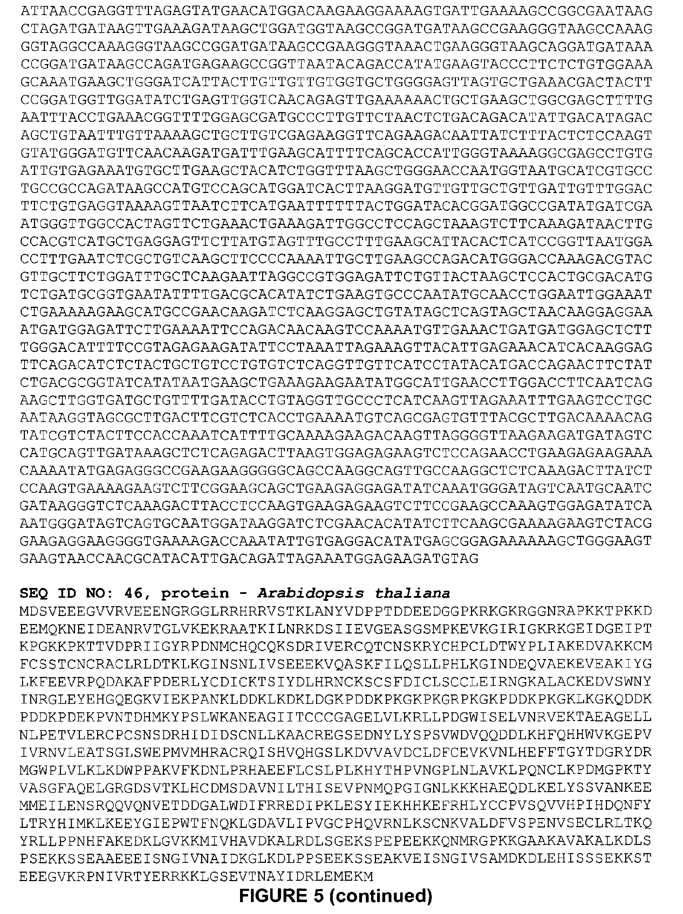

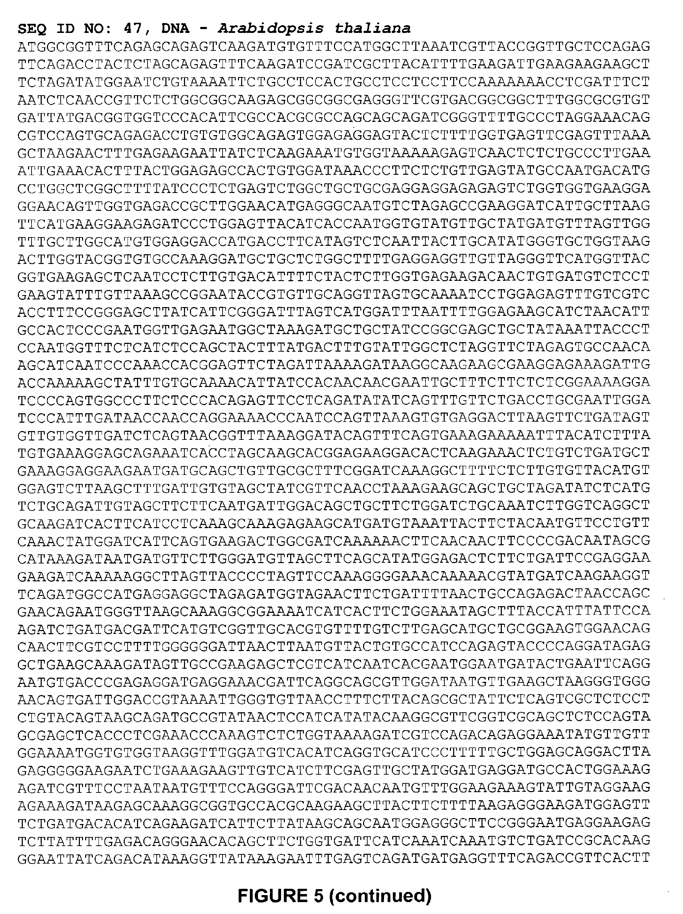

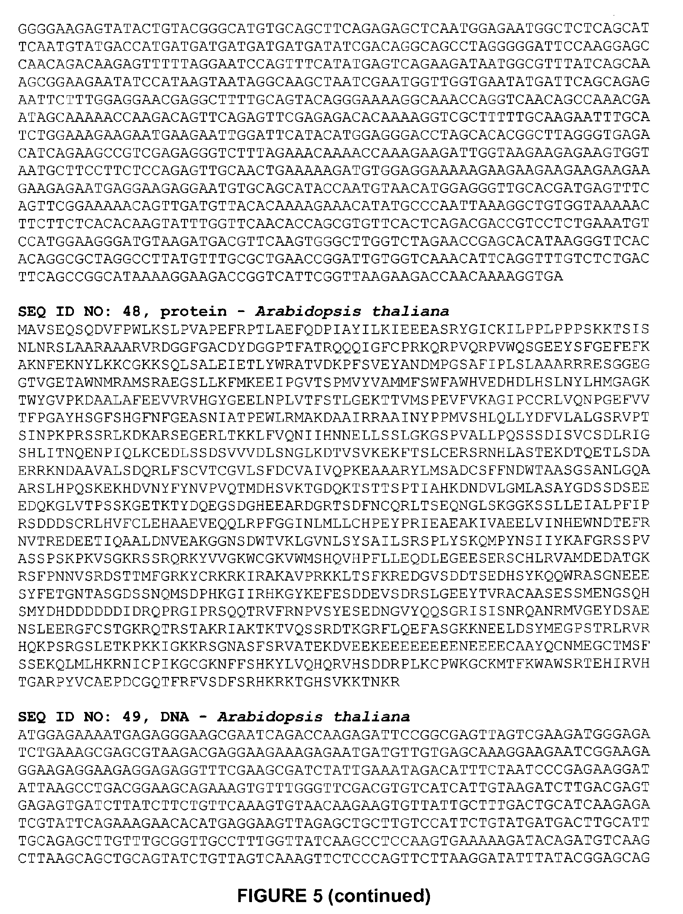

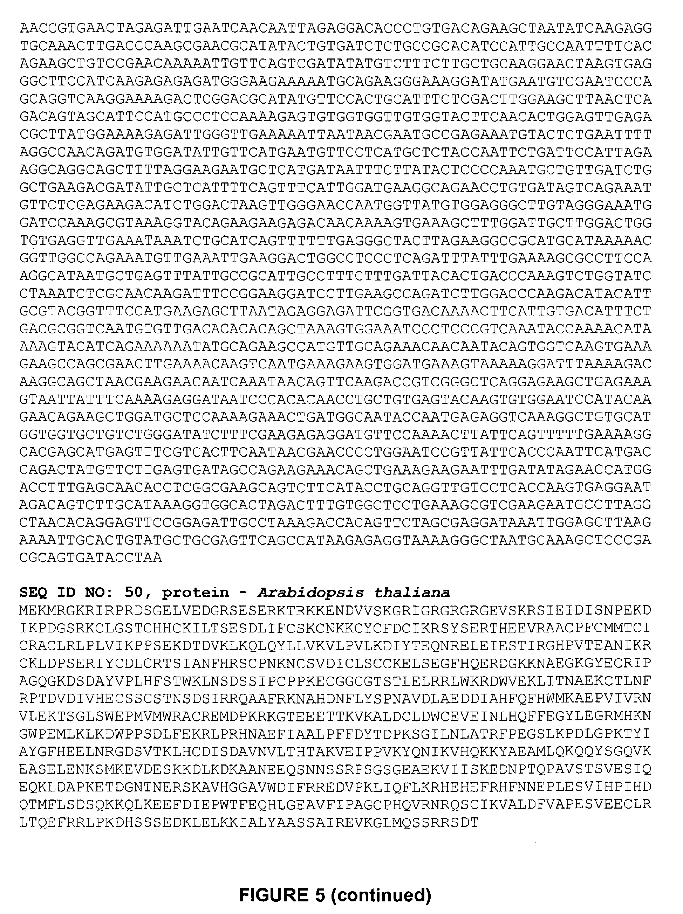

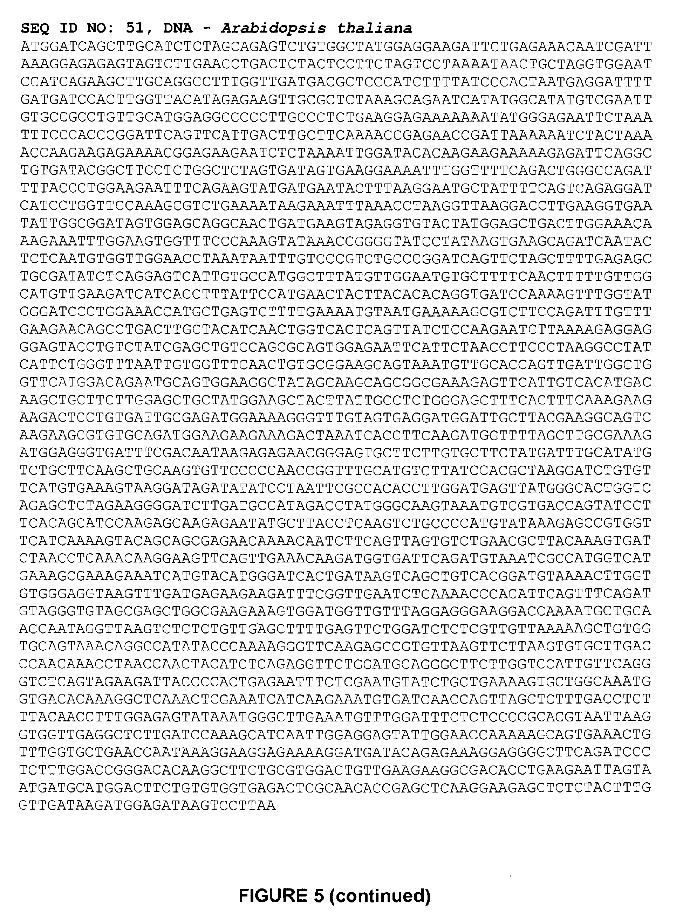

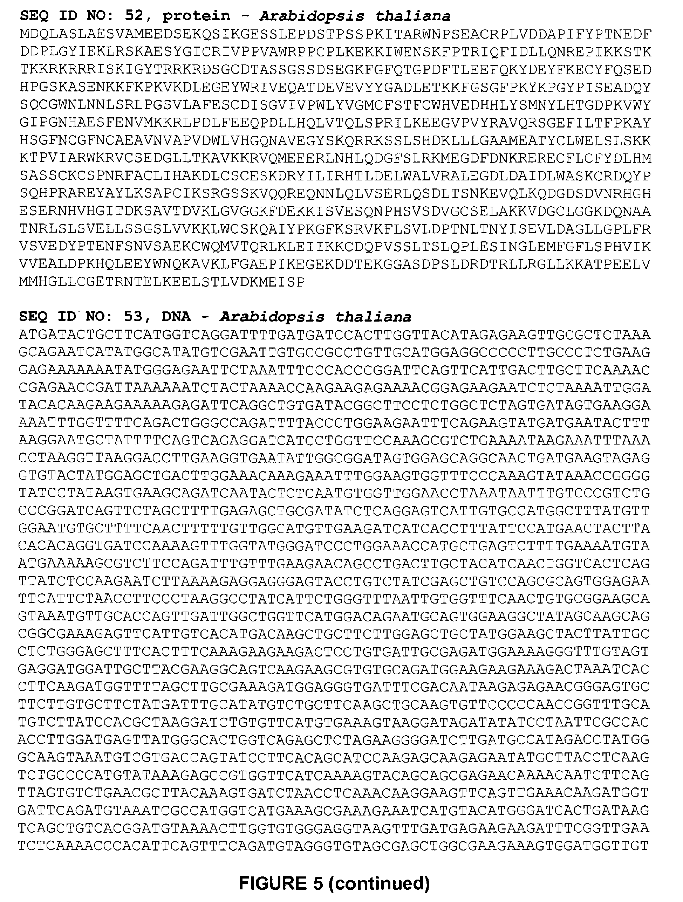

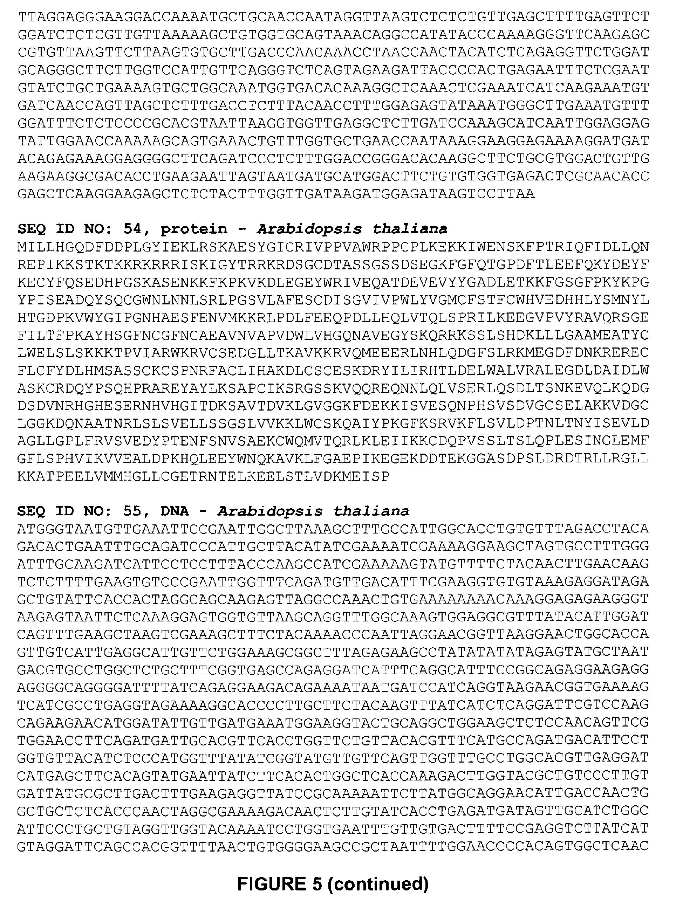

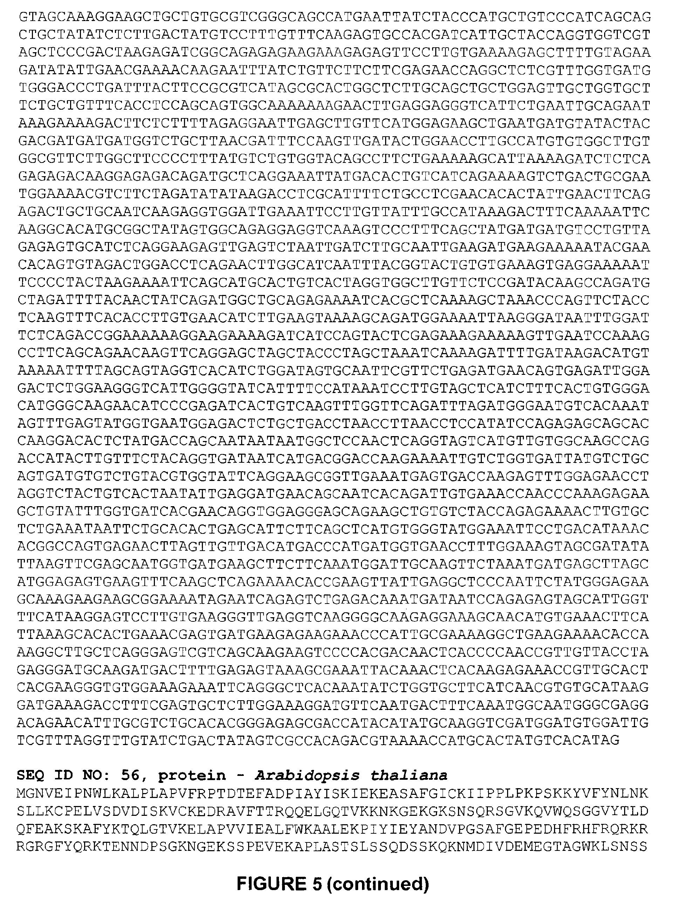

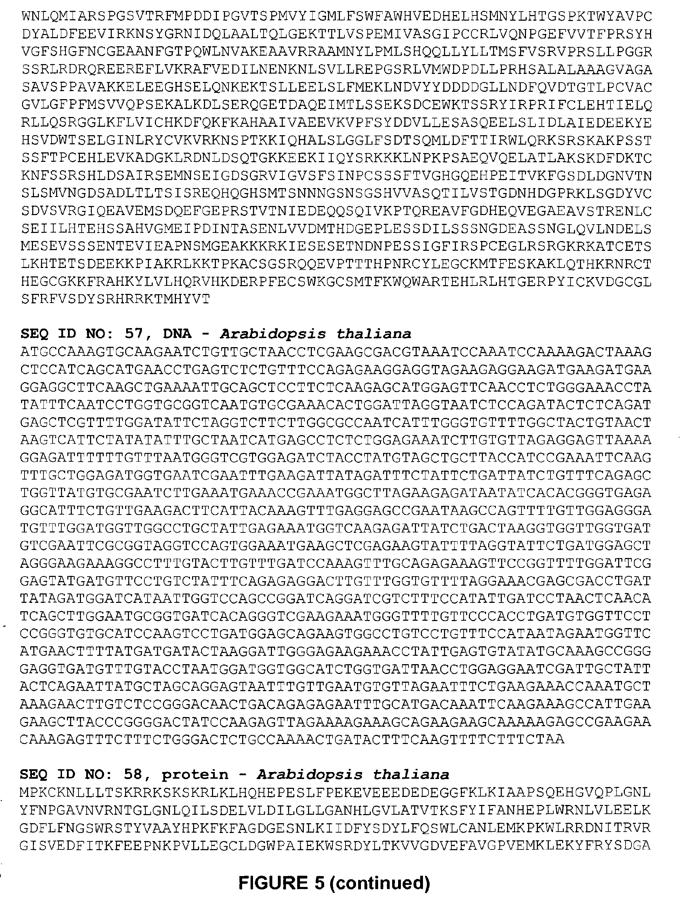

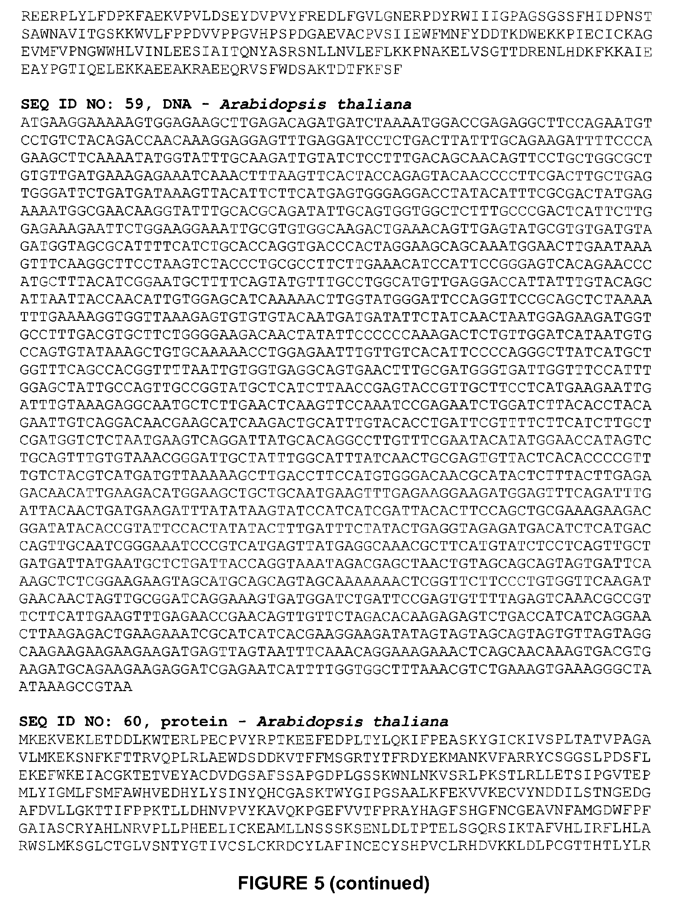

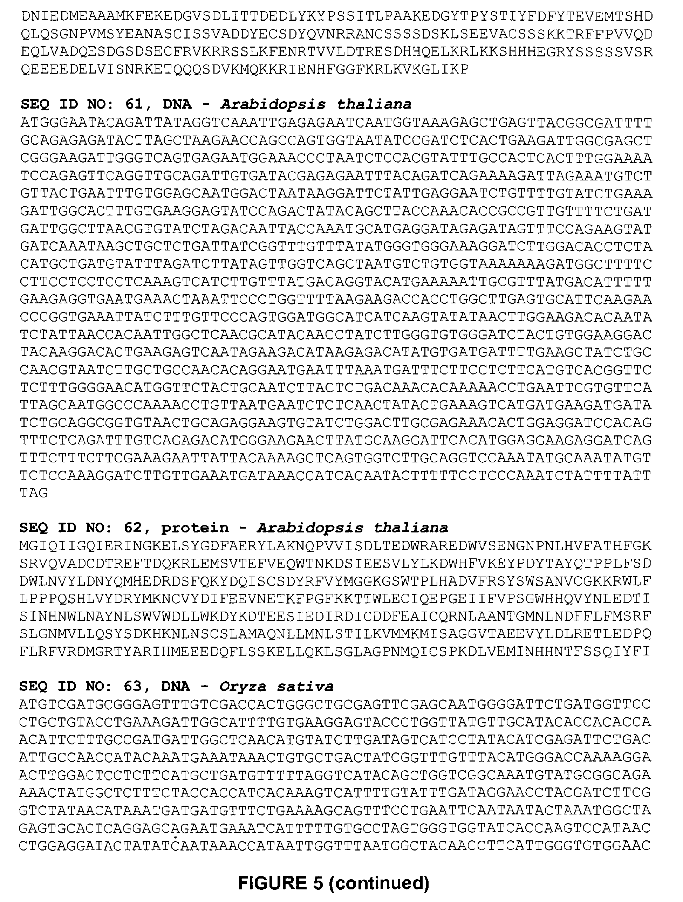

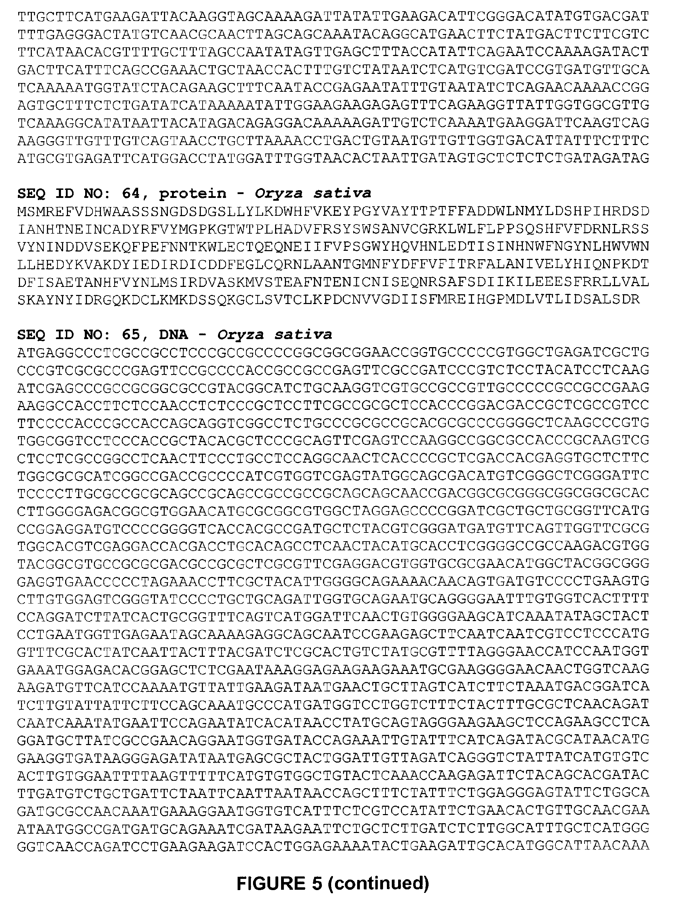

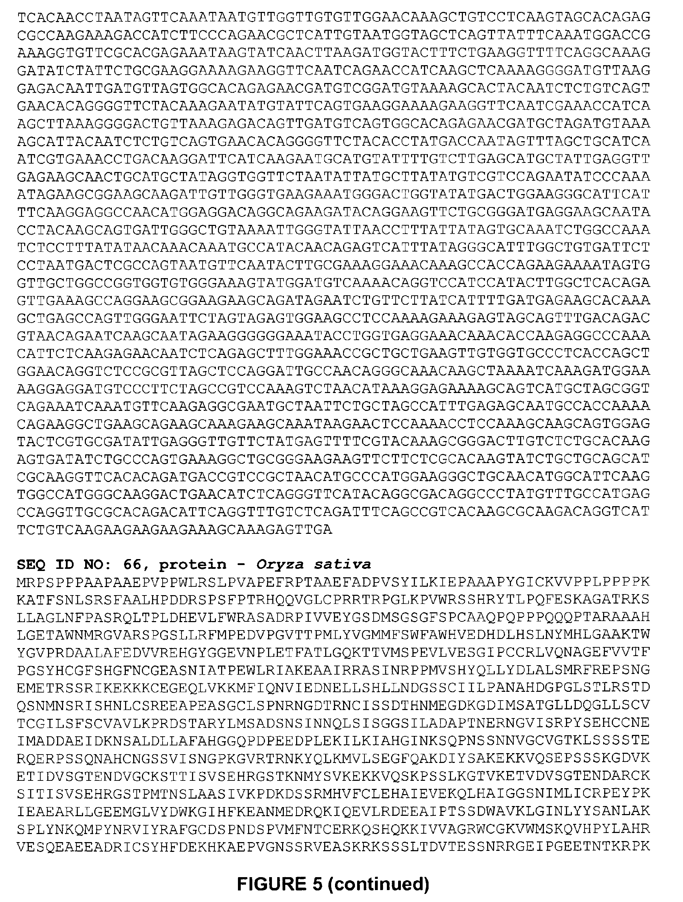

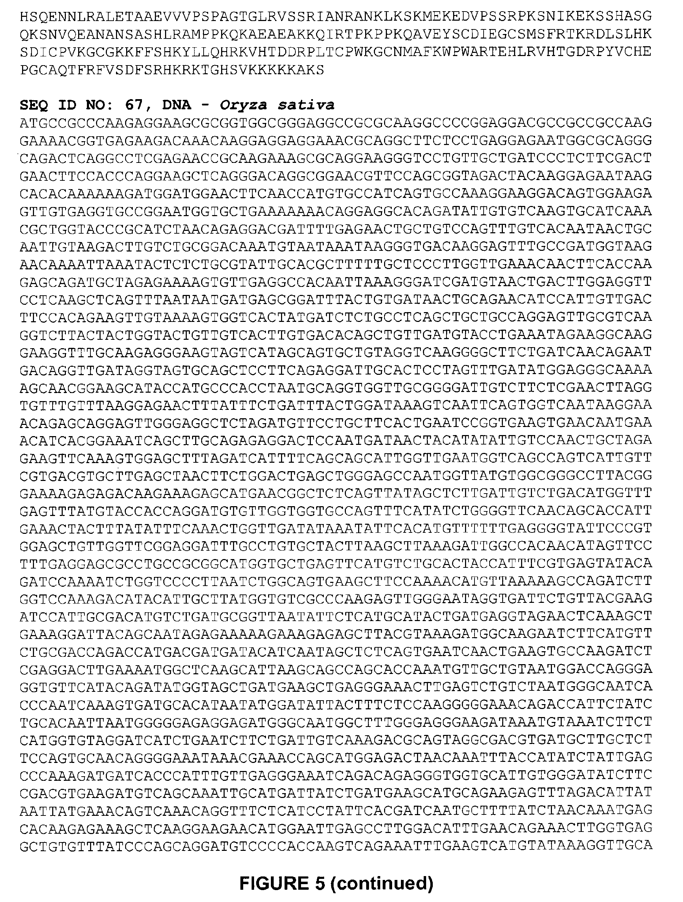

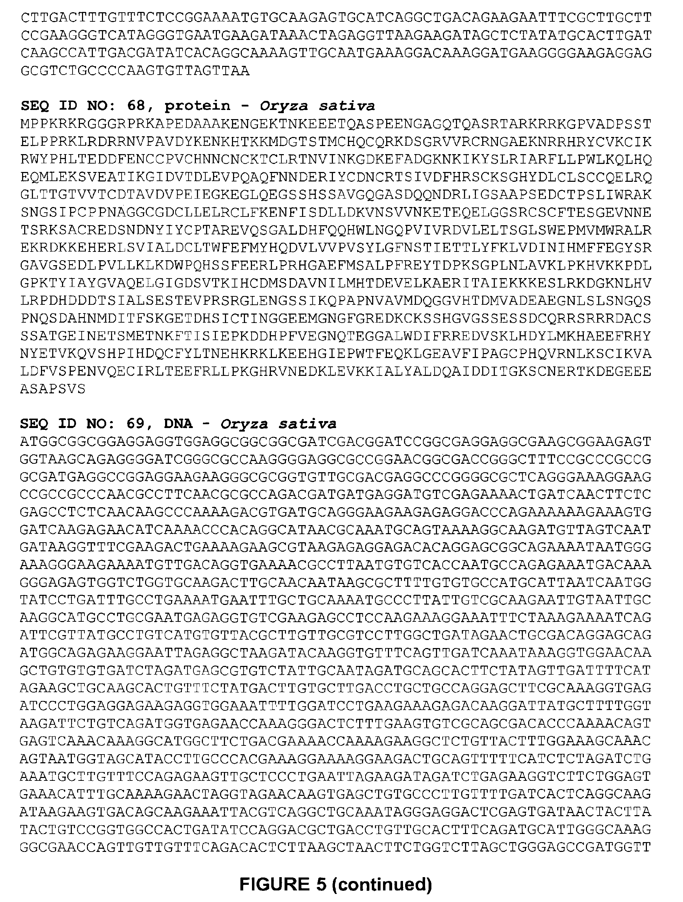

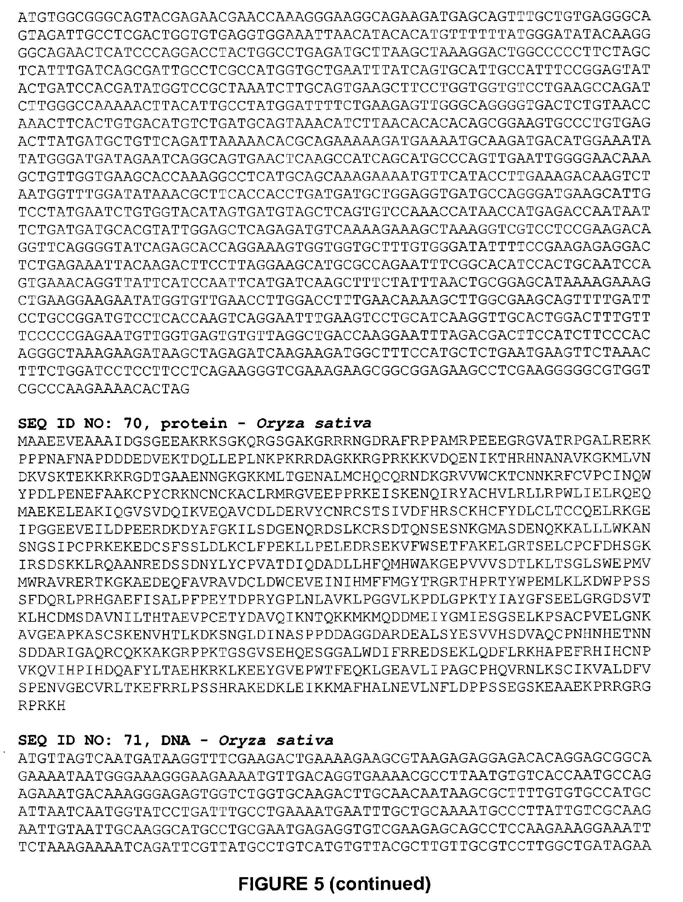

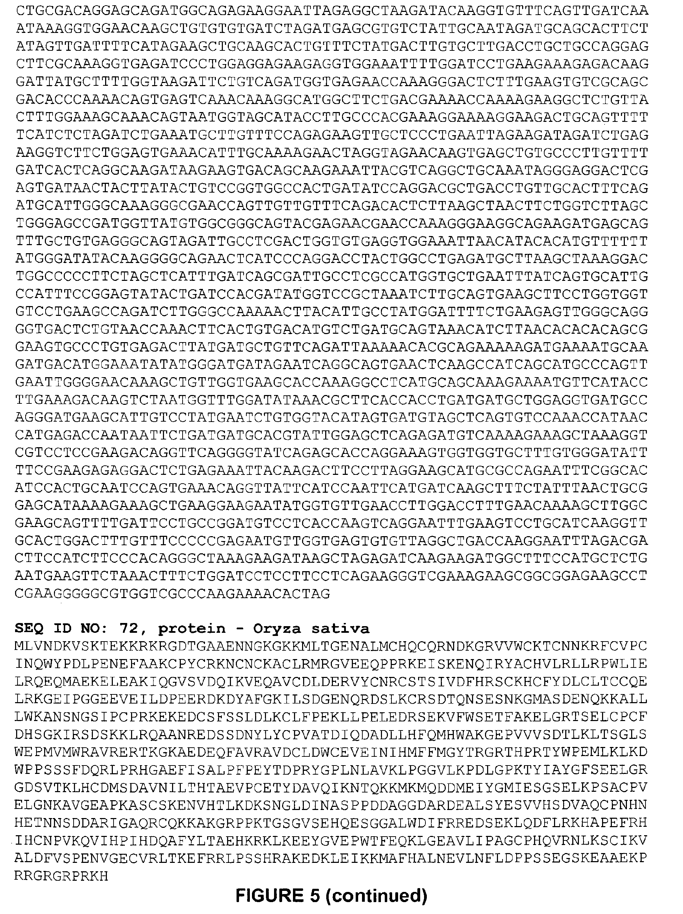

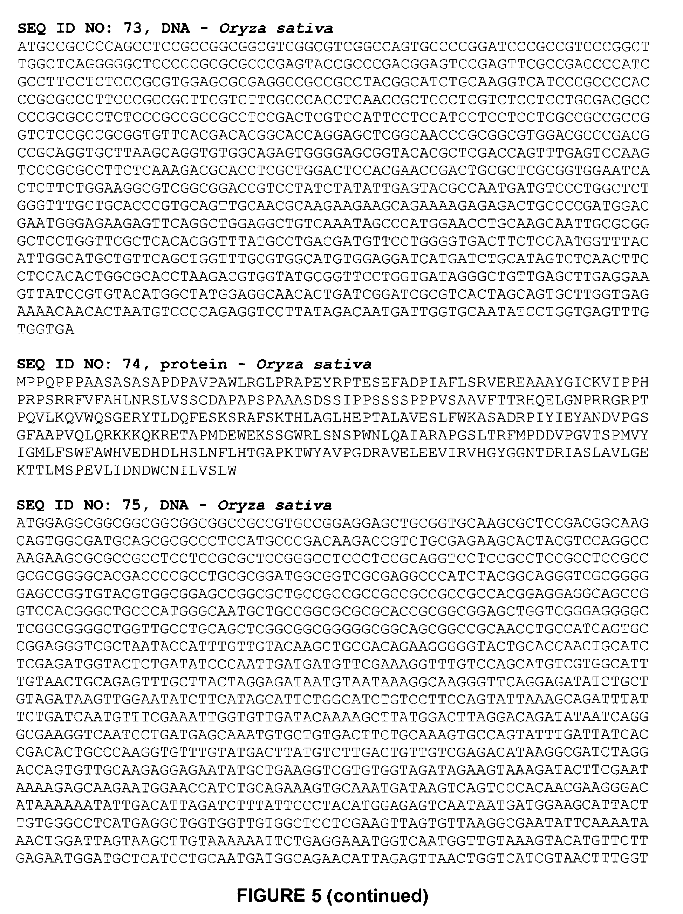

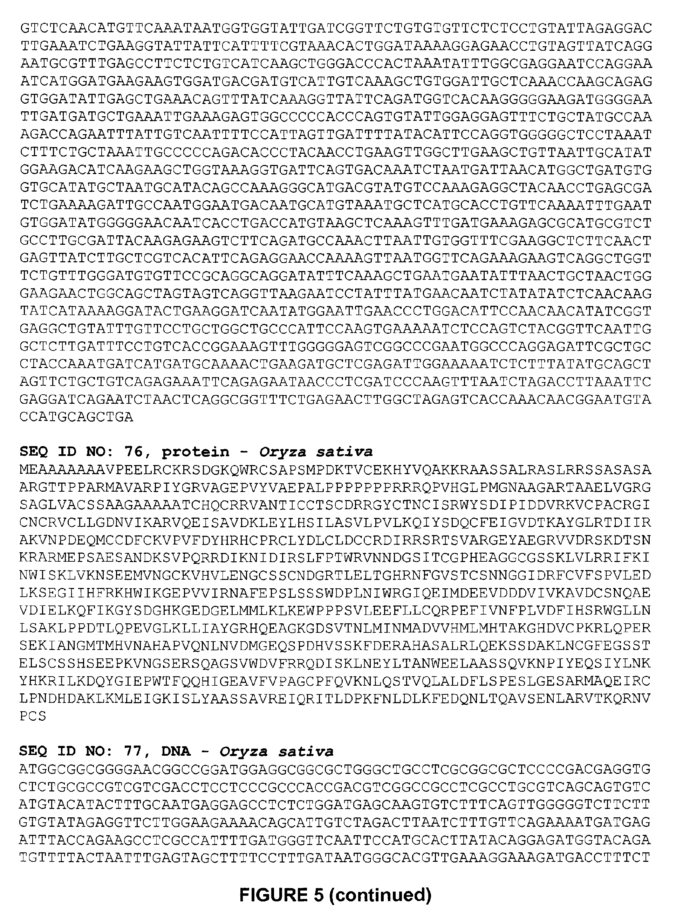

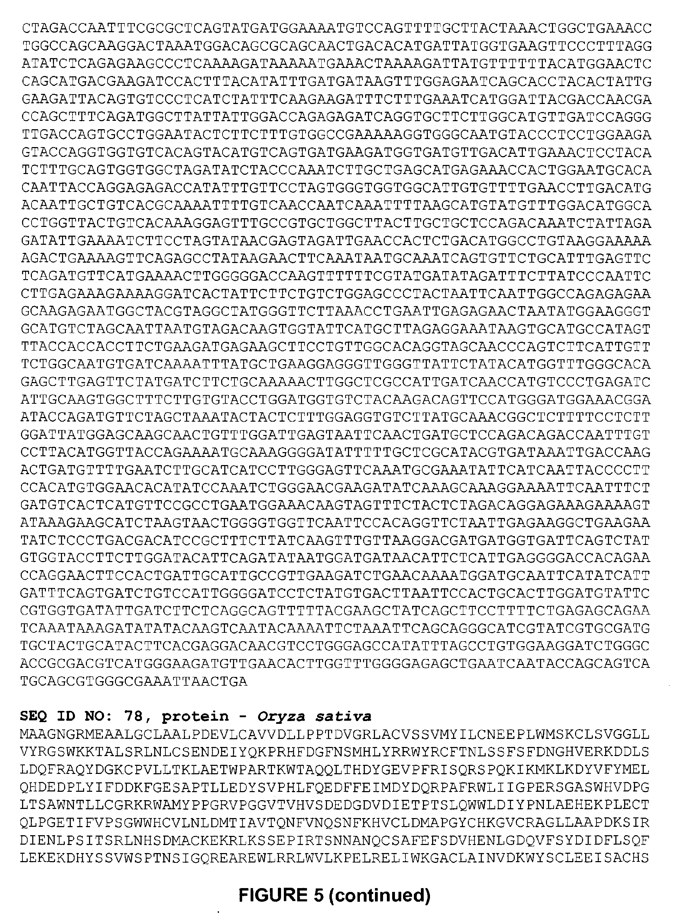

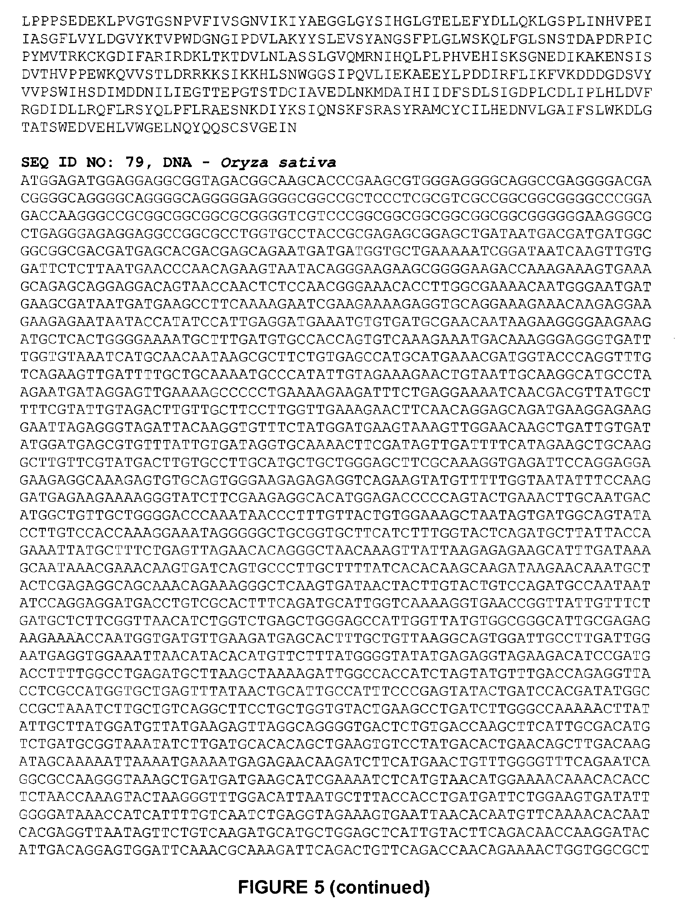

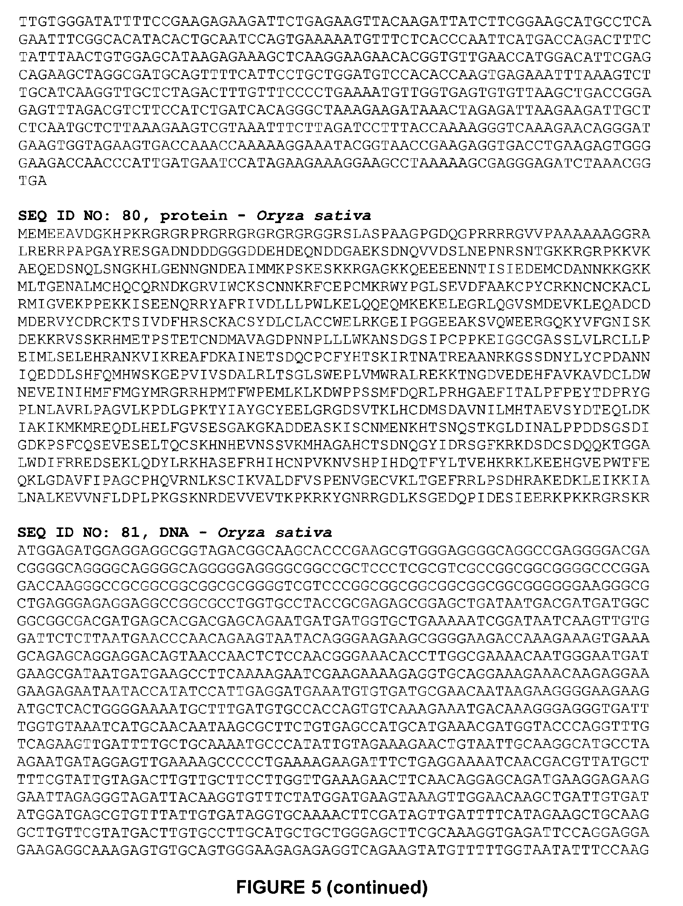

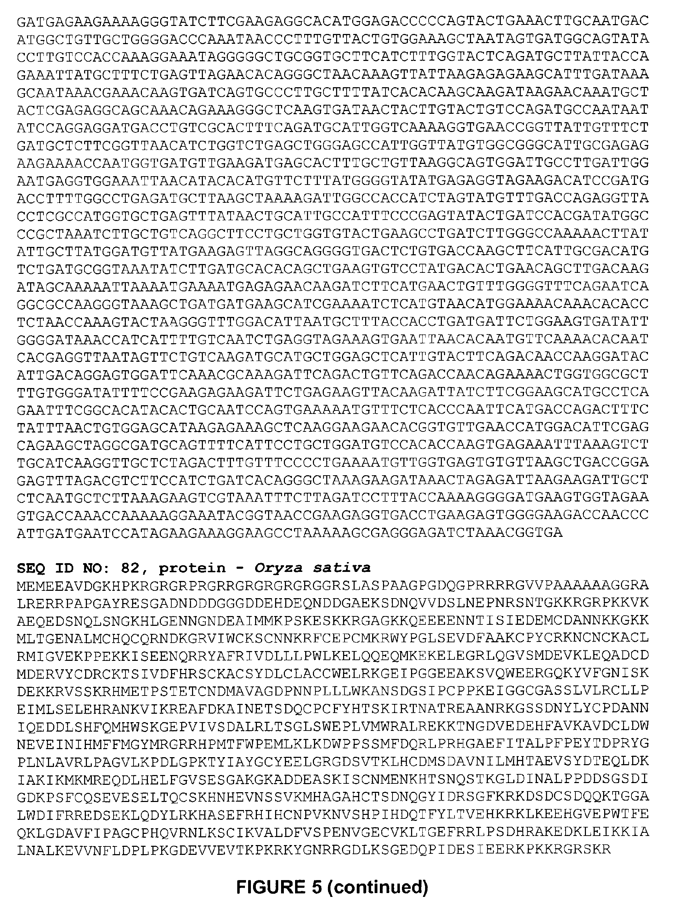

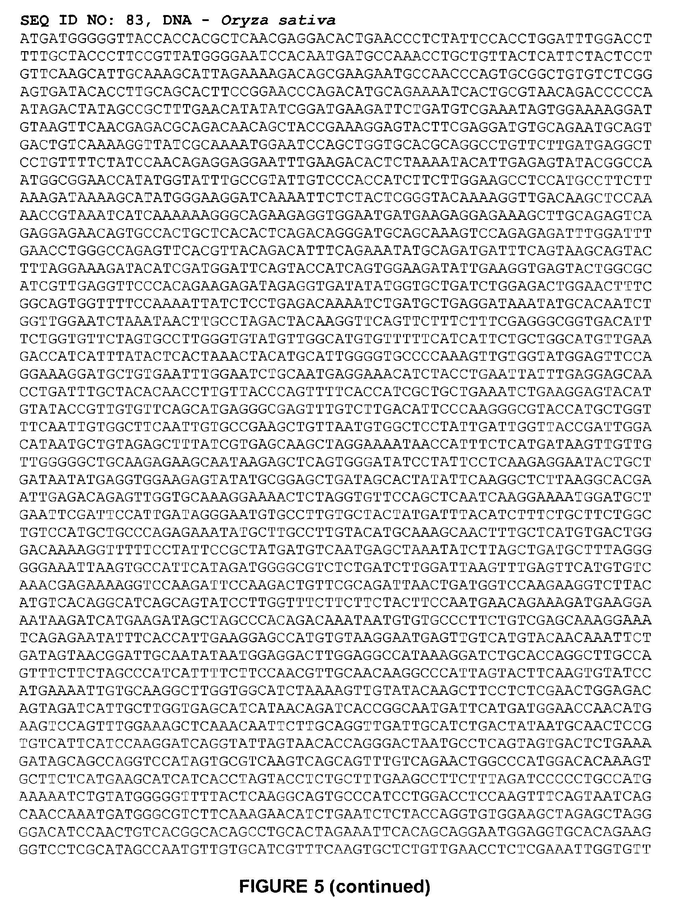

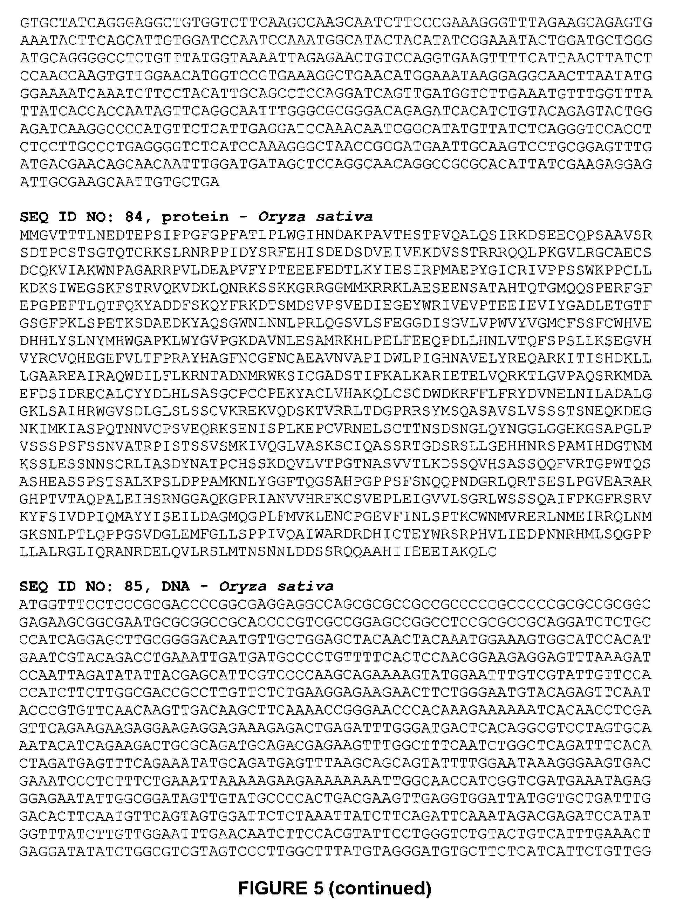

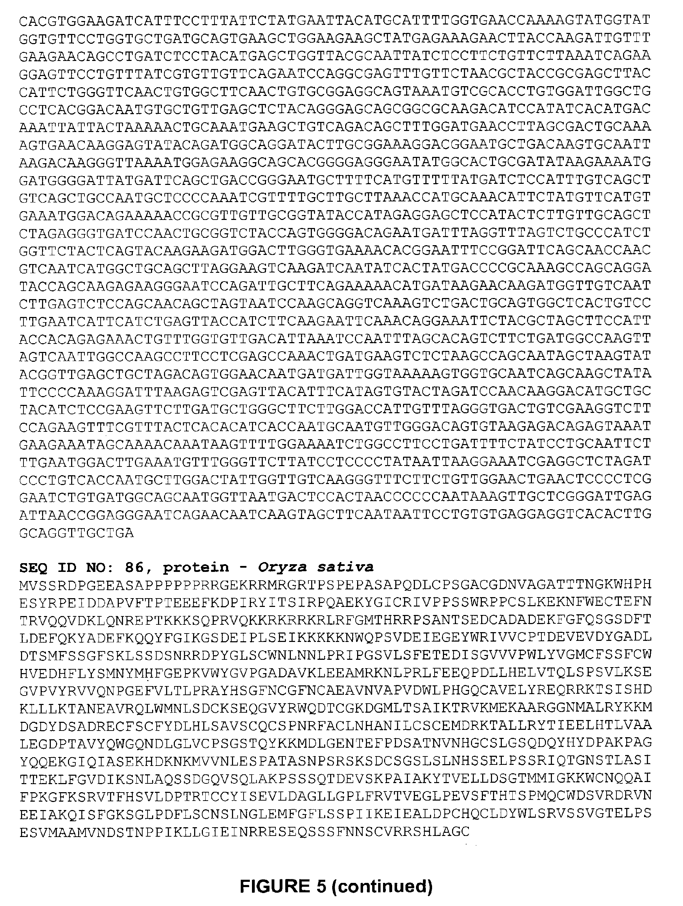

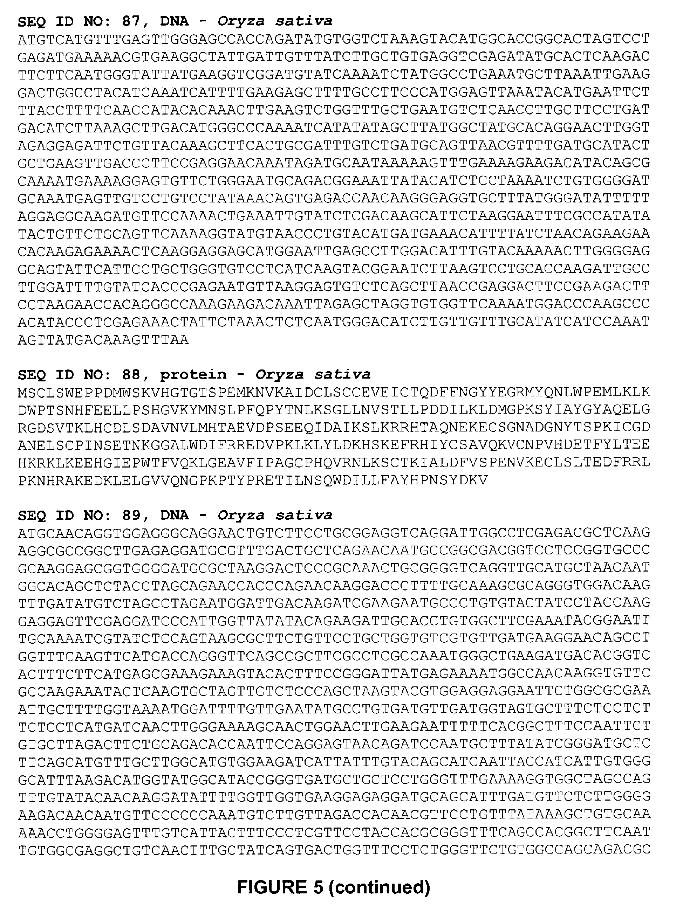

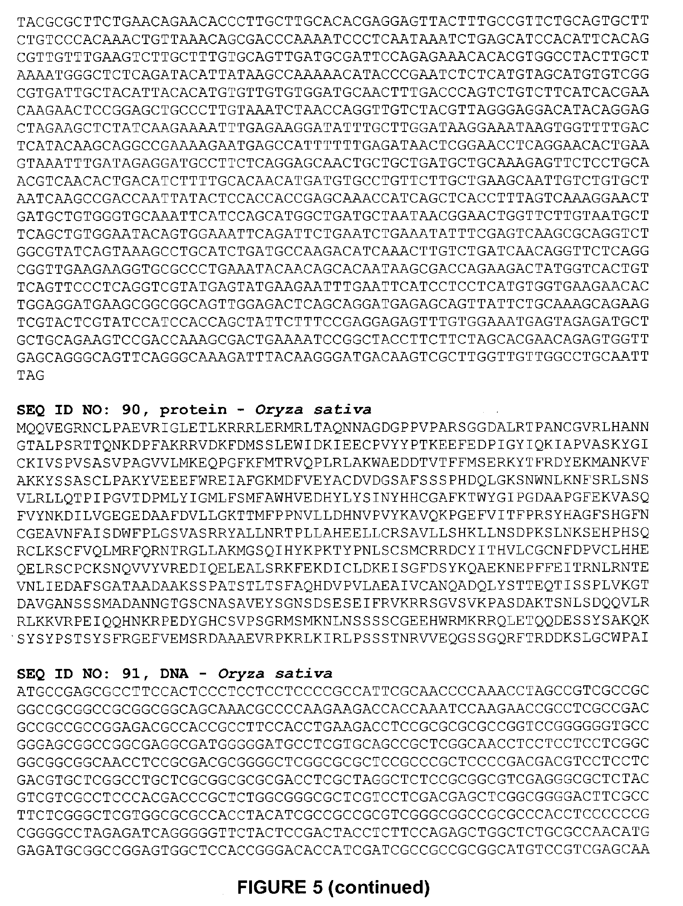

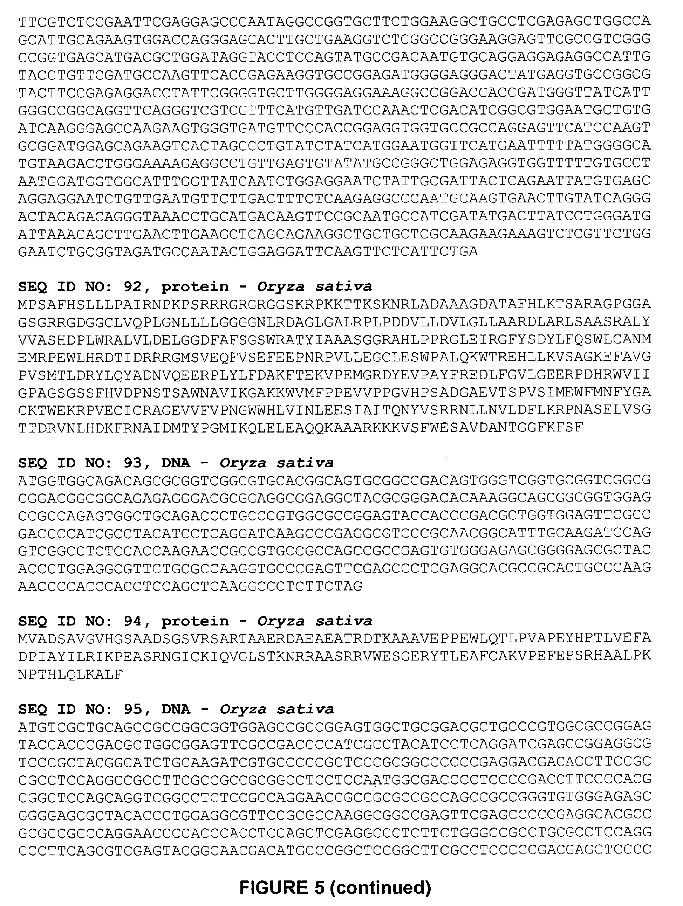

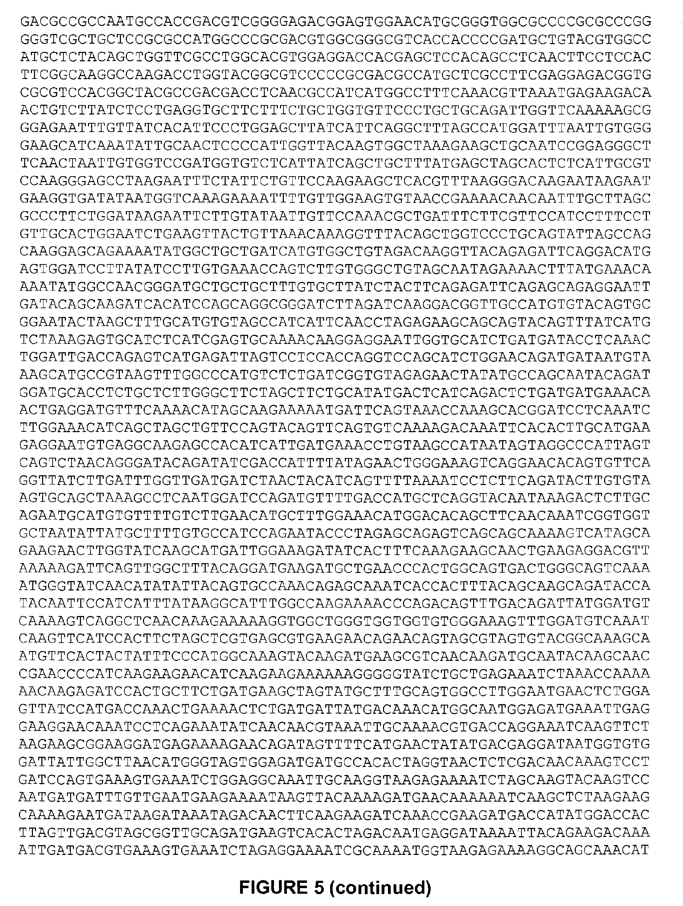

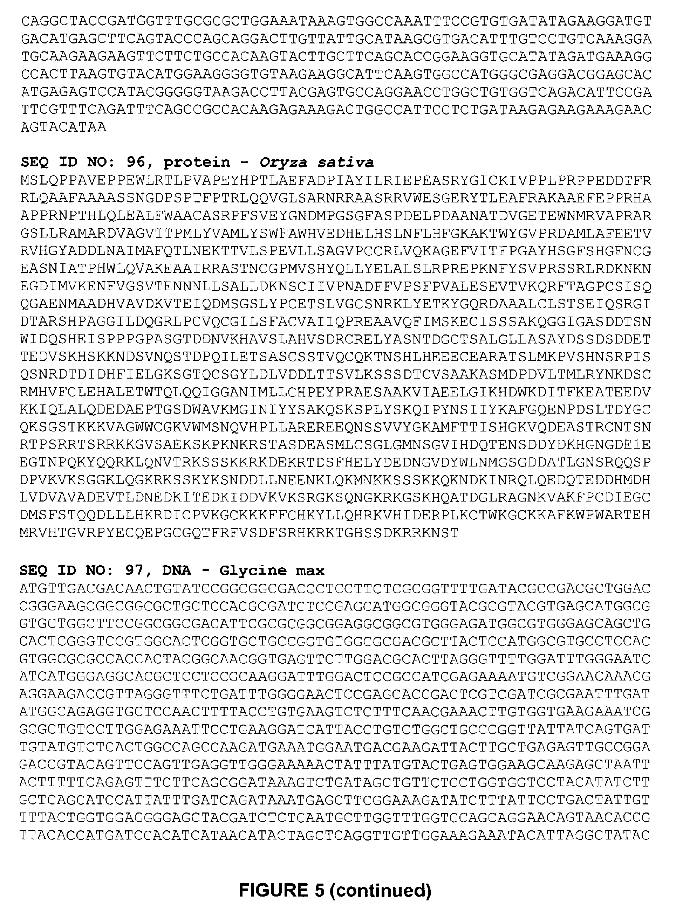

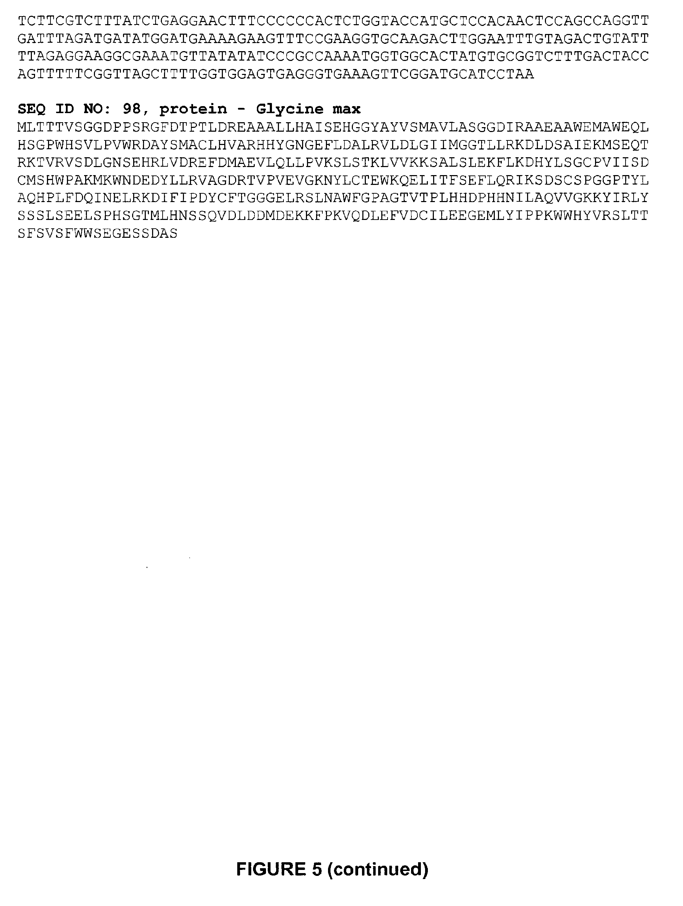

**Figure 5** details examples of JMJC sequences useful in performing the methods according to the present invention.

### Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in Sambrook (2001) Molecular Cloning: a laboratory manual, 3rd Edition Cold Spring Harbor Laboratory Press, CSH, New York, or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### Example 1: Identification of sequences related to the JMJC nucleic acid sequence used in the methods of the invention

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length..

Table A1 provides a list of nucleic acid sequences related to the JMJC nucleic acid sequence used in the methods of the present invention. Polypeptides with an accession extended by a dot and one digit represent splice variants.

**Table A1: Examples of JMJC polypeptides**

| **Plant Source** | **Genbank accession (or locus) number** | **Nucleic acid SEQ ID NO:** | **Protein SEQ ID NO:** |
|---|---|---|---|
| *Arabidopsis thaliana* | AT3G20810 | 1 | 2 |
| *Arabidopsis thaliana* | AT3G20810 | 11 | 12 |
| *Arabidopsis thaliana* | AT5G19840 | 13 | 14 |
| *Arabidopsis thaliana* | AT3G45880 | 15 | 16 |
| *Medicago truncatula* | ABE92082 | 17 | 18 |
| *Brachypodium sylvaticum* | CAJ26373 | 19 | 20 |
| *Oryza sativa* | Os09g0483600 | 21 | 22 |
| *Arabidopsis thaliana* | AT1G08620 | 23 | 24 |
| *Arabidopsis thaliana* | AT1G09060 | 25 | 26 |
| *Arabidopsis thaliana* | AT1G09060 | 27 | 28 |
| *Arabidopsis thaliana* | AT1G09060 | 29 | 30 |
| *Arabidopsis thaliana* | AT1G11950 | 31 | 32 |
| *Arabidopsis thaliana* | AT1G30810 | 33 | 34 |
| *Arabidopsis thaliana* | AT1G62310 | 35 | 36 |
| *Arabidopsis thaliana* | AT1G63490 | 37 | 38 |
| *Arabidopsis thaliana* | AT1G78280 | 39 | 40 |
| *Arabidopsis thaliana* | AT2G34880 | 41 | 42 |
| *Arabidopsis thaliana* | AT2G38950 | 43 | 44 |
| *Arabidopsis thaliana* | AT3G07610 | 45 | 46 |
| *Arabidopsis thaliana* | AT3G48430 | 47 | 48 |
| *Arabidopsis thaliana* | AT4G00990 | 49 | 50 |
| *Arabidopsis thaliana* | AT4G20400 | 51 | 52 |
| *Arabidopsis thaliana* | AT4G20400 | 53 | 54 |
| *Arabidopsis thaliana* | AT5G04240 | 55 | 56 |
| *Arabidopsis thaliana* | AT5G06550 | 57 | 58 |
| *Arabidopsis thaliana* | AT5G46910 | 59 | 60 |
| *Arabidopsis thaliana* | AT5G63080 | 61 | 62 |
| *Oryza sativa* | Os01g36630 | 63 | 64 |
| *Oryza sativa* | Os01g67970 | 65 | 66 |
| *Oryza sativa* | Os02g01940 | 67 | 68 |
| *Oryza sativa* | Os02g58210 | 69 | 70 |
| *Oryza sativa* | Os02g58210 | 71 | 72 |
| *Oryza sativa* | Os03g05680 | 73 | 74 |
| *Oryza sativa* | Os03g22540 | 75 | 76 |
| *Oryza sativa* | Os03g27250 | 77 | 78 |
| *Oryza sativa* | Os03g31594 | 79 | 80 |
| *Oryza sativa* | Os03g31594 | 81 | 82 |
| *Oryza sativa* | Os05g10770 | 83 | 84 |
| *Oryza sativa* | Os05g23670 | 85 | 86 |
| *Oryza sativa* | Os09g22540 | 87 | 88 |
| *Oryza sativa* | Os10g42690 | 89 | 90 |
| *Oryza sativa* | Os11g36450 | 91 | 92 |
| *Oryza sativa* | Os12g18149 | 93 | 94 |
| *Oryza sativa* | Os12g18150 | 95 | 96 |
| *Glycine max* | Gm_JMJ_1 | 97 | 98 |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest.

### Example 2: Alignment of JMJC polypeptide sequences

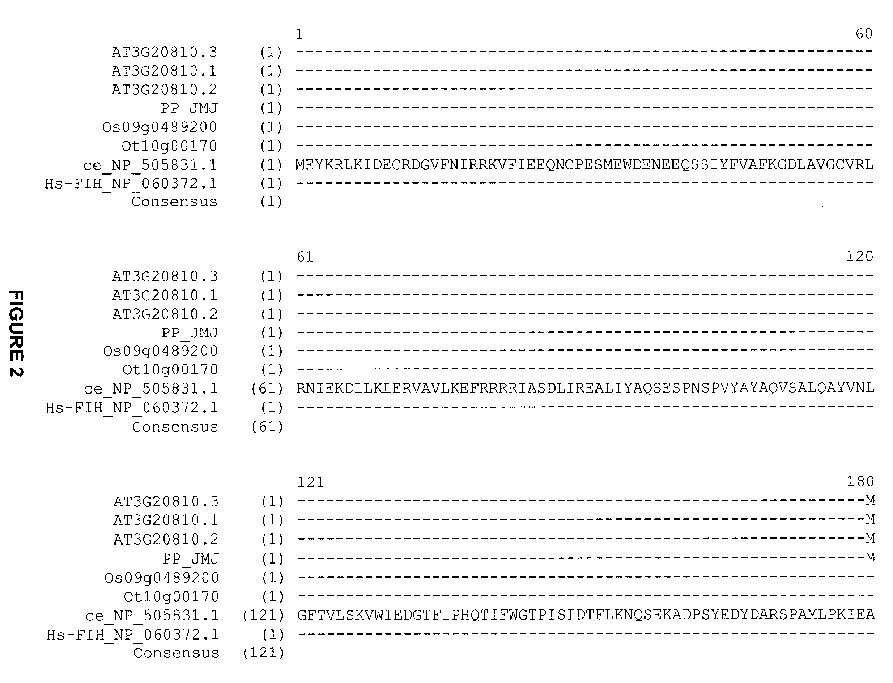

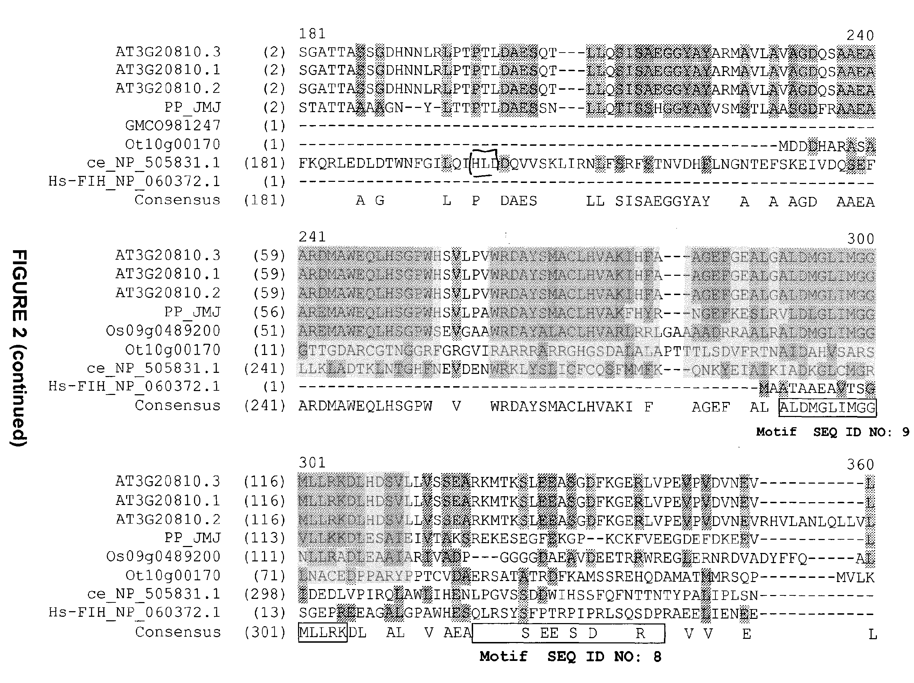

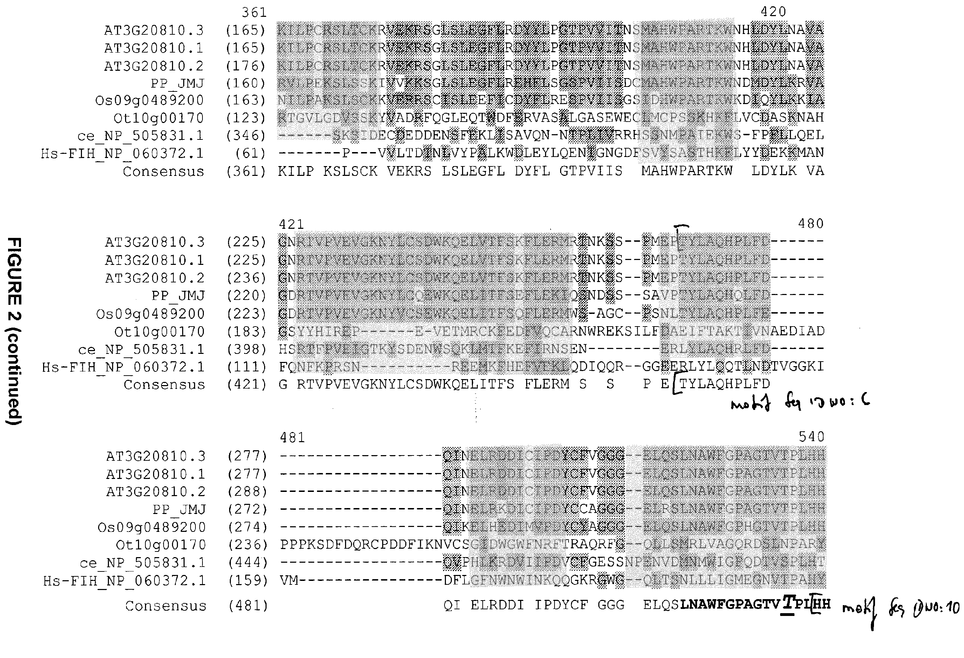

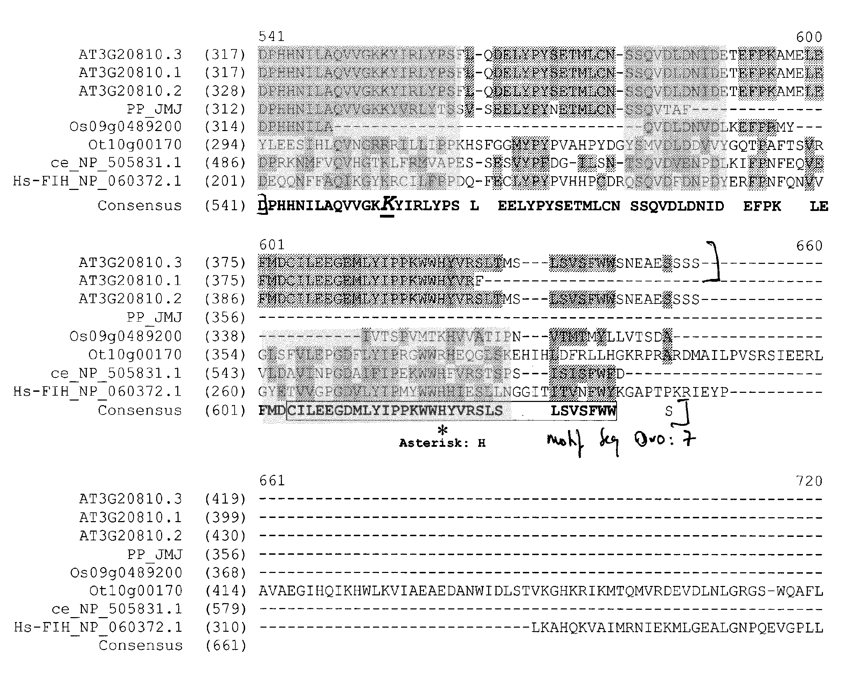

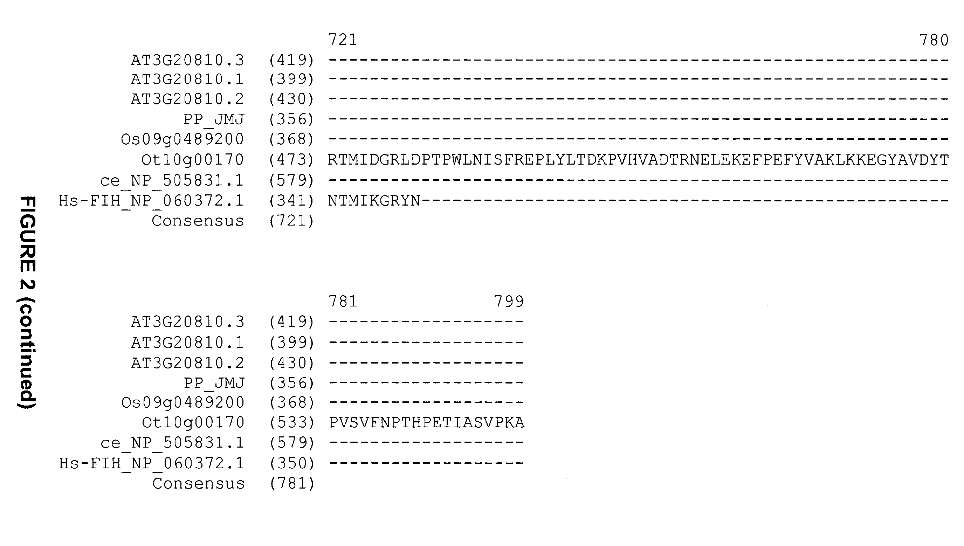

Alignment of polypeptide sequences was performed using the AlignX programme from the Vector NTI (Invitrogen) which is based on the popular Clustal W algorithm of progressive alignment (Thompson et al. (1997) Nucleic Acids Res 25:4876-4882; Chenna et al. (2003). Nucleic Acids Res 31:3497-3500). Default values are for the gap open penalty of 10, for the gap extension penalty of 0,1 and the selected weight matrix is Blosum 62 (if polypeptides are aligned). Minor manual editing may be done to further optimise the alignment. Sequence conservation among JMJC polypeptides is mostly in the JmjC domain of the polypeptides. The region corresponding to the motifs represented by SEQ ID NO: 7 and by SEQ ID NO: 9 is more conserved than that of motif 8. A consensus sequence is given. Amino acid residues in the consensus sequences are highly conserved. Blanks in the conserved sequences represent any amino acid. The JMJC polypeptides are aligned in Figure 2.

### Example 3: Calculation of global percentage identity between JMJC polypeptide sequences useful in performing the methods of the invention

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

| | |
|---|---|
| Scoring matrix: | Blosum62 |
| First Gap: | 12 |
| Extending gap: | 2 |

Results of the software analysis are shown in Table A2 for the global similarity and identity over the full length of the polypeptide sequences. Percentage identity is given above the diagonal in bold and percentage similarity is given below the diagonal (normal face).

The percentage identity between the JMJC polypeptide sequences useful in performing the methods of the invention can be as low as 15 % amino acid identity compared to SEQ ID NO: 2.

**Table A2: MatGAT results for global similarity and identity over the full length of the polypeptide sequences.**

| | | **SEQ ID NO: 2** | **SEQ ID NO: 12** | **SEQ ID NO: 14** | **SEQ ID NO: 18** | **SEQ ID NO: 20** | **SEQ ID NO: 22** | **SEQ ID NO: 64** |
|---|---|---|---|---|---|---|---|---|
| **AT3G20810.1** | **SEQ ID NO: 2** | | **97.4** | **16.1** | **15.5** | **16.9** | **17.3** | **15.5** |
| **AT3G20810.2** | **SEQ ID NO: 12** | 97.4 | | **15.5** | **14.5** | **17.5** | **17.1** | **14.9** |
| **AT5G19840** | **SEQ ID NO: 14** | 30.3 | 30.7 | | **16.9** | **17** | **16.9** | **16.9** |
| **ABE92082** | **SEQ ID NO: 18** | 28.2 | 27.7 | 28.5 | | **51.8** | **51.7** | **17.3** |
| **CAJ26373** | **SEQ ID NO: 20** | 30.1 | 29.6 | 27.7 | 67.6 | | **82.5** | **12.4** |
| **Os09g0483600** | **SEQ ID NO: 22** | 30.1 | 30.1 | 27.5 | 67.3 | 89.6 | | **16.1** |
| **Os01g36630** | **SEQ ID NO: 64** | 33.7 | 31.2 | 32.9 | 34.2 | 29.1 | 29.6 | |

### Example 4: Identification of domains comprised in JMJC polypeptide sequences useful in performing the methods of the invention

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Pfam is a large collection of multiple sequence alignments and hidden Markov models covering many common protein domains and families. Pfam is hosted at the Sanger Institute server in the United Kingdom. Interpro is hosted at the European Bioinformatics Institute in the United Kingdom.
Table A3 shows the settings (Gathering cut off, trusted cut off and noise cut off) as described in the Pfam database that were used to produce the HMMs_fs for the different domains.

**Table A3: HMMs_fs settings.**

| **Domain** | **Gathering cutoff** | **Trusted cutoff** | **Noise cutoff** |
|---|---|---|---|
| PF02373 | 16 | 16.1 | 15.9 |
| PF02375 | 15 | 16.8 | 13.8 |
| PF02928 | 25 | 44.4 | 21.1 |
| PF00646 | 17.7 | 17.7 | 17.6 |
| PF00096 | 22.5 | 22.5 | 22.4 |
| PF04967 | 15.4 | 15.4 | 3 |

The results of the Pfam scan for representative JMJC polypeptides of plant origin are presented in Table A4. The amino acid coordinates for each domain in the sequence of reference is indicated in the columns Start and End. The E-value of the alignment is also given. The InterPro ID accession number of each domain identified is also provided.

**Table A4: Pfam scan results (major accession numbers) of representative JMJC polypeptides of plant origin.**

| **Protein SEQ ID NO:** | **Genbank (genomic locus) accession number (name)** | **Database** | **Entry** | **E_value** | **Start** | **End** | **InterPro ID** | **InterPro Description** |
|---|---|---|---|---|---|---|---|---|
| 24 | AT1G08620 | Pfam | PF02373 | 1.10E-62 | 368 | 484 | IPR003347 | Transcription factor jumonji, jmjC |
| 24 | AT1G08620 | Pfam | PF02375 | 1.50E-16 | 127 | 165 | IPR003349 | Transcription factor jumonji, JmjN |
| 24 | AT1G08620 | Pfam | PF02928 | 9.10E-22 | 591 | 644 | IPR004198 | Zn-finger, C5HC2 type |
| 24 | AT1G08620 | SMART | SM00541 | 1.10E-17 | 962 | 1006 | IPR003888 | FY-rich domain, N-terminal |
| 24 | AT1G08620 | SMART | SM00542 | 1.30E-43 | 1012 | 1106 | IPR003889 | FY-rich domain, C-terminal |
| 26 | AT1G09060 | Pfam | PF02373 | 6.80E-06 | 644 | 856 | IPR003347 | Transcription factor jumonji, jmjC |
| 32 | AT1G11950 | Pfam | PF02373 | 1.00E-23 | 645 | 825 | IPR003347 | Transcription factor jumonji, jmjC |
| 34 | AT1G30810 | Pfam | PF02373 | 5.00E-30 | 294 | 378 | IPR003347 | Transcription factor jumonji, jmjC |
| 34 | AT1G30810 | Pfam | PF02375 | 3.10E-24 | 58 | 104 | IPR003349 | Transcription factor jumonji, JmjN |
| 34 | AT1G30810 | Pfam | PF02928 | 4.50E-30 | 487 | 540 | IPR004198 | Zn-finger, C5HC2 type |
| 34 | AT1G30810 | SMART | SM00541 | 1.30E-17 | 626 | 670 | IPR003888 | FY-rich domain, N-terminal |
| 34 | AT1G30810 | SMART | SM00542 | 1.20E-39 | 676 | 762 | IPR003889 | FY-rich domain, C-terminal |
| 36 | AT1G62310 | Pfam | PF02373 | 2.00E-27 | 733 | 846 | IPR003347 | Transcription factor jumonji, jmjC |
| 36 | AT1G62310 | ProSite | PS50089 | 10.173 | 209 | 256 | IPR001841 | Zn-finger, RING |
| 38 | AT1G63490 | Pfam | PF00628 | 5.40E-08 | 1009 | 1053 | IPR001965 | Zn-finger-like, PHD finger |
| 38 | AT1G63490 | Pfam | PF02373 | 3.10E-60 | 66 | 182 | IPR003347 | Transcription factor jumonji, jmjC |
| 38 | AT1G63490 | Pfam | PF02928 | 4.80E-33 | 276 | 329 | IPR004198 | Zn-finger, C5HC2 type |
| 40 | AT1G78280 | Pfam | PF00646 | 0.00012 | 15 | 62 | IPR001810 | Cyclin-like F-box |
| 40 | AT1G78280 | Pfam | PF02373 | 4.10E-12 | 249 | 362 | IPR003347 | Transcription factor jumonji, jmjC |
| 40 | AT1G78280 | ProSite | PS50181 | 9.603 | 14 | 60 | IPR001810 | Cyclin-like F-box |
| 42 | AT2G34880 | Pfam | PF02373 | 1.50E-67 | 294 | 410 | IPR003347 | Transcription factor jumonji, jmjC |
| 42 | AT2G34880 | Pfam | PF02375 | 3.70E-29 | 60 | 106 | IPR003349 | Transcription factor jumonji, JmjN |
| 42 | AT2G34880 | Pfam | PF02928 | 1.40E-23 | 514 | 567 | IPR004198 | Zn-finger, C5HC2 type |
| 42 | AT2G34880 | SMART | SM00541 | 9.90E-17 | 643 | 687 | IPR003888 | FY-rich domain, N-terminal |
| 42 | AT2G34880 | SMART | SM00542 | 4.60E-39 | 693 | 781 | IPR003889 | FY-rich domain, C-terminal |
| 44 | AT2G38950 | Pfam | PF02373 | 4.60E-47 | 321 | 437 | IPR003347 | Transcription factor jumonji, jmjC |
| 44 | AT2G38950 | Pfam | PF02375 | 9.30E-28 | 107 | 153 | IPR003349 | Transcription factor jumonji, JmjN |
| 44 | AT2G38950 | Pfam | PF02928 | 5.90E-27 | 544 | 597 | IPR004198 | Zn-finger, C5HC2 type |
| 46 | AT3G07610 | Pfam | PF02373 | 3.40E-15 | 726 | 843 | IPR003347 | Transcription factor jumonji, jmjC |
| 48 | AT3G48430 | Pfam | PF00096 | 0.014 | 1243 | 1268 | IPR007087 | Zn-finger, C2H2 type |
| 48 | AT3G48430 | Pfam | PF00096 | 0.0022 | 1296 | 1320 | IPR007087 | Zn-finger, C2H2 type |
| 48 | AT3G48430 | Pfam | PF00096 | 0.00026 | 1326 | 1352 | IPR007087 | Zn-finger, C2H2 type |
| 48 | AT3G48430 | Pfam | PF02373 | 9.00E-52 | 233 | 352 | IPR003347 | Transcription factor jumonji, jmjC |
| 48 | AT3G48430 | Pfam | PF02375 | 1.30E-07 | 19 | 62 | IPR003349 | Transcription factor jumonji, JmjN |
| 48 | AT3G48430 | ProSite | PS00028 | 0.00008 | 1268 | 1290 | IPR007087 | Zn-finger, C2H2 type |
| 50 | AT4G00990 | Pfam | PF02373 | 2.80E-16 | 607 | 781 | IPR003347 | Transcription factor jumonji, jmjC |
| 52 | AT4G20400 | Pfam | PF02373 | 4.60E-68 | 239 | 355 | IPR003347 | Transcription factor jumonji, jmjC |
| 52 | AT4G20400 | Pfam | PF02375 | 9.60E-11 | 7 | 44 | IPR003349 | Transcription factor jumonji, JmjN |
| 52 | AT4G20400 | Pfam | PF02928 | 5.70E-30 | 462 | 515 | IPR004198 | Zn-finger, C5HC2 type |
| 52 | AT4G20400 | SMART | SM00541 | 4.40E-15 | 683 | 727 | IPR003888 | FY-rich domain, N-terminal |
| 52 | AT4G20400 | SMART | SM00542 | 7.40E-41 | 733 | 830 | IPR003889 | FY-rich domain, C-terminal |
| 56 | AT5G04240 | Pfam | PF00096 | 0.41 | 1228 | 1253 | IPR007087 | Zn-finger, C2H2 type |
| 56 | AT5G04240 | Pfam | PF00096 | 0.0028 | 1281 | 1305 | IPR007087 | Zn-finger, C2H2 type |
| 56 | AT5G04240 | Pfam | PF00096 | 0.0016 | 1311 | 1337 | IPR007087 | Zn-finger, C2H2 type |
| 56 | AT5G04240 | Pfam | PF02373 | 1.10E-48 | 292 | 411 | IPR003347 | Transcription factor jumonji, jmjC |
| 56 | AT5G04240 | Pfam | PF02375 | 1.00E-09 | 15 | 58 | IPR003349 | Transcription factor jumonji, JmjN |
| 56 | AT5G06550 | Pfam | PF00646 | 0.0013 | 81 | 128 | IPR001810 | Cyclin-like F-box |
| 56 | AT5G06550 | Pfam | PF02373 | 3.20E-07 | 311 | 422 | IPR003347 | Transcription factor jumonji, jmjC |
| 58 | AT5G46910 | Pfam | PF02373 | 1.00E-49 | 199 | 322 | IPR003347 | Transcription factor jumonji, jmjC |
| 60 | AT5G46910 | Pfam | PF02375 | 2.90E-09 | 21 | 62 | IPR003349 | Transcription factor jumonji, JmjN |
| 62 | AT5G63080 | Pfam | PF02373 | 3.90E-16 | 164 | 270 | IPR003347 | Transcription factor jumonji, jmjC |
| 2 | AT3G20810 | Pfam | PF02373 | 0.00013 | 378 | 409 | IPR003347 | Transcription factor jumonji, jmjC |
| 12 | AT5G19840 | Pfam | PF02373 | 0.036 | 173 | 281 | IPR003347 | Transcription factor jumonji, jmjC |
| 14 | AT5G19840 | Pfam | PF04967 | 0.47 | 295 | 307 | IPR007050 | HTH DNA binding domain |
| 98 | 98Gm_JMJ_1 | Pfam | PF02373.11 | 9.70E-06 | 297 | 404 | IPR003347 | JmjC domain |

### Example 5: Cloning of the JMJC nucleic acid sequence used in the methods of the invention

The nucleic acid sequence used in the methods of the invention was amplified by PCR using as template a custom-made *Arabidopsis thaliana* seedlings cDNA library (in pCMV Sport 6.0; Invitrogen, Paisley, UK). PCR was performed using Hifi Taq DNA polymerase in standard conditions, using 200 ng of template in a 50 µl PCR mix. An uspstream and downstream primer as represented by SEQ ID NO: 3 and SEQ ID NO: 4 respectively were used to amplify by PCR (Polymerase Chain Reaction) the coding region of JMJC as represented by SEQ ID NO: 1. The primers include the AttB sites for Gateway recombination to facilitate the cloning of the amplified PCR DNA fragment into a Gateway cloning vector.

The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", pJMJC. Plasmid pDONR201 was purchased from Invitrogen, as part of the Gateway^{®} technology.

The entry clone comprising SEQ ID NO: 1 was then used in an LR reaction with a destination vector used for *Oryza sativa* transformation. This vector contained as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 5) for constitutive expression was located upstream of this Gateway cassette.

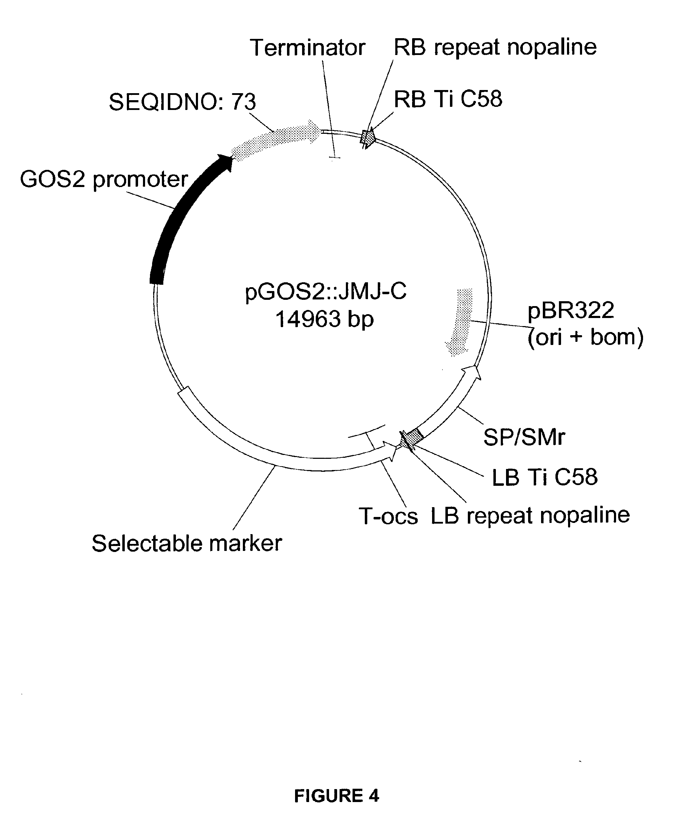

After the LR recombination step, the resulting expression vector pGOS2::JMJC (Figure 4) was transformed into Agrobacterium strain LBA4044 according to methods well known in the art.

### Example 6: Plant transformation

### Rice transformation

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2% HgCl₂, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were sub-cultured on fresh medium 3 days before co-cultivation (to boost cell division activity).

*Agrobacterium* strain LBA4404 containing the expression vector was used for co-cultivation. *Agrobacterium* was inoculated on AB medium with the appropriate antibiotics and cultured for 3 days at 28°C. The bacteria were then collected and suspended in liquid co-cultivation medium to a density (OD₆₀₀) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25°C. Co-cultivated calli were grown on 2,4-D-containing medium for 4 weeks in the dark at 28°C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

### Corn transformation

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 °C for 2-3 weeks, or until shoots develop. The green shoots are transferred from each embryo to maize rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Wheat transformation

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with *Agrobacterium,* the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

### Soybean transformation

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

### Rapeseed/canola transformation

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

### Alfalfa transformation

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 µm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

### Cotton transformation

Cotton (*Gossypium hirsutum* L.) transformation is performed using *Agrobacterium tumefaciens*, on hypocotyls explants. The commercial cultivars such as Coker 130 or Coker 312 (SeedCo, Lubbock, TX) are standard varieties used for transformation, but other varieties can also be used. The seeds are surface sterilized and germinated in the dark. Hypocotyl explants are cut from the germinated seedlings to lengths of about 1-1.5 centimeter. The hypotocyl explant is submersed in the *Agrobacterium tumefaciens* inoculum containing the expression vector, for 5 minutes then co-cultivated for about 48 hours on MS +1.8 mg/l KNO3 + 2% glucose at 24° C, in the dark. The explants are transferred the same medium containing appropriate bacterial and plant selectable markers (renewed several times), until embryogenic calli is seen. The calli are separated and subcultured until somatic embryos appear. Plantlets derived from the somatic embryos are matured on rooting medium until roots develop. The rooted shoots are transplanted to potting soil in the greenhouse. T1 seeds are produced from plants that exhibit tolerance to the selection agent and that contain a single copy of the T-DNA insert.

### Example 7: Phenotypic evaluation procedure

### 7.1 Evaluation setup

Approximately 35 independent T0 rice transformants were generated. The primary transformants were transferred from a tissue culture chamber to a greenhouse for growing and harvest of T1 seed. Eight events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%. Plants grown under non-stress conditions are supplied with water at regular intervals to ensure that water and nutrients are not limiting to satisfy plant needs to complete growth and development.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

### Drought screen

Plants from T2 seeds were grown in potting soil under normal conditions until they approached the heading stage. They were then transferred to a "dry" section where irrigation was withheld. Humidity probes were inserted in randomly chosen pots to monitor the soil water content (SWC). When SWC went below certain thresholds, the plants were automatically re-watered continuously until a normal level was reached again. The plants were then re-transferred again to normal conditions. The rest of the cultivation (plant maturation, seed harvest) was the same as for plants not grown under abiotic stress conditions. Growth and yield parameters are recorded as detailed for growth under normal conditions.

### Nitrogen use efficiency screen

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

### 7.2 Statistical analysis: F test

A two factor ANOVA (analysis of variants) was used as a statistical model for the overall evaluation of plant phenotypic characteristics. An F test was carried out on all the parameters measured of all the plants of all the events transformed with the gene of the present invention. The F test was carried out to check for an effect of the gene over all the transformation events and to verify for an overall effect of the gene, also known as a global gene effect. The threshold for significance for a true global gene effect was set at a 5% probability level for the F test. A significant F test value points to a gene effect, meaning that it is not only the mere presence or position of the gene that is causing the differences in phenotype.

Because two experiments with overlapping events were carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P values were obtained by comparing likelihood ratio test to chi square distributions.

### 7.3 Parameters measured

### Biomass-related parameter measurement

From the stage of sowing until the stage of maturity the plants were passed several times through a digital imaging cabinet. At each time digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the area measured at the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

Early vigour was determined by counting the total number of pixels from aboveground plant parts discriminated from the background. This value was averaged for the pictures taken on the same time from different angles and was converted to a physical surface value expressed in square mm by calibration. The results described below are for plants three weeks post-germination.

### Seed-related parameter measurements

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area (mm²), multiplied by a factor 10⁶. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

### Example 8: Results of the phenotypic evaluation of the transgenic plants

The results of the evaluation of transgenic rice plants expressing the JMJC nucleic acid as represented by SEQ ID NO: 1 under non-stress conditions are presented below. An increase of at least 5 % was observed for emergence vigour (early vigour), root/shoot index, total seed yield, harvest index, and of at least 3 % for thousand kernel weight in the transgenic plants of when compared to the control nullizygote plants.

The results of the evaluation of transgenic rice plants expressing SEQ ID NO: 1 under drought-stress conditions are presented hereunder. An increase of at least 5 % was observed for root/shoot index, total seed weight, number of filled seeds, fill rate, harvest index and of at least 3 % for thousand kernel weight in the transgenic plants of when compared to the control nullizygote plants.

## Claims

1. A method for increasing at least one yield-related trait selected from plant emergence vigour, root/shoot index, seed yield, seed filling rate, harvest index and thousand kernel weight in plants relative to control plants, comprising increasing expression in a plant of a nucleic acid encoding a JMJC polypeptide, wherein said JMJC polypeptide comprises a JmjC domain represented by a sequence having at least 60% or more sequence identity to:
(i) SEQ ID NO: 6; and/or
(ii) one of the JmjC domains comprised in the JMJC polypeptides represented by SEQ ID NO: 12; SEQ ID NO: 14; SEQ ID NO: 24; SEQ ID NO: 26; SEQ ID NO: 32; SEQ ID NO: 34, SEQ ID NO: 36; SEQ ID NO: 38; SEQ ID NO: 40; SEQ ID NO: 42; SEQ ID NO: 44; SEQ ID NO: 46; SEQ ID NO: 48; SEQ ID NO: 50; SEQ ID NO: 52; SEQ ID NO: 56; SEQ ID NO: 58; SEQ ID NO: 60; and SEQ ID NO: 62, whose amino acid coordinates are given in Table A4; and
(iii) wherein said JMJC polypeptides comprise a motif having at least 60% or more sequence identity to any one or more of:
(a) SEQ ID NO: 7,
(b) SEQ ID NO: 8,
(c) SEQ ID NO: 9,
(d) SEQ ID NO: 10.

2. Method according to claim 1, wherein said increased expression is effected by introducing and expressing in a plant a nucleic acid encoding a JMJC polypeptide.

3. Method according to claim 1 or 2, wherein said nucleic acid encoding a JMJC polypeptide encodes any one of the proteins listed in Table A1 or is a portion of such a nucleic acid, or a nucleic acid capable of hybridising with such a nucleic acid.

4. Method according to one of claims 1 to 3, wherein said nucleic acid sequence encodes an orthologue or paralogue of any of the proteins given in Table A1.

5. Method according to claim 4, wherein said nucleic acid encodes SEQ ID NO: 2.

6. Method according to any one of claims 1 to 5, wherein said increased yield-related traits comprise increased harvest index and/or seed yield relative to control plants.

7. Method according to any one of claims 1 to 6, wherein said increased yield-related trait comprises plant emergence vigour relative to control plants.

8. Method according to any one of claims 1 to 7, wherein said increased yield-related trait is obtained under non-stress conditions.

9. Method according to any one of claims 1 to 7, wherein said increased yield-related trait is obtained under mild drought stress growth conditions.

10. Method according to any one of claims 1 to 9, wherein said increased yield-related trait is obtained under growth conditions of nitrogen deficiency.

11. Method according to any one of claims 2 to 10, wherein said nucleic acid is operably linked to a constitutive promoter, preferably to a GOS2 promoter, most preferably to a GOS2 promoter from rice.

12. Method according to any one of claims 1 to 11, wherein said nucleic acid encoding a JMJC polypeptide is of plant origin, preferably from a dicotyledonous plant, further preferably from the family Brassicaceae, more preferably from the genus *Arabidopsis,* most preferably from *Arabidopsis thaliana.*

13. Plant or part thereof, including seeds, obtainable by a method according to any one of claims 1 to 12, wherein said plant or part thereof comprises a recombinant nucleic acid encoding a JMJC polypeptide as defined in claim 1 operably linked to a GOS2 promoter.

14. Construct comprising:
(i) nucleic acid encoding a JMJC polypeptide as defined in claim 1;
(ii) one or more control sequences capable of driving expression of the nucleic acid sequence of (i) comprising a GOS2 promoter; and optionally
(iii) a transcription termination sequence.

15. Construct according to claim 14, wherein said GOS2 promoter is from rice.

16. Use of a construct according to claim 14 or 15 in a method for making plants having increased yield, particularly plant emergence vigour and/or increased seed yield relative to control plants.

17. Plant, plant part or plant cell transformed with a construct according to claim 14 or 15.

18. Method for the production of a transgenic plant having increased yield, particularly plant emergence vigour and/or increased seed yield relative to control plants, comprising:
(i) introducing and expressing in a plant a nucleic acid encoding a JMJC polypeptide as defined in claim 1 under the control of a GOS2 promoter; and
(ii) cultivating the plant cell under conditions promoting plant growth and development.

19. Transgenic plant having increased yield, particularly increased plant seedling vigour and/or increased seed yield, relative to control plants, resulting from increased expression of a nucleic acid encoding a JMJC polypeptide as defined in claim 1 or 2, or a transgenic plant cell derived from said transgenic plant, wherein said plant comprises a construct according to claim 14 or 15.

20. Transgenic plant according to claim 13, 17, or 19, or a transgenic plant cell derived thereof, wherein said plant is a crop plant or a monocot or a cereal, such as rice, maize, wheat, barley, millet, rye, triticale, sorghum and oats.

21. Harvestable parts of a plant according to claim 20, wherein said harvestable parts are preferably shoot biomass and/or seeds and wherein said harvestable parts comprise a construct according to claim 14 or 15.

22. Use of a nucleic acid encoding a JMJC polypeptide as defined in claim 1 in increasing yield, particularly in increasing seed yield and/or shoot biomass in plants, relative to control plants.

## Patentansprüche

1. Verfahren zur Steigerung mindestens eines Ertragsmerkmals ausgewählt aus Pflanzenvitalität am Auslauf, Wurzel/Spross-Index, Samenertrag, Samenfüllungsrate, Harvest-Index und Tausendkomgewicht in Pflanzen im Vergleich zu Kontrollpflanzen, bei dem in einer Pflanze die Expression einer Nukleinsäure, die ein JMJC-Polypeptid kodiert, erhöht wird, wobei das JMJC-Polypeptid eine JmjC-Domäne umfasst, die durch eine Sequenz veranschaulicht wird, die mindestens 60% oder mehr Sequenzidentität aufweist zu:
(i) SEQ ID NO: 6; und/oder
(ii) einer der JmjC-Domänen, die sich in den JMJC-Polypeptiden befinden, die durch SEQ ID NO: 12; SEQ ID NO: 14; SEQ ID NO: 24; SEQ ID NO: 26; SEQ ID NO: 32; SEQ ID NO: 34; SEQ ID NO: 36; SEQ ID NO: 38; SEQ ID NO: 40; SEQ ID NO: 42; SEQ ID NO: 44; SEQ ID NO: 46; SEQ ID NO: 48; SEQ ID NO: 50; SEQ ID NO: 52; SEQ ID NO: 56; SEQ ID NO: 58; SEQ ID NO: 60; und SEQ ID NO: 62 veranschaulicht werden, deren Aminosäurekoordinaten in der Tabelle A4 angegeben sind; und
(iii) wobei die JMJC-Polypeptide ein Motiv umfassen, das mindestens 60% oder mehr Sequenzidentität aufweist zu einer oder mehreren Sequenz(en) von:
(a) SEQ ID NO: 7,
(b) SEQ ID NO: 8,
(c) SEQ ID NO: 9,
(d) SEQ ID NO: 10.

2. Verfahren nach Anspruch 1, wobei die erhöhte Expression durch Einführen und Exprimieren einer Nukleinsäure, die ein JMJC-Polypeptid kodiert, in eine(r) Pflanze herbeigeführt wird.

3. Verfahren nach Anspruch 1 oder 2, wobei die Nukleinsäure, die ein JMJC-Polypeptid kodiert, eines der in Tabelle A1 aufgeführten Proteine kodiert, oder ein Teil einer solchen Nukleinsäure oder eine Nukleinsäure ist, die mit einer solchen Nukleinsäure hybridisieren kann.

4. Verfahren nach einem der Ansprüche 1 bis 3, wobei die Nukleinsäuresequenz ein Orthologon oder Paralogon von einem der in Tabelle A1 angegebenen Proteine kodiert.

5. Verfahren nach Anspruch 4, wobei die Nukleinsäure SEQ ID NO: 2 kodiert.

6. Verfahren nach einem der Ansprüche 1 to 5, wobei die gesteigerten Ertragsmerkmale einen gesteigerten Harvest-Index und/oder Samenertrag im Vergleich zu Kontrollpflanzen umfassen.

7. Verfahren nach einem der Ansprüche 1 bis 6, wobei das gesteigerte Ertragsmerkmal Pflanzenvitalität am Auslauf im Vergleich zu Kontrollpflanzen umfasst.

8. Verfahren nach einem der Ansprüche 1 bis 7, wobei das gesteigerte Ertragsmerkmal unter Nichtstressbedingungen erhalten wird.

9. Verfahren nach einem der Ansprüche 1 bis 7, wobei das gesteigerte Ertragsmerkmal unter Wachstumsbedingungen eines leichten Trockenheitsstress erhalten wird.

10. Verfahren nach einem der Ansprüche 1 bis 9, wobei das gesteigerte Ertragsmerkmal unter Stickstoffmangel-Wachstumsbedingungen erhalten wird.

11. Verfahren nach einem der Ansprüche 2 bis 10, wobei die Nukleinsäure funktionsfähig mit einem konstitutiven Promotor, vorzugsweise einem GOS2-Promotor, am stärksten bevorzugt einem GOS2 Promotor aus Reis, verknüpft ist.

12. Verfahren nach einem der Ansprüche 1 bis 11, wobei die Nukleinsäure, die ein JMJC-Polypeptid kodiert, aus einer Pflanze, vorzugsweise aus einer dikotylen Pflanze, weiter bevorzugt aus der Familie Brassicaceae, stärker bevorzugt aus der Gattung *Arabidopsis,* am stärksten bevorzugt aus *Arabidopsis thaliana,* stammt.

13. Pflanze oder Teil davon, einschließlich Samen, die durch ein Verfahren nach einem der Ansprüche 1 bis 12 erhältlich sind, wobei die Pflanze oder das Teil davon eine rekombinante Nukleinsäure umfasst, die ein JMJC-Polypeptid nach Anspruch 1 kodiert und funktionsfähig mit einem GOS2-Promotor verknüpft ist.

14. Konstrukt, umfassend:
(i) eine Nukleinsäure, die ein JMJC-Polypeptid nach Anspruch 1 kodiert;
(ii) eine oder mehrere Kontrollsequenzen, die die Expression der Nukleinsäuresequenz von (i), die einen GOS2-Promotor umfasst, steuern können; und gegebenenfalls
(iii) eine Transkriptionsterminationssequenz.

15. Konstrukt nach Anspruch 14, wobei der GOS2-Promotor aus Reis ist.

16. Verwendung eines Konstrukts nach Anspruch 14 oder 15 in einem Verfahren zur Herstellung von Pflanzen mit gesteigertem Ertrag, insbesondere Pflanzenvitalität am Auslauf und/oder gesteigertem Samenertrag im Vergleich zu Kontrollpflanzen.

17. Pflanze, Pflanzenteil oder Pflanzenzelle, die mit einem Konstrukt nach Anspruch 14 oder 15 transformiert ist.

18. Verfahren zur Herstellung einer transgenen Pflanze mit gesteigertem Ertrag, insbesondere Pflanzenvitalität am Auslauf und/oder gesteigertem Samenertrag im Vergleich zu Kontrollpflanzen, umfassend:
(i) Einführen und Exprimieren einer Nukleinsäure, die ein JMJC-Polypeptid nach Anspruch 1 kodiert, in eine(r) Pflanze unter Kontrolle eines GOS2-Promotors; und
(ii) Züchten der Pflanzenzelle unter Bedingungen, die das Wachstum und die Entwicklung der Pflanze fördern.

19. Transgene Pflanze mit gesteigertem Ertrag, insbesondere gesteigerter Jungpflanzenvitalität beim Keimling und/oder gesteigertem Samenertrag im Vergleich zu Kontrollpflanzen, der sich aus der gesteigerten Expression einer Nukleinsäure ergibt, die ein JMJC-Polypeptid nach Anspruch 1 oder 2 kodiert, oder eine transgene Pflanzenzelle, die aus der transgenen Pflanze stammt, wobei die Pflanze ein Konstrukt nach Anspruch 14 oder 15 umfasst.

20. Transgene Pflanze nach Anspruch 13, 17 oder 19, oder eine daraus stammende transgene Pflanzenzelle, wobei die Pflanze eine Kulturpflanze oder eine Monokotyle oder ein Getreide, wie Reis, Mais, Weizen, Gerste, Hirse, Roggen, Triticale, Sorghum und Hafer ist.

21. Erntefähige Teile einer Pflanze nach Anspruch 20, wobei die erntefähigen Teile vorzugsweise Sprossbiomasse und/oder Samen sind und wobei die erntefähigen Teile ein Konstrukt nach Anspruch 14 oder 15 umfassen.

22. Verwendung einer Nukleinsäure, die ein JMJC-Polypeptid nach Anspruch 1 kodiert, bei der Steigerung des Ertrags, insbesondere bei der Steigerung des Samenertrags und/oder Sprossbiomasse in Pflanzen, im Vergleich zu Kontrollpflanzen.

## Revendications

1. Procédé pour augmenter au moins un caractère lié au rendement choisi parmi la vigueur de levée de plantes, l'indice de racine/pousse, le rendement en graines, le taux de graines pleines, l'indice de récolte et le poids de mille grains, dans des plantes, par rapport à des plantes témoins, comprenant l'augmentation de l'expression dans une plante d'un acide nucléique codant pour un polypeptide JMJC, ledit polypeptide JMJC comprenant un domaine JmjC représenté par une séquence ayant une identité de séquence d'au moins 60% ou plus avec .
(i) SEQ ID N0: 6; et/au
(ii) un des domaines JmjC compris dans les polypeptides compris dans les polypeptides JMJC représenté par SEQ ID N0: 12 ; SEQ ID NO: 14 ; SEQ ID NO: 24 ; SEQ ID NO: 26 ; SEQ ID NO: 32 ; SEQ ID NO: 34, SEQ ID NO: 36 ; SEQ ID NO: 38 ; SEQ ID NO: 40 ; SEQ ID NO: 42 ; SEQ ID NO: 44 ; SEQ ID NO: 46 ; SEQ ID NO: 48 ; SEQ ID NO: 50 ; SEQ ID NO: 52 ; SEQ ID NO: 56 ; SEQ ID NO: 58 ; SEQ ID NO: 60 et SEQ ID NO: 62, dont les coordonnées d'acides aminés sont décrites dans le tableau A4 ; et
(iii) lesdits polypeptides JMJC comprenant un motif ayant au moins 60 % ou plus d'identité de séquence avec l'un ou plusieurs quelconques :
(a) SEQ ID NO: 7,
(b) SEQ ID NO: 8,
(c) SEQ ID NO: 9,
(d) SEQ ID NO: 10.

2. Procédé selon la revendication 1, dans lequel ladite expression accrue est obtenue par l'introduction et l'expression dans une plante d'un acide nucléique codant pour un polypeptide JMJC.

3. Procédé selon la revendication 1 ou 2, dans lequel ledit acide nucléique codant pour un polypeptide JMJC code pour l'une quelconque des protéines listées dans le Tableau A1, ou est une portion d'un tel acide nucléique, ou un acide nucléique capable de s'hybrider à un tel acide nucléique.

4. Procédé selon l'une des revendications 1 à 3, dans lequel ladite séquence d'acide nucléique code pour un orthologue ou un paralogue de l'une quelconque des protéines consignées dans le Tableau A1.

5. Procédé selon la revendication 4, dans lequel ledit acide nucléique code pour SEQ ID NO: 2.

6. Procédé selon l'une quelconque des revendications 1 à 5, dans lequel lesdits caractères liés au rendement augmentés comprennent un indice de récolte et/ou un rendement en grains augmenté par rapport à des plantes témoins.

7. Procédé selon l'une quelconque des revendications 1 à 6, dans lequel ledit caractère lié au rendement augmenté comprend la vigueur de levée de plantes par rapport à des plantes témoins.

8. Procédé selon l'une quelconque des revendications 1 à 7, dans lequel ladite augmentation de caractère lié au rendement est obtenue dans des conditions sans stress.

9. Procédé selon l'une quelconque des revendications 1 à 7, dans lequel ladite augmentation de caractère lié au rendement est obtenue dans des conditions de croissance sous stress de légère sécheresse.

10. Procédé selon l'une quelconque des revendications 1 à 9, dans lequel ladite augmentation de caractère lié au rendement est obtenue dans les conditions de croissance d'une déficience en azote.

11. Procédé selon l'une quelconque des revendications 2 à 10, dans lequel ledit acide nucléique est fonctionnellement lié à un promoteur constitutif, de préférence à un promoteur GOS2, le plus préférablement à un promoteur GOS2 de riz.

12. Procédé selon l'une quelconque des revendications 1 à 11, dans lequel ledit acide nucléique codant pour un polypeptide JMJC est d'origine végétale, en provenant de préférence d'une plante dicotylédone, d'une manière plus préférée de la famille des Brassicaceae, d'une manière plus préférée du genre *Arabidopsis,* tout spécialement d'*Arabidopsis* thaliana.

13. Plante ou partie de cette dernière, y compris les graines, pouvant être obtenue par un procédé selon l'une quelconque des revendications 1 à 12, ladite plante ou partie de cette dernière comprenant un acide nucléique recombinant codant pour un polypeptide JMJC tel que défini dans la revendication 1 fonctionnellement lié à un promoteur GOS2.

14. Construction comprenant :
(i) un acide nucléique codant pour un polypeptide JMJC tel que défini dans la revendication 1 ;
(ii) une ou plusieurs séquences régulatrices capables de commander l'expression de la séquence d'acide nucléique de (i) comprenant un promoteur GOS2 ; et en option
(iii) une séquence de terminaison de transcription.

15. Construction selon la revendication 14, dans laquelle ledit promoteur GOS2 est de riz.

16. Utilisation d'une construction selon la revendication 14 ou 15 dans un procédé de fabrication de plantes ayant un rendement accru, en particulier une vigueur de levée de plantes et/ou un rendement en graines accru, par rapport à des plantes témoins.

17. Plante, partie de plante ou cellule végétale transformée avec une construction selon la revendication 14 ou 15.

18. Procédé de production d'une plante transgénique présentant un rendement accru, en particulier une vigueur de levée de plantes et/ou un rendement en graines accru, par rapport à des plantes témoins, comprenant :
(i) l'introduction et l'expression dans une plante d'un acide nucléique codant pour un polypeptide JMJC tel que défini dans la revendication 1 sous le contrôle d'un promoteur GOS2 ; et
(ii) la culture de la cellule végétale dans des conditions favorisant la croissance et le développement de la plante.

19. Plante transgénique ayant un rendement accru, en particulier une vigueur de semis de plantes et/ou un rendement en graines accru, par rapport à des plantes témoins, résultant de l'augmentation de l'expression d'un acide nucléique codant pour un polypeptide JMJC tel que défini dans la revendication 1 ou 2, ou cellule végétale transgénique dérivant de ladite plante transgénique, ladite plante comprenant une construction selon la revendication 14 ou 15.

20. Plante transgénique selon la revendication 13, 17 ou 19, ou cellule végétale transgénique qui en dérive, ladite plante étant une plante de culture, ou une monocotylédone, ou une céréale telle que le riz, le maïs, le blé, l'orge, le millet, le seigle, le triticale, le sorgho et l'avoine.

21. Parties récoltables d'une plante selon la revendication 20, lesdites parties récoltables étant de préférence la biomasse des pousses et/ou les graines, et lesdites parties récoltables comprenant une construction selon la revendication 14 ou 15.

22. Utilisation d'un acide nucléique codant pour un polypeptide JMJC tel que défini dans la revendication 1 pour l'augmentation du rendement, en particulier pour l'augmentation du rendement en graines et/ou de la biomasse des pousses dans des plantes, par rapport à des plantes témoins.


### SEQUENCE LISTING

<110> BASF Plant Science GmbH
<120> Plants having enhanced yield-related traits and a method for making the same
<130> PF59065-EP-4
<160> 98
<170> PatentIn version 3.3
<210> 1
   <211> 1257
   <212> DNA
   <213> Arabidopsis thaliana
<400> 1
   atgtcaggag ctaccaccgc ttcctccgga gaccacaaca acctccgtct cccaactccg      60
   acgcttgacg ccgaatcgca aactctgctg caatcgattt ctgctgaagg gggttacgct     120
   tatgctcgca tggcggtgtt agcggtggcc ggtgaccaaa gcgcggcgga ggcggcgcgt     180
   gatatggctt gggagcaact tcactctggt ccgtggcact cggttctccc cgtttggcgc     240
   gacgcttact caatggcttg tctccacgta gctaaaatcc atttcgccgc cggcgagttt     300
   ggtgaggcgc ttggtgcgct tgatatgggt cttatcatgg gaggtatgct tctccgcaag     360
   gatcttcatg attctgtttt gttggtctcc tctgaagctc gtaagatgac gaagagtctt     420
   gaagaagctt ctggagattt caaaggtgag agattggtcc cggaagttcc cgtcgacgtc     480
   aatgaggtcc ttaagattct accttgcagg tctttaactt gtaaaagagt agagaagagg     540
   tctggtttat ctttggaggg gtttctgcgt gattattatc tgccaggtac accagttgtt     600
   ataaccaatt ctatggctca ttggccagcc aggaccaagt ggaatcactt ggactacctc     660
   aatgctgttg ctggtaaccg caccgttccc gtggaggtgg ggaaaaacta tttgtgttct     720
   gattggaagc aagagcttgt gacattttct aagttccttg aacggatgcg gaccaataaa     780
   tcgtctccaa tggagcctac ttatcttgcc cagcatcctt tgtttgatca gataaatgaa     840
   ctgagagatg atatatgtat tcctgattac tgttttgtcg gtggtgggga actccaatca     900
   cttaatgcat ggtttggccc ggctgggaca gttactccgt tacaccatga tccacatcat     960
   aatatacttg ctcaggttgt tggcaagaag tatataaggc tttacccatc cttcctgcaa    1020
   gacgaacttt acccttactc tgagacaatg ctctgcaact ctagtcaggt tgatctagac    1080
   aatatcgacg aaaccgagtt cccaaaggcc atggagttgg agtttatgga ttgtattcta    1140
   gaagaaggtg aaatgttgta catccctccc aaatggtggc actatgtcag gtccttaaca    1200
   atgagtctct cggttagctt ctggtggagc aatgaagcag aatcttctag ctcgtag      1257
<210> 2
   <211> 418
   <212> PRT
   <213> Arabidopsis thaliana
<400> 2
   Met Ser Gly Ala Thr Thr Ala Ser Ser Gly Asp His Asn Asn Leu Arg
   1               5                   10                  15
   Leu Pro Thr Pro Thr Leu Asp Ala Glu Ser Gln Thr Leu Leu Gln Ser
               20                  25                  30
   Ile Ser Ala Glu Gly Gly Tyr Ala Tyr Ala Arg Met Ala Val Leu Ala
           35                  40                  45
   Val Ala Gly Asp Gln Ser Ala Ala Glu Ala Ala Arg Asp Met Ala Trp
       50                  55                  60
   Glu Gln Leu His Ser Gly Pro Trp His Ser Val Leu Pro Val Trp Arg
   65                  70                  75                  80
   Asp Ala Tyr Ser Met Ala Cys Leu His Val Ala Lys Ile His Phe Ala
                   85                  90                  95
   Ala Gly Glu Phe Gly Glu Ala Leu Gly Ala Leu Asp Met Gly Leu Ile
               100                 105                 110
   Met Gly Gly Met Leu Leu Arg Lys Asp Leu His Asp Ser Val Leu Leu
           115                 120                 125
   Val Ser Ser Glu Ala Arg Lys Met Thr Lys Ser Leu Glu Glu Ala Ser
       130                 135                 140
   Gly Asp Phe Lys Gly Glu Arg Leu Val Pro Glu Val Pro Val Asp Val
   145                 150                 155                 160
   Asn Glu Val Leu Lys Ile Leu Pro Cys Arg Ser Leu Thr Cys Lys Arg
                   165                 170                 175
   Val Glu Lys Arg Ser Gly Leu Ser Leu Glu Gly Phe Leu Arg Asp Tyr
               180                 185                 190
   Tyr Leu Pro Gly Thr Pro Val Val Ile Thr Asn Ser Met Ala His Trp
           195                 200                 205
   Pro Ala Arg Thr Lys Trp Asn His Leu Asp Tyr Leu Asn Ala Val Ala
       210                 215                 220
   Gly Asn Arg Thr Val Pro Val Glu Val Gly Lys Asn Tyr Leu Cys Ser
   225                 230                 235                 240
   Asp Trp Lys Gln Glu Leu Val Thr Phe Ser Lys Phe Leu Glu Arg Met
                   245                 250                 255
   Arg Thr Asn Lys Ser Ser Pro Met Glu Pro Thr Tyr Leu Ala Gln His
               260                 265                 270
   Pro Leu Phe Asp Gln Ile Asn Glu Leu Arg Asp Asp Ile Cys Ile Pro
           275                 280                 285
   Asp Tyr Cys Phe Val Gly Gly Gly Glu Leu Gln Ser Leu Asn Ala Trp
       290                 295                 300
   Phe Gly Pro Ala Gly Thr Val Thr Pro Leu His His Asp Pro His His
   305                 310                 315                 320
   Asn Ile Leu Ala Gln Val Val Gly Lys Lys Tyr Ile Arg Leu Tyr Pro
                   325                 330                 335
   Ser Phe Leu Gln Asp Glu Leu Tyr Pro Tyr Ser Glu Thr Met Leu Cys
               340                 345                 350
   Asn Ser Ser Gln Val Asp Leu Asp Asn Ile Asp Glu Thr Glu Phe Pro
           355                 360                 365
   Lys Ala Met Glu Leu Glu Phe Met Asp Cys Ile Leu Glu Glu Gly Glu
       370                 375                 380
   Met Leu Tyr Ile Pro Pro Lys Trp Trp His Tyr Val Arg Ser Leu Thr
   385                 390                 395                 400
   Met Ser Leu Ser Val Ser Phe Trp Trp Ser Asn Glu Ala Glu Ser Ser
                   405                 410                 415
   Ser Ser
<210> 3
   <211> 2194
   <212> DNA
   <213> Oryza sativa
<400> 3
   aatccgaaaa gtttctgcac cgttttcacc ccctaactaa caatataggg aacgtgtgct     60
   aaatataaaa tgagacctta tatatgtagc gctgataact agaactatgc aagaaaaact    120
   catccaccta ctttagtggc aatcgggcta aataaaaaag agtcgctaca ctagtttcgt    180
   tttccttagt aattaagtgg gaaaatgaaa tcattattgc ttagaatata cgttcacatc    240
   tctgtcatga agttaaatta ttcgaggtag ccataattgt catcaaactc ttcttgaata    300
   aaaaaatctt tctagctgaa ctcaatgggt aaagagagag atttttttta aaaaaataga    360
   atgaagatat tctgaacgta ttggcaaaga tttaaacata taattatata attttatagt    420
   ttgtgcattc gtcatatcgc acatcattaa ggacatgtct tactccatcc caatttttat    480
   ttagtaatta aagacaattg acttattttt attatttatc ttttttcgat tagatgcaag    540
   gtacttacgc acacactttg tgctcatgtg catgtgtgag tgcacctcct caatacacgt    600
   tcaactagca acacatctct aatatcactc gcctatttaa tacatttagg tagcaatatc    660
   tgaattcaag cactccacca tcaccagacc acttttaata atatctaaaa tacaaaaaat    720
   aattttacag aatagcatga aaagtatgaa acgaactatt taggtttttc acatacaaaa    780
   aaaaaaagaa ttttgctcgt gcgcgagcgc caatctccca tattgggcac acaggcaaca    840
   acagagtggc tgcccacaga acaacccaca aaaaacgatg atctaacgga ggacagcaag    900
   tccgcaacaa ccttttaaca gcaggctttg cggccaggag agaggaggag aggcaaagaa    960
   aaccaagcat cctccttctc ccatctataa attcctcccc ccttttcccc tctctatata   1020
   ggaggcatcc aagccaagaa gagggagagc accaaggaca cgcgactagc agaagccgag   1080
   cgaccgcctt ctcgatccat atcttccggt cgagttcttg gtcgatctct tccctcctcc   1140
   acctcctcct cacagggtat gtgcctccct tcggttgttc ttggatttat tgttctaggt   1200
   tgtgtagtac gggcgttgat gttaggaaag gggatctgta tctgtgatga ttcctgttct   1260
   tggatttggg atagaggggt tcttgatgtt gcatgttatc ggttcggttt gattagtagt   1320
   atggttttca atcgtctgga gagctctatg gaaatgaaat ggtttaggga tcggaatctt   1380
   gcgattttgt gagtaccttt tgtttgaggt aaaatcagag caccggtgat tttgcttggt   1440
   gtaataaagt acggttgttt ggtcctcgat tctggtagtg atgcttctcg atttgacgaa   1500
   gctatccttt gtttattccc tattgaacaa aaataatcca actttgaaga cggtcccgtt   1560
   gatgagattg aatgattgat tcttaagcct gtccaaaatt tcgcagctgg cttgtttaga   1620
   tacagtagtc cccatcacga aattcatgga aacagttata atcctcagga acaggggatt   1680
   ccctgttctt ccgatttgct ttagtcccag aatttttttt cccaaatatc ttaaaaagtc   1740
   actttctggt tcagttcaat gaattgattg ctacaaataa tgcttttata gcgttatcct   1800
   agctgtagtt cagttaatag gtaatacccc tatagtttag tcaggagaag aacttatccg   1860
   atttctgatc tccattttta attatatgaa atgaactgta gcataagcag tattcatttg   1920
   gattattttt tttattagct ctcacccctt cattattctg agctgaaagt ctggcatgaa   1980
   ctgtcctcaa ttttgttttc aaattcacat cgattatcta tgcattatcc tcttgtatct   2040
   acctgtagaa gtttcttttt ggttattcct tgactgcttg attacagaaa gaaatttatg   2100
   aagctgtaat cgggatagtt atactgcttg ttcttatgat tcatttcctt tgtgcagttc   2160
   ttggtgtagc ttgccacttt caccagcaaa gttc                                2194
<210> 4
   <211> 54
   <212> DNA
   <213> Artificial sequence
<220>
   <223> primer 1
<400> 4
   ggggacaagt ttgtacaaaa aagcaggctt aaacaatgtc aggagctacc accg 54
<210> 5
   <211> 50
   <212> DNA
   <213> Artificial sequence
<220>
   <223> primer 2
<400> 5
   ggggaccact ttgtacaaga aagctgggta aaataaatcc attcgctacg 50
<210> 6
   <211> 152
   <212> PRT
   <213> Artificial sequence
<220>
   <223> JMJ-C domain of SEQ ID NO: 02
<400> 6
   Thr Tyr Leu Ala Gln His Pro Leu Phe Asp Gln Ile Asn Glu Leu Arg
   1               5                   10                  15
   Asp Asp Ile Cys Ile Pro Asp Tyr Cys Phe Val Gly Gly Gly Glu Leu
               20                  25                  30
   Gln Ser Leu Asn Ala Trp Phe Gly Pro Ala Gly Thr Val Thr Pro Leu
           35                  40                  45
   His His Asp Pro His His Asn Ile Leu Ala Gln Val Val Gly Lys Lys
       50                  55                  60
   Tyr Ile Arg Leu Tyr Pro Ser Phe Leu Gln Asp Glu Leu Tyr Pro Tyr
   65                  70                  75                  80
   Ser Glu Thr Met Leu Cys Asn Ser Ser Gln Val Asp Leu Asp Asn Ile
                   85                  90                  95
   Asp Glu Thr Glu Phe Pro Lys Ala Met Glu Leu Glu Phe Met Asp Cys
               100                 105                 110
   Ile Leu Glu Glu Gly Glu Met Leu Tyr Ile Pro Pro Lys Trp Trp His
           115                 120                 125
   Tyr Val Arg Ser Leu Thr Met Ser Leu Ser Val Ser Phe Trp Trp Ser
       130                 135                 140
   Asn Glu Ala Glu Ser Ser Ser Ser
   145                 150
<210> 7
   <211> 32
   <212> PRT
   <213> Artificial sequence
<220>
   <223> conserved box in JMJ-C domain of SEQ ID NO: 02
<400> 7
   Cys Ile Leu Glu Glu Gly Glu Met Leu Tyr Ile Pro Pro Lys Trp Trp
   1               5                   10                  15
   His Tyr Val Arg Ser Leu Thr Met Ser Leu Ser Val Ser Phe Trp Trp
               20                  25                  30
<210> 8
   <211> 23
   <212> PRT
   <213> Artificial sequence
<220>
   <223> DUF335 domain
<400> 8
   Arg Lys Met Thr Lys Ser Leu Glu Glu Ala Ser Gly Asp Phe Lys Gly
   1               5                   10                  15
   Glu Arg Leu Val Pro Glu Val
               20
<210> 9
   <211> 17
   <212> PRT
   <213> Artificial sequence
<220>
   <223> conserved motif
<220>
   <221> UNSURE
   <222> (11)..(11)
   <223> Xaa can be any naturally occurring amino acid
<400> 9
   Ala Leu Asp Met Gly Leu Ile Met Gly Gly Xaa Leu Leu Arg Lys Asp
   1               5                   10                  15
   Leu
<210> 10
   <211> 3
   <212> PRT
   <213> Artificial sequence
<220>
   <223> FE(II) binding consensus seq
<220>
   <221> UNSURE
   <222> (2)..(2)
   <223> Xaa can be any naturally occurring amino acid
<220>
   <221> VARIANT
   <222> (3)..(3)
   <223> /replace = "Val"
<400> 10
   His Xaa Asp
   1
<210> 11
   <211> 1290
   <212> DNA
   <213> Arabidopsis thaliana
<400> 11
   atgtcaggag ctaccaccgc ttcctccgga gaccacaaca acctccgtct cccaactccg      60
   acgcttgacg ccgaatcgca aactctgctg caatcgattt ctgctgaagg gggttacgct     120
   tatgctcgca tggcggtgtt agcggtggcc ggtgaccaaa gcgcggcgga ggcggcgcgt     180
   gatatggctt gggagcaact tcactctggt ccgtggcact cggttctccc cgtttggcgc     240
   gacgcttact caatggcttg tctccacgta gctaaaatcc atttcgccgc cggcgagttt     300
   ggtgaggcgc ttggtgcgct tgatatgggt cttatcatgg gaggtatgct tctccgcaag     360
   gatcttcatg attctgtttt gttggtctcc tctgaagctc gtaagatgac gaagagtctt     420
   gaagaagctt ctggagattt caaaggtgag agattggtcc cggaagttcc cgtcgacgtc     480
   aatgaggtga gacatgtttt ggctaatcta caattacttg tccttaagat tctaccttgc     540
   aggtctttaa cttgtaaaag agtagagaag aggtctggtt tatctttgga ggggtttctg     600
   cgtgattatt atctgccagg tacaccagtt gttataacca attctatggc tcattggcca     660
   gccaggacca agtggaatca cttggactac ctcaatgctg ttgctggtaa ccgcaccgtt     720
   cccgtggagg tggggaaaaa ctatttgtgt tctgattgga agcaagagct tgtgacattt     780
   tctaagttcc ttgaacggat gcggaccaat aaatcgtctc caatggagcc tacttatctt     840
   gcccagcatc ctttgtttga tcagataaat gaactgagag atgatatatg tattcctgat     900
   tactgttttg tcggtggtgg ggaactccaa tcacttaatg catggtttgg cccggctggg     960
   acagttactc cgttacacca tgatccacat cataatatac ttgctcaggt tgttggcaag    1020
   aagtatataa ggctttaccc atccttcctg caagacgaac tttaccctta ctctgagaca    1080
   atgctctgca actctagtca ggttgatcta gacaatatcg acgaaaccga gttcccaaag    1140
   gccatggagt tggagtttat ggattgtatt ctagaagaag gtgaaatgtt gtacatccct    1200
   cccaaatggt ggcactatgt caggtcctta acaatgagtc tctcggttag cttctggtgg    1260
   agcaatgaag cagaatcttc tagctcgtag                                     1290
<210> 12
   <211> 429
   <212> PRT
   <213> Arabidopsis thaliana
<400> 12
   Met Ser Gly Ala Thr Thr Ala Ser Ser Gly Asp His Asn Asn Leu Arg
   1               5                   10                  15
   Leu Pro Thr Pro Thr Leu Asp Ala Glu Ser Gln Thr Leu Leu Gln Ser
               20                  25                  30
   Ile Ser Ala Glu Gly Gly Tyr Ala Tyr Ala Arg Met Ala Val Leu Ala
           35                  40                  45
   Val Ala Gly Asp Gln Ser Ala Ala Glu Ala Ala Arg Asp Met Ala Trp
       50                  55                  60
   Glu Gln Leu His Ser Gly Pro Trp His Ser Val Leu Pro Val Trp Arg
   65                  70                  75                  80
   Asp Ala Tyr Ser Met Ala Cys Leu His Val Ala Lys Ile His Phe Ala
                   85                  90                  95
   Ala Gly Glu Phe Gly Glu Ala Leu Gly Ala Leu Asp Met Gly Leu Ile
               100                 105                 110
   Met Gly Gly Met Leu Leu Arg Lys Asp Leu His Asp Ser Val Leu Leu
           115                 120                 125
   Val Ser Ser Glu Ala Arg Lys Met Thr Lys Ser Leu Glu Glu Ala Ser
       130                 135                 140
   Gly Asp Phe Lys Gly Glu Arg Leu Val Pro Glu Val Pro Val Asp Val
   145                 150                 155                 160
   Asn Glu Val Arg His Val Leu Ala Asn Leu Gln Leu Leu Val Leu Lys
                   165                 170                 175
   Ile Leu Pro Cys Arg Ser Leu Thr Cys Lys Arg Val Glu Lys Arg Ser
               180                 185                 190
   Gly Leu Ser Leu Glu Gly Phe Leu Arg Asp Tyr Tyr Leu Pro Gly Thr
           195                 200                 205
   Pro Val Val Ile Thr Asn Ser Met Ala His Trp Pro Ala Arg Thr Lys
       210                 215                 220
   Trp Asn His Leu Asp Tyr Leu Asn Ala Val Ala Gly Asn Arg Thr Val
   225                 230                 235                 240
   Pro Val Glu Val Gly Lys Asn Tyr Leu Cys Ser Asp Trp Lys Gln Glu
                   245                 250                 255
   Leu Val Thr Phe Ser Lys Phe Leu Glu Arg Met Arg Thr Asn Lys Ser
               260                 265                 270
   Ser Pro Met Glu Pro Thr Tyr Leu Ala Gln His Pro Leu Phe Asp Gln
           275                 280                 285
   Ile Asn Glu Leu Arg Asp Asp Ile Cys Ile Pro Asp Tyr Cys Phe Val
       290                 295                 300
   Gly Gly Gly Glu Leu Gln Ser Leu Asn Ala Trp Phe Gly Pro Ala Gly
   305                 310                 315                 320
   Thr Val Thr Pro Leu His His Asp Pro His His Asn Ile Leu Ala Gln
                   325                 330                 335
   Val Val Gly Lys Lys Tyr Ile Arg Leu Tyr Pro Ser Phe Leu Gln Asp
               340                 345                 350
   Glu Leu Tyr Pro Tyr Ser Glu Thr Met Leu Cys Asn Ser Ser Gln Val
           355                 360                 365
   Asp Leu Asp Asn Ile Asp Glu Thr Glu Phe Pro Lys Ala Met Glu Leu
       370                 375                 380
   Glu Phe Met Asp Cys Ile Leu Glu Glu Gly Glu Met Leu Tyr Ile Pro
   385                 390                 395                 400
   Pro Lys Trp Trp His Tyr Val Arg Ser Leu Thr Met Ser Leu Ser Val
                   405                 410                 415
   Ser Phe Trp Trp Ser Asn Glu Ala Glu Ser Ser Ser Ser
               420                 425
<210> 13
   <211> 1518
   <212> DNA
   <213> Arabidopsis thaliana
<400> 13
   atgccgacgg gatgcgagtt tccgatgatc aaaaccttcg agaatgctct tactgccgcc     60
   gacttcgagt ctaccgtcga gctcaccaac ttcccagcag tttttcgggg atgtgctagt    120
   gtctgggatg cgtattctaa gtggaatcca ttcaacagtg gcctcgatta tctggaggaa    180
   cgtgcaggtt cagttgaagt ggaggcaatg ctttcgagga cggctccagt tttcaatggt    240
   gatatcagaa gccatgagag ggtgtcattg cctttctcgg attttatcag attctgcaag     300
   cagcatatga gaggcaaagg gaatggttct ggtgttgatg ctaagtctgc agatttaaat    360
   cctatgtgtg aggactatag acctggacaa atatacctag cacagtttcc aattctgaat    420
   gatgagaaag aagagaaggt tctacttaag attctgagac aagatatcca gacgcccaca    480
   tttttagacg caaagtcact ttcttctatc aacttttgga tgaattctgc agaagctcga    540
   tcaagtaccc actatgatcc acatcataac cttctatgcg tagtctcagg gcgtaaaaaa    600
   gttgtattgt ggcccccttc cgccagtccc tcgctctacc cgatgcctat atacggagaa    660
   gcatcaaacc atagttcggt tggtctagaa aatccaaatt tatcagatta tccaagagca    720
   gagcattcac tgaagcagtc acaggagatt actcttaacg ctggtgatgc agtattcatt    780
   cctgaaggtt ggttccatca ggtggatagt gatgaactga ctgttgctgt caacttttgg    840
   tggcaatcaa actatatgtc taacatgcct gaacacatgg attcgtatta tctgcgtcga    900
   atcacaagaa gattgataga cagagaaatg agcctgttag tctcgaagcc ttcttcaact    960
   gatttaaggc acctgtctga gcatattgac caatctcgca ttgagatggc agaaggtggg   1020
   aatgataata taggcaatga aagcattaaa aagggcctca gcacattgca tgaaaaagcg   1080
   tcgctgcacg atctagatcc ttctgcttcc caggcgcttc atgatcttat ttctttagtc   1140
   catgaccacg ttaatgctgt tgatacaaaa gatgatcgcg ttgcccattt gctttggaat   1200
   cttgaagcct ctagacttcg ggatgttctc cttgcaatgg cacgttattt cccaagaact   1260
   ttagaagcat taatactcca catgctctca cccattgctg cggaagttct aacacagaag   1320
   tttgatgaga ttgaccaaca gactggtgaa gaagatagga cccagttctt tcgggaattt   1380
   tatagtgcat tcgatgatga agcagctgcg atggatataa ttttaagtag gaaagaggct   1440
   tttgcatttc aggcatttaa gaatgtacta gacaagtttt tgggggtcaa cattgcttca   1500
   ccaacaacaa acatttga                                                 1518
<210> 14
   <211> 505
   <212> PRT
   <213> Arabidopsis thaliana
<400> 14
   Met Pro Thr Gly Cys Glu Phe Pro Met Ile Lys Thr Phe Glu Asn Ala
   1               5                   10                  15
   Leu Thr Ala Ala Asp Phe Glu Ser Thr Val Glu Leu Thr Asn Phe Pro
               20                  25                  30
   Ala Val Phe Arg Gly Cys Ala Ser Val Trp Asp Ala Tyr Ser Lys Trp
           35                  40                  45
   Asn Pro Phe Asn Ser Gly Leu Asp Tyr Leu Glu Glu Arg Ala Gly Ser
       50                  55                  60
   Val Glu Val Glu Ala Met Leu Ser Arg Thr Ala Pro Val Phe Asn Gly
   65                  70                  75                  80
   Asp Ile Arg Ser His Glu Arg Val Ser Leu Pro Phe Ser Asp Phe Ile
                   85                  90                  95
   Arg Phe Cys Lys Gln His Met Arg Gly Lys Gly Asn Gly Ser Gly Val
               100                 105                 110
   Asp Ala Lys Ser Ala Asp Leu Asn Pro Met Cys Glu Asp Tyr Arg Pro
           115                 120                 125
   Gly Gln Ile Tyr Leu Ala Gln Phe Pro Ile Leu Asn Asp Glu Lys Glu
       130                 135                 140
   Glu Lys Val Leu Leu Lys Ile Leu Arg Gln Asp Ile Gln Thr Pro Thr
   145                 150                 155                 160
   Phe Leu Asp Ala Lys Ser Leu Ser Ser Ile Asn Phe Trp Met Asn Ser
                   165                 170                 175
   Ala Glu Ala Arg Ser Ser Thr His Tyr Asp Pro His His Asn Leu Leu
               180                 185                 190
   Cys Val Val Ser Gly Arg Lys Lys Val Val Leu Trp Pro Pro Ser Ala
           195                 200                 205
   Ser Pro Ser Leu Tyr Pro Met Pro Ile Tyr Gly Glu Ala Ser Asn His
       210                 215                 220
   Ser Ser Val Gly Leu Glu Asn Pro Asn Leu Ser Asp Tyr Pro Arg Ala
   225                 230                 235                 240
   Glu His Ser Leu Lys Gln Ser Gln Glu Ile Thr Leu Asn Ala Gly Asp
                   245                 250                 255
   Ala Val Phe Ile Pro Glu Gly Trp Phe His Gln Val Asp Ser Asp Glu
               260                 265                 270
   Leu Thr Val Ala Val Asn Phe Trp Trp Gln Ser Asn Tyr Met Ser Asn
           275                 280                 285
   Met Pro Glu His Met Asp Ser Tyr Tyr Leu Arg Arg Ile Thr Arg Arg
       290                 295                 300
   Leu Ile Asp Arg Glu Met Ser Leu Leu Val Ser Lys Pro Ser Ser Thr
   305                 310                 315                 320
   Asp Leu Arg His Leu Ser Glu His Ile Asp Gln Ser Arg Ile Glu Met
                   325                 330                 335
   Ala Glu Gly Gly Asn Asp Asn Ile Gly Asn Glu Ser Ile Lys Lys Gly
               340                 345                 350
   Leu Ser Thr Leu His Glu Lys Ala Ser Leu His Asp Leu Asp Pro Ser
           355                 360                 365
   Ala Ser Gln Ala Leu His Asp Leu Ile Ser Leu Val His Asp His Val
       370                 375                 380
   Asn Ala Val Asp Thr Lys Asp Asp Arg Val Ala His Leu Leu Trp Asn
   385                 390                 395                 400
   Leu Glu Ala Ser Arg Leu Arg Asp Val Leu Leu Ala Met Ala Arg Tyr
                   405                 410                 415
   Phe Pro Arg Thr Leu Glu Ala Leu Ile Leu His Met Leu Ser Pro Ile
               420                 425                 430
   Ala Ala Glu Val Leu Thr Gln Lys Phe Asp Glu Ile Asp Gln Gln Thr
           435                 440                 445
   Gly Glu Glu Asp Arg Thr Gln Phe Phe Arg Glu Phe Tyr Ser Ala Phe
       450                 455                 460
   Asp Asp Glu Ala Ala Ala Met Asp Ile Ile Leu Ser Arg Lys Glu Ala
   465                 470                 475                 480
   Phe Ala Phe Gln Ala Phe Lys Asn Val Leu Asp Lys Phe Leu Gly Val
                   485                 490                 495
   Asn Ile Ala Ser Pro Thr Thr Asn Ile
               500                 505
<210> 15
   <211> 1038
   <212> DNA
   <213> Arabidopsis thaliana
<400> 15
   atggctaaag agatagagaa tttatggaga gaagtgagag aattgagctt aggaactaaa      60
   atcgatcgtt ttgattctca accgtctccg gtgaagtttc tccgaaacta cgtgtctcag     120
   agcaagcctt gtgtaatctc caaagctatt actcattggc cggcgttaaa actctggtca     180
   gatccggctt acctcaccgg agctttgtca gatgacgttg tctcgctaca tctcactcct     240
   aacggctgcg cagacgccgt caccggagat agtgatcttt gtttcgcttc ggctcatgtt     300
   gagaaagttc tgttcccgga ggctcttaag gttgttcaat cgtcgtgtaa aggactgaag     360
   gttgggtatt tgcaacagca aaacgattgt tttagaacgg agtattcgac ggttgcgtta     420
   gactgcgacg gtgatattga gtgggctacg gaggcatttg gttgttcgcc ggaagctgtg     480
   aacctttgga ttggtactga tgactccgtg acttcatttc ataaagatca ctatgagaat     540
   ctttacgctg ttgtttcagg ggagaaacat ttccttcttc ttcctccgac tgatgttcac     600
   cgtctctata tcgaacagta tccggcagct aattactctt accaccgaga tactgatgcg     660
   ttcaagcttg aggttgagga gccggttagg catgtacctt ggtctagtgt tgatccatac     720
   ccttcgccag agaaggaagc aagcgagaga ttgaaatttc cgttgttctt tgatggcccg     780
   aagccgtttc attgtactgt caaggctggc gaggttcttt acctgccaag catgtggttt     840
   caccatgtta gtcaaacccc aggagatgga ggttatacca ttgcagtgaa ctattggtat     900
   gacatgcagt ttgacatcaa gtatgcttat tttaacttct tgcaatcttt attatacaag     960
   tcatcttcac tcaatccagt tctttcttgg agagaagacg aagattcaga atccagtgat    1020
   gcagagattg ctccctga                                                  1038

<210> 16
   <211> 431
   <212> PRT
   <213> Arabidopsis thaliana
<400> 16
   Met Ala Lys Glu Ile Glu Asn Leu Trp Arg Glu Val Arg Glu Leu Ser
   1               5                   10                  15
   Leu Gly Thr Lys Ile Asp Arg Phe Asp Ser Gln Pro Ser Pro Val Lys
               20                  25                  30
   Phe Leu Arg Asn Tyr Val Ser Gln Ser Lys Pro Cys Val Ile Ser Lys
           35                  40                  45
   Ala Ile Thr His Trp Pro Ala Leu Lys Leu Trp Ser Asp Pro Ala Tyr
       50                  55                  60
   Leu Thr Gly Ala Leu Ser Asp Asp Val Val Ser Leu His Leu Thr Pro
   65                  70                  75                  80
   Asn Gly Cys Ala Asp Ala Val Thr Gly Asp Ser Asp Leu Cys Phe Ala
                   85                  90                  95
   Ser Ala His Val Glu Lys Val Leu Phe Pro Glu Ala Leu Lys Val Val
               100                 105                 110
   Gln Ser Ser Cys Lys Gly Leu Lys Val Gly Tyr Leu Gln Gln Gln Asn
           115                 120                 125
   Asp Cys Phe Arg Thr Glu Tyr Ser Thr Val Ala Leu Asp Cys Asp Gly
       130                 135                 140
   Asp Ile Glu Trp Ala Thr Glu Ala Phe Gly Cys Ser Pro Glu Ala Val
   145                 150                 155                 160
   Asn Leu Trp Ile Gly Thr Asp Asp Ser Val Thr Ser Phe His Lys Asp
                   165                 170                 175
   His Tyr Glu Asn Leu Tyr Ala Val Val Ser Gly Glu Lys His Phe Leu
               180                 185                 190
   Leu Leu Pro Pro Thr Asp Val His Arg Leu Tyr Ile Glu Gln Tyr Pro
           195                 200                 205
   Ala Ala Asn Tyr Ser Tyr His Arg Asp Thr Asp Ala Phe Lys Leu Glu
       210                 215                 220
   Val Glu Glu Pro Val Arg His Val Pro Trp Ser Ser Val Asp Pro Tyr
   225                 230                 235                 240
   Pro Ser Pro Glu Lys Glu Ala Ser Glu Arg Leu Lys Phe Pro Leu Phe
                   245                 250                 255
   Phe Asp Gly Pro Lys Pro Phe His Cys Thr Val Lys Ala Gly Glu Val
               260                 265                 270
   Leu Tyr Leu Pro Ser Met Trp Phe His His Val Ser Gln Thr Pro Gly
           275                 280                 285
   Asp Gly Gly Tyr Thr Ile Ala Val Asn Tyr Trp Tyr Asp Met Gln Phe
       290                 295                 300
   Asp Ile Lys Tyr Ala Tyr Phe Asn Phe Leu Gln Ser Leu Leu Tyr Lys
   305                 310                 315                 320
   Ser Ser Ser Leu Asn Pro Val Leu Ser Trp Arg Glu Asp Glu Asp Ser
                   325                 330                 335
   Glu Ser Ser Asp Ala Glu Gly Ser Lys Phe Thr Pro Ser Ala Thr Asn
               340                 345                 350
   Leu Tyr Leu Ser Ser Thr Tyr Val Ser Asp Gly Ala Arg Phe Trp Lys
           355                 360                 365
   Arg Val His Val Asn Ser Ile Lys Leu Gly Phe Glu Ser Asn Gly Leu
       370                 375                 380
   Ile Gln Ser Gly Leu Ile Asp Met Tyr Arg Lys Tyr Arg Leu Val Ile
   385                 390                 395                 400
   Asn Ala Glu Lys Val Phe Arg Ala Met Glu Gly Gln Thr Val Leu Thr
                   405                 410                 415
   Val Gly Gly Tyr Gly Phe Glu Tyr Tyr Ala Glu Trp Phe Ser His
               420                 425                 430
<210> 17
   <211> 1142
   <212> DNA
   <213> Medicago truncatula
<400> 17
   atgaatgaga agatagaaga actatggaga gaagtacgag aattaagtct aggaagcaaa      60
   agaagcatag aacgtttaga atcagcacca acaccactcc aattccatag aaacttcatc     120
   actccaaaca aaccttgcat catctccaac tccatctccc attggccctc cctctctctc     180
   tggtcccacc cttcttacct cactcaatcc ctctcttcca ccaccgtctc cctccatctc     240
   acccccaccg gctccgccga ctctttaacc cctctcccct cttccccctc ctccctctgc     300
   ttcgcctctg cccatgtcca aaacctccct ttccccgaag ccctccgtct catcaactcc     360
   tctaaccctt cccaatgtgt cgcctacgcg cagcaacaga acgattgttt tcgttcagag     420
   tatgattcca ttgttaaaga ttgtgatcaa catattgctt gggctactga agcatttggt     480
   ttagagcctg aagctgttaa tctttggatt ggaaacaaac attcatctac ttggtttcat     540
   aaggatcatt atgagaatct ttatgctgtt gttactggtc aaaaacactt tcttttgttt     600
   cctcctactg atgttcatcg cttttatatt cggaattacc ctgctgctac ttataaatat     660
   tacatggaaa ctggagagtt tgatttggaa cttgacaagc caactaggta tgtgccttgg     720
   tgtagtgtga acccttttcc ttctccggag aacttggagg acgagatttc aaagtttcct     780
   ttatacttca atggccctcc accgtttgag tgtactgtca aggctgggga gattctttac     840
   ttgtatgatt ctcttctttt gcattgcatc atgccaagca tgtggtttca tcatgttaga     900
   caaagtgggg atgatggaga attaactatt gcagtaaatt attggtatga tatgcaattt     960
   gacattaagt atgcttattt caacttttta caatctattg attaccgatc tcctacgagt    1020
   ccaatgatgc ctgagaaatt gtgtgaggag atagattttg gtcctgatga cgatgagact     1080
   agatgaatta tcatttctca gtcggtgata ttcggcaatg tggtccttcg atcgaatgct    1140
   ga                                                                   1142
<210> 18
   <211> 361
   <212> PRT
   <213> Medicago truncatula
<400> 18
   Met Asn Glu Lys Ile Glu Glu Leu Trp Arg Glu Val Arg Glu Leu Ser
   1               5                   10                  15
   Leu Gly Ser Lys Arg Ser Ile Glu Arg Leu Glu Ser Ala Pro Thr Pro
               20                  25                  30
   Leu Gln Phe His Arg Asn Phe Ile Thr Pro Asn Lys Pro Cys Ile Ile
           35                  40                  45
   Ser Asn Ser Ile Ser His Trp Pro Ser Leu Ser Leu Trp Ser His Pro
       50                  55                  60
   Ser Tyr Leu Thr Gln Ser Leu Ser Ser Thr Thr Val Ser Leu His Leu
   65                  70                  75                  80
   Thr Pro Thr Gly Ser Ala Asp Ser Leu Thr Pro Leu Pro Ser Ser Pro
                   85                  90                  95
   Ser Ser Leu Cys Phe Ala Ser Ala His Val Gln Asn Leu Pro Phe Pro
               100                 105                 110
   Glu Ala Leu Arg Leu Ile Asn Ser Ser Asn Pro Ser Gln Cys Val Ala
           115                 120                 125
   Tyr Ala Gln Gln Gln Asn Asp Cys Phe Arg Ser Glu Tyr Asp Ser Ile
       130                 135                 140
   Val Lys Asp Cys Asp Gln His Ile Ala Trp Ala Thr Glu Ala Phe Gly
   145                 150                 155                 160
   Leu Glu Pro Glu Ala Val Asn Leu Trp Ile Gly Asn Lys His Ser Ser
                   165                 170                 175
   Thr Trp Phe His Lys Asp His Tyr Glu Asn Leu Tyr Ala Val Val Thr
               180                 185                 190
   Gly Gln Lys His Phe Leu Leu Phe Pro Pro Thr Asp Val His Arg Phe
           195                 200                 205
   Tyr Ile Arg Asn Tyr Pro Ala Ala Thr Tyr Lys Tyr Tyr Met Glu Thr
       210                 215                 220
   Gly Glu Phe Asp Leu Glu Leu Asp Lys Pro Thr Arg Tyr Val Pro Trp
   225                 230                 235                 240
   Cys Ser Val Asn Pro Phe Pro Ser Pro Glu Asn Leu Glu Asp Glu Ile
                   245                 250                 255
   Ser Lys Phe Pro Leu Tyr Phe Asn Gly Pro Pro Pro Phe Glu Cys Thr
               260                 265                 270
   Val Lys Ala Gly Glu Ile Leu Tyr Leu Tyr Asp Ser Leu Leu Leu His
           275                 280                 285
   Cys Ile Met Pro Ser Met Trp Phe His His Val Arg Gln Ser Gly Asp
       290                 295                 300
   Asp Gly Glu Leu Thr Ile Ala Val Asn Tyr Trp Tyr Asp Met Gln Phe
   305                 310                 315                 320
   Asp Ile Lys Tyr Ala Tyr Phe Asn Phe Leu Gln Ser Ile Asp Tyr Arg
                   325                 330                 335
   Ser Pro Thr Ser Pro Met Met Pro Glu Lys Leu Cys Glu Glu Ile Asp
               340                 345                 350
   Phe Gly Pro Asp Asp Asp Glu Thr Arg
           355                 360
<210> 19
   <211> 1071
   <212> DNA
   <213> Brachypodium sylvaticum
<400> 19
   tcaatcgttc ttctcctcaa gatcaccttc caaggcagca tctttgttgt ccagcggaga     60
   attactgatc tccagtgacc tcaggaagtt aaagtacgca tacttgatgt caaactgcat    120
   gtcataccag taattcacag caatggtgag cccgttaggc ccggggctct ggctgacatg    180
   gtgaaaccac atgctcggca agtagagcac ctcgccagca cggacggtgc accgtatcgg    240
   cctgggcccc tcgaagtaga gcgggaagga tgagacctgc gccgccatct cctccggcga    300
   cgacgggttc gggtccacgc tgctccacgg cacgatcctc tcgggctctt ccatctccag    360
   cttaagcccc gtcagctcct cctctccttc gttctccgtg acgtagcggg cggcggggta    420
   gtcgcggacg tagaggcggt ggtgctcggt ggggggcaag aggaggaaat gcttctcgcc    480
   ggagaggacg gcgtagacgt tgtcgtagtg gtccttgtgg aaggaggtga cggagcagga    540
   gttgccgatc cagaggttga cggcctccgg gaggcagccg agcgcctcgc tggcccaggg    600
   cacgtgcgcg tccacgtcgc cggccaccgc cgcgtactcc ccgcgcaggc agtcgtcctg    660
   ctgctgcgcg tacgccacca ggccgccagc ggccgcccgg tcggagcccc tgatgagccg    720
   cacggcggta gggaagtcga cccggcggac gtgcgcggac gcgaagcacc tggcgccggg    780
   gaggcacggg tgaggggcga gggcgtcggc gcggccgtcg ggggtgaggt ggacggagac    840
   gtcggtggag cggagcgcgt cggtgaggta ggattcggtg ggccagaggg aggcggcggg    900
   ccagtggcgg gtggcggcgg cggagacgag gagcgggcgg cccggggaga cgtggtcgcg    960
   gaggaaggcg agcggggtcg ggggcaggtc ggcgcgcggc acggcctccg gagagtggag   1020
   acccagcagg tcccgtgatt ccgcccacag ctcccgcacc gcgcgctcca t            1071
<210> 20
   <211> 356
   <212> PRT
   <213> Brachypodium sylvaticum
<400> 20
   Met Glu Arg Ala Val Arg Glu Leu Trp Ala Glu Ser Arg Asp Leu Leu
   1               5                   10                  15
   Gly Leu His Ser Pro Glu Ala Val Pro Arg Ala Asp Leu Pro Pro Thr
               20                  25                  30
   Pro Leu Ala Phe Leu Arg Asp His Val Ser Pro Gly Arg Pro Leu Leu
           35                  40                  45
   Val Ser Ala Ala Ala Thr Arg His Trp Pro Ala Ala Ser Leu Trp Pro
       50                  55                  60
   Thr Glu Ser Tyr Leu Thr Asp Ala Leu Arg Ser Thr Asp Val Ser Val
   65                  70                  75                  80
   His Leu Thr Pro Asp Gly Arg Ala Asp Ala Leu Ala Pro His Pro Cys
                   85                  90                  95
   Leu Pro Gly Ala Arg Cys Phe Ala Ser Ala His Val Arg Arg Val Asp
               100                 105                 110
   Phe Pro Thr Ala Val Arg Leu Ile Arg Gly Ser Asp Arg Ala Ala Ala
           115                 120                 125
   Gly Gly Leu Val Ala Tyr Ala Gln Gln Gln Asp Asp Cys Leu Arg Gly
       130                 135                 140
   Glu Tyr Ala Ala Val Ala Gly Asp Val Asp Ala His Val Pro Trp Ala
   145                 150                 155                 160
   Ser Glu Ala Leu Gly Cys Leu Pro Glu Ala Val Asn Leu Trp Ile Gly
                   165                 170                 175
   Asn Ser Cys Ser Val Thr Ser Phe His Lys Asp His Tyr Asp Asn Val
               180                 185                 190
   Tyr Ala Val Leu Ser Gly Glu Lys His Phe Leu Leu Leu Pro Pro Thr
           195                 200                 205
   Glu His His Arg Leu Tyr Val Arg Asp Tyr Pro Ala Ala Arg Tyr Val
       210                 215                 220
   Thr Glu Asn Glu Gly Glu Glu Glu Leu Thr Gly Leu Lys Leu Glu Met
   225                 230                 235                 240
   Glu Glu Pro Glu Arg Ile Val Pro Trp Ser Ser Val Asp Pro Asn Pro
                   245                 250                 255
   Ser Ser Pro Glu Glu Met Ala Ala Gln Val Ser Ser Phe Pro Leu Tyr
               260                 265                 270
   Phe Glu Gly Pro Arg Pro Ile Arg Cys Thr Val Arg Ala Gly Glu Val
           275                 280                 285
   Leu Tyr Leu Pro Ser Met Trp Phe His His Val Ser Gln Ser Pro Gly
       290                 295                 300
   Pro Asn Gly Leu Thr Ile Ala Val Asn Tyr Trp Tyr Asp Met Gln Phe
   305                 310                 315                 320
   Asp Ile Lys Tyr Ala Tyr Phe Asn Phe Leu Arg Ser Leu Glu Ile Ser
                   325                 330                 335
   Asn Ser Pro Leu Asp Asn Lys Asp Ala Ala Leu Glu Gly Asp Leu Glu
               340                 345                 350
   Glu Lys Asn Asp
           355
<210> 21
   <211> 1080
   <212> DNA
   <213> Oryza sativa
<400> 21
   atggagcgcg cagtgcggga gctgtgggcg gagtcgcggg acctgctggg cctccactcc      60
   ccggacgacg ccgccgccgc cgacgccgcg atgccccgcg ccgagatgcc cccgacgccg     120
   ctcgcgtttc tccgcgacca cgtctcgccg gggcgccctc tcctcgtgtc ctccgccgcc      180
   accagccact ggccggccgc ctcgctgtgg ccgaccgact cctacctcac cgacgcgctc     240
   cgctccaccg ccgtctcgct ccacctcacc cccgacggcc gcgccgacgc cctcgccccg     300
   cacccgcgcc cgagccaccc cggcgccaag tgcttcgcct ccgcgcacgt ccgccaggtc     360
   gacttcccca ccgccgtgcg cctcatccgg agctccgatc cggcctccgg cctggtggcc     420
   tacgcgcagc agcaggacga ctgcctgcgc ggggagtacg ccgccgtcgc cggcgacgtg     480
   gacgcgcacg tgccctgggc cagcgacgcg ctcggctgcc tccccgaggc cgtcaacctc     540
   tggatcggca gcgcctgctc ccagacctcc ttccacaagg accactacga caacatctac     600
   gtcgtcgtct ccggcgagaa gcacttcctc ctcttgcccc ccaccgagca ccaccgcctc     660
   tacgtccgcg actaccccgc cgcccactac gccgcggaag atgaagcgga gctgaggctt     720
   aagctggagc tggaggagcc cgagaggatc gtgccatgga gcagcgttga cccctacccg     780
   ccgtcgccgg aggaggcggc cgcgcaggcg tcatccttcc cgctctactt cgaggggccg    840
   aggccgatcc gctgcacggt gcgcgccggc gagatgctct acttgccgag catgtggttt     900
   caccatgtga gccagagccc cgggccgaac gggctcacca ttgcagtgaa ctactggtat     960
   gacatgcagt ttgacattaa gtatgcctac ttcaacttct tgaggtcatt ggagatcgat    1020
   ggtagttcgt cgaaaaagac ggatgctttg gaagacgatc tcgaggagac gaatgattga    1080
<210> 22
   <211> 354
   <212> PRT
   <213> Oryza sativa
<400> 22
   Met Glu Arg Ala Val Arg Glu Leu Trp Ala Glu Ser Arg Asp Leu Leu
   1               5                   10                  15
   Gly Leu His Ser Pro Asp Asp Ala Ala Ala Ala Asp Ala Ala Met Pro
               20                  25                  30
   Arg Ala Glu Met Pro Pro Thr Pro Leu Ala Phe Leu Arg Asp His Val
           35                  40                  45
   Ser Pro Gly Arg Pro Leu Leu Val Ser Ser Ala Ala Thr Ser His Trp
       50                  55                  60
   Pro Ala Ala Ser Leu Trp Pro Thr Asp Ser Tyr Leu Thr Asp Ala Leu
   65                  70                  75                  80
   Arg Ser Thr Ala Val Ser Leu His Leu Thr Pro Asp Gly Arg Ala Asp
                   85                  90                  95
   Ala Leu Ala Pro His Pro Arg Pro Ser His Pro Gly Ala Lys Cys Phe
               100                 105                 110
   Ala Ser Ala His Val Arg Gln Val Asp Phe Pro Thr Ala Val Arg Leu
           115                 120                 125
   Ile Arg Ser Ser Asp Pro Ala Ser Gly Leu Val Ala Tyr Ala Gln Gln
       130                 135                 140
   Gln Asp Asp Cys Leu Arg Gly Glu Tyr Ala Ala Val Ala Gly Asp Val
   145                 150                 155                 160
   Asp Ala His Val Pro Trp Ala Ser Asp Ala Leu Gly Cys Leu Pro Glu
                   165                 170                 175
   Ala Val Asn Leu Trp Ile Gly Ser Ala Cys Ser Gln Thr Ser Phe His
               180                 185                 190
   Lys Asp His Tyr Asp Asn Ile Tyr Val Val Val Ser Gly Glu Lys His
           195                 200                 205
   Phe Leu Leu Leu Pro Pro Thr Glu His His Arg Leu Tyr Val Arg Asp
       210                 215                 220
   Tyr Pro Ala Ala His Tyr Ala Ala Glu Asp Glu Ala Glu Leu Arg Leu
   225                 230                 235                 240
   Lys Leu Glu Leu Glu Glu Pro Glu Arg Ile Val Pro Trp Ser Ser Val
                   245                 250                 255
   Asp Pro Tyr Pro Pro Ser Pro Glu Glu Ala Ala Ala Gln Ala Ser Ser
               260                 265                 270
   Phe Pro Leu Tyr Phe Glu Gly Pro Arg Pro Ile Arg Cys Thr Val Arg
           275                 280                 285
   Ala Gly Glu Met Leu Tyr Leu Pro Ser Met Trp Phe His His Val Ser
       290                 295                 300
   Gln Ser Pro Gly Pro Asn Gly Leu Thr Ile Ala Val Asn Tyr Trp Tyr
   305                 310                 315                 320
   Asp Met Gln Phe Asp Ile Lys Tyr Ala Tyr Phe Asn Phe Leu Arg Ser
                   325                 330                 335
   Leu Glu Ile Asp Gly Ser Ser Ser Lys Lys Thr Asp Ala Leu Glu Asp
               340                 345                 350
   Asp Leu
<210> 23
   <211> 3552
   <212> DNA
   <213> Arabidopsis thaliana
<400> 23
   atggggacag agctaatgag aatctgtgtg aaagaagata gtgatgattt gccatctgtt      60
   cctcctggtt ttgaatcata tgcaactttt accttaaaaa gagtagtccc tgctactact     120
   tctgataaag ccaagactcc tgcaattgaa agtgtttctg caaccgaaca agctaagatg     180
   gaagttgaat ctgatgaagc caaagctgct cgggctctac gccgtagacc ttggattaat     240
   catagcggat gtgatgatga tggtgattgt gctgcaaaca atgacaatgc tgcatctcaa     300
   aatcctgatc aaaattgtga tgtgaagcca gctcttccaa agggtgtggt caggggatgt     360
   gaagaatgta aagattgtca gaaggagttt gaagacactt taaattatat cgctaaaata     420
   agaccggagg ctgaaaagta tggaatctgt cgcattgttc ctcctccttc ctggaaaccg     480
   ccgtgcccac tgaaagaaaa gcaagtatgg gagggttcta aatttacaac acgtgtccaa     540
   agagttgata aacttcagaa tcggagttca atgaagaaga tttcaaagct tcctaatcaa     600
   atgagaaaga agaaaagaaa gtgcatgaaa atgggaatgg attctgtcac taatggtatg     660
   ggcgatccct gttctgcaag cactggaatg aatgaacttg agacgtttgg gtttgaacct     720
   ggtccagggt tcacactaaa agacttccaa aaatatgctg atgagttcaa ggctcagtac     780
   tttaaaaaga gtgaaacttc tacagacgat aaatgtaaag ttgataattc aatagactgc     840
   tgggagccag cacttgagga tgttgaaggt gaatattggc ggatagtaga taaagcaact     900
   gaagagatag aggtgctata tggtgctgac cttgaaactg gggtatttgg tagcggattc       960
   cccaagatat catcaagtca caatgcttct tcttcagaag ataaatatgc aaaatcaggc    1020
   tggaacttaa ataactttcc aaggcttccg ggatccctcc ttaagtacga gggcagtgat    1080
   atttccggtg tccttgtgcc gtggctgtat attgggatgt gcttttcttc tttctgctgg    1140
   catgttgaag atcaccactt gtattcgttg aattatatgc actggggtgc accgaaattg    1200
   tggtatggtg ttggggggaa ggatgctgtt aaactcgagg aggcaatgag gaagcatttg     1260
   cctgaccttt ttgaagaaca gcctgactta cttcataagc tagttacaca actctccccg   1320
   tcaaaactga aaaccgcagg agtacctgtg caccgttgcg tccagcatgc tggagagttt   1380
   gtcttgactt ttcctcgggc atatcatgct ggatttaatt ctggtttcaa ctgtgctgaa   1440
   gctgtgaatg tagcacccgt tgactggtta cctcatgggc agattgccat agagttatac   1500
   tgtcaacagg gtagaaaaac gtccatctcg catgataaat tgttgcttgg ggcagcaagg   1560
   gaagtagtga aagccgactg ggagctgaac ttactaagga aaaatactgt agataactta   1620
   aggtggaaag cattcagtgc gaaggatgga attttggcaa aaacattaaa ggcacgcatt   1680
   gacatggaac gtacgagaag agaatttctg tgcaactctt cacttgcatt gaaaatgcac   1740
   agtaatttcg atgctaccaa cgagagagag tgctgtatat gcttctttga tttgcacctg   1800
   tctgctgctg gctgtcgttg ttccccggag aagtattcat gtttgactca tgtgaaggag   1860
   ttgtgttcat gtccatgggt tactaaatat tttctatttc gctatgatat cgatgaactg   1920
   aatgttcttg ttgaggcagt ggaaggaaaa ctgagttctg tatatagatg ggcgcgtcaa   1980
   gatttaggat tggcattaag tacagacgtc tcaggaagca agatggagat agatgaagaa   2040
   ggaaaagtcc acaaggaccc gactccacaa acaactgcac tttcagggaa ggatttgcag   2100
   ttgaaagtaa catcgaaaga agtaagtaaa gagttagaaa agactagtaa attatctcat   2160
   gttaatttac ttctgaaaga aaaggaggag caaataacgt caagtcactg tatgaagcct   2220
   gtcaaagaag aaactgtttg tgattcttct gatccaaacg tttcagcttg ccaaccatct   2280
   gaaggaggca taatctgtat gacagctgtt aagtccgcaa gtggtaaaaa gaattcacag   2340
   agtctaccca atgatgtgat actcctcagc gatgatgagt atgacatacc taggaaacga   2400
   ggttctgtga gaagagatgc aatttcttct ggcaagaaat tagaaatacg agagagaccg   2460
   acccacgttt tggcgttaga agcttctgcc aaaattgctg ctccaatttg ccaaagggaa   2520
   ggagattcgc tgcgtgatac acgaaacact atatcattgc ctactaatga ccaaaaaaca   2580
   atgagaaggg atgtaccgag ttctacttcg cacgcggaag ttaatgcgga ggctactggg   2640
   cttactcaag atatatgtaa cagaatggca actaacagcc acggtggtgg aaaacctact   2700
   agttgtaaat ccaaaaactc tggaggtttg gctatagtgg atgttgtgga tgggacaaga   2760
   agtagttcag gtacaccgtc ttgttcgcaa aataatagtc cggataggtt catccgccaa   2820
   aagggtcccc gcattgcaaa ggtcgtgagg agaatcaatt gcaatgtaga gcctttaagt   2880
   tatggatgtg tgctgtctgg aaaatcatgg tgcagccggc gagcaatttt tcctaaagga   2940
   tttcgtagcc gggttaagta cataaacatt ttggatccaa caaatatgtg cttctacatt   3000
   tcagaaattc tggatgctgg acgcaacagt ccattgttca tggtttactt ggagagtaat   3060
   cccagtgagg tgttcgttca tatgtcgcct acaagatgct gggagatggt aagagagaga   3120
   gtaaatcaag agataactaa gcagcacaaa gctggaaaat cagatcttcc tcctttgcaa   3180
   ccctctggga gtcccgacgg ttttgaaatg tttggatatt cgtcacccgc aatcgtacag   3240
   gctatcgaag cattagacgt gaatcgagtt tgcacagact attgggattc caggccttat   3300
   tcccggccac aagttcagtt ccctgcaaat cctcttctca gagaagccaa cacaagtggc   3360
   agatcgaatg tgggaaatct ccagctaaac cctggacacc atatatcgcc tactggaata   3420
   aattctattc ttaaagttct gttcaagaaa gctagcatgg aggaactaag ctcacttcaa   3480
   gaggttttaa gtgagactaa ctcagatatg gtgaccgaac ttgtgaagga agagatccag   3540
   aaccgtcgct ga                                                       3552
<210> 24
   <211> 1183
   <212> PRT
   <213> Arabidopsis thaliana
<400> 24
   Met Gly Thr Glu Leu Met Arg Ile Cys Val Lys Glu Asp Ser Asp Asp
   1               5                   10                  15
   Leu Pro Ser Val Pro Pro Gly Phe Glu Ser Tyr Ala Thr Phe Thr Leu
               20                  25                  30
   Lys Arg Val Val Pro Ala Thr Thr Ser Asp Lys Ala Lys Thr Pro Ala
           35                  40                  45
   Ile Glu Ser Val Ser Ala Thr Glu Gln Ala Lys Met Glu Val Glu Ser
       50                  55                  60
   Asp Glu Ala Lys Ala Ala Arg Ala Leu Arg Arg Arg Pro Trp Ile Asn
   65                  70                  75                  80
   His Ser Gly Cys Asp Asp Asp Gly Asp Cys Ala Ala Asn Asn Asp Asn
                   85                  90                  95
   Ala Ala Ser Gln Asn Pro Asp Gln Asn Cys Asp Val Lys Pro Ala Leu
               100                 105                 110
   Pro Lys Gly Val Val Arg Gly Cys Glu Glu Cys Lys Asp Cys Gln Lys
           115                 120                 125
   Glu Phe Glu Asp Thr Leu Asn Tyr Ile Ala Lys Ile Arg Pro Glu Ala
       130                 135                 140
   Glu Lys Tyr Gly Ile Cys Arg Ile Val Pro Pro Pro Ser Trp Lys Pro
   145                 150                 155                 160
   Pro Cys Pro Leu Lys Glu Lys Gln Val Trp Glu Gly Ser Lys Phe Thr
                   165                 170                 175
   Thr Arg Val Gln Arg Val Asp Lys Leu Gln Asn Arg Ser Ser Met Lys
               180                 185                 190
   Lys Ile Ser Lys Leu Pro Asn Gln Met Arg Lys Lys Lys Arg Lys Cys
           195                 200                 205
   Met Lys Met Gly Met Asp Ser Val Thr Asn Gly Met Gly Asp Pro Cys
       210                 215                 220
   Ser Ala Ser Thr Gly Met Asn Glu Leu Glu Thr Phe Gly Phe Glu Pro
   225                 230                 235                 240
   Gly Pro Gly Phe Thr Leu Lys Asp Phe Gln Lys Tyr Ala Asp Glu Phe
                   245                 250                 255
   Lys Ala Gln Tyr Phe Lys Lys Ser Glu Thr Ser Thr Asp Asp Lys Cys
               260                 265                 270
   Lys Val Asp Asn Ser Ile Asp Cys Trp Glu Pro Ala Leu Glu Asp Val
           275                 280                 285
   Glu Gly Glu Tyr Trp Arg Ile Val Asp Lys Ala Thr Glu Glu Ile Glu
       290                 295                 300
   Val Leu Tyr Gly Ala Asp Leu Glu Thr Gly Val Phe Gly Ser Gly Phe
   305                 310                 315                 320
   Pro Lys Ile Ser Ser Ser His Asn Ala Ser Ser Ser Glu Asp Lys Tyr
                   325                 330                 335
   Ala Lys Ser Gly Trp Asn Leu Asn Asn Phe Pro Arg Leu Pro Gly Ser
               340                 345                 350
   Leu Leu Lys Tyr Glu Gly Ser Asp Ile Ser Gly Val Leu Val Pro Trp
           355                 360                 365
   Leu Tyr Ile Gly Met Cys Phe Ser Ser Phe Cys Trp His Val Glu Asp
       370                 375                 380
   His His Leu Tyr Ser Leu Asn Tyr Met His Trp Gly Ala Pro Lys Leu
   385                 390                 395                 400
   Trp Tyr Gly Val Gly Gly Lys Asp Ala Val Lys Leu Glu Glu Ala Met
                   405                 410                 415
   Arg Lys His Leu Pro Asp Leu Phe Glu Glu Gln Pro Asp Leu Leu His
               420                 425                 430
   Lys Leu Val Thr Gln Leu Ser Pro Ser Lys Leu Lys Thr Ala Gly Val
           435                 440                 445
   Pro Val His Arg Cys Val Gln His Ala Gly Glu Phe Val Leu Thr Phe
       450                 455                 460
   Pro Arg Ala Tyr His Ala Gly Phe Asn Ser Gly Phe Asn Cys Ala Glu
   465                 470                 475                 480
   Ala Val Asn Val Ala Pro Val Asp Trp Leu Pro His Gly Gln Ile Ala
                   485                 490                 495
   Ile Glu Leu Tyr Cys Gln Gln Gly Arg Lys Thr Ser Ile Ser His Asp
               500                 505                 510
   Lys Leu Leu Leu Gly Ala Ala Arg Glu Val Val Lys Ala Asp Trp Glu
           515                 520                 525
   Leu Asn Leu Leu Arg Lys Asn Thr Val Asp Asn Leu Arg Trp Lys Ala
       530                 535                 540
   Phe Ser Ala Lys Asp Gly Ile Leu Ala Lys Thr Leu Lys Ala Arg Ile
   545                 550                 555                 560
   Asp Met Glu Arg Thr Arg Arg Glu Phe Leu Cys Asn Ser Ser Leu Ala
                   565                 570                 575
   Leu Lys Met His Ser Asn Phe Asp Ala Thr Asn Glu Arg Glu Cys Cys
               580                 585                 590
   Ile Cys Phe Phe Asp Leu His Leu Ser Ala Ala Gly Cys Arg Cys Ser
           595                 600                 605
   Pro Glu Lys Tyr Ser Cys Leu Thr His Val Lys Glu Leu Cys Ser Cys
       610                 615                 620
   Pro Trp Val Thr Lys Tyr Phe Leu Phe Arg Tyr Asp Ile Asp Glu Leu
   625                 630                 635                 640
   Asn Val Leu Val Glu Ala Val Glu Gly Lys Leu Ser Ser Val Tyr Arg
                   645                 650                 655
   Trp Ala Arg Gln Asp Leu Gly Leu Ala Leu Ser Thr Asp Val Ser Gly
               660                 665                 670
   Ser Lys Met Glu Ile Asp Glu Glu Gly Lys Val His Lys Asp Pro Thr
           675                 680                 685
   Pro Gln Thr Thr Ala Leu Ser Gly Lys Asp Leu Gln Leu Lys Val Thr
       690                 695                 700
   Ser Lys Glu Val Ser Lys Glu Leu Glu Lys Thr Ser Lys Leu Ser His
   705                 710                 715                 720
   Val Asn Leu Leu Leu Lys Glu Lys Glu Glu Gln Ile Thr Ser Ser His
                   725                 730                 735
   Cys Met Lys Pro Val Lys Glu Glu Thr Val Cys Asp Ser Ser Asp Pro
               740                 745                 750
   Asn Val Ser Ala Cys Gln Pro Ser Glu Gly Gly Ile Ile Cys Met Thr
           755                 760                 765
   Ala Val Lys Ser Ala Ser Gly Lys Lys Asn Ser Gln Ser Leu Pro Asn
       770                 775                 780
   Asp Val Ile Leu Leu Ser Asp Asp Glu Tyr Asp Ile Pro Arg Lys Arg
   785                 790                 795                 800
   Gly Ser Val Arg Arg Asp Ala Ile Ser Ser Gly Lys Lys Leu Glu Ile
                   805                 810                 815
   Arg Glu Arg Pro Thr His Val Leu Ala Leu Glu Ala Ser Ala Lys Ile
               820                 825                 830
   Ala Ala Pro Ile Cys Gln Arg Glu Gly Asp Ser Leu Arg Asp Thr Arg
           835                 840                 845
   Asn Thr Ile Ser Leu Pro Thr Asn Asp Gln Lys Thr Met Arg Arg Asp
       850                 855                 860
   Val Pro Ser Ser Thr Ser His Ala Glu Val Asn Ala Glu Ala Thr Gly
   865                 870                 875                 880
   Leu Thr Gln Asp Ile Cys Asn Arg Met Ala Thr Asn Ser His Gly Gly
                   885                 890                 895
   Gly Lys Pro Thr Ser Cys Lys Ser Lys Asn Ser Gly Gly Leu Ala Ile
               900                 905                 910
   Val Asp Val Val Asp Gly Thr Arg Ser Ser Ser Gly Thr Pro Ser Cys
           915                 920                 925
   Ser Gln Asn Asn Ser Pro Asp Arg Phe Ile Arg Gln Lys Gly Pro Arg
       930                 935                 940
   Ile Ala Lys Val Val Arg Arg Ile Asn Cys Asn Val Glu Pro Leu Ser
   945                 950                 955                 960
   Tyr Gly Cys Val Leu Ser Gly Lys Ser Trp Cys Ser Arg Arg Ala Ile
                   965                 970                 975
   Phe Pro Lys Gly Phe Arg Ser Arg Val Lys Tyr Ile Asn Ile Leu Asp
               980                 985                 990
   Pro Thr Asn Met Cys Phe Tyr Ile  Ser Glu Ile Leu Asp  Ala Gly Arg
           995                 1000                1005
   Asn Ser  Pro Leu Phe Met Val  Tyr Leu Glu Ser Asn  Pro Ser Glu
       1010                1015                1020
   Val Phe  Val His Met Ser Pro  Thr Arg Cys Trp Glu  Met Val Arg
       1025                1030                1035
   Glu Arg  Val Asn Gln Glu Ile  Thr Lys Gln His Lys  Ala Gly Lys
       1040                1045                1050
   Ser Asp  Leu Pro Pro Leu Gln  Pro Ser Gly Ser Pro  Asp Gly Phe
       1055                1060                1065
   Glu Met  Phe Gly Tyr Ser Ser  Pro Ala Ile Val Gln  Ala Ile Glu
       1070                1075                1080
   Ala Leu  Asp Val Asn Arg Val  Cys Thr Asp Tyr Trp  Asp Ser Arg
       1085                1090                1095
   Pro Tyr  Ser Arg Pro Gln Val  Gln Phe Pro Ala Asn  Pro Leu Leu
       1100                1105                1110
   Arg Glu  Ala Asn Thr Ser Gly  Arg Ser Asn Val Gly  Asn Leu Gln
       1115                1120                1125
   Leu Asn  Pro Gly His His Ile  Ser Pro Thr Gly Ile  Asn Ser Ile
       1130                1135                1140
   Leu Lys  Val Leu Phe Lys Lys  Ala Ser Met Glu Glu  Leu Ser Ser
       1145                1150                1155
   Leu Gln  Glu Val Leu Ser Glu  Thr Asn Ser Asp Met  Val Thr Glu
       1160                1165                1170
   Leu Val  Lys Glu Glu Ile Gln  Asn Arg Arg
       1175                1180
<210> 25
   <211> 2793
   <212> DNA
   <213> Arabidopsis thaliana
<400> 25
   atgaatgcta atgagcaaac tcgatccgcc aatggcattg gcaatggcaa tggtgagtct     60
   attcccggga ttccagatga cttacggtgc aagagatcgg atggtaaaca gtggagatgc    120
   actgcaatgt ccatggctga taagactgtt tgtgagaagc actacatcca agcaaagaag    180
   cgggcggcta attctgcttt cagggcgaac cagaagaaag cgaaaaggcg atcatcgtta    240
   ggcgaaacag atacgtattc ggaagggaag atggatgatt tcgagttacc agtcaccagc    300
   attgaccact ataataacgg tcttgcctct gcttccaaga gtaatggtag actagagaag    360
   agacataata aaagcctgat gcggtactcg cccgagacac cgatgatgag gagtttctct    420
   ccacgtgttg cagtggattt gaatgatgac ttgggtagag atgttgtaat gtttgaagag    480
   ggctacagat cttataggac accaccatct gttgctgtta tggatccgac acgaaacaga    540
   tcacaccaaa gcaccagtcc tatggaatac tcagcagcaa gcacagatgt gtctgcagag    600
   tctttggggg aaatctgcca tcaatgccag agaaaagata gagagagaat catttcttgc    660
   ctcaaatgca atcaaagagc cttctgccac aattgtctat cggcaaggta ctcggagata    720
   tcacttgaag aagtcgagaa agtttgccct gcatgtcgtg gcttgtgtga ttgcaaatct    780
   tgcctgcgtt cagataatac aataaaggtt cggatccggg aaatacccgt tttggacaag    840
   ttgcagtatc tttatcgtct attatcagct gtcctaccag tcataaagca gatccatctt    900
   gaacaatgta tggaagttga actagagaag aggcttcgtg aagttgagat tgatcttgtc    960
   agggcaagat tgaaagcaga tgagcagatg tgctgcaacg tgtgtcggat accagttgtt   1020
   gactactacc gtcactgtcc gaactgctca tatgaccttt gcctgagatg ctgtcaagat   1080
   ctacgggaag agtcttcagt gacgattagt gggactaacc aaaacgtaca agatagaaaa   1140
   ggagctccca aactaaaact aaacttttca tacaagtttc ctgagtggga agccaacggt   1200
   gatgggagca tcccttgccc tcctaaggag tatggaggct gcggttcaca ttctttgaat   1260
   cttgcccgca ttttcaagat gaattgggtt gcaaagcttg tgaaaaatgc tgaggagatt   1320
   gttagtggct gcaaattatc tgatcttctg aaccctgata tgtgtgattc aagattctgc   1380
   aaatttgctg agagagaaga gagcggtgac aactacgtgt acagcccgtc gcttgaaacg   1440
   attaaaactg atggagtagc taagtttgag caacaatggg cagagggtcg gcttgttact   1500
   gtgaaaatgg tacttgatga ctcatcttgc tctagatggg atcctgagac tatttggagg   1560
   gatatagacg agctttcgga cgagaaactg agagaacatg atccattctt gaaggccatt   1620
   aattgcttgg atggtttaga ggttgatgta agacttgggg agtttacaag agcatataaa   1680
   gatggaaaga accaagagac aggtcttccg ctattgtgga agttaaagga ctggccgagc   1740
   ccaagtgctt ccgaggagtt cattttctac caaagacctg agtttatcag aagttttccg   1800
   tttctcgagt acattcatcc ccggttaggc cttctgaatg ttgcagccaa gttacctcat   1860
   tactcgctcc aaaacgattc aggtccaaag atttatgtgt cttgtgggac gtaccaagaa   1920
   atcagtgctg gcgattcatt gactggtatt cactacaaca tgcgtgacat ggtataccta   1980
   ttggtgcaca cgtctgaaga aacaacattc gaaagggtga gaaaaacaaa acctgttcca   2040
   gaggaacctg accagaagat gagcgaaaat gagtcacttc ttagccctga gcagaaatta   2100
   agggacggag agttacatga tctatcactt ggtgaagcca gtatggagaa gaatgaacct   2160
   gagttggcgt tgactgtgaa tccagagaac ttaacggaaa acggtgacaa catggaatct   2220
   tcttgcacat cttcatgtgc aggaggagcc cagtgggatg tctttcgacg ccaagacgtc   2280
   ccaaagttgt ccgggtattt gcagagaaca ttccagaagc ctgataatat ccagactgat   2340
   tttgtgtcac gcccgttgta tgaaggattg ttcttaaatg aacaccacaa gagacaacta   2400
   agagacgagt ttggagttga gccatggaca tttgagcaac atcgtggtga ggctatcttc   2460
   attccggctg gatgtccgtt ccaaatcact aatcttcagt cgaatattca ggtggcactt   2520
   gacttcttgt gccctgaaag cgttggagag tcagcaagac tagctgaaga aatccggtgt   2580
   ttaccaaacg accacgaggc aaaacttcag attctagaga ttggaaagat atcattatac   2640
   gcagctagct cagccattaa agaggttcag aaactggtct tggatccaaa gtttggagca   2700
   gagcttggat ttgaagactc taacttaacc aaagcagtct ctcacaactt agacgaggca   2760
   accaagcggc cgcagcaaaa cagctgcact taa                                2793
<210> 26
   <211> 930
   <212> PRT
   <213> Arabidopsis thaliana
<400> 26
   Met Asn Ala Asn Glu Gln Thr Arg Ser Ala Asn Gly Ile Gly Asn Gly
   1               5                   10                  15
   Asn Gly Glu Ser Ile Pro Gly Ile Pro Asp Asp Leu Arg Cys Lys Arg
               20                  25                  30
   Ser Asp Gly Lys Gln Trp Arg Cys Thr Ala Met Ser Met Ala Asp Lys
           35                  40                  45
   Thr Val Cys Glu Lys His Tyr Ile Gln Ala Lys Lys Arg Ala Ala Asn
       50                  55                  60
   Ser Ala Phe Arg Ala Asn Gln Lys Lys Ala Lys Arg Arg Ser Ser Leu
   65                  70                  75                  80
   Gly Glu Thr Asp Thr Tyr Ser Glu Gly Lys Met Asp Asp Phe Glu Leu
                   85                  90                  95
   Pro Val Thr Ser Ile Asp His Tyr Asn Asn Gly Leu Ala Ser Ala Ser
               100                 105                 110
   Lys Ser Asn Gly Arg Leu Glu Lys Arg His Asn Lys Ser Leu Met Arg
           115                 120                 125
   Tyr Ser Pro Glu Thr Pro Met Met Arg Ser Phe Ser Pro Arg Val Ala
       130                 135                 140
   Val Asp Leu Asn Asp Asp Leu Gly Arg Asp Val Val Met Phe Glu Glu
   145                 150                 155                 160
   Gly Tyr Arg Ser Tyr Arg Thr Pro Pro Ser Val Ala Val Met Asp Pro
                   165                 170                 175
   Thr Arg Asn Arg Ser His Gln Ser Thr Ser Pro Met Glu Tyr Ser Ala
               180                 185                 190
   Ala Ser Thr Asp Val Ser Ala Glu Ser Leu Gly Glu Ile Cys His Gln
           195                 200                 205
   Cys Gln Arg Lys Asp Arg Glu Arg Ile Ile Ser Cys Leu Lys Cys Asn
       210                 215                 220
   Gln Arg Ala Phe Cys His Asn Cys Leu Ser Ala Arg Tyr Ser Glu Ile
   225                 230                 235                 240
   Ser Leu Glu Glu Val Glu Lys Val Cys Pro Ala Cys Arg Gly Leu Cys
                   245                 250                 255
   Asp Cys Lys Ser Cys Leu Arg Ser Asp Asn Thr Ile Lys Val Arg Ile
               260                 265                 270
   Arg Glu Ile Pro Val Leu Asp Lys Leu Gln Tyr Leu Tyr Arg Leu Leu
           275                 280                 285
   Ser Ala Val Leu Pro Val Ile Lys Gln Ile His Leu Glu Gln Cys Met
       290                 295                 300
   Glu Val Glu Leu Glu Lys Arg Leu Arg Glu Val Glu Ile Asp Leu Val
   305                 310                 315                 320
   Arg Ala Arg Leu Lys Ala Asp Glu Gln Met Cys Cys Asn Val Cys Arg
                   325                 330                 335
   Ile Pro Val Val Asp Tyr Tyr Arg His Cys Pro Asn Cys Ser Tyr Asp
               340                 345                 350
   Leu Cys Leu Arg Cys Cys Gln Asp Leu Arg Glu Glu Ser Ser Val Thr
           355                 360                 365
   Ile Ser Gly Thr Asn Gln Asn Val Gln Asp Arg Lys Gly Ala Pro Lys
       370                 375                 380
   Leu Lys Leu Asn Phe Ser Tyr Lys Phe Pro Glu Trp Glu Ala Asn Gly
   385                 390                 395                 400
   Asp Gly Ser Ile Pro Cys Pro Pro Lys Glu Tyr Gly Gly Cys Gly Ser
                   405                 410                 415
   His Ser Leu Asn Leu Ala Arg Ile Phe Lys Met Asn Trp Val Ala Lys
               420                 425                 430
   Leu Val Lys Asn Ala Glu Glu Ile Val Ser Gly Cys Lys Leu Ser Asp
           435                 440                 445
   Leu Leu Asn Pro Asp Met Cys Asp Ser Arg Phe Cys Lys Phe Ala Glu
       450                 455                 460
   Arg Glu Glu Ser Gly Asp Asn Tyr Val Tyr Ser Pro Ser Leu Glu Thr
   465                 470                 475                 480
   Ile Lys Thr Asp Gly Val Ala Lys Phe Glu Gln Gln Trp Ala Glu Gly
                   485                 490                 495
   Arg Leu Val Thr Val Lys Met Val Leu Asp Asp Ser Ser Cys Ser Arg
               500                 505                 510
   Trp Asp Pro Glu Thr Ile Trp Arg Asp Ile Asp Glu Leu Ser Asp Glu
           515                 520                 525
   Lys Leu Arg Glu His Asp Pro Phe Leu Lys Ala Ile Asn Cys Leu Asp
       530                 535                 540
   Gly Leu Glu Val Asp Val Arg Leu Gly Glu Phe Thr Arg Ala Tyr Lys
   545                 550                 555                 560
   Asp Gly Lys Asn Gln Glu Thr Gly Leu Pro Leu Leu Trp Lys Leu Lys
                   565                 570                 575
   Asp Trp Pro Ser Pro Ser Ala Ser Glu Glu Phe Ile Phe Tyr Gln Arg
               580                 585                 590
   Pro Glu Phe Ile Arg Ser Phe Pro Phe Leu Glu Tyr Ile His Pro Arg
           595                 600                 605
   Leu Gly Leu Leu Asn Val Ala Ala Lys Leu Pro His Tyr Ser Leu Gln
       610                 615                 620
   Asn Asp Ser Gly Pro Lys Ile Tyr Val Ser Cys Gly Thr Tyr Gln Glu
   625                 630                 635                 640
   Ile Ser Ala Gly Asp Ser Leu Thr Gly Ile His Tyr Asn Met Arg Asp
                   645                 650                 655
   Met Val Tyr Leu Leu Val His Thr Ser Glu Glu Thr Thr Phe Glu Arg
               660                 665                 670
   Val Arg Lys Thr Lys Pro Val Pro Glu Glu Pro Asp Gln Lys Met Ser
           675                 680                 685
   Glu Asn Glu Ser Leu Leu Ser Pro Glu Gln Lys Leu Arg Asp Gly Glu
       690                 695                 700
   Leu His Asp Leu Ser Leu Gly Glu Ala Ser Met Glu Lys Asn Glu Pro
   705                 710                 715                 720
   Glu Leu Ala Leu Thr Val Asn Pro Glu Asn Leu Thr Glu Asn Gly Asp
                   725                 730                 735
   Asn Met Glu Ser Ser Cys Thr Ser Ser Cys Ala Gly Gly Ala Gln Trp
               740                 745                 750
   Asp Val Phe Arg Arg Gln Asp Val Pro Lys Leu Ser Gly Tyr Leu Gln
           755                 760                 765
   Arg Thr Phe Gln Lys Pro Asp Asn Ile Gln Thr Asp Phe Val Ser Arg
       770                 775                 780
   Pro Leu Tyr Glu Gly Leu Phe Leu Asn Glu His His Lys Arg Gln Leu
   785                 790                 795                 800
   Arg Asp Glu Phe Gly Val Glu Pro Trp Thr Phe Glu Gln His Arg Gly
                   805                 810                 815
   Glu Ala Ile Phe Ile Pro Ala Gly Cys Pro Phe Gln Ile Thr Asn Leu
               820                 825                 830
   Gln Ser Asn Ile Gln Val Ala Leu Asp Phe Leu Cys Pro Glu Ser Val
           835                 840                 845
   Gly Glu Ser Ala Arg Leu Ala Glu Glu Ile Arg Cys Leu Pro Asn Asp
       850                 855                 860
   His Glu Ala Lys Leu Gln Ile Leu Glu Ile Gly Lys Ile Ser Leu Tyr
   865                 870                 875                 880
   Ala Ala Ser Ser Ala Ile Lys Glu Val Gln Lys Leu Val Leu Asp Pro
                   885                 890                 895
   Lys Phe Gly Ala Glu Leu Gly Phe Glu Asp Ser Asn Leu Thr Lys Ala
               900                 905                 910
   Val Ser His Asn Leu Asp Glu Ala Thr Lys Arg Pro Gln Gln Asn Ser
           915                 920                 925
   Cys Thr
       930
<210> 27
   <211> 2793
   <212> DNA
   <213> Arabidopsis thaliana
<400> 27
   atgaatgcta atgagcaaac tcgatccgcc aatggcattg gcaatggcaa tggtgagtct      60
   attcccggga ttccagatga cttacggtgc aagagatcgg atggtaaaca gtggagatgc     120
   actgcaatgt ccatggctga taagactgtt tgtgagaagc actacatcca agcaaagaag     180
   cgggcggcta attctgcttt cagggcgaac cagaagaaag cgaaaaggcg atcatcgtta     240
   ggcgaaacag atacgtattc ggaagggaag atggatgatt tcgagttacc agtcaccagc     300
   attgaccact ataataacgg tcttgcctct gcttccaaga gtaatggtag actagagaag     360
   agacataata aaagcctgat gcggtactcg cccgagacac cgatgatgag gagtttctct     420
   ccacgtgttg cagtggattt gaatgatgac ttgggtagag atgttgtaat gtttgaagag     480
   ggctacagat cttataggac accaccatct gttgctgtta tggatccgac acgaaacaga     540
   tcacaccaaa gcaccagtcc tatggaatac tcagcagcaa gcacagatgt gtctgcagag     600
   tctttggggg aaatctgcca tcaatgccag agaaaagata gagagagaat catttcttgc     660
   ctcaaatgca atcaaagagc cttctgccac aattgtctat cggcaaggta ctcggagata     720
   tcacttgaag aagtcgagaa agtttgccct gcatgtcgtg gcttgtgtga ttgcaaatct     780
   tgcctgcgtt cagataatac aataaaggtt cggatccggg aaatacccgt tttggacaag     840
   ttgcagtatc tttatcgtct attatcagct gtcctaccag tcataaagca gatccatctt     900
   gaacaatgta tggaagttga actagagaag aggcttcgtg aagttgagat tgatcttgtc     960
   agggcaagat tgaaagcaga tgagcagatg tgctgcaacg tgtgtcggat accagttgtt    1020
   gactactacc gtcactgtcc gaactgctca tatgaccttt gcctgagatg ctgtcaagat    1080
   ctacgggaag agtcttcagt gacgattagt gggactaacc aaaacgtaca agatagaaaa    1140
   ggagctccca aactaaaact aaacttttca tacaagtttc ctgagtggga agccaacggt    1200
   gatgggagca tcccttgccc tcctaaggag tatggaggct gcggttcaca ttctttgaat    1260
   cttgcccgca ttttcaagat gaattgggtt gcaaagcttg tgaaaaatgc tgaggagatt    1320
   gttagtggct gcaaattatc tgatcttctg aaccctgata tgtgtgattc aagattctgc    1380
   aaatttgctg agagagaaga gagcggtgac aactacgtgt acagcccgtc gcttgaaacg    1440
   attaaaactg atggagtagc taagtttgag caacaatggg cagagggtcg gcttgttact    1500
   gtgaaaatgg tacttgatga ctcatcttgc tctagatggg atcctgagac tatttggagg    1560
   gatatagacg agctttcgga cgagaaactg agagaacatg atccattctt gaaggccatt    1620
   aattgcttgg atggtttaga ggttgatgta agacttgggg agtttacaag agcatataaa    1680
   gatggaaaga accaagagac aggtcttccg ctattgtgga agttaaagga ctggccgagc    1740
   ccaagtgctt ccgaggagtt cattttctac caaagacctg agtttatcag aagttttccg    1800
   tttctcgagt acattcatcc ccggttaggc cttctgaatg ttgcagccaa gttacctcat    1860
   tactcgctcc aaaacgattc aggtccaaag atttatgtgt cttgtgggac gtaccaagaa    1920
   atcagtgctg gcgattcatt gactggtatt cactacaaca tgcgtgacat ggtataccta    1980
   ttggtgcaca cgtctgaaga aacaacattc gaaagggtga gaaaaacaaa acctgttcca    2040
   gaggaacctg accagaagat gagcgaaaat gagtcacttc ttagccctga gcagaaatta    2100
   agggacggag agttacatga tctatcactt ggtgaagcca gtatggagaa gaatgaacct    2160
   gagttggcgt tgactgtgaa tccagagaac ttaacggaaa acggtgacaa catggaatct    2220
   tcttgcacat cttcatgtgc aggaggagcc cagtgggatg tctttcgacg ccaagacgtc    2280
   ccaaagttgt ccgggtattt gcagagaaca ttccagaagc ctgataatat ccagactgat    2340
   tttgtgtcac gcccgttgta tgaaggattg ttcttaaatg aacaccacaa gagacaacta    2400
   agagacgagt ttggagttga gccatggaca tttgagcaac atcgtggtga ggctatcttc    2460
   attccggctg gatgtccgtt ccaaatcact aatcttcagt cgaatattca ggtggcactt    2520
   gacttcttgt gccctgaaag cgttggagag tcagcaagac tagctgaaga aatccggtgt    2580
   ttaccaaacg accacgaggc aaaacttcag attctagaga ttggaaagat atcattatac    2640
   gcagctagct cagccattaa agaggttcag aaactggtct tggatccaaa gtttggagca    2700
   gagcttggat ttgaagactc taacttaacc aaagcagtct ctcacaactt agacgaggca    2760
   accaagcggc cgcagcaaaa cagctgcact taa                                2793
<210> 28
   <211> 930
   <212> PRT
   <213> Arabidopsis thaliana
<400> 28
   Met Asn Ala Asn Glu Gln Thr Arg Ser Ala Asn Gly Ile Gly Asn Gly
   1               5                   10                  15
   Asn Gly Glu Ser Ile Pro Gly Ile Pro Asp Asp Leu Arg Cys Lys Arg
               20                  25                  30
   Ser Asp Gly Lys Gln Trp Arg Cys Thr Ala Met Ser Met Ala Asp Lys
           35                  40                  45
   Thr Val Cys Glu Lys His Tyr Ile Gln Ala Lys Lys Arg Ala Ala Asn
       50                  55                  60
   Ser Ala Phe Arg Ala Asn Gln Lys Lys Ala Lys Arg Arg Ser Ser Leu
   65                  70                  75                  80
   Gly Glu Thr Asp Thr Tyr Ser Glu Gly Lys Met Asp Asp Phe Glu Leu
                   85                  90                  95
   Pro Val Thr Ser Ile Asp His Tyr Asn Asn Gly Leu Ala Ser Ala Ser
               100                 105                 110
   Lys Ser Asn Gly Arg Leu Glu Lys Arg His Asn Lys Ser Leu Met Arg
           115                 120                 125
   Tyr Ser Pro Glu Thr Pro Met Met Arg Ser Phe Ser Pro Arg Val Ala
       130                 135                 140
   Val Asp Leu Asn Asp Asp Leu Gly Arg Asp Val Val Met Phe Glu Glu
   145                 150                 155                 160
   Gly Tyr Arg Ser Tyr Arg Thr Pro Pro Ser Val Ala Val Met Asp Pro
                   165                 170                 175
   Thr Arg Asn Arg Ser His Gln Ser Thr Ser Pro Met Glu Tyr Ser Ala
               180                 185                 190
   Ala Ser Thr Asp Val Ser Ala Glu Ser Leu Gly Glu Ile Cys His Gln
           195                 200                 205
   Cys Gln Arg Lys Asp Arg Glu Arg Ile Ile Ser Cys Leu Lys Cys Asn
       210                 215                 220
   Gln Arg Ala Phe Cys His Asn Cys Leu Ser Ala Arg Tyr Ser Glu Ile
   225                 230                 235                 240
   Ser Leu Glu Glu Val Glu Lys Val Cys Pro Ala Cys Arg Gly Leu Cys
                   245                 250                 255
   Asp Cys Lys Ser Cys Leu Arg Ser Asp Asn Thr Ile Lys Val Arg Ile
               260                 265                 270
   Arg Glu Ile Pro Val Leu Asp Lys Leu Gln Tyr Leu Tyr Arg Leu Leu
           275                 280                 285
   Ser Ala Val Leu Pro Val Ile Lys Gln Ile His Leu Glu Gln Cys Met
       290                 295                 300
   Glu Val Glu Leu Glu Lys Arg Leu Arg Glu Val Glu Ile Asp Leu Val
   305                 310                 315                 320
   Arg Ala Arg Leu Lys Ala Asp Glu Gln Met Cys Cys Asn Val Cys Arg
                   325                 330                 335
   Ile Pro Val Val Asp Tyr Tyr Arg His Cys Pro Asn Cys Ser Tyr Asp
               340                 345                 350
   Leu Cys Leu Arg Cys Cys Gln Asp Leu Arg Glu Glu Ser Ser Val Thr
           355                 360                 365
   Ile Ser Gly Thr Asn Gln Asn Val Gln Asp Arg Lys Gly Ala Pro Lys
       370                 375                 380
   Leu Lys Leu Asn Phe Ser Tyr Lys Phe Pro Glu Trp Glu Ala Asn Gly
   385                 390                 395                 400
   Asp Gly Ser Ile Pro Cys Pro Pro Lys Glu Tyr Gly Gly Cys Gly Ser
                   405                 410                 415
   His Ser Leu Asn Leu Ala Arg Ile Phe Lys Met Asn Trp Val Ala Lys
               420                 425                 430
   Leu Val Lys Asn Ala Glu Glu Ile Val Ser Gly Cys Lys Leu Ser Asp
           435                 440                 445
   Leu Leu Asn Pro Asp Met Cys Asp Ser Arg Phe Cys Lys Phe Ala Glu
       450                 455                 460
   Arg Glu Glu Ser Gly Asp Asn Tyr Val Tyr Ser Pro Ser Leu Glu Thr
   465                 470                 475                 480
   Ile Lys Thr Asp Gly Val Ala Lys Phe Glu Gln Gln Trp Ala Glu Gly
                   485                 490                 495
   Arg Leu Val Thr Val Lys Met Val Leu Asp Asp Ser Ser Cys Ser Arg
               500                 505                 510
   Trp Asp Pro Glu Thr Ile Trp Arg Asp Ile Asp Glu Leu Ser Asp Glu
           515                 520                 525
   Lys Leu Arg Glu His Asp Pro Phe Leu Lys Ala Ile Asn Cys Leu Asp
       530                 535                 540
   Gly Leu Glu Val Asp Val Arg Leu Gly Glu Phe Thr Arg Ala Tyr Lys
   545                 550                 555                 560
   Asp Gly Lys Asn Gln Glu Thr Gly Leu Pro Leu Leu Trp Lys Leu Lys
                   565                 570                 575
   Asp Trp Pro Ser Pro Ser Ala Ser Glu Glu Phe Ile Phe Tyr Gln Arg
               580                 585                 590
   Pro Glu Phe Ile Arg Ser Phe Pro Phe Leu Glu Tyr Ile His Pro Arg
           595                 600                 605
   Leu Gly Leu Leu Asn Val Ala Ala Lys Leu Pro His Tyr Ser Leu Gln
       610                 615                 620
   Asn Asp Ser Gly Pro Lys Ile Tyr Val Ser Cys Gly Thr Tyr Gln Glu
   625                 630                 635                 640
   Ile Ser Ala Gly Asp Ser Leu Thr Gly Ile His Tyr Asn Met Arg Asp
                   645                 650                 655
   Met Val Tyr Leu Leu Val His Thr Ser Glu Glu Thr Thr Phe Glu Arg
               660                 665                 670
   Val Arg Lys Thr Lys Pro Val Pro Glu Glu Pro Asp Gln Lys Met Ser
           675                 680                 685
   Glu Asn Glu Ser Leu Leu Ser Pro Glu Gln Lys Leu Arg Asp Gly Glu
       690                 695                 700
   Leu His Asp Leu Ser Leu Gly Glu Ala Ser Met Glu Lys Asn Glu Pro
   705                 710                 715                 720
   Glu Leu Ala Leu Thr Val Asn Pro Glu Asn Leu Thr Glu Asn Gly Asp
                   725                 730                 735
   Asn Met Glu Ser Ser Cys Thr Ser Ser Cys Ala Gly Gly Ala Gln Trp
               740                 745                 750
   Asp Val Phe Arg Arg Gln Asp Val Pro Lys Leu Ser Gly Tyr Leu Gln
           755                 760                 765
   Arg Thr Phe Gln Lys Pro Asp Asn Ile Gln Thr Asp Phe Val Ser Arg
       770                 775                 780
   Pro Leu Tyr Glu Gly Leu Phe Leu Asn Glu His His Lys Arg Gln Leu
   785                 790                 795                 800
   Arg Asp Glu Phe Gly Val Glu Pro Trp Thr Phe Glu Gln His Arg Gly
                   805                 810                 815
   Glu Ala Ile Phe Ile Pro Ala Gly Cys Pro Phe Gln Ile Thr Asn Leu
               820                 825                 830
   Gln Ser Asn Ile Gln Val Ala Leu Asp Phe Leu Cys Pro Glu Ser Val
           835                 840                 845
   Gly Glu Ser Ala Arg Leu Ala Glu Glu Ile Arg Cys Leu Pro Asn Asp
       850                 855                 860
   His Glu Ala Lys Leu Gln Ile Leu Glu Ile Gly Lys Ile Ser Leu Tyr
   865                 870                 875                 880
   Ala Ala Ser Ser Ala Ile Lys Glu Val Gln Lys Leu Val Leu Asp Pro
                   885                 890                 895
   Lys Phe Gly Ala Glu Leu Gly Phe Glu Asp Ser Asn Leu Thr Lys Ala
               900                 905                 910
   Val Ser His Asn Leu Asp Glu Ala Thr Lys Arg Pro Gln Gln Asn Ser
           915                 920                 925
   Cys Thr
       930
<210> 29
   <211> 2835
   <212> DNA
   <213> Arabidopsis thaliana
<400> 29
   atgcaggtca attttgatga aacttgtgat tcagtgatta gaatgaatgc taatgagcaa      60
   actcgatccg ccaatggcat tggcaatggc aatggtgagt ctattcccgg gattccagat     120
   gacttacggt gcaagagatc ggatggtaaa cagtggagat gcactgcaat gtccatggct     180
   gataagactg tttgtgagaa gcactacatc caagcaaaga agcgggcggc taattctgct     240
   ttcagggcga accagaagaa agcgaaaagg cgatcatcgt taggcgaaac agatacgtat     300
   tcggaaggga agatggatga tttcgagtta ccagtcacca gcattgacca ctataataac     360
   ggtcttgcct ctgcttccaa gagtaatggt agactagaga agagacataa taaaagcctg     420
   atgcggtact cgcccgagac accgatgatg aggagtttct ctccacgtgt tgcagtggat     480
   ttgaatgatg acttgggtag agatgttgta atgtttgaag agggctacag atcttatagg     540
   acaccaccat ctgttgctgt tatggatccg acacgaaaca gatcacacca aagcaccagt     600
   cctatggaat actcagcagc aagcacagat gtgtctgcag agtctttggg ggaaatctgc     660
   catcaatgcc agagaaaaga tagagagaga atcatttctt gcctcaaatg caatcaaaga     720
   gccttctgcc acaattgtct atcggcaagg tactcggaga tatcacttga agaagtcgag     780
   aaagtttgcc ctgcatgtcg tggcttgtgt gattgcaaat cttgcctgcg ttcagataat     840
   acaataaagg ttcggatccg ggaaataccc gttttggaca agttgcagta tctttatcgt     900
   ctattatcag ctgtcctacc agtcataaag cagatccatc ttgaacaatg tatggaagtt     960
   gaactagaga agaggcttcg tgaagttgag attgatcttg tcagggcaag attgaaagca    1020
   gatgagcaga tgtgctgcaa cgtgtgtcgg ataccagttg ttgactacta ccgtcactgt    1080
   ccgaactgct catatgacct ttgcctgaga tgctgtcaag atctacggga agagtcttca    1140
   gtgacgatta gtgggactaa ccaaaacgta caagatagaa aaggagctcc caaactaaaa    1200
   ctaaactttt catacaagtt tcctgagtgg gaagccaacg gtgatgggag catcccttgc    1260
   cctcctaagg agtatggagg ctgcggttca cattctttga atcttgcccg cattttcaag    1320
   atgaattggg ttgcaaagct tgtgaaaaat gctgaggaga ttgttagtgg ctgcaaatta    1380
   tctgatcttc tgaaccctga tatgtgtgat tcaagattct gcaaatttgc tgagagagaa    1440
   gagagcggtg acaactacgt gtacagcccg tcgcttgaaa cgattaaaac tgatggagta    1500
   gctaagtttg agcaacaatg ggcagagggt cggcttgtta ctgtgaaaat ggtacttgat    1560
   gactcatctt gctctagatg ggatcctgag actatttgga gggatataga cgagctttcg    1620
   gacgagaaac tgagagaaca tgatccattc ttgaaggcca ttaattgctt ggatggttta    1680
   gaggttgatg taagacttgg ggagtttaca agagcatata aagatggaaa gaaccaagag    1740
   acaggtcttc cgctattgtg gaagttaaag gactggccga gcccaagtgc ttccgaggag    1800
   ttcattttct accaaagacc tgagtttatc agaagttttc cgtttctcga gtacattcat     1860
   ccccggttag gccttctgaa tgttgcagcc aagttacctc attactcgct ccaaaacgat    1920
   tcaggtccaa agatttatgt gtcttgtggg acgtaccaag aaatcagtgc tggcgattca    1980
   ttgactggta ttcactacaa catgcgtgac atggtatacc tattggtgca cacgtctgaa   2040
   gaaacaacat tcgaaagggt gagaaaaaca aaacctgttc cagaggaacc tgaccagaag   2100
   atgagcgaaa atgagtcact tcttagccct gagcagaaat taagggacgg agagttacat   2160
   gatctatcac ttggtgaagc cagtatggag aagaatgaac ctgagttggc gttgactgtg   2220
   aatccagaga acttaacgga aaacggtgac aacatggaat cttcttgcac atcttcatgt   2280
   gcaggaggag cccagtggga tgtctttcga cgccaagacg tcccaaagtt gtccgggtat   2340
   ttgcagagaa cattccagaa gcctgataat atccagactg attttgtgtc acgcccgttg   2400
   tatgaaggat tgttcttaaa tgaacaccac aagagacaac taagagacga gtttggagtt   2460
   gagccatgga catttgagca acatcgtggt gaggctatct tcattccggc tggatgtccg   2520
   ttccaaatca ctaatcttca gtcgaatatt caggtggcac ttgacttctt gtgccctgaa   2580
   agcgttggag agtcagcaag actagctgaa gaaatccggt gtttaccaaa cgaccacgag   2640
   gcaaaacttc agattctaga gattggaaag atatcattat acgcagctag ctcagccatt   2700
   aaagaggttc agaaactggt cttggatcca aagtttggag cagagcttgg atttgaagac   2760
   tctaacttaa ccaaagcagt ctctcacaac ttagacgagg caaccaagcg gccgcagcaa   2820
   aacagctgca cttaa                                                    2835
<210> 30
   <211> 944
   <212> PRT
   <213> Arabidopsis thaliana
<400> 30
   Met Gln Val Asn Phe Asp Glu Thr Cys Asp Ser Val Ile Arg Met Asn
   1               5                   10                  15
   Ala Asn Glu Gln Thr Arg Ser Ala Asn Gly Ile Gly Asn Gly Asn Gly
               20                  25                  30
   Glu Ser Ile Pro Gly Ile Pro Asp Asp Leu Arg Cys Lys Arg Ser Asp
           35                  40                  45
   Gly Lys Gln Trp Arg Cys Thr Ala Met Ser Met Ala Asp Lys Thr Val
       50                  55                  60
   Cys Glu Lys His Tyr Ile Gln Ala Lys Lys Arg Ala Ala Asn Ser Ala
   65                  70                  75                  80
   Phe Arg Ala Asn Gln Lys Lys Ala Lys Arg Arg Ser Ser Leu Gly Glu
                   85                  90                  95
   Thr Asp Thr Tyr Ser Glu Gly Lys Met Asp Asp Phe Glu Leu Pro Val
               100                 105                 110
   Thr Ser Ile Asp His Tyr Asn Asn Gly Leu Ala Ser Ala Ser Lys Ser
           115                 120                 125
   Asn Gly Arg Leu Glu Lys Arg His Asn Lys Ser Leu Met Arg Tyr Ser
       130                 135                 140
   Pro Glu Thr Pro Met Met Arg Ser Phe Ser Pro Arg Val Ala Val Asp
   145                 150                 155                 160
   Leu Asn Asp Asp Leu Gly Arg Asp Val Val Met Phe Glu Glu Gly Tyr
                   165                 170                 175
   Arg Ser Tyr Arg Thr Pro Pro Ser Val Ala Val Met Asp Pro Thr Arg
               180                 185                 190
   Asn Arg Ser His Gln Ser Thr Ser Pro Met Glu Tyr Ser Ala Ala Ser
           195                 200                 205
   Thr Asp Val Ser Ala Glu Ser Leu Gly Glu Ile Cys His Gln Cys Gln
       210                 215                 220
   Arg Lys Asp Arg Glu Arg Ile Ile Ser Cys Leu Lys Cys Asn Gln Arg
   225                 230                 235                 240
   Ala Phe Cys His Asn Cys Leu Ser Ala Arg Tyr Ser Glu Ile Ser Leu
                   245                 250                 255
   Glu Glu Val Glu Lys Val Cys Pro Ala Cys Arg Gly Leu Cys Asp Cys
               260                 265                 270
   Lys Ser Cys Leu Arg Ser Asp Asn Thr Ile Lys Val Arg Ile Arg Glu
           275                 280                 285
   Ile Pro Val Leu Asp Lys Leu Gln Tyr Leu Tyr Arg Leu Leu Ser Ala
       290                 295                 300
   Val Leu Pro Val Ile Lys Gln Ile His Leu Glu Gln Cys Met Glu Val
   305                 310                 315                 320
   Glu Leu Glu Lys Arg Leu Arg Glu Val Glu Ile Asp Leu Val Arg Ala
                   325                 330                 335
   Arg Leu Lys Ala Asp Glu Gln Met Cys Cys Asn Val Cys Arg Ile Pro
               340                 345                 350
   Val Val Asp Tyr Tyr Arg His Cys Pro Asn Cys Ser Tyr Asp Leu Cys
           355                 360                 365
   Leu Arg Cys Cys Gln Asp Leu Arg Glu Glu Ser Ser Val Thr Ile Ser
       370                 375                 380
   Gly Thr Asn Gln Asn Val Gln Asp Arg Lys Gly Ala Pro Lys Leu Lys
   385                 390                 395                 400
   Leu Asn Phe Ser Tyr Lys Phe Pro Glu Trp Glu Ala Asn Gly Asp Gly
                   405                 410                 415
   Ser Ile Pro Cys Pro Pro Lys Glu Tyr Gly Gly Cys Gly Ser His Ser
               420                 425                 430
   Leu Asn Leu Ala Arg Ile Phe Lys Met Asn Trp Val Ala Lys Leu Val
           435                 440                 445
   Lys Asn Ala Glu Glu Ile Val Ser Gly Cys Lys Leu Ser Asp Leu Leu
       450                 455                 460
   Asn Pro Asp Met Cys Asp Ser Arg Phe Cys Lys Phe Ala Glu Arg Glu
   465                 470                 475                 480
   Glu Ser Gly Asp Asn Tyr Val Tyr Ser Pro Ser Leu Glu Thr Ile Lys
                   485                 490                 495
   Thr Asp Gly Val Ala Lys Phe Glu Gln Gln Trp Ala Glu Gly Arg Leu
               500                 505                 510
   Val Thr Val Lys Met Val Leu Asp Asp Ser Ser Cys Ser Arg Trp Asp
           515                 520                 525
   Pro Glu Thr Ile Trp Arg Asp Ile Asp Glu Leu Ser Asp Glu Lys Leu
       530                 535                 540
   Arg Glu His Asp Pro Phe Leu Lys Ala Ile Asn Cys Leu Asp Gly Leu
   545                 550                 555                 560
   Glu Val Asp Val Arg Leu Gly Glu Phe Thr Arg Ala Tyr Lys Asp Gly
                   565                 570                 575
   Lys Asn Gln Glu Thr Gly Leu Pro Leu Leu Trp Lys Leu Lys Asp Trp
               580                 585                 590
   Pro Ser Pro Ser Ala Ser Glu Glu Phe Ile Phe Tyr Gln Arg Pro Glu
           595                 600                 605
   Phe Ile Arg Ser Phe Pro Phe Leu Glu Tyr Ile His Pro Arg Leu Gly
       610                 615                 620
   Leu Leu Asn Val Ala Ala Lys Leu Pro His Tyr Ser Leu Gln Asn Asp
   625                 630                 635                 640
   Ser Gly Pro Lys Ile Tyr Val Ser Cys Gly Thr Tyr Gln Glu Ile Ser
                   645                 650                 655
   Ala Gly Asp Ser Leu Thr Gly Ile His Tyr Asn Met Arg Asp Met Val
               660                 665                 670
   Tyr Leu Leu Val His Thr Ser Glu Glu Thr Thr Phe Glu Arg Val Arg
           675                 680                 685
   Lys Thr Lys Pro Val Pro Glu Glu Pro Asp Gln Lys Met Ser Glu Asn
       690                 695                 700
   Glu Ser Leu Leu Ser Pro Glu Gln Lys Leu Arg Asp Gly Glu Leu His
   705                 710                 715                 720
   Asp Leu Ser Leu Gly Glu Ala Ser Met Glu Lys Asn Glu Pro Glu Leu
                   725                 730                 735
   Ala Leu Thr Val Asn Pro Glu Asn Leu Thr Glu Asn Gly Asp Asn Met
               740                 745                 750
   Glu Ser Ser Cys Thr Ser Ser Cys Ala Gly Gly Ala Gln Trp Asp Val
           755                 760                 765
   Phe Arg Arg Gln Asp Val Pro Lys Leu Ser Gly Tyr Leu Gln Arg Thr
       770                 775                 780
   Phe Gln Lys Pro Asp Asn Ile Gln Thr Asp Phe Val Ser Arg Pro Leu
   785                 790                 795                 800
   Tyr Glu Gly Leu Phe Leu Asn Glu His His Lys Arg Gln Leu Arg Asp
                   805                 810                 815
   Glu Phe Gly Val Glu Pro Trp Thr Phe Glu Gln His Arg Gly Glu Ala
               820                 825                 830
   Ile Phe Ile Pro Ala Gly Cys Pro Phe Gln Ile Thr Asn Leu Gln Ser
           835                 840                 845
   Asn Ile Gln Val Ala Leu Asp Phe Leu Cys Pro Glu Ser Val Gly Glu
       850                 855                 860
   Ser Ala Arg Leu Ala Glu Glu Ile Arg Cys Leu Pro Asn Asp His Glu
   865                 870                 875                 880
   Ala Lys Leu Gln Ile Leu Glu Ile Gly Lys Ile Ser Leu Tyr Ala Ala
                   885                 890                 895
   Ser Ser Ala Ile Lys Glu Val Gln Lys Leu Val Leu Asp Pro Lys Phe
               900                 905                 910
   Gly Ala Glu Leu Gly Phe Glu Asp Ser Asn Leu Thr Lys Ala Val Ser
           915                 920                 925
   His Asn Leu Asp Glu Ala Thr Lys Arg Pro Gln Gln Asn Ser Cys Thr
       930                 935                 940
<210> 31
   <211> 2643
   <212> DNA
   <213> Arabidopsis thaliana
<400> 31
   atggagggtg aagttgctac taacggagtg attctcaagc ataatggtgt aaaggatatt      60
   tctttagaaa cttgttggcc agagaagaag aaaccggttg aggcaacgag tcttagcagt     120
   ggttcatcag acatagaaga agagattagt gtggaatgtc ctaagcgagt ggctaatcaa     180
   agacggaaac ggtctaaagc tgatgagatt aaaacgaaat cttcaagaaa gaggaaatgt     240
   gatgatgaga acaagtgtga agagaatgag aagaagcaga ggagctctgt taaaaagaga     300
   gcaactacat ggaaagaaga agaagttgtt gttgatgatg aaaagaaatg cgaacaacaa     360
   ctccaactag ttccttccag caaggcaaca tctcgaagca ggagcaagaa atcagtcagt     420
   gtggatactt ggttggttaa caacgagatt gatgttagcg ctttgagttc gcgttctgag     480
   tctgaacttt cggattctta tctgaaaacc gagtacttta atgactgcag aagcatgaca     540
   aggagtttaa aggcaaattt gggagagctt gcaatttgcc atcaatgctc taaaggggaa     600
   agaagatatc ttttcatttg caccttttgt gaagtgaggt tgtattgttt tccatgcata     660
   aagaaatggt atccccattt gtccacggat gatatccttg aaaaatgtcc cttttgccga     720
   ggaacttgca actgctgcac atgtttgcac tctagtggtt taatcgagac atctaagaga     780
   aagctcgata aatacgaaag attttatcat cttcgcttct tgattgtggc gatgcttcct     840
   ttcctgaaaa agttatgcaa agcacaagat caagaaatcg aaaccgaggc taaggttcaa     900
   gattcaatgg cttctcaagt tgatatatcc gagagtctat gctcaaatga agagcgtgtc    960
   ttctgtaacc actgtgcaac atcaattgtt gacttgcatc gaagctgtcc aaagtgttca   1020
   tatgaattgt gtctgaactg ttgccaagag atccgtggag gttggctttc tgaccgcccg   1080
   gaatgtcagc tacaattcga gtacagaggg acccggtata tacatggcga agctgcagaa   1140
   ccgagctctt cttctgtttc cgaggatgaa actaaaactc catccatcaa gtggaatgct   1200
   gatgaaaatg gaagcatacg gtgtgcgcca aaagagctag gaggttgcgg ggactctgtg   1260
   ctggagctta agcgaatctt accagtgact tggatgtcag atttggagca aaaggcagaa   1320
   acattcttag cctcttatag tatcaaaccg ccaatgtcat attgtagatg ttcttctgat   1380
   atgagtagta tgaagaggaa ggcagcttcg agggacggat ctagtgacaa ttacctttat   1440
   tctcctgatt ctttggatgt cctgaagcaa gaagaacttc tgcatttcca agagcattgg   1500
   tccaaaggtg aaccagtgat tgttcgaaat gctcttaaca acacagctgg tttaagctgg   1560
   gagccaatgg tgatgtggcg ggctttatgt gagaatgttg attcagcaat tagctctaat   1620
   atgtctgatg tcaaagctat cgattgttta gctaattgtg aggtgaagat aaatactcta   1680
   tgtttctttg aaggatatag caaaggaaga acatatgaaa acttctggcc tgagatgctg   1740
   aagctgaagg attggcctcc ttcagacaag tttgaaaacc ttttacctcg tcactgcgat   1800
   gagttcattt ccgcattacc atttcaagaa tacagcgatc ctagatcagg aattctcaac   1860
   attgctacaa aacttcctga aggacttctt aaaccagatc ttggtccaaa aacttatgtt   1920
   gcatatggga cttcagatga gctcggtaga ggagattcag tcactaagct tcactgtgat   1980
   atgtcagatg ctgtaagttt cagaattgtt aatattctga tgcatactgc tgaagtaaca   2040
   ctaagtgagg agcaaaggtc tgcaattgca gatttgaaac agaaacataa gcaacagaat   2100
   gagaaagagc ttcaggagca aaatggttta gaggaagaag aggttgtgag tgatgagatt   2160
   gtggtttatg atgaaacaag tggtgcactt tgggacatct ttaaaagaga agatgttcct   2220
   aagcttgaag agtatctaag gaagcattgc atagaattca gacatactta ttgctctcgt   2280
   gttacaaagg tgtatcatcc aatacatgat cagtcatatt tcttaaccgt ggagcataaa   2340
   agaaaactca aggcagaatt tgggattgag ccatggacat ttgtgcaaaa gctaggagaa   2400
   gcagtgttta ttccagcagg ttgtcctcat caagttcgga atctcaagtc gtgcacaaag   2460
   gtagctgttg attttgtttc ccctgaaaac atcgatgagt gtctccgttt gactgatgag   2520
   tttcgacaac tccctaagaa ccataaagcc agagaagaca aacttgagat aaaaaagatg   2580
   gtgatctatg cagttgaaca agctctgaaa gaagtggaga cattattgct agatcgcagc   2640
   taa                                                                 2643
<210> 32
   <211> 880
   <212> PRT
   <213> Arabidopsis thaliana
<400> 32
   Met Glu Gly Glu Val Ala Thr Asn Gly Val Ile Leu Lys His Asn Gly
   1               5                   10                  15
   Val Lys Asp Ile Ser Leu Glu Thr Cys Trp Pro Glu Lys Lys Lys Pro
               20                  25                  30
   Val Glu Ala Thr Ser Leu Ser Ser Gly Ser Ser Asp Ile Glu Glu Glu
           35                  40                  45
   Ile Ser Val Glu Cys Pro Lys Arg Val Ala Asn Gln Arg Arg Lys Arg
       50                  55                  60
   Ser Lys Ala Asp Glu Ile Lys Thr Lys Ser Ser Arg Lys Arg Lys Cys
   65                  70                  75                  80
   Asp Asp Glu Asn Lys Cys Glu Glu Asn Glu Lys Lys Gln Arg Ser Ser
                   85                  90                  95
   Val Lys Lys Arg Ala Thr Thr Trp Lys Glu Glu Glu Val Val Val Asp
               100                 105                 110
   Asp Glu Lys Lys Cys Glu Gln Gln Leu Gln Leu Val Pro Ser Ser Lys
           115                 120                 125
   Ala Thr Ser Arg Ser Arg Ser Lys Lys Ser Val Ser Val Asp Thr Trp
       130                 135                 140
   Leu Val Asn Asn Glu Ile Asp Val Ser Ala Leu Ser Ser Arg Ser Glu
   145                 150                 155                 160
   Ser Glu Leu Ser Asp Ser Tyr Leu Lys Thr Glu Tyr Phe Asn Asp Cys
                   165                 170                 175
   Arg Ser Met Thr Arg Ser Leu Lys Ala Asn Leu Gly Glu Leu Ala Ile
               180                 185                 190
   Cys His Gln Cys Ser Lys Gly Glu Arg Arg Tyr Leu Phe Ile Cys Thr
           195                 200                 205
   Phe Cys Glu Val Arg Leu Tyr Cys Phe Pro Cys Ile Lys Lys Trp Tyr
       210                 215                 220
   Pro His Leu Ser Thr Asp Asp Ile Leu Glu Lys Cys Pro Phe Cys Arg
   225                 230                 235                 240
   Gly Thr Cys Asn Cys Cys Thr Cys Leu His Ser Ser Gly Leu Ile Glu
                   245                 250                 255
   Thr Ser Lys Arg Lys Leu Asp Lys Tyr Glu Arg Phe Tyr His Leu Arg
               260                 265                 270
   Phe Leu Ile Val Ala Met Leu Pro Phe Leu Lys Lys Leu Cys Lys Ala
           275                 280                 285
   Gln Asp Gln Glu Ile Glu Thr Glu Ala Lys Val Gln Asp Ser Met Ala
       290                 295                 300
   Ser Gln Val Asp Ile Ser Glu Ser Leu Cys Ser Asn Glu Glu Arg Val
   305                 310                 315                 320
   Phe Cys Asn His Cys Ala Thr Ser Ile Val Asp Leu His Arg Ser Cys
                   325                 330                 335
   Pro Lys Cys Ser Tyr Glu Leu Cys Leu Asn Cys Cys Gln Glu Ile Arg
               340                 345                 350
   Gly Gly Trp Leu Ser Asp Arg Pro Glu Cys Gln Leu Gln Phe Glu Tyr
           355                 360                 365
   Arg Gly Thr Arg Tyr Ile His Gly Glu Ala Ala Glu Pro Ser Ser Ser
       370                 375                 380
   Ser Val Ser Glu Asp Glu Thr Lys Thr Pro Ser Ile Lys Trp Asn Ala
   385                 390                 395                 400
   Asp Glu Asn Gly Ser Ile Arg Cys Ala Pro Lys Glu Leu Gly Gly Cys
                   405                 410                 415
   Gly Asp Ser Val Leu Glu Leu Lys Arg Ile Leu Pro Val Thr Trp Met
               420                 425                 430
   Ser Asp Leu Glu Gln Lys Ala Glu Thr Phe Leu Ala Ser Tyr Ser Ile
           435                 440                 445
   Lys Pro Pro Met Ser Tyr Cys Arg Cys Ser Ser Asp Met Ser Ser Met
       450                 455                 460
   Lys Arg Lys Ala Ala Ser Arg Asp Gly Ser Ser Asp Asn Tyr Leu Tyr
   465                 470                 475                 480
   Ser Pro Asp Ser Leu Asp Val Leu Lys Gln Glu Glu Leu Leu His Phe
                   485                 490                 495
   Gln Glu His Trp Ser Lys Gly Glu Pro Val Ile Val Arg Asn Ala Leu
               500                 505                 510
   Asn Asn Thr Ala Gly Leu Ser Trp Glu Pro Met Val Met Trp Arg Ala
           515                 520                 525
   Leu Cys Glu Asn Val Asp Ser Ala Ile Ser Ser Asn Met Ser Asp Val
       530                 535                 540
   Lys Ala Ile Asp Cys Leu Ala Asn Cys Glu Val Lys Ile Asn Thr Leu
   545                 550                 555                 560
   Cys Phe Phe Glu Gly Tyr Ser Lys Gly Arg Thr Tyr Glu Asn Phe Trp
                                   565                 570                 575
   Pro Glu Met Leu Lys Leu Lys Asp Trp Pro Pro Ser Asp Lys Phe Glu
               580                 585                 590
   Asn Leu Leu Pro Arg His Cys Asp Glu Phe Ile Ser Ala Leu Pro Phe
           595                 600                 605
   Gln Glu Tyr Ser Asp Pro Arg Ser Gly Ile Leu Asn Ile Ala Thr Lys
       610                 615                 620
   Leu Pro Glu Gly Leu Leu Lys Pro Asp Leu Gly Pro Lys Thr Tyr Val
   625                 630                 635                 640
   Ala Tyr Gly Thr Ser Asp Glu Leu Gly Arg Gly Asp Ser Val Thr Lys
                   645                 650                 655
   Leu His Cys Asp Met Ser Asp Ala Val Ser Phe Arg Ile Val Asn Ile
               660                 665                 670
   Leu Met His Thr Ala Glu Val Thr Leu Ser Glu Glu Gln Arg Ser Ala
           675                 680                 685
   Ile Ala Asp Leu Lys Gln Lys His Lys Gln Gln Asn Glu Lys Glu Leu
       690                 695                 700
   Gln Glu Gln Asn Gly Leu Glu Glu Glu Glu Val Val Ser Asp Glu Ile
   705                 710                 715                 720
   Val Val Tyr Asp Glu Thr Ser Gly Ala Leu Trp Asp Ile Phe Lys Arg
                   725                 730                 735
   Glu Asp Val Pro Lys Leu Glu Glu Tyr Leu Arg Lys His Cys Ile Glu
               740                 745                 750
   Phe Arg His Thr Tyr Cys Ser Arg Val Thr Lys Val Tyr His Pro Ile
           755                 760                 765
   His Asp Gln Ser Tyr Phe Leu Thr Val Glu His Lys Arg Lys Leu Lys
       770                 775                 780
   Ala Glu Phe Gly Ile Glu Pro Trp Thr Phe Val Gln Lys Leu Gly Glu
   785                 790                 795                 800
   Ala Val Phe Ile Pro Ala Gly Cys Pro His Gln Val Arg Asn Leu Lys
                   805                 810                 815
   Ser Cys Thr Lys Val Ala Val Asp Phe Val Ser Pro Glu Asn Ile Asp
               820                 825                 830
   Glu Cys Leu Arg Leu Thr Asp Glu Phe Arg Gln Leu Pro Lys Asn His
           835                 840                 845
   Lys Ala Arg Glu Asp Lys Leu Glu Ile Lys Lys Met Val Ile Tyr Ala
       850                 855                 860
   Val Glu Gln Ala Leu Lys Glu Val Glu Thr Leu Leu Leu Asp Arg Ser
   865                 870                 875                 880
<210> 33
   <211> 2364
   <212> DNA
   <213> Arabidopsis thaliana
<400> 33
   atggaaaatc ctccattaga atctgagatc aaagaggata tgtctttgaa gaatcatcct      60
   ccagacaagg ataaggacaa agacactatc atggaacaac ctagtagtcc acgtcatcgg     120
   aaggttgttg ctagatggtt acctgatgaa gcacagaggc caattatcaa tgacgctccc     180
   gtattcactc catcactaga ggagtttgta gatccacttg cgtatatcga gaagatacgt     240
   ccactagcag agccgtatgg tatctgtcga atcatcccac catcgacatg gaagcctcct     300
   tgccggctta aggagaagag tatatgggag cagacaaagt ttcctactcg gattcaaacc     360
   gtagacctgc ttcagaaccg tgagccgatg aagaagaaac cgaagagtag aaagcggaaa     420
   cggagaagaa actcgagaat gggttcgtct aagagacgat ctggttcttc tcccgctgaa     480
   tcgacttcat cacccgaggc tgaggagaag tttggtttca attctggttc agacttcact     540
   cttgatgagt ttgagaaata tgctctgcat tttaaagact cgtactttga aaagaaagac     600
   tctggtggag atatagtaaa atggacaccg tctgtggatg atatagaagg ggaatactgg     660
   cggatagttg agcaaccaac agatgaagtg gaggtttact atggagctga cttggagaat     720
   ggggtgcttg gaagcgggtt ttataaaaga gccgagaagt ttaccggtag cgatatggag     780
   cagtacacat tatctggctg gaacttgaat aacttaccac ggcttcctgg ctctgtactc     840
   tcttttgaag attgtgacat atccggagtt ctagttccat ggctatatgt gggaatgtgt     900
   ttttcatcat tttgttggca tgtggaggac catcatttat attcacttaa ctatcatcac     960
   tttggggagc caaaagtatg gtatggtgtt ccaggaagca atgcaaccgc tctcgagaag    1020
   gcgatgagga aacatttacc tgacttgttc gaagacagcc tgatctacta catggcctgg    1080
   gcgtatcatg ctggattcaa ctgcggtttc aactgcgcag aagcggttaa tgtggctccg    1140
   gttgattggt tggctcatgg acagaacgct gtggaactat atagtaaaga gacaaggaag    1200
   acttctctgt ctcacgacaa gcttcttctt ggagcagctt atgaagcggt gaaggctctt    1260
   tgggaactct cagcttctga gggaaaggaa aatacaacaa atttgagatg gaagagtttc    1320
   tgtgggaaga acgggacact taccaacgcg atacaagctc ggttacaaat ggaagagggg    1380
   agaattacag ctcttggtag ggattcttca agcttgaaga agatggagaa ggactttgat    1440
   tcaaactgtg aaagggaatg tttctcatgc ttttatgatt tgcatctctc ggcttctggc    1500
   tgcaagtgct ctcccgaaga atacgcgtgc cttaaacatg cagatgatct ttgttcatgt    1560
   gatgtgaaag atggatttat tcttctccgg tacacaatgg atgagttaag ctcgttggtc    1620
   agagcattgg aaggggaatc agatgattta aagatatggg cttccaaggt tttaggcatt    1680
   gaacatagtg atgaagatca gacaaagact agttcagtta tcagtgagga gaagaagtta    1740
   aaggaaggtt cttttgatct gaacattgac ttagagatgg attatcaaga agacgttaaa    1800
   gaagaagcca gcactagtgg cggcgagtta accgcctcag agaaccttgg tgtatcggtc    1860
   gagcctataa acctcgggtt cttgattttt ggaaagcttt ggtgcaataa gtatgctata    1920
   ttcccaaaag gattcaggag tcgtgtcaag ttctacaatg ttcttgatcc aacaagaatg    1980
   agcaattaca tctctgaggt tttggatgca ggactcatgg gtccattgtt cagggttact    2040
   ctggaagaat ctccagacga gagcttcttc aatgtctctg cacaacaatg ctgggaaatg    2100
   gtgatgcgga gagttaaaga tacatcaaca agtcttggtc ttcctatttt accacagttt    2160
   gagagtatca acgggcttca aatgtttggt ttcctctcac cctctatagt tcaggccatt    2220
   gaagctcttg acccaaacca tcgactagtt gagtactgga accacaagaa ccaaacttca    2280
   tcagactcaa aagatcactt catatcatca aactgttccg caagtttaac caaaggaaaa    2340
   cttttcggag tagatttgat gtaa                                            2364
<210> 34
   <211> 787
   <212> PRT
   <213> Arabidopsis thaliana
<400> 34
   Met Glu Asn Pro Pro Leu Glu Ser Glu Ile Lys Glu Asp Met Ser Leu
   1               5                   10                  15
   Lys Asn His Pro Pro Asp Lys Asp Lys Asp Lys Asp Thr Ile Met Glu
               20                  25                  30
   Gln Pro Ser Ser Pro Arg His Arg Lys Val Val Ala Arg Trp Leu Pro
           35                  40                  45
   Asp Glu Ala Gln Arg Pro Ile Ile Asn Asp Ala Pro Val Phe Thr Pro
       50                  55                  60
   Ser Leu Glu Glu Phe Val Asp Pro Leu Ala Tyr Ile Glu Lys Ile Arg
   65                  70                  75                  80
   Pro Leu Ala Glu Pro Tyr Gly Ile Cys Arg Ile Ile Pro Pro Ser Thr
                   85                  90                  95
   Trp Lys Pro Pro Cys Arg Leu Lys Glu Lys Ser Ile Trp Glu Gln Thr
               100                 105                 110
   Lys Phe Pro Thr Arg Ile Gln Thr Val Asp Leu Leu Gln Asn Arg Glu
           115                 120                 125
   Pro Met Lys Lys Lys Pro Lys Ser Arg Lys Arg Lys Arg Arg Arg Asn
       130                 135                 140
   Ser Arg Met Gly Ser Ser Lys Arg Arg Ser Gly Ser Ser Pro Ala Glu
   145                 150                 155                 160
   Ser Thr Ser Ser Pro Glu Ala Glu Glu Lys Phe Gly Phe Asn Ser Gly
                   165                 170                 175
   Ser Asp Phe Thr Leu Asp Glu Phe Glu Lys Tyr Ala Leu His Phe Lys
               180                 185                 190
   Asp Ser Tyr Phe Glu Lys Lys Asp Ser Gly Gly Asp Ile Val Lys Trp
           195                 200                 205
   Thr Pro Ser Val Asp Asp Ile Glu Gly Glu Tyr Trp Arg Ile Val Glu
       210                 215                 220
   Gln Pro Thr Asp Glu Val Glu Val Tyr Tyr Gly Ala Asp Leu Glu Asn
   225                 230                 235                 240
   Gly Val Leu Gly Ser Gly Phe Tyr Lys Arg Ala Glu Lys Phe Thr Gly
                   245                 250                 255
   Ser Asp Met Glu Gln Tyr Thr Leu Ser Gly Trp Asn Leu Asn Asn Leu
               260                 265                 270
   Pro Arg Leu Pro Gly Ser Val Leu Ser Phe Glu Asp Cys Asp Ile Ser
           275                 280                 285
   Gly Val Leu Val Pro Trp Leu Tyr Val Gly Met Cys Phe Ser Ser Phe
       290                 295                 300
   Cys Trp His Val Glu Asp His His Leu Tyr Ser Leu Asn Tyr His His
   305                 310                 315                 320
   Phe Gly Glu Pro Lys Val Trp Tyr Gly Val Pro Gly Ser Asn Ala Thr
                   325                 330                 335
   Ala Leu Glu Lys Ala Met Arg Lys His Leu Pro Asp Leu Phe Glu Asp
               340                 345                 350
   Ser Leu Ile Tyr Tyr Met Ala Trp Ala Tyr His Ala Gly Phe Asn Cys
           355                 360                 365
   Gly Phe Asn Cys Ala Glu Ala Val Asn Val Ala Pro Val Asp Trp Leu
       370                 375                 380
   Ala His Gly Gln Asn Ala Val Glu Leu Tyr Ser Lys Glu Thr Arg Lys
   385                 390                 395                 400
   Thr Ser Leu Ser His Asp Lys Leu Leu Leu Gly Ala Ala Tyr Glu Ala
                   405                 410                 415
   Val Lys Ala Leu Trp Glu Leu Ser Ala Ser Glu Gly Lys Glu Asn Thr
               420                 425                 430
   Thr Asn Leu Arg Trp Lys Ser Phe Cys Gly Lys Asn Gly Thr Leu Thr
           435                 440                 445
   Asn Ala Ile Gln Ala Arg Leu Gln Met Glu Glu Gly Arg Ile Thr Ala
       450                 455                 460
   Leu Gly Arg Asp Ser Ser Ser Leu Lys Lys Met Glu Lys Asp Phe Asp
   465                 470                 475                 480
   Ser Asn Cys Glu Arg Glu Cys Phe Ser Cys Phe Tyr Asp Leu His Leu
                   485                 490                 495
   Ser Ala Ser Gly Cys Lys Cys Ser Pro Glu Glu Tyr Ala Cys Leu Lys
               500                 505                 510
   His Ala Asp Asp Leu Cys Ser Cys Asp Val Lys Asp Gly Phe Ile Leu
           515                 520                 525
   Leu Arg Tyr Thr Met Asp Glu Leu Ser Ser Leu Val Arg Ala Leu Glu
       530                 535                 540
   Gly Glu Ser Asp Asp Leu Lys Ile Trp Ala Ser Lys Val Leu Gly Ile
   545                 550                 555                 560
   Glu His Ser Asp Glu Asp Gln Thr Lys Thr Ser Ser Val Ile Ser Glu
                   565                 570                 575
   Glu Lys Lys Leu Lys Glu Gly Ser Phe Asp Leu Asn Ile Asp Leu Glu
               580                 585                 590
   Met Asp Tyr Gln Glu Asp Val Lys Glu Glu Ala Ser Thr Ser Gly Gly
           595                 600                 605
   Glu Leu Thr Ala Ser Glu Asn Leu Gly Val Ser Val Glu Pro Ile Asn
       610                 615                 620
   Leu Gly Phe Leu Ile Phe Gly Lys Leu Trp Cys Asn Lys Tyr Ala Ile
   625                 630                 635                 640
   Phe Pro Lys Gly Phe Arg Ser Arg Val Lys Phe Tyr Asn Val Leu Asp
                   645                 650                 655
   Pro Thr Arg Met Ser Asn Tyr Ile Ser Glu Val Leu Asp Ala Gly Leu
               660                 665                 670
   Met Gly Pro Leu Phe Arg Val Thr Leu Glu Glu Ser Pro Asp Glu Ser
           675                 680                 685
   Phe Phe Asn Val Ser Ala Gln Gln Cys Trp Glu Met Val Met Arg Arg
       690                 695                 700
   Val Lys Asp Thr Ser Thr Ser Leu Gly Leu Pro Ile Leu Pro Gln Phe
   705                 710                 715                 720
   Glu Ser Ile Asn Gly Leu Gln Met Phe Gly Phe Leu Ser Pro Ser Ile
                   725                 730                 735
   Val Gln Ala Ile Glu Ala Leu Asp Pro Asn His Arg Leu Val Glu Tyr
               740                 745                 750
   Trp Asn His Lys Asn Gln Thr Ser Ser Asp Ser Lys Asp His Phe Ile
           755                 760                 765
   Ser Ser Asn Cys Ser Ala Ser Leu Thr Lys Gly Lys Leu Phe Gly Val
       770                 775                 780
   Asp Leu Met
   785

<210> 35
   <211> 2652
   <212> DNA
   <213> Arabidopsis thaliana
<400> 35
   atggattctg gagttaaatt ggagcatatg aattgtttcc aattatctta tcaatattcg     60
   tggacgacta ggaagaagag aactctgaaa ccattcatgt caaaaggttc tagccctagt    120
   agtagtagtg atagtaggaa acggaagctt tcgagagctg aagatagcga tgattcagcg    180
   gttaaacgga atgcgaaacg aaggagaaag atatgcaaag tagaagaata ttatgaagat    240
   gatgattgca tattaagtga ttgggttcag aggaatactg cgaaaaggat tgataagcgc    300
   aacgaagaag ttgaagttat ggttaagata gagtcaggtg atgattgcac aataggtaag    360
   tggttttctg atgttagtag taaaaggaaa gataaacgac aagtagaagt tgatgaagat    420
   gaggaatggg aagaagaagt tacactgtgt tctaagatca aggcaacgtc gtcgcgaagc    480
   agaacccata gtttaagtgc taacagtcca gaaaatgtta cagatgttat tagtccttgt    540
   cgttcacggt ctccagcttc taatgtatca gattccattc agaaaaatga ctgcaccagt    600
   agtagaaaac agagtggccc aatttgtcat cagtgcttga aaggggaaag gataacactt    660
   ctcatttgta gtgaatgtga gaagacaatg ttttgtcttc aatgtataag gaaatggtat    720
   ccaaatctat ctgaggatga tgttgttgag aaatgtcctt tatgccgtca aaattgtaat    780
   tgtagcaaat gcttgcattt gaatggttta attgagacat cgaagaggga actcgcgaaa    840
   tctgaaagac gtcatcatct ccagtatttg attacgttga tgcttccttt tctgaataag    900
   ttatccatat tccagaagct agagattgaa tttgaggcaa ctgttcaagg aaaactgcct    960
   tctgaagttg aaataactgc ggctataagc tacactgatg aacgtgtata ctgtgatcat   1020
   tgtgcaactt caattgttga cttgcatcga agctgtccaa agtgctctta tgaactttgt   1080
   ttgaagtgct gtcaagagat tcgtgaagga tcgctttctg aacgccctga aatgaagttc   1140
   cattatgttg atagaggaca tcgatatatg catggtttag atgctgcgga accgagcttg   1200
   tcttccactt ttgaagacga agaagctaac ccatctgacg ctaagtggag ccttggtgaa   1260
   aatggaagca tcacttgtgc accagaaaag ctaggtggtt gtggtgaacg gatgctagag   1320
   cttaggcgga tcctaccact cacgtggatg tcagatttag agcacaaagc agagactttc   1380
   ttatcatcat acaatataag ccctagaatg ttgaactgta ggtgttcttc tttggagaca   1440
   gagctgacaa ggaaatcagc ttcgaggaca acatcaagtg acaactacct tttctgtcct   1500
   gaatctctcg gtgtcttgaa ggaagaagag cttcttcatt ttcaagagca ttgggcaaaa   1560
   ggcgaaccag taatcgttag gaacgctctt gacaacacac ctggtttaag ctgggagccg   1620
   atggttatgt ggcgggcttt atgcgaaaat gtgaattcaa catcaagctc tgagatgtcc   1680
   caagtcaaag caattgattg cttagctaat tgtgaggtgg agatcaatac tcgtcagttc   1740
   tttgaaggtt atagcaaagg aagaacatat gaaaatttct ggcctgagat gttgaagctt   1800
   aaggactggc ctccttctga taagtttgaa gatcttctac ctcgtcactg tgatgagttc   1860
   atatccgcgt tacctttcca ggaatatagt gatcctagaa ctggaattct taacattgca   1920
   acaaagcttc ctgaaggatt tatcaaacca gatttaggtc caaaaactta catcgcctat   1980
   gggattccag atgagcttgg tagaggcgat tccgtgacta agcttcactg cgatatgtca   2040
   gacgcggtga atattctaac gcatacggcc gaagtgaccc taagtcaaga acaaatatcc   2100
   tcagtcaaag cactgaaaca gaaacacaag ttacagaata aggtcgataa acagagtact   2160
   gaagactgta acgaaaagga agaagaagaa gaagaagaat tgaacatgcc agagatttcg   2220
   agcaatgaaa atgaagaaac gggcagtgct ctttgggaca tatttaggag agaagacgtt   2280
   cccaagttag aagagtatct aagaaagcat tgcaaagaat ttagacacac ttattgtagt   2340
   ccagttacaa aggtttatca tccgatacat gaccaatcat gctatttaac cttagagcac   2400
   aaaagaaaac tcaaggcgga atatgggatc gagccatgga catttgtgca aaagctaggt   2460
   gaagcggtgt ttattcccgc gggttgtcct catcaagttc ggaatctaaa gtcgtgcaca   2520
   aaagtcgcag ttgactttgt gtcaccagag aacatccatg agtgtctgcg tttgaccgaa   2580
   gagtttcgcc aacttcccaa aaaccacaag gccagagagg acaaactcga ggcaagtctt   2640
   ttatctcttt ga                                                       2652
<210> 36
   <211> 883
   <212> PRT
   <213> Arabidopsis thaliana
<400> 36
   Met Asp Ser Gly Val Lys Leu Glu His Met Asn Cys Phe Gln Leu Ser
   1               5                   10                  15
   Tyr Gln Tyr Ser Trp Thr Thr Arg Lys Lys Arg Thr Leu Lys Pro Phe
               20                  25                  30
   Met Ser Lys Gly Ser Ser Pro Ser Ser Ser Ser Asp Ser Arg Lys Arg
           35                  40                  45
   Lys Leu Ser Arg Ala Glu Asp Ser Asp Asp Ser Ala Val Lys Arg Asn
       50                  55                  60
   Ala Lys Arg Arg Arg Lys Ile Cys Lys Val Glu Glu Tyr Tyr Glu Asp
   65                  70                  75                  80
   Asp Asp Cys Ile Leu Ser Asp Trp Val Gln Arg Asn Thr Ala Lys Arg
                   85                  90                  95
   Ile Asp Lys Arg Asn Glu Glu Val Glu Val Met Val Lys Ile Glu Ser
               100                 105                 110
   Gly Asp Asp Cys Thr Ile Gly Lys Trp Phe Ser Asp Val Ser Ser Lys
           115                 120                 125
   Arg Lys Asp Lys Arg Gln Val Glu Val Asp Glu Asp Glu Glu Trp Glu
       130                 135                 140
   Glu Glu Val Thr Leu Cys Ser Lys Ile Lys Ala Thr Ser Ser Arg Ser
   145                 150                 155                 160
   Arg Thr His Ser Leu Ser Ala Asn Ser Pro Glu Asn Val Thr Asp Val
                   165                 170                 175
   Ile Ser Pro Cys Arg Ser Arg Ser Pro Ala Ser Asn Val Ser Asp Ser
               180                 185                 190
   Ile Gln Lys Asn Asp Cys Thr Ser Ser Arg Lys Gln Ser Gly Pro Ile
           195                 200                 205
   Cys His Gln Cys Leu Lys Gly Glu Arg Ile Thr Leu Leu Ile Cys Ser
       210                 215                 220
   Glu Cys Glu Lys Thr Met Phe Cys Leu Gln Cys Ile Arg Lys Trp Tyr
   225                 230                 235                 240
   Pro Asn Leu Ser Glu Asp Asp Val Val Glu Lys Cys Pro Leu Cys Arg
                   245                 250                 255
   Gln Asn Cys Asn Cys Ser Lys Cys Leu His Leu Asn Gly Leu Ile Glu
               260                 265                 270
   Thr Ser Lys Arg Glu Leu Ala Lys Ser Glu Arg Arg His His Leu Gln
           275                 280                 285
   Tyr Leu Ile Thr Leu Met Leu Pro Phe Leu Asn Lys Leu Ser Ile Phe
       290                 295                 300
   Gln Lys Leu Glu Ile Glu Phe Glu Ala Thr Val Gln Gly Lys Leu Pro
   305                 310                 315                 320
   Ser Glu Val Glu Ile Thr Ala Ala Ile Ser Tyr Thr Asp Glu Arg Val
                   325                 330                 335
   Tyr Cys Asp His Cys Ala Thr Ser Ile Val Asp Leu His Arg Ser Cys
               340                 345                 350
   Pro Lys Cys Ser Tyr Glu Leu Cys Leu Lys Cys Cys Gln Glu Ile Arg
           355                 360                 365
   Glu Gly Ser Leu Ser Glu Arg Pro Glu Met Lys Phe His Tyr Val Asp
       370                 375                 380
   Arg Gly His Arg Tyr Met His Gly Leu Asp Ala Ala Glu Pro Ser Leu
   385                 390                 395                 400
   Ser Ser Thr Phe Glu Asp Glu Glu Ala Asn Pro Ser Asp Ala Lys Trp
                   405                 410                 415
   Ser Leu Gly Glu Asn Gly Ser Ile Thr Cys Ala Pro Glu Lys Leu Gly
               420                 425                 430
   Gly Cys Gly Glu Arg Met Leu Glu Leu Arg Arg Ile Leu Pro Leu Thr
           435                 440                 445
   Trp Met Ser Asp Leu Glu His Lys Ala Glu Thr Phe Leu Ser Ser Tyr
       450                 455                 460
   Asn Ile Ser Pro Arg Met Leu Asn Cys Arg Cys Ser Ser Leu Glu Thr
   465                 470                 475                 480
   Glu Leu Thr Arg Lys Ser Ala Ser Arg Thr Thr Ser Ser Asp Asn Tyr
                   485                 490                 495
   Leu Phe Cys Pro Glu Ser Leu Gly Val Leu Lys Glu Glu Glu Leu Leu
               500                 505                 510
   His Phe Gln Glu His Trp Ala Lys Gly Glu Pro Val Ile Val Arg Asn
           515                 520                 525
   Ala Leu Asp Asn Thr Pro Gly Leu Ser Trp Glu Pro Met Val Met Trp
       530                 535                 540
   Arg Ala Leu Cys Glu Asn Val Asn Ser Thr Ser Ser Ser Glu Met Ser
   545                 550                 555                 560
   Gln Val Lys Ala Ile Asp Cys Leu Ala Asn Cys Glu Val Glu Ile Asn
                   565                 570                 575
   Thr Arg Gln Phe Phe Glu Gly Tyr Ser Lys Gly Arg Thr Tyr Glu Asn
               580                 585                 590
   Phe Trp Pro Glu Met Leu Lys Leu Lys Asp Trp Pro Pro Ser Asp Lys
           595                 600                 605
   Phe Glu Asp Leu Leu Pro Arg His Cys Asp Glu Phe Ile Ser Ala Leu
       610                 615                 620
   Pro Phe Gln Glu Tyr Ser Asp Pro Arg Thr Gly Ile Leu Asn Ile Ala
   625                 630                 635                 640
   Thr Lys Leu Pro Glu Gly Phe Ile Lys Pro Asp Leu Gly Pro Lys Thr
                   645                 650                 655
   Tyr Ile Ala Tyr Gly Ile Pro Asp Glu Leu Gly Arg Gly Asp Ser Val
               660                 665                 670
   Thr Lys Leu His Cys Asp Met Ser Asp Ala Val Asn Ile Leu Thr His
           675                 680                 685
   Thr Ala Glu Val Thr Leu Ser Gln Glu Gln Ile Ser Ser Val Lys Ala
       690                 695                 700
   Leu Lys Gln Lys His Lys Leu Gln Asn Lys Val Asp Lys Gln Ser Thr
   705                 710                 715                 720
   Glu Asp Cys Asn Glu Lys Glu Glu Glu Glu Glu Glu Glu Leu Asn Met
                   725                 730                 735
   Pro Glu Ile Ser Ser Asn Glu Asn Glu Glu Thr Gly Ser Ala Leu Trp
               740                 745                 750
   Asp Ile Phe Arg Arg Glu Asp Val Pro Lys Leu Glu Glu Tyr Leu Arg
           755                 760                 765
   Lys His Cys Lys Glu Phe Arg His Thr Tyr Cys Ser Pro Val Thr Lys
       770                 775                 780
   Val Tyr His Pro Ile His Asp Gln Ser Cys Tyr Leu Thr Leu Glu His
   785                 790                 795                 800
   Lys Arg Lys Leu Lys Ala Glu Tyr Gly Ile Glu Pro Trp Thr Phe Val
                   805                 810                 815
   Gln Lys Leu Gly Glu Ala Val Phe Ile Pro Ala Gly Cys Pro His Gln
               820                 825                 830
   Val Arg Asn Leu Lys Ser Cys Thr Lys Val Ala Val Asp Phe Val Ser
           835                 840                 845
   Pro Glu Asn Ile His Glu Cys Leu Arg Leu Thr Glu Glu Phe Arg Gln
       850                 855                 860
   Leu Pro Lys Asn His Lys Ala Arg Glu Asp Lys Leu Glu Ala Ser Leu
   865                 870                 875                 880
   Leu Ser Leu
<210> 37
   <211> 3351
   <212> DNA
   <213> Arabidopsis thaliana
<400> 37
   atgtatggta atgacttgga tacttctgtt tacgggagtg gttttcctcg aataggcgac      60
   caaagaccag aatcagttga ggcagatata tgggatgagt attgtggtag cccctggaat     120
   ctcaataaca tgcctaagtt gaaaggatct atgcttcagg ccattcgaca taacattaat     180
   ggtgttacag tgccttggct atatcttgga atgctcttct cttctttttg ttggcatttt      240
   gaggaccatt gtttttactc tgtcaattat ctacactggg gagaggcaaa atgttggtat     300
   ggtattccag gcagtgctgc tagtgctttt gaaaaggtca tgcgaaaaac cctacccgat     360
   ctctttgatg ctcagccaga tttgctcttt caactagtta ccatgttgag tccgactgtt     420
   ttacaagaaa ataaagtccc ggtctacaca gtattacagg agccaggaaa cttcgtgatc     480
   acatttccaa aatcctttca tgctggattc aattttggtc taaattgtgc agaggccgtc     540
   aactttgcta ctgctgattg gctaccttat ggtggttctg gggcggagct gtatcggctg     600
   taccggaaac cttcagtcat atctcatgaa gagcttctct gcgtggtagc taagggaaac     660
   tgctgcaata acgaagggtc aatacatttg aagaaagaat tgctcagaat atacagcaag     720
   gaaaaaacct ggagagagca gctctggaaa agtggtattt tgagatcttc tcctatgttt     780
   gtgcctgaat gcgctgattc tgtgggcatc gaagaggatc caacatgcat catatgccag     840
   cagtttcttc atctttctgc tatcgtctgc aattgcaggc catctgtttt tgcatgctta     900
   gagcactgga agcatctttg tgaatgtgaa cctacgaagt tgcgacttga atatcgttat     960
   acccttgccg agttagatat gatggtacaa gaagttgaaa agtttggtgg gtgcaaaaca    1020
   caagaaacca aaatctcaca acggccgagt tcaggcacca aacgatctat tgctttaaat    1080
   aaaaagcagg agggaatgca agtgagtcag gcacggccag cagacaaatg gcttcttaga    1140
   gcatcaaagg ttcttgacgc tgcgttttct agtgttgaat atgccaccct tttgaaggaa    1200
   tcagaacagt ttctttgggc tggatcagaa atggaccgtg tacgagatgt tacaaaaagt    1260
   ttgaacaaag caaagatatg ggctgaagct gttagtgact gtctttcaaa agtcgaaggt    1320
   gaagtcaacg atgattcaat gaaagttcac ttggaattta ttgatgagtt gctgagagtt    1380
   aatcctgttc cttgctttaa ttctggttat ctgaaactaa aggactatgc tgaagaagct    1440
   agaaaattgt ccgagaaaat cgattcagct ctctcgagta gcccaactat cacccagctg    1500
   gagctattgc attctgaagt ctccagatcg ccaatctccc taaaaaaaca cgagatcttg    1560
   tcaaagaaaa tctcttccgc aaagatgtta gctaaaaggg cgaaacgcta tctcacagac    1620
   gctaaacctc caggaattga aatggatgca cttttcaagc taaattcaga gatgttagag    1680
   cttcatgttc aacttccaga aacagaaggg attctggatt tggtaaagaa atcagaatca    1740
   gcccgtgata aaagtaacaa agttttgact ggttctttat ctctcgagaa tgtggaagag    1800
   ttgcttcatg aattcgatag tttcagcatt aacgttcctg agttgaatat cctgaggcag    1860
   tatcatgttg acactttgtc ttggatttca cgctttaatg atgtaatggt tgatgttcgt    1920
   gaaggcaagg accaacgaaa gctaattagt gacctcagtt cccttctacg ggatggagca    1980
   tccttaggca ttcaagttga aggactccct cttgttgaag ttgaattgaa gaaggcatct    2040
   tgccgggaaa aagctcgaac ggtttatact gcaagaaaat ctctggattt cattgagcaa    2100
   ctgctctcgg aagctgttat actacacatc gaagaggagg agatatttgt tgagatctcg    2160
   ggaattttgt caacagcacg gtgttgggag gaaagagcaa gcactatcct tgaaaatgaa    2220
   actcagatgt acgagcttaa agatctcgta aggatgtcag tcaacattga tgcggtttta    2280
   cctactctgc aaggcataga gaacacaatc tcgtcggctg aaacttggct tcagaaatct    2340
   gagccttttc tatcagccac ttcctctatg gcatcatctc catgttctat gcttgaactt    2400
   cctgtattaa aggatctagt tactcaggct aaattgctca atgttcagct tcaagagcca    2460
   cgaattcttg aaacattgtt gcttaactgc gagaggtggc agtgtgataa tcatcagctc    2520
   ttgcaagaaa ctgaagattt gttggacaat gcgaaaatag atgatggcac gcatagcaat    2580
   atccttccga agataatgga tttgataacc agagtggact ccgctaggag atctggtctg    2640
   gcccttggtc tcaatttcga tgaacttccc aaacttcgaa ctgcaagtct aaaactagga    2700
   tggtgttgta agaccatcac gttaagctct agctcaccta cctctgagtt gctagaagat    2760
   gttggaaagc cctcgttaca gcatattcag cagcacttaa aagagggaca aacacttgaa    2820
   atattacctg aagagtatta cttaggcaag agactcatgg agttaaaaga cactggactg    2880
   gagtgggcaa aacgagctag aaaggtggta acagactcag gtgctcttgc cttggaagat    2940
   gttttcgagc ttatttctga gggtgaaaac ttacctgttc atgcagagca ggaacttcag    3000
   tctttacgag ctcggagtat gttacactgc atttgtctaa agccatacaa ctcaagatcc    3060
   atggtttctt gtagtcaatg tggcgaatgg tatcacacct attgtttaaa gcttcattgg    3120
   cggcctaagg cttatgtctg ctccgcttgc tgtccactgg cagaaaccac tccacagatc    3180
   gatcccgcca gagcaacaga gccagagaga ccgtctctga accaaagacg gacaagaatg    3240
   gtcgcgactg atgcagcagt taatgactta aagtggaaaa cccgaaaaca catcaaacgg    3300
   acaactaaac ggagtcctca ggttcatatt cttccctggt ttttcactta a             3351
<210> 38
   <211> 1116
   <212> PRT
   <213> Arabidopsis thaliana
<400> 38
   Met Tyr Gly Asn Asp Leu Asp Thr Ser Val Tyr Gly Ser Gly Phe Pro
   1               5                   10                  15
   Arg Ile Gly Asp Gln Arg Pro Glu Ser Val Glu Ala Asp Ile Trp Asp
               20                  25                  30
   Glu Tyr Cys Gly Ser Pro Trp Asn Leu Asn Asn Met Pro Lys Leu Lys
           35                  40                  45
   Gly Ser Met Leu Gln Ala Ile Arg His Asn Ile Asn Gly Val Thr Val
       50                  55                  60
   Pro Trp Leu Tyr Leu Gly Met Leu Phe Ser Ser Phe Cys Trp His Phe
   65                  70                  75                  80
   Glu Asp His Cys Phe Tyr Ser Val Asn Tyr Leu His Trp Gly Glu Ala
                   85                  90                  95
   Lys Cys Trp Tyr Gly Ile Pro Gly Ser Ala Ala Ser Ala Phe Glu Lys
               100                 105                 110
   Val Met Arg Lys Thr Leu Pro Asp Leu Phe Asp Ala Gln Pro Asp Leu
           115                 120                 125
   Leu Phe Gln Leu Val Thr Met Leu Ser Pro Thr Val Leu Gln Glu Asn
       130                 135                 140
   Lys Val Pro Val Tyr Thr Val Leu Gln Glu Pro Gly Asn Phe Val Ile
   145                 150                 155                 160
   Thr Phe Pro Lys Ser Phe His Ala Gly Phe Asn Phe Gly Leu Asn Cys
                   165                 170                 175
   Ala Glu Ala Val Asn Phe Ala Thr Ala Asp Trp Leu Pro Tyr Gly Gly
               180                 185                 190
   Ser Gly Ala Glu Leu Tyr Arg Leu Tyr Arg Lys Pro Ser Val Ile Ser
           195                 200                 205
   His Glu Glu Leu Leu Cys Val Val Ala Lys Gly Asn Cys Cys Asn Asn
       210                 215                 220
   Glu Gly Ser Ile His Leu Lys Lys Glu Leu Leu Arg Ile Tyr Ser Lys
   225                 230                 235                 240
   Glu Lys Thr Trp Arg Glu Gln Leu Trp Lys Ser Gly Ile Leu Arg Ser
                   245                 250                 255
   Ser Pro Met Phe Val Pro Glu Cys Ala Asp Ser Val Gly Ile Glu Glu
               260                 265                 270
   Asp Pro Thr Cys Ile Ile Cys Gln Gln Phe Leu His Leu Ser Ala Ile
           275                 280                 285
   Val Cys Asn Cys Arg Pro Ser Val Phe Ala Cys Leu Glu His Trp Lys
       290                 295                 300
   His Leu Cys Glu Cys Glu Pro Thr Lys Leu Arg Leu Glu Tyr Arg Tyr
   305                 310                 315                 320
   Thr Leu Ala Glu Leu Asp Met Met Val Gln Glu Val Glu Lys Phe Gly
                   325                 330                 335
   Gly Cys Lys Thr Gln Glu Thr Lys Ile Ser Gln Arg Pro Ser Ser Gly
               340                 345                 350
   Thr Lys Arg Ser Ile Ala Leu Asn Lys Lys Gln Glu Gly Met Gln Val
           355                 360                 365
   Ser Gln Ala Arg Pro Ala Asp Lys Trp Leu Leu Arg Ala Ser Lys Val
       370                 375                 380
   Leu Asp Ala Ala Phe Ser Ser Val Glu Tyr Ala Thr Leu Leu Lys Glu
   385                 390                 395                 400
   Ser Glu Gln Phe Leu Trp Ala Gly Ser Glu Met Asp Arg Val Arg Asp
                   405                 410                 415
   Val Thr Lys Ser Leu Asn Lys Ala Lys Ile Trp Ala Glu Ala Val Ser
               420                 425                 430
   Asp Cys Leu Ser Lys Val Glu Gly Glu Val Asn Asp Asp Ser Met Lys
           435                 440                 445
   Val His Leu Glu Phe Ile Asp Glu Leu Leu Arg Val Asn Pro Val Pro
       450                 455                 460
   Cys Phe Asn Ser Gly Tyr Leu Lys Leu Lys Asp Tyr Ala Glu Glu Ala
   465                 470                 475                 480
   Arg Lys Leu Ser Glu Lys Ile Asp Ser Ala Leu Ser Ser Ser Pro Thr
                   485                 490                 495
   Ile Thr Gln Leu Glu Leu Leu His Ser Glu Val Ser Arg Ser Pro Ile
               500                 505                 510
   Ser Leu Lys Lys His Glu Ile Leu Ser Lys Lys Ile Ser Ser Ala Lys
           515                 520                 525
   Met Leu Ala Lys Arg Ala Lys Arg Tyr Leu Thr Asp Ala Lys Pro Pro
       530                 535                 540
   Gly Ile Glu Met Asp Ala Leu Phe Lys Leu Asn Ser Glu Met Leu Glu
   545                 550                 555                 560
   Leu His Val Gln Leu Pro Glu Thr Glu Gly Ile Leu Asp Leu Val Lys
                   565                 570                 575
   Lys Ser Glu Ser Ala Arg Asp Lys Ser Asn Lys Val Leu Thr Gly Ser
               580                 585                 590
   Leu Ser Leu Glu Asn Val Glu Glu Leu Leu His Glu Phe Asp Ser Phe
           595                 600                 605
   Ser Ile Asn Val Pro Glu Leu Asn Ile Leu Arg Gln Tyr His Val Asp
       610                 615                 620
   Thr Leu Ser Trp Ile Ser Arg Phe Asn Asp Val Met Val Asp Val Arg
   625                 630                 635                 640
   Glu Gly Lys Asp Gln Arg Lys Leu Ile Ser Asp Leu Ser Ser Leu Leu
                   645                 650                 655
   Arg Asp Gly Ala Ser Leu Gly Ile Gln Val Glu Gly Leu Pro Leu Val
               660                 665                 670
   Glu Val Glu Leu Lys Lys Ala Ser Cys Arg Glu Lys Ala Arg Thr Val
           675                 680                 685
   Tyr Thr Ala Arg Lys Ser Leu Asp Phe Ile Glu Gln Leu Leu Ser Glu
       690                 695                 700
   Ala Val Ile Leu His Ile Glu Glu Glu Glu Ile Phe Val Glu Ile Ser
   705                 710                 715                 720
   Gly Ile Leu Ser Thr Ala Arg Cys Trp Glu Glu Arg Ala Ser Thr Ile
                   725                 730                 735
   Leu Glu Asn Glu Thr Gln Met Tyr Glu Leu Lys Asp Leu Val Arg Met
               740                 745                 750
   Ser Val Asn Ile Asp Ala Val Leu Pro Thr Leu Gln Gly Ile Glu Asn
           755                 760                 765
   Thr Ile Ser Ser Ala Glu Thr Trp Leu Gln Lys Ser Glu Pro Phe Leu
       770                 775                 780
   Ser Ala Thr Ser Ser Met Ala Ser Ser Pro Cys Ser Met Leu Glu Leu
   785                 790                 795                 800
   Pro Val Leu Lys Asp Leu Val Thr Gln Ala Lys Leu Leu Asn Val Gln
                   805                 810                 815
   Leu Gln Glu Pro Arg Ile Leu Glu Thr Leu Leu Leu Asn Cys Glu Arg
               820                 825                 830
   Trp Gln Cys Asp Asn His Gln Leu Leu Gln Glu Thr Glu Asp Leu Leu
           835                 840                 845
   Asp Asn Ala Lys Ile Asp Asp Gly Thr His Ser Asn Ile Leu Pro Lys
       850                 855                 860
   Ile Met Asp Leu Ile Thr Arg Val Asp Ser Ala Arg Arg Ser Gly Leu
   865                 870                 875                 880
   Ala Leu Gly Leu Asn Phe Asp Glu Leu Pro Lys Leu Arg Thr Ala Ser
                   885                 890                 895
   Leu Lys Leu Gly Trp Cys Cys Lys Thr Ile Thr Leu Ser Ser Ser Ser
               900                 905                 910
   Pro Thr Ser Glu Leu Leu Glu Asp Val Gly Lys Pro Ser Leu Gln His
           915                 920                 925
   Ile Gln Gln His Leu Lys Glu Gly Gln Thr Leu Glu Ile Leu Pro Glu
       930                 935                 940
   Glu Tyr Tyr Leu Gly Lys Arg Leu Met Glu Leu Lys Asp Thr Gly Leu
   945                 950                 955                 960
   Glu Trp Ala Lys Arg Ala Arg Lys Val Val Thr Asp Ser Gly Ala Leu
                   965                 970                 975
   Ala Leu Glu Asp Val Phe Glu Leu Ile Ser Glu Gly Glu Asn Leu Pro
               980                 985                 990
   Val His Ala Glu Gln Glu Leu Gln  Ser Leu Arg Ala Arg  Ser Met Leu
           995                 1000                1005
   His Cys  Ile Cys Leu Lys Pro  Tyr Asn Ser Arg Ser  Met Val Ser
       1010                1015                1020
   Cys Ser  Gln Cys Gly Glu Trp  Tyr His Thr Tyr Cys  Leu Lys Leu
       1025                1030                1035
   His Trp  Arg Pro Lys Ala Tyr  Val Cys Ser Ala Cys  Cys Pro Leu
       1040                1045                1050
   Ala Glu  Thr Thr Pro Gln Ile  Asp Pro Ala Arg Ala  Thr Glu Pro
       1055                1060                1065
   Glu Arg  Pro Ser Leu Asn Gln  Arg Arg Thr Arg Met  Val Ala Thr
       1070                1075                1080
   Asp Ala  Ala Val Asn Asp Leu  Lys Trp Lys Thr Arg  Lys His Ile
       1085                1090                1095
   Lys Arg  Thr Thr Lys Arg Ser  Pro Gln Val His Ile  Leu Pro Trp
       1100                1105                1110
   Phe Phe  Thr
       1115
<210> 39
   <211> 2751
   <212> DNA
   <213> Arabidopsis thaliana
<400> 39
   atgacgacgt taggacaaag agatcgccga ccagatgctc tcggtagtct cagtgttcta    60
   ccagatgaga ccatctgtgt tcttctcgaa taccttgctc cccgagatat tgctcacctc   120
   gcttgtgtca gcagtgtgat gtatattcta tgtaatgaag aaccattgtg gatgagtttg   180
   tgtctcagaa gagcaaaagg tcctcttgaa tacaaaggtt cttggaaaaa aacaacattg   240
   catctagaag gagttactca agaaaatgac gcatacagaa aatgttttca ttttgatgga   300
   ttcatgtcgt tgtacttgta taaacgattc tataggtgta atacatctct tgacgggttc   360
   tcttttgata atgggaatgt ggaacgtagg agaaatatct ccttggatga gttttctaag   420
   gaatacgacg ccaagaaacc tgttttgctt tctggccttg ctgattcttg gccagccagt   480
   aatacgtgga ctatcgacca actctcagag aaatatggtg aagtcccgtt tagaatatct   540
   cagaggagcc ccaacaaaat ttccatgaag ttcaaggatt acatcgcata tatgaaaact   600
   cagcgggatg aagatcctct atacgttttc gatgacaagt ttggagaagc tgctccagaa   660
   ttattgaaag actatagtgt gccccatttg tttcaagaag attggtttga gatcttagac     720
   aaggaaagtc gtcctccata cagatggctt atagttggtc cggagaggtc tggtgcatct     780
   tggcatgttg acccagctct taccagcgcc tggaacaccc tgctttgcgg tcggaaaagg     840
   tgggcattgt atccccctgg aaaagtgcct ctaggtgtta cagtccatgt caatgaagat     900
   gagggtgatg tcagcattga tacaccctca tctctgcagt ggtggctaga ctattatccc     960
   cttcttgcgg acgaagacaa accgattgag tgcacactac tacctggcga aacgatttat    1020
   gttccaagtg gttggtggca ctgtatcctt aatcttgaac caacagtggc tgttacccag    1080
   aattttgtga acaaagaaaa ctttgggttc gtgtgcttgg atatggcgcc tggttaccat    1140
   cacaaaggag tttgccgtgc tgggcttctt gctcttgatg atgaaaattc tgaagatttg    1200
   gaagaagaaa cacacgatga agaagataat actttgagct attcagacct taccaggaaa    1260
   gagaagagga cacggatgaa tggaggtgga gagactgaaa accgcgaaga ggatgtgaat    1320
   ggagtatcga agagatataa tatgtggaag aatggatttt catatgatat cgatttcctc    1380
   gcttcatttc ttgataaaga aagagatcat tacaatttcc catggtcaat ggggaactct    1440
   gtaggccaac gagaaatgag agcctggcta tccaagcttt gggttctgaa gcctgagatg    1500
   agagaactaa tatggaaggg agcatgcatt gctttaaatg ctgagaaatg gttgcgatgc    1560
   ctagaggaag tatgtacttt ccacaacttg ccgttggtta ccgaagatga aaagcttcca    1620
   gttggaactg gcagcaaccc tcttgagttt tatgacattc ttggccgggc tgattctcct    1680
   ttgaaaacac atattcctga agttctagca agcgggattc ttttcttcga aaaaggatct    1740
   tacaaagttg tcccttggga tggcaagaga atcccagata ttatctctag ctccagtttc    1800
   gattttgatg catccatgtt aaacagcgaa ttcccatttg gtatttggaa taaaacgcta    1860
   cgtgaacata aaaaccaggg gaagccagca cccgattctt ttggttcatt gagttcacat    1920
   gtttggccat atattataac caaaagatgc aaagggaaga tttttgccca actaagagat    1980
   gatctaacct ggaacgacgc tcagaacctg gctttctttc tcggacaaca actacgcaac    2040
   cttcatctac ttccatatcc accggtcaca cgtccggagt tgttgaatgt gaatgctgtt    2100
   catgaggagt tgaacattcc agcagaatgg aaagtttttg tcgatgctct gtgccaaaag    2160
   aagaaggacg tcactagtcg tctggaaaac tggggaaatc cgatacctcg ggctctgatg    2220
   accaagatag acgaatacat tcccgatgac ttttttgtag atttactcca cgtcttcaag    2280
   gagacaaatg gtggagatga aatcaagccc tgcacctgga tacactcaga cgtaatggat    2340
   gacaacattc acatggaacc atacgcagat gattcagttg atggtcaaca caactcatgg    2400
   cgtcccagtc atattcttga cttcagcgat ttaaccatag gagatcccat ctgcgacttg    2460
   ataccaatat acttggacgt ctttagagga gacgctgatc ttcttaagaa gcttctagaa    2520
   aattatggac ttccattaat cagaagtaga tcttcagaga acggaacaac aaaaacagca    2580
   gacagtacga ggaagaaagt attgtctcca tcctaccgta caatgtgtta ctgtatatta    2640
   catgaagaaa atgtgttggg ttcaatattc agtatttggg acgaacttcg gactgctgaa    2700
   tcatgggaac aagttgagca aactgtttgg agtctactta acacctacta a             2751
<210> 40
   <211> 916
   <212> PRT
   <213> Arabidopsis thaliana
<400> 40
   Met Thr Thr Leu Gly Gln Arg Asp Arg Arg Pro Asp Ala Leu Gly Ser
   1               5                   10                  15
   Leu Ser Val Leu Pro Asp Glu Thr Ile Cys Val Leu Leu Glu Tyr Leu
               20                  25                  30
   Ala Pro Arg Asp Ile Ala His Leu Ala Cys Val Ser Ser Val Met Tyr
           35                  40                  45
   Ile Leu Cys Asn Glu Glu Pro Leu Trp Met Ser Leu Cys Leu Arg Arg
       50                  55                  60
   Ala Lys Gly Pro Leu Glu Tyr Lys Gly Ser Trp Lys Lys Thr Thr Leu
   65                  70                  75                  80
   His Leu Glu Gly Val Thr Gln Glu Asn Asp Ala Tyr Arg Lys Cys Phe
                   85                  90                  95
   His Phe Asp Gly Phe Met Ser Leu Tyr Leu Tyr Lys Arg Phe Tyr Arg
   100 105 110
   Cys Asn Thr Ser Leu Asp Gly Phe Ser Phe Asp Asn Gly Asn Val Glu
   115 120 125
   Arg Arg Arg Asn Ile Ser Leu Asp Glu Phe Ser Lys Glu Tyr Asp Ala
   130 135 140
   Lys Lys Pro Val Leu Leu Ser Gly Leu Ala Asp Ser Trp Pro Ala Ser
   145 150 155 160
   Asn Thr Trp Thr Ile Asp Gln Leu Ser Glu Lys Tyr Gly Glu Val Pro
   165 170 175
   Phe Arg Ile Ser Gln Arg Ser Pro Asn Lys Ile Ser Met Lys Phe Lys
   180 185 190
   Asp Tyr Ile Ala Tyr Met Lys Thr Gln Arg Asp Glu Asp Pro Leu Tyr
   195 200 205
   Val Phe Asp Asp Lys Phe Gly Glu Ala Ala Pro Glu Leu Leu Lys Asp
   210 215 220
   Tyr Ser Val Pro His Leu Phe Gln Glu Asp Trp Phe Glu Ile Leu Asp
   225 230 235 240
   Lys Glu Ser Arg Pro Pro Tyr Arg Trp Leu Ile Val Gly Pro Glu Arg
   245 250 255
   Ser Gly Ala Ser Trp His Val Asp Pro Ala Leu Thr Ser Ala Trp Asn
   260 265 270
   Thr Leu Leu Cys Gly Arg Lys Arg Trp Ala Leu Tyr Pro Pro Gly Lys
   275 280 285
   Val Pro Leu Gly Val Thr Val His Val Asn Glu Asp Glu Gly Asp Val
   290 295 300
   Ser Ile Asp Thr Pro Ser Ser Leu Gln Trp Trp Leu Asp Tyr Tyr Pro
   305 310 315 320
   Leu Leu Ala Asp Glu Asp Lys Pro Ile Glu Cys Thr Leu Leu Pro Gly
   325 330 335
   Glu Thr Ile Tyr Val Pro Ser Gly Trp Trp His Cys Ile Leu Asn Leu
   340 345 350
   Glu Pro Thr Val Ala Val Thr Gln Asn Phe Val Asn Lys Glu Asn Phe
   355 360 365
   Gly Phe Val Cys Leu Asp Met Ala Pro Gly Tyr His His Lys Gly Val
   370 375 380
   Cys Arg Ala Gly Leu Leu Ala Leu Asp Asp Glu Asn Ser Glu Asp Leu
   385 390 395 400
   Glu Glu Glu Thr His Asp Glu Glu Asp Asn Thr Leu Ser Tyr Ser Asp
   405 410 415
   Leu Thr Arg Lys Glu Lys Arg Thr Arg Met Asn Gly Gly Gly Glu Thr
   420 425 430
   Glu Asn Arg Glu Glu Asp Val Asn Gly Val Ser Lys Arg Tyr Asn Met
   435 440 445
   Trp Lys Asn Gly Phe Ser Tyr Asp Ile Asp Phe Leu Ala Ser Phe Leu
   450 455 460
   Asp Lys Glu Arg Asp His Tyr Asn Phe Pro Trp Ser Met Gly Asn Ser
   465 470 475 480
   Val Gly Gln Arg Glu Met Arg Ala Trp Leu Ser Lys Leu Trp Val Leu
   485 490 495
   Lys Pro Glu Met Arg Glu Leu Ile Trp Lys Gly Ala Cys Ile Ala Leu
   500 505 510
   Asn Ala Glu Lys Trp Leu Arg Cys Leu Glu Glu Val Cys Thr Phe His
   515 520 525
   Asn Leu Pro Leu Val Thr Glu Asp Glu Lys Leu Pro Val Gly Thr Gly
       530                 535                 540
   Ser Asn Pro Leu Glu Phe Tyr Asp Ile Leu Gly Arg Ala Asp Ser Pro
   545                 550                 555                 560
   Leu Lys Thr His Ile Pro Glu Val Leu Ala Ser Gly Ile Leu Phe Phe
                   565                 570                 575
   Glu Lys Gly Ser Tyr Lys Val Val Pro Trp Asp Gly Lys Arg Ile Pro
               580                 585                 590
   Asp Ile Ile Ser Ser Ser Ser Phe Asp Phe Asp Ala Ser Met Leu Asn
           595                 600                 605
   Ser Glu Phe Pro Phe Gly Ile Trp Asn Lys Thr Leu Arg Glu His Lys
       610                 615                 620
   Asn Gln Gly Lys Pro Ala Pro Asp Ser Phe Gly Ser Leu Ser Ser His
   625                 630                 635                 640
   Val Trp Pro Tyr Ile Ile Thr Lys Arg Cys Lys Gly Lys Ile Phe Ala
                   645                 650                 655
   Gln Leu Arg Asp Asp Leu Thr Trp Asn Asp Ala Gln Asn Leu Ala Phe
               660                 665                 670
   Phe Leu Gly Gln Gln Leu Arg Asn Leu His Leu Leu Pro Tyr Pro Pro
           675                 680                 685
   Val Thr Arg Pro Glu Leu Leu Asn Val Asn Ala Val His Glu Glu Leu
       690                 695                 700
   Asn Ile Pro Ala Glu Trp Lys Val Phe Val Asp Ala Leu Cys Gln Lys
   705                 710                 715                 720
   Lys Lys Asp Val Thr Ser Arg Leu Glu Asn Trp Gly Asn Pro Ile Pro
                   725                 730                 735
   Arg Ala Leu Met Thr Lys Ile Asp Glu Tyr Ile Pro Asp Asp Phe Phe
               740                 745                 750
   Val Asp Leu Leu His Val Phe Lys Glu Thr Asn Gly Gly Asp Glu Ile
           755                 760                 765
   Lys Pro Cys Thr Trp Ile His Ser Asp Val Met Asp Asp Asn Ile His
       770                 775                 780
   Met Glu Pro Tyr Ala Asp Asp Ser Val Asp Gly Gln His Asn Ser Trp
   785                 790                 795                 800
   Arg Pro Ser His Ile Leu Asp Phe Ser Asp Leu Thr Ile Gly Asp Pro
                   805                 810                 815
   Ile Cys Asp Leu Ile Pro Ile Tyr Leu Asp Val Phe Arg Gly Asp Ala
               820                 825                 830
   Asp Leu Leu Lys Lys Leu Leu Glu Asn Tyr Gly Leu Pro Leu Ile Arg
           835                 840                 845
   Ser Arg Ser Ser Glu Asn Gly Thr Thr Lys Thr Ala Asp Ser Thr Arg
       850                 855                 860
   Lys Lys Val Leu Ser Pro Ser Tyr Arg Thr Met Cys Tyr Cys Ile Leu
   865                 870                 875                 880
   His Glu Glu Asn Val Leu Gly Ser Ile Phe Ser Ile Trp Asp Glu Leu
                   885                 890                 895
   Arg Thr Ala Glu Ser Trp Glu Gln Val Glu Gln Thr Val Trp Ser Leu
               900                 905                 910
   Leu Asn Thr Tyr
           915
<210> 41
   <211> 2421
   <212> DNA
   <213> Arabidopsis thaliana
<400> 41
   atggagcctt tcagtgctgc tcagaacaaa gaggacaagg acactagcgt agaacctcct      60
   cgtaggcgtt gtcatcgaaa gaacaagggc actaatgtag aacctcctag tagtccgtat     120
   catccgaagg ttcttgctag atgggatccg gctaatgaaa agaggccaga cattggtgaa     180
   gctccagtat tccaccctac atcagaggag tttgaagata cgcttgctta catagaaaaa     240
   atacgtccct tagctgaatc atttggtatt tgtcgaatcg tgccaccgtc gaattggtct     300
   cctccttgcc ggcttaaagg ggatagtata tggaagaata aaaatttccc aacccgtgtt     360
   cagtttgttg acctgcttca gaacagagga cccgtgaaga agaagacacc taaaggaagg     420
   aaacggaaac gaggaaaata ctcaagaacc gtcgccccca agaaacgaaa tggctctgtt     480
   tctaaatcag tttctacccc caaggcgacc gaagaagaga attttggttt cgaatctggt     540
   ccagaattta ctcttgagaa gtttgagaaa tacgctcaag atttcaaaga ctcctacttt     600
   gaaagaaaag acaatgttgg agatccatct gttgaggaga tcgaaggaga gtactggaga     660
   atcattgaga aagaaacaaa tgaagttaag gtattgtatg ggacggattt ggagaatccc     720
   atacttggaa gcggtttctc caaaggggta aaaattccaa ccagaagaaa tgatatggat     780
   aaatacatat cttctggttg gaatttgaac aacctcgccc gtctgcaggg atctctacta     840
   tcctttgagg attgtgagat ctcaggggtt caggtgccat ggctctatgt gggcatgtgc     900
   ttttcaacgt tttgttggca tgttgaggac aatcatctat attcactgaa ctatcatcac     960
   tttggcgagc caaaagtatg gtacggagtt ccgggaagcc atgcaactgg gctagagaag    1020
   gcaatgagaa aacatctacc agatttgttc gatgaacaac ctgatctact tcatgaactg    1080
   gttactcagt tttctccaac gattttgaaa aatgagggag tcccggttta tcgagctgtt    1140
   cagaatgcag gggagtatgt tctaacattc cctagagcat atcattcggg tttcaactgc    1200
   ggattcaact gtgcagaagc ggtgaatgtg gctccggttg attggctggc tcatggacag    1260
   aatgctgtgg aaatatatag ccaagagact cggaaaacgt ctctatctca tgacaaaatt    1320
   ctccttggag cagcttttga agctgttaag tccctctcag cacatgggga agataataca    1380
   aaaaggttca gctggaagag attctgtggg aaggacggta tcataactaa ggccatcgag    1440
   gcacggttac ggatggaaga aaaaagaatt gaggctcttg gaaatggttt cagcttggta    1500
   aagatggata aagactttga ttcaaactgt gaaagggaat gcatctcttg ctttagtgac    1560
   ttgcatctct ctgctactgg ctgcaagaat tgctcatctc tcgaagaata tggttgcacg    1620
   aaacacgata tttgttcatg tgaagggaaa gaccgtttca tttttcttcg ttacaccata    1680
   gatgagttaa gctcattggt aagagcattg gaaggtgaat cagatgatct aaaagcttgg    1740
   ctttccaaag tcatggaagg ttgttcagag actcagaagg gagaatctag tgggattatc    1800
   gttaaggaaa agcaggtaca agaagaatgt tttgatctga atggtgagtg taataaatct    1860
   tctgagatat gtgaggatgc atccatcatg gacttagctg cttatcatgt tgaacctata    1920
   aatcttgggt tcttggtcgt gggaaagctt tggtgtaaca agcatgctat atttccaaaa    1980
   gggtttaaga gtcgtgttaa gttctataac gtgcaagatc caatgagaat aagctattac    2040
   gtctctgaga ttgtagatgc aggacttctg ggtccactct tcaaggttac tttggaagaa    2100
   tctcaagacg agagcttctc ttatgcctca cctcagaagt gctgggaaat ggtgttgctt    2160
   agagttaagg aagagatcat gagacgcagc aaccaaaaac aagatgtcca tatgttggaa    2220
   agcatcgatg ggttaaaaat gtttggcttt cgctctccgt ttattgttca ggccactgag    2280
   gctcttgacc cgaaccatgg ccaagtggag tattggaacc ataagaatga gaaggattcg    2340
   ctggaaatga aagattgctt catgtcaaat tcttcccaga gcttaagcaa agcaagactt    2400
   tttggggttg atttgaattg a                                              2421
<210> 42
   <211> 806
   <212> PRT
   <213> Arabidopsis thaliana
<400> 42
<210> 43
   <211> 2127
   <212> DNA
   <213> Arabidopsis thaliana
<400> 43
   atgggcattg aaggtgtatc aacgtatttg aaatctggaa atatggacac aatttctgct      60
   ccgccaggtt ttgtatctca aacatcgttt gtgctgagga atgtaccccg agacaaagaa     120
   agtcccaggt ccgtgtcgag acaagagcag acaactggat tcggcacaga tgataaagat     180
   agttgcaata tgtttcttaa aagccgaccg tggatagtgc atggtcacac aattcccagc     240
   tcagaggcct taaggccgaa aaaaactgag gtacggagaa gacggcccct caaagtgtct     300
   gagaccaaag tccttgagga agctcctgtg ttcaacccaa ccgaagagga attcagggac     360
   acactctcat atatatcaag cttgcgtgac agagctgagc cgtatggaat ctgttgtgtt     420
   gttcctccac cttcatggaa acctccatgt cttctcaagg agaaacaaat atgggaggct     480
   tctacatttt tccctcaagt tcagctcttt ggaattcaga ccgagaaccg aaagattaaa     540
   aaggaggttg atgcagatag taatgatgct gcatctgaag gggttcagtt atgtagagta    600
   gaacgtggtc ctgggtatac tttgaagtcc tttaaaaact ttgcagatac atacaagaag     660
   agtcattttg gcatgaagga tgaggttttg ggttcagaga actcatctcc atcacttaaa     720
   ccaaatgagc taattgtggc agacattgaa aaagagtaca gacaaattgt tgagagtcca     780
   cttattgaaa ttggggtgct ttatggcaat gatttagaca ctgcaacgtt tggaagtgga     840
   tttcccttat ctgcaccatc tgaatctagc aaatattcat caggttggaa tctgaacagt     900
   acggctaagc ttcctggttc tcttctatca ttagaagact gcgaatcagt ttgtgttcct     960
   cgtctaagtg taggaatgtg cttgtcttcc cagttctgga aatctgagaa ggagagactc    1020
   tactctctct gctatttgca tgtgggcgct cctagagtgt ggtattccgt agcaggatgt    1080
   catcgatcaa agtttaaggc tgccatgaaa agcttcatcc ttgagatgtc aggagaacag    1140
   ccaaagaaga gtcataatcc tgtgatgatg atgtctccat atcaattgag cgtggagggt    1200
   ataccagtga cccgctgtgt ccagcaccct ggccagtatg ttattatatt tccagggtcg    1260
   tattactctg cctttgactg tggcttcaac tgtttggaga aagcaaactt tgctcccctt    1320
   gattggttgc cccatgggga tattgctgtt caggtgaatc aagagatgag taaaacgtcg    1380
   ctgatatcgt atgataagct attatttagt gctgcaaggg aagctgtaaa atgtcttaag    1440
   gaatatgggt tgtcgaagaa aaacacagca tgttacacga gatggaatga ttcttgtggg    1500
   acggatgggt tgttctccaa cataatcaag tcacggatca aactggagaa aaatagacgt    1560
   gaatttctta tcagttcact agaatcgcaa aggatggaca agagttatga tgctgtgaac    1620
   aaaagggaat gctgtgtatg tcttggggat ttgtatcttt ccgcggttaa ctgttcatgc    1680
   tctgctaatc gttactcgtg tctgaaccac atgaggaagc tctgtgcatg tccgtgtgac    1740
   agaaagagtt ttctctatag gtacactatg gatgagttga atcttctcgt tgaagccctg    1800
   gaaggtaaaa agctcagctc tatgttcaga tgggcaggca tagatcaaaa attctgtgct    1860
   tctccagcca ccacaagttc aaaacccgaa gaagacaaag gcaaggaaac agatgaggtt    1920
   acaccctgta atatcacgag gaaagatgtc gcagctggaa ctaaggacca gacaagagtg    1980
   aaggcgagat ctttggctga tatactgaat gttaaagatg gaaacaatga tgcaaaggag    2040
   actttagaat cttgttctaa gaaatcaaac aggccatgcg ataacgattc ctctgaggcc    2100
   aacgcgccaa agaaacagaa gcagtga                                        2127
<210> 44
   <211> 708
   <212> PRT
   <213> Arabidopsis thaliana
<400> 44
   Met Gly Ile Glu Gly Val Ser Thr Tyr Leu Lys Ser Gly Asn Met Asp
   1               5                   10                  15
   Thr Ile Ser Ala Pro Pro Gly Phe Val Ser Gln Thr Ser Phe Val Leu
               20                  25                  30
   Arg Asn Val Pro Arg Asp Lys Glu Ser Pro Arg Ser Val Ser Arg Gln
           35                  40                  45
   Glu Gln Thr Thr Gly Phe Gly Thr Asp Asp Lys Asp Ser Cys Asn Met
       50                  55                  60
   Phe Leu Lys Ser Arg Pro Trp Ile Val His Gly His Thr Ile Pro Ser
   65                  70                  75                  80
   Ser Glu Ala Leu Arg Pro Lys Lys Thr Glu Val Arg Arg Arg Arg Pro
                   85                  90                  95
   Leu Lys Val Ser Glu Thr Lys Val Leu Glu Glu Ala Pro Val Phe Asn
                   100                 105                 110
   Pro Thr Glu Glu Glu Phe Arg Asp Thr Leu Ser Tyr Ile Ser Ser Leu
           115                 120                 125
   Arg Asp Arg Ala Glu Pro Tyr Gly Ile Cys Cys Val Val Pro Pro Pro
       130                 135                 140
   Ser Trp Lys Pro Pro Cys Leu Leu Lys Glu Lys Gln Ile Trp Glu Ala
   145                 150                 155                 160
   Ser Thr Phe Phe Pro Gln Val Gln Leu Phe Gly Ile Gln Thr Glu Asn
                   165                 170                 175
   Arg Lys Ile Lys Lys Glu Val Asp Ala Asp Ser Asn Asp Ala Ala Ser
               180                 185                 190
   Glu Gly Val Gln Leu Cys Arg Val Glu Arg Gly Pro Gly Tyr Thr Leu
           195                 200                 205
   Lys Ser Phe Lys Asn Phe Ala Asp Thr Tyr Lys Lys Ser His Phe Gly
       210                 215                 220
   Met Lys Asp Glu Val Leu Gly Ser Glu Asn Ser Ser Pro Ser Leu Lys
   225                 230                 235                 240
   Pro Asn Glu Leu Ile Val Ala Asp Ile Glu Lys Glu Tyr Arg Gln Ile
                   245                 250                 255
   Val Glu Ser Pro Leu Ile Glu Ile Gly Val Leu Tyr Gly Asn Asp Leu
               260                 265                 270
   Asp Thr Ala Thr Phe Gly Ser Gly Phe Pro Leu Ser Ala Pro Ser Glu
           275                 280                 285
   Ser Ser Lys Tyr Ser Ser Gly Trp Asn Leu Asn Ser Thr Ala Lys Leu
       290                 295                 300
   Pro Gly Ser Leu Leu Ser Leu Glu Asp Cys Glu Ser Val Cys Val Pro
   305                 310                 315                 320
   Arg Leu Ser Val Gly Met Cys Leu Ser Ser Gln Phe Trp Lys Ser Glu
                   325                 330                 335
   Lys Glu Arg Leu Tyr Ser Leu Cys Tyr Leu His Val Gly Ala Pro Arg
               340                 345                 350
   Val Trp Tyr Ser Val Ala Gly Cys His Arg Ser Lys Phe Lys Ala Ala
           355                 360                 365
   Met Lys Ser Phe Ile Leu Glu Met Ser Gly Glu Gln Pro Lys Lys Ser
       370                 375                 380
   His Asn Pro Val Met Met Met Ser Pro Tyr Gln Leu Ser Val Glu Gly
   385                 390                 395                 400
   Ile Pro Val Thr Arg Cys Val Gln His Pro Gly Gln Tyr Val Ile Ile
                   405                 410                 415
   Phe Pro Gly Ser Tyr Tyr Ser Ala Phe Asp Cys Gly Phe Asn Cys Leu
               420                 425                 430
   Glu Lys Ala Asn Phe Ala Pro Leu Asp Trp Leu Pro His Gly Asp Ile
           435                 440                 445
   Ala Val Gln Val Asn Gln Glu Met Ser Lys Thr Ser Leu Ile Ser Tyr
       450                 455                 460
   Asp Lys Leu Leu Phe Ser Ala Ala Arg Glu Ala Val Lys Cys Leu Lys
   465                 470                 475                 480
   Glu Tyr Gly Leu Ser Lys Lys Asn Thr Ala Cys Tyr Thr Arg Trp Asn
                   485                 490                 495
   Asp Ser Cys Gly Thr Asp Gly Leu Phe Ser Asn Ile Ile Lys Ser Arg
               500                 505                 510
   Ile Lys Leu Glu Lys Asn Arg Arg Glu Phe Leu Ile Ser Ser Leu Glu
           515                 520                 525
   Ser Gln Arg Met Asp Lys Ser Tyr Asp Ala Val Asn Lys Arg Glu Cys
       530                     535                     540
   Cys Val Cys Leu Gly Asp Leu Tyr Leu Ser Ala Val Asn Cys Ser Cys
   545                 550                 555                 560
   Ser Ala Asn Arg Tyr Ser Cys Leu Asn His Met Arg Lys Leu Cys Ala
                   565                 570                 575
   Cys Pro Cys Asp Arg Lys Ser Phe Leu Tyr Arg Tyr Thr Met Asp Glu
               580                 585                 590
   Leu Asn Leu Leu Val Glu Ala Leu Glu Gly Lys Lys Leu Ser Ser Met
           595                 600                 605
   Phe Arg Trp Ala Gly Ile Asp Gln Lys Phe Cys Ala Ser Pro Ala Thr
       610                 615                 620
   Thr Ser Ser Lys Pro Glu Glu Asp Lys Gly Lys Glu Thr Asp Glu Val
   625                 630                 635                 640
   Thr Pro Cys Asn Ile Thr Arg Lys Asp Val Ala Ala Gly Thr Lys Asp
                   645                 650                 655
   Gln Thr Arg Val Lys Ala Arg Ser Leu Ala Asp Ile Leu Asn Val Lys
               660                 665                 670
   Asp Gly Asn Asn Asp Ala Lys Glu Thr Leu Glu Ser Cys Ser Lys Lys
           675                 680                 685
   Ser Asn Arg Pro Cys Asp Asn Asp Ser Ser Glu Ala Asn Ala Pro Lys
       690                 695                 700
   Lys Gln Lys Gln
   705
<210> 45
   <211> 3084
   <212> DNA
   <213> Arabidopsis thaliana
<400> 45
   atggattctg tggaggaaga aggtgttgtt agggttgaag aagagaacgg acgcggtggt      60
   ctccgtaggc atcggagggt ttctacgaag ctggcgaatt acgtagatcc tccgactgac     120
   gacgaagaag atggaggacc caagcgaaag ggcaaaaggg gtggaaatag ggctccgaaa     180
   aagacaccga agaaagacga ggagatgcaa aagaacgaaa ttgatgaagc aaaccgagtc     240
   actggtctgg tgaaggaaaa aagagccgcg actaagattc tgaatcgcaa agactccatt     300
   attgaagtag gagaagcttc tggaagcatg cccaaggaag taaaggaaat taggattggg     360
   aaaaggaagg gagaaatcga tggtgagatt ccaactaagc cgggcaagaa gccaaagact     420
   acagtagacc cgagaatcat tggttacaga ccggacaata tgtgccatca atgtcaaaag     480
   agcgatagga ttgttgaacg gtgtcagacc tgcaatagta agcgttattg tcatccatgc     540
   ttggacactt ggtaccccct tatagcaaaa gaagatgttg ccaagaaatg catgttctgc     600
   tctagtactt gcaattgcag agcatgcttg cgtctagata ctaaattgaa agggataaat     660
   tcgaacctca tagtcagcga ggaagaaaag gtccaagcct ctaagtttat tctgcagagc     720
   cttctcccac atctgaaagg aataaatgat gaacaagttg ctgagaagga agttgaggcc     780
   aaaatatacg gactgaagtt tgaagaggtg aggccccaag acgctaaagc tttccctgat     840
   gaaagactat actgtgatat ctgcaagact tctatctatg atctccatag gaattgcaag     900
   tcctgtagtt ttgatatctg ccttagctgt tgccttgaga tccgcaatgg aaaggctctg     960
   gcatgtaagg aggatgtgtc ttggaattat attaaccgag gtttagagta tgaacatgga    1020
   caagaaggaa aagtgattga aaagccggcg aataagctag atgataagtt gaaagataag    1080
   ctggatggta agccggatga taagccgaag ggtaagccaa agggtaggcc aaagggtaag    1140
   ccggatgata agccgaaggg taaactgaag ggtaagcagg atgataaacc ggatgataag    1200
   ccagatgaga agccggttaa tacagaccat atgaagtacc cttctctgtg gaaagcaaat    1260
   gaagctggga tcattacttg ttgttgtggt gctggggagt tagtgctgaa acgactactt    1320
   ccggatggtt ggatatctga gttggtcaac agagttgaaa aaactgctga agctggcgag    1380
   cttttgaatt tacctgaaac ggttttggag cgatgccctt gttctaactc tgacagacat    1440
   attgacatag acagctgtaa tttgttaaaa gctgcttgtc gagaaggttc agaagacaat   1500
   tatctttact ctccaagtgt atgggatgtt caacaagatg atttgaagca ttttcagcac   1560
   cattgggtaa aaggcgagcc tgtgattgtg agaaatgtgc ttgaagctac atctggttta   1620
   agctgggaac caatggtaat gcatcgtgcc tgccgccaga taagccatgt ccagcatgga   1680
   tcacttaagg atgttgttgc tgttgattgt ttggacttct gtgaggtaaa agttaatctt   1740
   catgaatttt ttactggata cacggatggc cgatatgatc gaatgggttg gccactagtt   1800
   ctgaaactga aagattggcc tccagctaaa gtcttcaaag ataacttgcc acgtcatgct   1860
   gaggagttct tatgtagttt gcctttgaag cattacactc atccggttaa tggacctttg   1920
   aatctcgctg tcaagcttcc ccaaaattgc ttgaagccag acatgggacc aaagacgtac   1980
   gttgcttctg gatttgctca agaattaggc cgtggagatt ctgttactaa gctccactgc   2040
   gacatgtctg atgcggtgaa tattttgacg cacatatctg aagtgcccaa tatgcaacct   2100
   ggaattggaa atctgaaaaa gaagcatgcc gaacaagatc tcaaggagct gtatagctca   2160
   gtagctaaca aggaggaaat gatggagatt cttgaaaatt ccagacaaca agtccaaaat   2220
   gttgaaactg atgatggagc tctttgggac attttccgta gagaagatat tcctaaatta   2280
   gaaagttaca ttgagaaaca tcacaaggag ttcagacatc tctactgctg tcctgtgtct   2340
   caggttgttc atcctataca tgaccagaac ttctatctga cgcggtatca tataatgaag   2400
   ctgaaagaag aatatggcat tgaaccttgg accttcaatc agaagcttgg tgatgctgtt   2460
   ttgatacctg taggttgccc tcatcaagtt agaaatttga agtcctgcaa taaggtagcg   2520
   cttgacttcg tctcacctga aaatgtcagc gagtgtttac gcttgacaaa acagtatcgt   2580
   ctacttccac caaatcattt tgcaaaagaa gacaagttag gggttaagaa gatgatagtc   2640
   catgcagttg ataaagctct cagagactta agtggagaga agtctccaga acctgaagag   2700
   aagaaacaaa atatgagagg gccgaagaag gggcagccaa aggcagttgc caaggctctc   2760
   aaagacttat ctccaagtga aaagaagtct tcggaagcag ctgaagagga gatatcaaat   2820
   gggatagtca atgcaatcga taagggtctc aaagacttac ctccaagtga agagaagtct   2880
   tccgaagcca aagtggagat atcaaatggg atagtcagtg caatggataa ggatctcgaa   2940
   cacatatctt caagcgaaaa gaagtctacg gaagaggaag gggtgaaaag accaaatatt   3000
   gtgaggacat atgagcggag aaaaaagctg ggaagtgaag taaccaacgc atacattgac   3060
   agattagaaa tggagaagat gtag                                          3084
<210> 46
   <211> 1027
   <212> PRT
   <213> Arabidopsis thaliana
<400> 46
   Met Asp Ser Val Glu Glu Glu Gly Val Val Arg Val Glu Glu Glu Asn
   1               5                   10                  15
   Gly Arg Gly Gly Leu Arg Arg His Arg Arg Val Ser Thr Lys Leu Ala
               20                  25                  30
   Asn Tyr Val Asp Pro Pro Thr Asp Asp Glu Glu Asp Gly Gly Pro Lys
           35                  40                  45
   Arg Lys Gly Lys Arg Gly Gly Asn Arg Ala Pro Lys Lys Thr Pro Lys
       50                  55                  60
   Lys Asp Glu Glu Met Gln Lys Asn Glu Ile Asp Glu Ala Asn Arg Val
   65                  70                  75                  80
   Thr Gly Leu Val Lys Glu Lys Arg Ala Ala Thr Lys Ile Leu Asn Arg
                   85                  90                  95
   Lys Asp Ser Ile Ile Glu Val Gly Glu Ala Ser Gly Ser Met Pro Lys
               100                 105                 110
   Glu Val Lys Gly Ile Arg Ile Gly Lys Arg Lys Gly Glu Ile Asp Gly
           115                 120                 125
   Glu Ile Pro Thr Lys Pro Gly Lys Lys Pro Lys Thr Thr Val Asp Pro
       130                 135                 140
   Arg Ile Ile Gly Tyr Arg Pro Asp Asn Met Cys His Gln Cys Gln Lys
   145                 150                 155                 160
   Ser Asp Arg Ile Val Glu Arg Cys Gln Thr Cys Asn Ser Lys Arg Tyr
                   165                 170                 175
   Cys His Pro Cys Leu Asp Thr Trp Tyr Pro Leu Ile Ala Lys Glu Asp
               180                 185                 190
   Val Ala Lys Lys Cys Met Phe Cys Ser Ser Thr Cys Asn Cys Arg Ala
           195                 200                 205
   Cys Leu Arg Leu Asp Thr Lys Leu Lys Gly Ile Asn Ser Asn Leu Ile
       210                 215                 220
   Val Ser Glu Glu Glu Lys Val Gln Ala Ser Lys Phe Ile Leu Gln Ser
   225                 230                 235                 240
   Leu Leu Pro His Leu Lys Gly Ile Asn Asp Glu Gln Val Ala Glu Lys
                   245                 250                 255
   Glu Val Glu Ala Lys Ile Tyr Gly Leu Lys Phe Glu Glu Val Arg Pro
               260                 265                 270
   Gln Asp Ala Lys Ala Phe Pro Asp Glu Arg Leu Tyr Cys Asp Ile Cys
           275                 280                 285
   Lys Thr Ser Ile Tyr Asp Leu His Arg Asn Cys Lys Ser Cys Ser Phe
       290                 295                 300
   Asp Ile Cys Leu Ser Cys Cys Leu Glu Ile Arg Asn Gly Lys Ala Leu
   305                 310                 315                 320
   Ala Cys Lys Glu Asp Val Ser Trp Asn Tyr Ile Asn Arg Gly Leu Glu
                   325                 330                 335
   Tyr Glu His Gly Gln Glu Gly Lys Val Ile Glu Lys Pro Ala Asn Lys
               340                 345                 350
   Leu Asp Asp Lys Leu Lys Asp Lys Leu Asp Gly Lys Pro Asp Asp Lys
           355                 360                 365
   Pro Lys Gly Lys Pro Lys Gly Arg Pro Lys Gly Lys Pro Asp Asp Lys
       370                 375                 380
   Pro Lys Gly Lys Leu Lys Gly Lys Gln Asp Asp Lys Pro Asp Asp Lys
   385                 390                 395                 400
   Pro Asp Glu Lys Pro Val Asn Thr Asp His Met Lys Tyr Pro Ser Leu
                   405                 410                 415
   Trp Lys Ala Asn Glu Ala Gly Ile Ile Thr Cys Cys Cys Gly Ala Gly
               420                 425                 430
   Glu Leu Val Leu Lys Arg Leu Leu Pro Asp Gly Trp Ile Ser Glu Leu
           435                 440                 445
   Val Asn Arg Val Glu Lys Thr Ala Glu Ala Gly Glu Leu Leu Asn Leu
       450                 455                 460
   Pro Glu Thr Val Leu Glu Arg Cys Pro Cys Ser Asn Ser Asp Arg His
   465                 470                 475                 480
   Ile Asp Ile Asp Ser Cys Asn Leu Leu Lys Ala Ala Cys Arg Glu Gly
                   485                 490                 495
   Ser Glu Asp Asn Tyr Leu Tyr Ser Pro Ser Val Trp Asp Val Gln Gln
               500                 505                 510
   Asp Asp Leu Lys His Phe Gln His His Trp Val Lys Gly Glu Pro Val
           515                 520                 525
   Ile Val Arg Asn Val Leu Glu Ala Thr Ser Gly Leu Ser Trp Glu Pro
       530                 535                 540
   Met Val Met His Arg Ala Cys Arg Gln Ile Ser His Val Gln His Gly
   545                 550                 555                 560
   Ser Leu Lys Asp Val Val Ala Val Asp Cys Leu Asp Phe Cys Glu Val
                   565                 570                 575
   Lys Val Asn Leu His Glu Phe Phe Thr Gly Tyr Thr Asp Gly Arg Tyr
                   580                     585                     590
   Asp Arg Met Gly Trp Pro Leu Val Leu Lys Leu Lys Asp Trp Pro Pro
               595                     600                     605
   Ala Lys Val Phe Lys Asp Asn Leu Pro Arg His Ala Glu Glu Phe Leu
       610                     615                     620
   Cys Ser Leu Pro Leu Lys His Tyr Thr His Pro Val Asn Gly Pro Leu
   625                     630                     635                     640
   Asn Leu Ala Val Lys Leu Pro Gln Asn Cys Leu Lys Pro Asp Met Gly
                   645                     650                     655
   Pro Lys Thr Tyr Val Ala Ser Gly Phe Ala Gln Glu Leu Gly Arg Gly
                   660                     665                     670
   Asp Ser Val Thr Lys Leu His Cys Asp Met Ser Asp Ala Val Asn Ile
               675                     680                     685
   Leu Thr His Ile Ser Glu Val Pro Asn Met Gln Pro Gly Ile Gly Asn
       690                     695                     700
   Leu Lys Lys Lys His Ala Glu Gln Asp Leu Lys Glu Leu Tyr Ser Ser
   705                     710                     715                     720
   Val Ala Asn Lys Glu Glu Met Met Glu Ile Leu Glu Asn Ser Arg Gln
                   725                     730                     735
   Gln Val Gln Asn Val Glu Thr Asp Asp Gly Ala Leu Trp Asp Ile Phe
                   740                     745                     750
   Arg Arg Glu Asp Ile Pro Lys Leu Glu Ser Tyr Ile Glu Lys His His
               755                     760                     765
   Lys Glu Phe Arg His Leu Tyr Cys Cys Pro Val Ser Gln Val Val His
       770                     775                     780
   Pro Ile His Asp Gln Asn Phe Tyr Leu Thr Arg Tyr His Ile Met Lys
   785                     790                     795                     800
   Leu Lys Glu Glu Tyr Gly Ile Glu Pro Trp Thr Phe Asn Gln Lys Leu
                   805                     810                     815
   Gly Asp Ala Val Leu Ile Pro Val Gly Cys Pro His Gln Val Arg Asn
                   820                     825                     830
   Leu Lys Ser Cys Asn Lys Val Ala Leu Asp Phe Val Ser Pro Glu Asn
               835                     840                     845
   Val Ser Glu Cys Leu Arg Leu Thr Lys Gln Tyr Arg Leu Leu Pro Pro
       850                     855                     860
   Asn His Phe Ala Lys Glu Asp Lys Leu Gly Val Lys Lys Met Ile Val
   865                     870                     875                     880
   His Ala Val Asp Lys Ala Leu Arg Asp Leu Ser Gly Glu Lys Ser Pro
                   885                     890                     895
   Glu Pro Glu Glu Lys Lys Gln Asn Met Arg Gly Pro Lys Lys Gly Ala
                   900                     905                     910
   Ala Lys Ala Val Ala Lys Ala Leu Lys Asp Leu Ser Pro Ser Glu Lys
               915                     920                     925
   Lys Ser Ser Glu Ala Ala Glu Glu Glu Ile Ser Asn Gly Ile Val Asn
       930                     935                     940
   Ala Ile Asp Lys Gly Leu Lys Asp Leu Pro Pro Ser Glu Glu Lys Ser
   945                     950                     955                     960
   Ser Glu Ala Lys Val Glu Ile Ser Asn Gly Ile Val Ser Ala Met Asp
                   965                     970                     975
   Lys Asp Leu Glu His Ile Ser Ser Ser Glu Lys Lys Ser Thr Glu Glu
                   980                     985                     990
   Glu Gly Val Lys Arg Pro Asn Ile  Val Arg Thr Tyr Glu  Arg Arg Lys
               995                     1000                    1005
   Lys Leu  Gly Ser Glu Val Thr  Asn Ala Tyr Ile Asp  Arg Leu Glu
                  1010                     1015                     1020
   Met Glu  Lys Met
       1025
<210> 47
   <211> 4083
   <212> DNA
   <213> Arabidopsis thaliana
<400> 47
   atggcggttt cagagcagag tcaagatgtg tttccatggc ttaaatcgtt accggttgct     60
   ccagagttca gacctactct agcagagttt caagatccga tcgcttacat tttgaagatt    120
   gaagaagaag cttctagata tggaatctgt aaaattctgc ctccactgcc tcctccttcc    180
   aaaaaaacct cgatttctaa tctcaaccgt tctctggcgg caagagcggc ggcgagggtt    240
   cgtgacggcg gctttggcgc gtgtgattat gacggtggtc ccacattcgc cacgcgccag    300
   cagcagatcg ggttttgccc taggaaacag cgtccagtgc agagacctgt gtggcagagt    360
   ggagaggagt actcttttgg tgagttcgag tttaaagcta agaactttga gaagaattat    420
   ctcaagaaat gtggtaaaaa gagtcaactc tctgcccttg aaattgaaac actttactgg    480
   agagccactg tggataaacc cttctctgtt gagtatgcca atgacatgcc tggctcggct    540
   tttatccctc tgagtctggc tgctgcgagg aggagagagt ctggtggtga aggaggaaca    600
   gttggtgaga ccgcttggaa catgagggca atgtctagag ccgaaggatc attgcttaag    660
   ttcatgaagg aagagatccc tggagttaca tcaccaatgg tgtatgttgc tatgatgttt    720
   agttggtttg cttggcatgt ggaggaccat gaccttcata gtctcaatta cttgcatatg    780
   ggtgctggta agacttggta cggtgtgcca aaggatgctg ctctggcttt tgaggaggtt    840
   gttagggttc atggttacgg tgaagagctc aatcctcttg tgacattttc tactcttggt    900
   gagaagacaa ctgtgatgtc tcctgaagta tttgttaaag ccggaatacc gtgttgcagg    960
   ttagtgcaaa atcctggaga gtttgtcgtc acctttccgg gagcttatca ttcgggattt   1020
   agtcatggat ttaattttgg agaagcatct aacattgcca ctcccgaatg gttgagaatg   1080
   gctaaagatg ctgctatccg gcgagctgct ataaattacc ctccaatggt ttctcatctc   1140
   cagctacttt atgactttgt attggctcta ggttctagag tgccaacaag catcaatccc   1200
   aaaccacgga gttctagatt aaaagataag gcaagaagcg aaggagaaag attgaccaaa   1260
   aagctatttg tgcaaaacat tatccacaac aacgaattgc tttcttctct cggaaaagga   1320
   tccccagtgg cccttctccc acagagttcc tcagatatat cagtttgttc tgacctgcga   1380
   attggatccc atttgataac caaccaggaa aacccaatcc agttaaagtg tgaggactta   1440
   agttctgata gtgttgtggt tgatctcagt aacggtttaa aggatacagt ttcagtgaaa   1500
   gaaaaattta catctttatg tgaaaggagc agaaatcacc tagcaagcac ggagaaggac   1560
   actcaagaaa ctctgtctga tgctgaaagg aggaagaatg atgcagctgt tgcgctttcg   1620
   gatcaaaggc ttttctcttg tgttacatgt ggagtcttaa gctttgattg tgtagctatc   1680
   gttcaaccta aagaagcagc tgctagatat ctcatgtctg cagattgtag cttcttcaat   1740
   gattggacag ctgcttctgg atctgcaaat cttggtcagg ctgcaagatc acttcatcct   1800
   caaagcaaag agaagcatga tgtaaattac ttctacaatg ttcctgttca aactatggat   1860
   cattcagtga agactggcga tcaaaaaact tcaacaactt ccccgacaat agcgcataaa   1920
   gataatgatg ttcttgggat gttagcttca gcatatggag actcttctga ttccgaggaa   1980
   gaagatcaaa aaggcttagt tacccctagt tccaaagggg aaacaaaaac gtatgatcaa   2040
   gaaggttcag atggccatga ggaggctaga gatggtagaa cttctgattt taactgccag   2100
   agactaacca gcgaacagaa tgggttaagc aaaggcggaa aatcatcact tctggaaata   2160
   gctttaccat ttattccaag atctgatgac gattcatgtc ggttgcacgt gttttgtctt   2220
   gagcatgctg cggaagtgga acagcaactt cgtccttttg gggggattaa cttaatgtta   2280
   ctgtgccatc cagagtaccc caggatagag gctgaagcaa agatagttgc cgaagagctc   2340
   gtcatcaatc acgaatggaa tgatactgaa ttcaggaatg tgacccgaga ggatgaggaa   2400
   acgattcagg cagcgttgga taatgttgaa gctaagggtg ggaacagtga ttggaccgta   2460
   aaattgggtg ttaacctttc ttacagcgct attctcagtc gctctcctct gtacagtaag   2520
   cagatgccgt ataactccat catatacaag gcgttcggtc gcagctctcc agtagcgagc   2580
   tcaccctcga aacccaaagt ctctggtaaa agatcgtcca gacagaggaa atatgttgtt   2640
   ggaaaatggt gtggtaaggt ttggatgtca catcaggtgc atcccttttt gctggagcag   2700
   gacttagagg gggaagaatc tgaaagaagt tgtcatcttc gagttgctat ggatgaggat   2760
   gccactggaa agagatcgtt tcctaataat gtttccaggg attcgacaac aatgtttgga   2820
   agaaagtatt gtaggaagag aaagataaga gcaaaggcgg tgccacgcaa gaagcttact   2880
   tcttttaaga gggaagatgg agtttctgat gacacatcag aagatcattc ttataagcag   2940
   caatggaggg cttccgggaa tgaggaagag tcttattttg agacagggaa cacagcttct   3000
   ggtgattcat caaatcaaat gtctgatccg cacaagggaa ttatcagaca taaaggttat   3060
   aaagaatttg agtcagatga tgaggtttca gaccgttcac ttggggaaga gtatactgta   3120
   cgggcatgtg cagcttcaga gagctcaatg gagaatggct ctcagcattc aatgtatgac   3180
   catgatgatg atgatgatga tatcgacagg cagcctaggg ggattccaag gagccaacag   3240
   acaagagttt ttaggaatcc agtttcatat gagtcagaag ataatggcgt ttatcagcaa   3300
   agcggaagaa tatccataag taataggcaa gctaatcgaa tggttggtga atatgattca   3360
   gcagagaatt ctttggagga acgaggcttt tgcagtacag ggaaaaggca aaccaggtca   3420
   acagccaaac gaatagcaaa aaccaagaca gttcagagtt cgagagacac aaaaggtcgc   3480
   tttttgcaag aatttgcatc tggaaagaag aatgaagaat tggattcata catggaggga   3540
   cctagcacac ggcttagggt gagacatcag aagccgtcga gagggtcttt agaaacaaaa   3600
   ccaaagaaga ttggtaagaa gagaagtggt aatgcttcct tctccagagt tgcaactgaa   3660
   aaagatgtgg aggaaaaaga agaagaagaa gaagaagaag agaatgagga agaggaatgt   3720
   gcagcatacc aatgtaacat ggagggttgc acgatgagtt tcagttcgga aaaacagttg   3780
   atgttacaca aaagaaacat atgcccaatt aaaggctgtg gtaaaaactt cttctcacac   3840
   aagtatttgg ttcaacacca gcgtgttcac tcagacgacc gtcctctgaa atgtccatgg   3900
   aagggatgta agatgacgtt caagtgggct tggtctagaa ccgagcacat aagggttcac   3960
   acaggcgcta ggccttatgt ttgcgctgaa ccggattgtg gtcaaacatt caggtttgtc   4020
   tctgacttca gccggcataa aaggaagacc ggtcattcgg ttaagaagac caacaaaagg   4080
   tga                                                                 4083
<210> 48
   <211> 1360
   <212> PRT
   <213> Arabidopsis thaliana
<400> 48
   Met Ala Val Ser Glu Gln Ser Gln Asp Val Phe Pro Trp Leu Lys Ser
   1               5                   10                  15
   Leu Pro Val Ala Pro Glu Phe Arg Pro Thr Leu Ala Glu Phe Gln Asp
               20                  25                  30
   Pro Ile Ala Tyr Ile Leu Lys Ile Glu Glu Glu Ala Ser Arg Tyr Gly
           35                  40                  45
   Ile Cys Lys Ile Leu Pro Pro Leu Pro Pro Pro Ser Lys Lys Thr Ser
       50                  55                  60
   Ile Ser Asn Leu Asn Arg Ser Leu Ala Ala Arg Ala Ala Ala Arg Val
   65                  70                  75                  80
   Arg Asp Gly Gly Phe Gly Ala Cys Asp Tyr Asp Gly Gly Pro Thr Phe
                   85                  90                  95
   Ala Thr Arg Gln Gln Gln Ile Gly Phe Cys Pro Arg Lys Gln Arg Pro
               100                 105                 110
   Val Gln Arg Pro Val Trp Gln Ser Gly Glu Glu Tyr Ser Phe Gly Glu
           115                 120                 125
   Phe Glu Phe Lys Ala Lys Asn Phe Glu Lys Asn Tyr Leu Lys Lys Cys
       130                 135                 140
   Gly Lys Lys Ser Gln Leu Ser Ala Leu Glu Ile Glu Thr Leu Tyr Trp
   145                 150                 155                 160
   Arg Ala Thr Val Asp Lys Pro Phe Ser Val Glu Tyr Ala Asn Asp Met
                   165                 170                 175
   Pro Gly Ser Ala Phe Ile Pro Leu Ser Leu Ala Ala Ala Arg Arg Arg
               180                 185                 190
   Glu Ser Gly Gly Glu Gly Gly Thr Val Gly Glu Thr Ala Trp Asn Met
           195                 200                 205
   Arg Ala Met Ser Arg Ala Glu Gly Ser Leu Leu Lys Phe Met Lys Glu
       210                 215                 220
   Glu Ile Pro Gly Val Thr Ser Pro Met Val Tyr Val Ala Met Met Phe
   225                 230                 235                 240
   Ser Trp Phe Ala Trp His Val Glu Asp His Asp Leu His Ser Leu Asn
                   245                 250                 255
   Tyr Leu His Met Gly Ala Gly Lys Thr Trp Tyr Gly Val Pro Lys Asp
               260                 265                 270
   Ala Ala Leu Ala Phe Glu Glu Val Val Arg Val His Gly Tyr Gly Glu
           275                 280                 285
   Glu Leu Asn Pro Leu Val Thr Phe Ser Thr Leu Gly Glu Lys Thr Thr
       290                 295                 300
   Val Met Ser Pro Glu Val Phe Val Lys Ala Gly Ile Pro Cys Cys Arg
   305                 310                 315                 320
   Leu Val Gln Asn Pro Gly Glu Phe Val Val Thr Phe Pro Gly Ala Tyr
                   325                 330                 335
   His Ser Gly Phe Ser His Gly Phe Asn Phe Gly Glu Ala Ser Asn Ile
               340                 345                 350
   Ala Thr Pro Glu Trp Leu Arg Met Ala Lys Asp Ala Ala Ile Arg Arg
           355                 360                 365
   Ala Ala Ile Asn Tyr Pro Pro Met Val Ser His Leu Gln Leu Leu Tyr
       370                 375                 380
   Asp Phe Val Leu Ala Leu Gly Ser Arg Val Pro Thr Ser Ile Asn Pro
   385                 390                 395                 400
   Lys Pro Arg Ser Ser Arg Leu Lys Asp Lys Ala Arg Ser Glu Gly Glu
                   405                 410                 415
   Arg Leu Thr Lys Lys Leu Phe Val Gln Asn Ile Ile His Asn Asn Glu
               420                 425                 430
   Leu Leu Ser Ser Leu Gly Lys Gly Ser Pro Val Ala Leu Leu Pro Gln
           435                 440                 445
   Ser Ser Ser Asp Ile Ser Val Cys Ser Asp Leu Arg Ile Gly Ser His
       450                 455                 460
   Leu Ile Thr Asn Gln Glu Asn Pro Ile Gln Leu Lys Cys Glu Asp Leu
   465                 470                 475                 480
   Ser Ser Asp Ser Val Val Val Asp Leu Ser Asn Gly Leu Lys Asp Thr
                   485                 490                 495
   Val Ser Val Lys Glu Lys Phe Thr Ser Leu Cys Glu Arg Ser Arg Asn
               500                 505                 510
   His Leu Ala Ser Thr Glu Lys Asp Thr Gln Glu Thr Leu Ser Asp Ala
           515                 520                 525
   Glu Arg Arg Lys Asn Asp Ala Ala Val Ala Leu Ser Asp Gln Arg Leu
       530                 535                 540
   Phe Ser Cys Val Thr Cys Gly Val Leu Ser Phe Asp Cys Val Ala Ile
   545                 550                 555                 560
   Val Gln Pro Lys Glu Ala Ala Ala Arg Tyr Leu Met Ser Ala Asp Cys
                   565                 570                 575
   Ser Phe Phe Asn Asp Trp Thr Ala Ala Ser Gly Ser Ala Asn Leu Gly
               580                 585                 590
   Gln Ala Ala Arg Ser Leu His Pro Gln Ser Lys Glu Lys His Asp Val
           595                 600                 605
   Asn Tyr Phe Tyr Asn Val Pro Val Gln Thr Met Asp His Ser Val Lys
       610                 615                 620
   Thr Gly Asp Gln Lys Thr Ser Thr Thr Ser Pro Thr Ile Ala His Lys
   625                 630                 635                 640
   Asp Asn Asp Val Leu Gly Met Leu Ala Ser Ala Tyr Gly Asp Ser Ser
                   645                 650                 655
   Asp Ser Glu Glu Glu Asp Gln Lys Gly Leu Val Thr Pro Ser Ser Lys
               660                 665                 670
   Gly Glu Thr Lys Thr Tyr Asp Gln Glu Gly Ser Asp Gly His Glu Glu
           675                 680                 685
   Ala Arg Asp Gly Arg Thr Ser Asp Phe Asn Cys Gln Arg Leu Thr Ser
       690                 695                 700
   Glu Gln Asn Gly Leu Ser Lys Gly Gly Lys Ser Ser Leu Leu Glu Ile
   705                 710                 715                 720
   Ala Leu Pro Phe Ile Pro Arg Ser Asp Asp Asp Ser Cys Arg Leu His
                   725                 730                 735
   Val Phe Cys Leu Glu His Ala Ala Glu Val Glu Gln Gln Leu Arg Pro
               740                 745                 750
   Phe Gly Gly Ile Asn Leu Met Leu Leu Cys His Pro Glu Tyr Pro Arg
           755                 760                 765
   Ile Glu Ala Glu Ala Lys Ile Val Ala Glu Glu Leu Val Ile Asn His
       770                 775                 780
   Glu Trp Asn Asp Thr Glu Phe Arg Asn Val Thr Arg Glu Asp Glu Glu
   785                 790                 795                 800
   Thr Ile Gln Ala Ala Leu Asp Asn Val Glu Ala Lys Gly Gly Asn Ser
                   805                 810                 815
   Asp Trp Thr Val Lys Leu Gly Val Asn Leu Ser Tyr Ser Ala Ile Leu
               820                 825                 830
   Ser Arg Ser Pro Leu Tyr Ser Lys Gln Met Pro Tyr Asn Ser Ile Ile
           835                 840                 845
   Tyr Lys Ala Phe Gly Arg Ser Ser Pro Val Ala Ser Ser Pro Ser Lys
       850                 855                 860
   Pro Lys Val Ser Gly Lys Arg Ser Ser Arg Gln Arg Lys Tyr Val Val
   865                 870                 875                 880
   Gly Lys Trp Cys Gly Lys Val Trp Met Ser His Gln Val His Pro Phe
                   885                 890                 895
   Leu Leu Glu Gln Asp Leu Glu Gly Glu Glu Ser Glu Arg Ser Cys His
               900                 905                 910
   Leu Arg Val Ala Met Asp Glu Asp Ala Thr Gly Lys Arg Ser Phe Pro
           915                 920                 925
   Asn Asn Val Ser Arg Asp Ser Thr Thr Met Phe Gly Arg Lys Tyr Cys
       930                 935                 940
   Arg Lys Arg Lys Ile Arg Ala Lys Ala Val Pro Arg Lys Lys Leu Thr
   945                 950                 955                 960
   Ser Phe Lys Arg Glu Asp Gly Val Ser Asp Asp Thr Ser Glu Asp His
                   965                 970                 975
   Ser Tyr Lys Gln Gln Trp Arg Ala Ser Gly Asn Glu Glu Glu Ser Tyr
               980                 985                 990
   Phe Glu Thr Gly Asn Thr Ala Ser  Gly Asp Ser Ser Asn  Gln Met Ser
           995                 1000                1005
   Asp Pro  His Lys Gly Ile Ile  Arg His Lys Gly Tyr  Lys Glu Phe
       1010                1015                1020
   Glu Ser  Asp Asp Glu Val Ser  Asp Arg Ser Leu Gly  Glu Glu Tyr
       1025                1030                1035
   Thr Val  Arg Ala Cys Ala Ala  Ser Glu Ser Ser Met  Glu Asn Gly
       1040                 1045                 1050
   Ser Gln  His Ser Met Tyr Asp  His Asp Asp Asp Asp  Asp Asp Ile
       1055                 1060                 1065
   Asp Arg  Gln Pro Arg Gly Ile  Pro Arg Ser Gln Gln  Thr Arg Val
       1070                 1075                 1080
   Phe Arg  Asn Pro Val Ser Tyr  Glu Ser Glu Asp Asn  Gly Val Tyr
       1085                 1090                 1095
   Gln Gln  Ser Gly Arg Ile Ser  Ile Ser Asn Arg Gln  Ala Asn Arg
       1100                 1105                 1110
   Met Val  Gly Glu Tyr Asp Ser  Ala Glu Asn Ser Leu  Glu Glu Arg
       1115                 1120                 1125
   Gly Phe  Cys Ser Thr Gly Lys  Arg Gln Thr Arg Ser  Thr Ala Lys
       1130                 1135                 1140
   Arg Ile  Ala Lys Thr Lys Thr  Val Gln Ser Ser Arg  Asp Thr Lys
       1145                 1150                 1155
   Gly Arg  Phe Leu Gln Glu Phe  Ala Ser Gly Lys Lys  Asn Glu Glu
       1160                 1165                 1170
   Leu Asp  Ser Tyr Met Glu Gly  Pro Ser Thr Arg Leu  Arg Val Arg
       1175                 1180                 1185
   His Gln  Lys Pro Ser Arg Gly  Ser Leu Glu Thr Lys  Pro Lys Lys
       1190                 1195                 1200
   Ile Gly  Lys Lys Arg Ser Gly  Asn Ala Ser Phe Ser  Arg Val Ala
       1205                 1210                 1215
   Thr Glu  Lys Asp Val Glu Glu  Lys Glu Glu Glu Glu  Glu Glu Glu
       1220                 1225                 1230
   Glu Asn  Glu Glu Glu Glu Cys  Ala Ala Tyr Gln Cys  Asn Met Glu
       1235                 1240                 1245
   Gly Cys  Thr Met Ser Phe Ser  Ser Glu Lys Gln Leu  Met Leu His
       1250                 1255                 1260
   Lys Arg  Asn Ile Cys Pro Ile  Lys Gly Cys Gly Lys  Asn Phe Phe
       1265                 1270                 1275
   Ser His  Lys Tyr Leu Val Gln  His Gln Arg Val His  Ser Asp Asp
       1280                 1285                 1290
   Arg Pro  Leu Lys Cys Pro Trp  Lys Gly Cys Lys Met  Thr Phe Lys
       1295                 1300                 1305
   Trp Ala  Trp Ser Arg Thr Glu  His Ile Arg Val His  Thr Gly Ala
       1310                 1315                 1320
   Arg Pro  Tyr Val Cys Ala Glu  Pro Asp Cys Gly Gln  Thr Phe Arg
       1325                 1330                 1335
   Phe Val  Ser Asp Phe Ser Arg  His Lys Arg Lys Thr  Gly His Ser
       1340                 1345                 1350
   Val Lys  Lys Thr Asn Lys Arg
       1355                 1360
<210> 49
   <211> 2523
   <212> DNA
   <213> Arabidopsis thaliana
<400> 49
   atggagaaaa tgagagggaa gcgaatcaga ccaagagatt ccggcgagtt agtcgaagat     60
   gggagatctg aaagcgagcg taagacgagg aagaaagaga atgatgttgt gagcaaagga    120
   agaatcggaa gaggaagagg aagaggagag gtttcgaagc gatctattga aatagacatt    180
   tctaatcccg agaaggatat taagcctgac ggaagcagaa agtgtttggg ttcgacgtgt     240
   catcattgta agatcttgac gagtgagagt gatcttatct tctgttcaaa gtgtaacaag     300
   aagtgttatt gctttgactg catcaagaga tcgtattcag aaagaacaca tgaggaagtt     360
   agagctgctt gtccattctg tatgatgact tgcatttgca gagcttgttt gcggttgcct     420
   ttggttatca agcctccaag tgaaaaagat acagatgtca agcttaagca gctgcagtat     480
   ctgttagtca aagttctccc agttcttaag gatatttata cggagcagaa ccgtgaacta     540
   gagattgaat caacaattag aggacaccct gtgacagaag ctaatatcaa gaggtgcaaa     600
   cttgacccaa gcgaacgcat atactgtgat ctctgccgca catccattgc caattttcac     660
   agaagctgtc cgaacaaaaa ttgttcagtc gatatatgtc tttcttgctg caaggaacta     720
   agtgagggct tccatcaaga gagagatggg aagaaaaatg cagaagggaa aggatatgaa     780
   tgtcgaatcc cagcaggtca aggaaaagac tcggacgcat atgttccact gcatttctcg     840
   acttggaagc ttaactcaga cagtagcatt ccatgccctc caaaagagtg tggtggttgt     900
   ggtacttcaa cactggagtt gagacgctta tggaaaagag attgggttga aaaattaata     960
   acgaatgccg agaaatgtac tctgaatttt aggccaacag atgtggatat tgttcatgaa    1020
   tgttcctcat gctctaccaa ttctgattcc attagaaggc aggcagcttt taggaagaat    1080
   gctcatgata atttcttata ctccccaaat gctgttgatc tggctgaaga cgatattgct    1140
   cattttcagt ttcattggat gaaggcagaa cctgtgatag tcagaaatgt tctcgagaag    1200
   acatctggac taagttggga accaatggtt atgtggaggg cttgtaggga aatggatcca    1260
   aagcgtaaag gtacagaaga agagacaaca aaagtgaaag ctttggattg cttggactgg    1320
   tgtgaggttg aaataaatct gcatcagttt tttgagggct acttagaagg ccgcatgcat    1380
   aaaaacggtt ggccagaaat gttgaaattg aaggactggc ctccctcaga tttatttgaa    1440
   aagcgccttc caaggcataa tgctgagttt attgccgcat tgcctttctt tgattacact    1500
   gacccaaagt ctggtatcct aaatctcgca acaagatttc cggaaggatc cttgaagcca    1560
   gatcttggac ccaagacata cattgcgtac ggtttccatg aagagcttaa tagaggagat    1620
   tcggtgacaa aacttcattg tgacatttct gacgcggtca atgtgttgac acacacagct    1680
   aaagtggaaa tccctcccgt caaataccaa aacataaaag tacatcagaa aaaatatgca    1740
   gaagccatgt tgcagaaaca acaatacagt ggtcaagtga aagaagccag cgaacttgaa    1800
   aacaagtcaa tgaaagaagt ggatgaaagt aaaaaggatt taaaagacaa ggcagctaac    1860
   gaagaacaat caaataacag ttcaagaccg tcgggctcag gagaagctga gaaagtaatt    1920
   atttcaaaag aggataatcc cacacaacct gctgtgagta caagtgtgga atccatacaa    1980
   gaacagaagc tggatgctcc aaaagaaact gatggcaata ccaatgagag gtcaaaggct    2040
   gtgcatggtg gtgctgtctg ggatatcttt cgaagagagg atgttccaaa acttattcag    2100
   tttttgaaaa ggcacgagca tgagtttcgt cacttcaata acgaacccct ggaatccgtt    2160
   attcacccaa ttcatgacca gactatgttc ttgagtgata gccagaagaa acagctgaaa    2220
   gaagaatttg atatagaacc atggaccttt gagcaacacc tcggcgaagc agtcttcata    2280
   cctgcaggtt gtcctcacca agtgaggaat agacagtctt gcataaaggt ggcactagac    2340
   tttgtggctc ctgaaagcgt cgaagaatgc cttaggctaa cacaggagtt ccggagattg    2400
   cctaaagacc acagttctag cgaggataaa ttggagctta agaaaattgc actgtatgct    2460
   gcgagttcag ccataagaga ggtaaaaggg ctaatgcaaa gctcccgacg cagtgatacc    2520
   taa                                                                  2523

<210> 50
   <211> 840
   <212> PRT
   <213> Arabidopsis thaliana
<400> 50
   Met Glu Lys Met Arg Gly Lys Arg Ile Arg Pro Arg Asp Ser Gly Glu
   1               5                   10                  15
   Leu Val Glu Asp Gly Arg Ser Glu Ser Glu Arg Lys Thr Arg Lys Lys
               20                  25                  30
   Glu Asn Asp Val Val Ser Lys Gly Arg Ile Gly Arg Gly Arg Gly Arg
           35                  40                  45
   Gly Glu Val Ser Lys Arg Ser Ile Glu Ile Asp Ile Ser Asn Pro Glu
       50                  55                  60
   Lys Asp Ile Lys Pro Asp Gly Ser Arg Lys Cys Leu Gly Ser Thr Cys
   65                  70                  75                  80
   His His Cys Lys Ile Leu Thr Ser Glu Ser Asp Leu Ile Phe Cys Ser
                   85                  90                  95
   Lys Cys Asn Lys Lys Cys Tyr Cys Phe Asp Cys Ile Lys Arg Ser Tyr
               100                 105                 110
   Ser Glu Arg Thr His Glu Glu Val Arg Ala Ala Cys Pro Phe Cys Met
           115                 120                 125
   Met Thr Cys Ile Cys Arg Ala Cys Leu Arg Leu Pro Leu Val Ile Lys
       130                 135                 140
   Pro Pro Ser Glu Lys Asp Thr Asp Val Lys Leu Lys Gln Leu Gln Tyr
   145                 150                 155                 160
   Leu Leu Val Lys Val Leu Pro Val Leu Lys Asp Ile Tyr Thr Glu Gln
                   165                 170                 175
   Asn Arg Glu Leu Glu Ile Glu Ser Thr Ile Arg Gly His Pro Val Thr
               180                 185                 190
   Glu Ala Asn Ile Lys Arg Cys Lys Leu Asp Pro Ser Glu Arg Ile Tyr
           195                 200                 205
   Cys Asp Leu Cys Arg Thr Ser Ile Ala Asn Phe His Arg Ser Cys Pro
       210                 215                 220
   Asn Lys Asn Cys Ser Val Asp Ile Cys Leu Ser Cys Cys Lys Glu Leu
   225                 230                 235                 240
   Ser Glu Gly Phe His Gln Glu Arg Asp Gly Lys Lys Asn Ala Glu Gly
                   245                 250                 255
   Lys Gly Tyr Glu Cys Arg Ile Pro Ala Gly Gln Gly Lys Asp Ser Asp
               260                 265                 270
   Ala Tyr Val Pro Leu His Phe Ser Thr Trp Lys Leu Asn Ser Asp Ser
           275                 280                 285
   Ser Ile Pro Cys Pro Pro Lys Glu Cys Gly Gly Cys Gly Thr Ser Thr
       290                 295                 300
   Leu Glu Leu Arg Arg Leu Trp Lys Arg Asp Trp Val Glu Lys Leu Ile
   305                 310                 315                 320
   Thr Asn Ala Glu Lys Cys Thr Leu Asn Phe Arg Pro Thr Asp Val Asp
                   325                 330                 335
   Ile Val His Glu Cys Ser Ser Cys Ser Thr Asn Ser Asp Ser Ile Arg
               340                 345                 350
   Arg Gln Ala Ala Phe Arg Lys Asn Ala His Asp Asn Phe Leu Tyr Ser
           355                 360                 365
   Pro Asn Ala Val Asp Leu Ala Glu Asp Asp Ile Ala His Phe Gln Phe
       370                 375                 380
   His Trp Met Lys Ala Glu Pro Val Ile Val Arg Asn Val Leu Glu Lys
   385                 390                 395                 400
   Thr Ser Gly Leu Ser Trp Glu Pro Met Val Met Trp Arg Ala Cys Arg
                   405                 410                 415
   Glu Met Asp Pro Lys Arg Lys Gly Thr Glu Glu Glu Thr Thr Lys Val
               420                 425                 430
   Lys Ala Leu Asp Cys Leu Asp Trp Cys Glu Val Glu Ile Asn Leu His
           435                 440                 445
   Gln Phe Phe Glu Gly Tyr Leu Glu Gly Arg Met His Lys Asn Gly Trp
       450                 455                 460
   Pro Glu Met Leu Lys Leu Lys Asp Trp Pro Pro Ser Asp Leu Phe Glu
   465                 470                 475                 480
   Lys Arg Leu Pro Arg His Asn Ala Glu Phe Ile Ala Ala Leu Pro Phe
                       485                 490                 495
   Phe Asp Tyr Thr Asp Pro Lys Ser Gly Ile Leu Asn Leu Ala Thr Arg
                   500                 505                 510
   Phe Pro Glu Gly Ser Leu Lys Pro Asp Leu Gly Pro Lys Thr Tyr Ile
               515                 520                 525
   Ala Tyr Gly Phe His Glu Glu Leu Asn Arg Gly Asp Ser Val Thr Lys
           530                 535                 540
   Leu His Cys Asp Ile Ser Asp Ala Val Asn Val Leu Thr His Thr Ala
   545                 550                 555                 560
   Lys Val Glu Ile Pro Pro Val Lys Tyr Gln Asn Ile Lys Val His Gln
                       565                 570                 575
   Lys Lys Tyr Ala Glu Ala Met Leu Gln Lys Gln Gln Tyr Ser Gly Gln
                   580                 585                 590
   Val Lys Glu Ala Ser Glu Leu Glu Asn Lys Ser Met Lys Glu Val Asp
               595                 600                 605
   Glu Ser Lys Lys Asp Leu Lys Asp Lys Ala Ala Asn Glu Glu Gln Ser
           610                 615                 620
   Asn Asn Ser Ser Arg Pro Ser Gly Ser Gly Glu Ala Glu Lys Val Ile
   625                 630                 635                 640
   Ile Ser Lys Glu Asp Asn Pro Thr Gln Pro Ala Val Ser Thr Ser Val
                       645                 650                 655
   Glu Ser Ile Gln Glu Gln Lys Leu Asp Ala Pro Lys Glu Thr Asp Gly
                   660                 665                 670
   Asn Thr Asn Glu Arg Ser Lys Ala Val His Gly Gly Ala Val Trp Asp
               675                 680                 685
   Ile Phe Arg Arg Glu Asp Val Pro Lys Leu Ile Gln Phe Leu Lys Arg
           690                 695                 700
   His Glu His Glu Phe Arg His Phe Asn Asn Glu Pro Leu Glu Ser Val
   705                 710                 715                 720
   Ile His Pro Ile His Asp Gln Thr Met Phe Leu Ser Asp Ser Gln Lys
                       725                 730                 735
   Lys Gln Leu Lys Glu Glu Phe Asp Ile Glu Pro Trp Thr Phe Glu Gln
                   740                 745                 750
   His Leu Gly Glu Ala Val Phe Ile Pro Ala Gly Cys Pro His Gln Val
               755                 760                 765
   Arg Asn Arg Gln Ser Cys Ile Lys Val Ala Leu Asp Phe Val Ala Pro
           770                 775                 780
   Glu Ser Val Glu Glu Cys Leu Arg Leu Thr Gln Glu Phe Arg Arg Leu
   785                 790                 795                 800
   Pro Lys Asp His Ser Ser Ser Glu Asp Lys Leu Glu Leu Lys Lys Ile
                       805                 810                 815
   Ala Leu Tyr Ala Ala Ser Ser Ala Ile Arg Glu Val Lys Gly Leu Met
                   820                 825                 830
   Gln Ser Ser Arg Arg Ser Asp Thr
               835                 840
<210> 51
   <211> 2865
   <212> DNA
   <213> Arabidopsis thaliana
<400> 51
   atggatcagc ttgcatctct agcagagtct gtggctatgg aggaagattc tgagaaacaa      60
   tcgattaaag gagagagtag tcttgaacct gactctactc cttctagtcc taaaataact     120
   gctaggtgga atccatcaga agcttgcagg cctttggttg atgacgctcc catcttttat    180
   cccactaatg aggattttga tgatccactt ggttacatag agaagttgcg ctctaaagca    240
   gaatcatatg gcatatgtcg aattgtgccg cctgttgcat ggaggccccc ttgccctctg    300
   aaggagaaaa aaatatggga gaattctaaa tttcccaccc ggattcagtt cattgacttg    360
   cttcaaaacc gagaaccgat taaaaaatct actaaaacca agaagagaaa acggagaaga    420
   atctctaaaa ttggatacac aagaagaaaa agagattcag gctgtgatac ggcttcctct    480
   ggctctagtg atagtgaagg aaaatttggt tttcagactg ggccagattt taccctggaa    540
   gaatttcaga agtatgatga atactttaag gaatgctatt ttcagtcaga ggatcatcct    600
   ggttccaaag cgtctgaaaa taagaaattt aaacctaagg ttaaggacct tgaaggtgaa    660
   tattggcgga tagtggagca ggcaactgat gaagtagagg tgtactatgg agctgacttg    720
   gaaacaaaga aatttggaag tggtttccca aagtataaac cggggtatcc tataagtgaa    780
   gcagatcaat actctcaatg tggttggaac ctaaataatt tgtcccgtct gcccggatca    840
   gttctagctt ttgagagctg cgatatctca ggagtcattg tgccatggct ttatgttgga    900
   atgtgctttt caactttttg ttggcatgtt gaagatcatc acctttattc catgaactac    960
   ttacacacag gtgatccaaa agtttggtat gggatccctg gaaaccatgc tgagtctttt   1020
   gaaaatgtaa tgaaaaagcg tcttccagat ttgtttgaag aacagcctga cttgctacat   1080
   caactggtca ctcagttatc tccaagaatc ttaaaagagg agggagtacc tgtctatcga   1140
   gctgtccagc gcagtggaga attcattcta accttcccta aggcctatca ttctgggttt   1200
   aattgtggtt tcaactgtgc ggaagcagta aatgttgcac cagttgattg gctggttcat   1260
   ggacagaatg cagtggaagg ctatagcaag cagcggcgaa agagttcatt gtcacatgac   1320
   aagctgcttc ttggagctgc tatggaagct acttattgcc tctgggagct ttcactttca   1380
   aagaagaaga ctcctgtgat tgcgagatgg aaaagggttt gtagtgagga tggattgctt   1440
   acgaaggcag tcaagaagcg tgtgcagatg gaagaagaaa gactaaatca ccttcaagat   1500
   ggttttagct tgcgaaagat ggagggtgat ttcgacaata agagagaacg ggagtgcttc   1560
   ttgtgcttct atgatttgca tatgtctgct tcaagctgca agtgttcccc caaccggttt   1620
   gcatgtctta tccacgctaa ggatctgtgt tcatgtgaaa gtaaggatag atatatccta   1680
   attcgccaca ccttggatga gttatgggca ctggtcagag ctctagaagg ggatcttgat   1740
   gccatagacc tatgggcaag taaatgtcgt gaccagtatc cttcacagca tccaagagca   1800
   agagaatatg cttacctcaa gtctgcccca tgtataaaga gccgtggttc atcaaaagta   1860
   cagcagcgag aacaaaacaa tcttcagtta gtgtctgaac gcttacaaag tgatctaacc   1920
   tcaaacaagg aagttcagtt gaaacaagat ggtgattcag atgtaaatcg ccatggtcat   1980
   gaaagcgaaa gaaatcatgt acatgggatc actgataagt cagctgtcac ggatgtaaaa   2040
   cttggtgtgg gaggtaagtt tgatgagaag aagatttcgg ttgaatctca aaacccacat   2100
   tcagtttcag atgtagggtg tagcgagctg gcgaagaaag tggatggttg tttaggaggg   2160
   aaggaccaaa atgctgcaac caataggtta agtctctctg ttgagctttt gagttctgga   2220
   tctctcgttg ttaaaaagct gtggtgcagt aaacaggcca tatacccaaa agggttcaag   2280
   agccgtgtta agttcttaag tgtgcttgac ccaacaaacc taaccaacta catctcagag   2340
   gttctggatg cagggcttct tggtccattg ttcagggtct cagtagaaga ttaccccact   2400
   gagaatttct cgaatgtatc tgctgaaaag tgctggcaaa tggtgacaca aaggctcaaa   2460
   ctcgaaatca tcaagaaatg tgatcaacca gttagctctt tgacctcttt acaacctttg   2520
   gagagtataa atgggcttga aatgtttgga tttctctccc cgcacgtaat taaggtggtt   2580
   gaggctcttg atccaaagca tcaattggag gagtattgga accaaaaagc agtgaaactg   2640
   tttggtgctg aaccaataaa ggaaggagaa aaggatgata cagagaaagg aggggcttca   2700
   gatccctctt tggaccggga cacaaggctt ctgcgtggac tgttgaagaa ggcgacacct   2760
   gaagaattag taatgatgca tggacttctg tgtggtgaga ctcgcaacac cgagctcaag   2820
   gaagagctct ctactttggt tgataagatg gagataagtc cttaa                  2865
<210> 52
   <211> 954
   <212> PRT
   <213> Arabidopsis thaliana
<400> 52
   Met Asp Gln Leu Ala Ser Leu Ala Glu Ser Val Ala Met Glu Glu Asp
   1               5                   10                  15
   Ser Glu Lys Gln Ser Ile Lys Gly Glu Ser Ser Leu Glu Pro Asp Ser
               20                  25                  30
   Thr Pro Ser Ser Pro Lys Ile Thr Ala Arg Trp Asn Pro Ser Glu Ala
           35                  40                  45
   Cys Arg Pro Leu Val Asp Asp Ala Pro Ile Phe Tyr Pro Thr Asn Glu
       50                  55                  60
   Asp Phe Asp Asp Pro Leu Gly Tyr Ile Glu Lys Leu Arg Ser Lys Ala
   65                  70                  75                  80
   Glu Ser Tyr Gly Ile Cys Arg Ile Val Pro Pro Val Ala Trp Arg Pro
                   85                  90                  95
   Pro Cys Pro Leu Lys Glu Lys Lys Ile Trp Glu Asn Ser Lys Phe Pro
               100                 105                 110
   Thr Arg Ile Gln Phe Ile Asp Leu Leu Gln Asn Arg Glu Pro Ile Lys
           115                 120                 125
   Lys Ser Thr Lys Thr Lys Lys Arg Lys Arg Arg Arg Ile Ser Lys Ile
       130                 135                 140
   Gly Tyr Thr Arg Arg Lys Arg Asp Ser Gly Cys Asp Thr Ala Ser Ser
   145                 150                 155                 160
   Gly Ser Ser Asp Ser Glu Gly Lys Phe Gly Phe Gln Thr Gly Pro Asp
                   165                 170                 175
   Phe Thr Leu Glu Glu Phe Gln Lys Tyr Asp Glu Tyr Phe Lys Glu Cys
               180                 185                 190
   Tyr Phe Gln Ser Glu Asp His Pro Gly Ser Lys Ala Ser Glu Asn Lys
           195                 200                 205
   Lys Phe Lys Pro Lys Val Lys Asp Leu Glu Gly Glu Tyr Trp Arg Ile
       210                 215                 220
   Val Glu Gln Ala Thr Asp Glu Val Glu Val Tyr Tyr Gly Ala Asp Leu
   225                 230                 235                 240
   Glu Thr Lys Lys Phe Gly Ser Gly Phe Pro Lys Tyr Lys Pro Gly Tyr
                   245                 250                 255
   Pro Ile Ser Glu Ala Asp Gln Tyr Ser Gln Cys Gly Trp Asn Leu Asn
               260                 265                 270
   Asn Leu Ser Arg Leu Pro Gly Ser Val Leu Ala Phe Glu Ser Cys Asp
           275                 280                 285
   Ile Ser Gly Val Ile Val Pro Trp Leu Tyr Val Gly Met Cys Phe Ser
       290                 295                 300
   Thr Phe Cys Trp His Val Glu Asp His His Leu Tyr Ser Met Asn Tyr
   305                 310                 315                 320
   Leu His Thr Gly Asp Pro Lys Val Trp Tyr Gly Ile Pro Gly Asn His
                   325                 330                 335
   Ala Glu Ser Phe Glu Asn Val Met Lys Lys Arg Leu Pro Asp Leu Phe
               340                 345                 350
   Glu Glu Gln Pro Asp Leu Leu His Gln Leu Val Thr Gln Leu Ser Pro
           355                 360                 365
   Arg Ile Leu Lys Glu Glu Gly Val Pro Val Tyr Arg Ala Val Gln Arg
       370                 375                 380
   Ser Gly Glu Phe Ile Leu Thr Phe Pro Lys Ala Tyr His Ser Gly Phe
   385                 390                 395                 400
   Asn Cys Gly Phe Asn Cys Ala Glu Ala Val Asn Val Ala Pro Val Asp
                   405                 410                 415
   Trp Leu Val His Gly Gln Asn Ala Val Glu Gly Tyr Ser Lys Gln Arg
               420                 425                 430
   Arg Lys Ser Ser Leu Ser His Asp Lys Leu Leu Leu Gly Ala Ala Met
   435 440 445
   Glu Ala Thr Tyr Cys Leu Trp Glu Leu Ser Leu Ser Lys Lys Lys Thr
   450 455 460
   Pro Val Ile Ala Arg Trp Lys Arg Val Cys Ser Glu Asp Gly Leu Leu
   465 470 475 480
   Thr Lys Ala Val Lys Lys Arg Val Gln Met Glu Glu Glu Arg Leu Asn
   485 490 495
   His Leu Gln Asp Gly Phe Ser Leu Arg Lys Met Glu Gly Asp Phe Asp
   500 505 510
   Asn Lys Arg Glu Arg Glu Cys Phe Leu Cys Phe Tyr Asp Leu His Met
   515 520 525
   Ser Ala Ser Ser Cys Lys Cys Ser Pro Asn Arg Phe Ala Cys Leu Ile
   530 535 540
   His Ala Lys Asp Leu Cys Ser Cys Glu Ser Lys Asp Arg Tyr Ile Leu
   545 550 555 560
   Ile Arg His Thr Leu Asp Glu Leu Trp Ala Leu Val Arg Ala Leu Glu
   565 570 575
   Gly Asp Leu Asp Ala Ile Asp Leu Trp Ala Ser Lys Cys Arg Asp Gln
   580 585 590
   Tyr Pro Ser Gln His Pro Arg Ala Arg Glu Tyr Ala Tyr Leu Lys Ser
   595 600 605
   Ala Pro Cys Ile Lys Ser Arg Gly Ser Ser Lys Val Gln Gln Arg Glu
   610 615 620
   Gln Asn Asn Leu Gln Leu Val Ser Glu Arg Leu Gln Ser Asp Leu Thr
   625 630 635 640
   Ser Asn Lys Glu Val Gln Leu Lys Gln Asp Gly Asp Ser Asp Val Asn
   645 650 655
   Arg His Gly His Glu Ser Glu Arg Asn His Val His Gly Ile Thr Asp
   660 665 670
   Lys Ser Ala Val Thr Asp Val Lys Leu Gly Val Gly Gly Lys Phe Asp
   675 680 685
   Glu Lys Lys Ile Ser Val Glu Ser Gln Asn Pro His Ser Val Ser Asp
   690 695 700
   Val Gly Cys Ser Glu Leu Ala Lys Lys Val Asp Gly Cys Leu Gly Gly
   705 710 715 720
   Lys Asp Gln Asn Ala Ala Thr Asn Arg Leu Ser Leu Ser Val Glu Leu
   725 730 735
   Leu Ser Ser Gly Ser Leu Val Val Lys Lys Leu Trp Cys Ser Lys Gln
   740 745 750
   Ala Ile Tyr Pro Lys Gly Phe Lys Ser Arg Val Lys Phe Leu Ser Val
   755 760 765
   Leu Asp Pro Thr Asn Leu Thr Asn Tyr Ile Ser Glu Val Leu Asp Ala
   770 775 780
   Gly Leu Leu Gly Pro Leu Phe Arg Val Ser Val Glu Asp Tyr Pro Thr
   785 790 795 800
   Glu Asn Phe Ser Asn Val Ser Ala Glu Lys Cys Trp Gln Met Val Thr
   805 810 815
   Gln Arg Leu Lys Leu Glu Ile Ile Lys Lys Cys Asp Gln Pro Val Ser
   820 825 830
   Ser Leu Thr Ser Leu Gln Pro Leu Glu Ser Ile Asn Gly Leu Glu Met
   835 840 845
   Phe Gly Phe Leu Ser Pro His Val Ile Lys Val Val Glu Ala Leu Asp
   850 855 860
   Pro Lys His Gln Leu Glu Glu Tyr Trp Asn Gln Lys Ala Val Lys Leu
   865                 870                 875                 880
   Phe Gly Ala Glu Pro Ile Lys Glu Gly Glu Lys Asp Asp Thr Glu Lys
                   885                 890                 895
   Gly Gly Ala Ser Asp Pro Ser Leu Asp Arg Asp Thr Arg Leu Leu Arg
               900                 905                 910
   Gly Leu Leu Lys Lys Ala Thr Pro Glu Glu Leu Val Met Met His Gly
           915                 920                 925
   Leu Leu Cys Gly Glu Thr Arg Asn Thr Glu Leu Lys Glu Glu Leu Ser
       930                 935                 940
   Thr Leu Val Asp Lys Met Glu Ile Ser Pro
   945                 950
<210> 53
   <211> 2694
   <212> DNA
   <213> Arabidopsis thaliana
<400> 53
   atgatactgc ttcatggtca ggattttgat gatccacttg gttacataga gaagttgcgc    60
   tctaaagcag aatcatatgg catatgtcga attgtgccgc ctgttgcatg gaggccccct   120
   tgccctctga aggagaaaaa aatatgggag aattctaaat ttcccacccg gattcagttc   180
   attgacttgc ttcaaaaccg agaaccgatt aaaaaatcta ctaaaaccaa gaagagaaaa   240
   cggagaagaa tctctaaaat tggatacaca agaagaaaaa gagattcagg ctgtgatacg   300
   gcttcctctg gctctagtga tagtgaagga aaatttggtt ttcagactgg gccagatttt   360
   accctggaag aatttcagaa gtatgatgaa tactttaagg aatgctattt tcagtcagag   420
   gatcatcctg gttccaaagc gtctgaaaat aagaaattta aacctaaggt taaggacctt   480
   gaaggtgaat attggcggat agtggagcag gcaactgatg aagtagaggt gtactatgga   540
   gctgacttgg aaacaaagaa atttggaagt ggtttcccaa agtataaacc ggggtatcct   600
   ataagtgaag cagatcaata ctctcaatgt ggttggaacc taaataattt gtcccgtctg   660
   cccggatcag ttctagcttt tgagagctgc gatatctcag gagtcattgt gccatggctt   720
   tatgttggaa tgtgcttttc aactttttgt tggcatgttg aagatcatca cctttattcc   780
   atgaactact tacacacagg tgatccaaaa gtttggtatg ggatccctgg aaaccatgct   840
   gagtcttttg aaaatgtaat gaaaaagcgt cttccagatt tgtttgaaga acagcctgac   900
   ttgctacatc aactggtcac tcagttatct ccaagaatct taaaagagga gggagtacct   960
   gtctatcgag ctgtccagcg cagtggagaa ttcattctaa ccttccctaa ggcctatcat  1020
   tctgggttta attgtggttt caactgtgcg gaagcagtaa atgttgcacc agttgattgg  1080
   ctggttcatg gacagaatgc agtggaaggc tatagcaagc agcggcgaaa gagttcattg  1140
   tcacatgaca agctgcttct tggagctgct atggaagcta cttattgcct ctgggagctt  1200
   tcactttcaa agaagaagac tcctgtgatt gcgagatgga aaagggtttg tagtgaggat  1260
   ggattgctta cgaaggcagt caagaagcgt gtgcagatgg aagaagaaag actaaatcac  1320
   cttcaagatg gttttagctt gcgaaagatg gagggtgatt tcgacaataa gagagaacgg  1380
   gagtgcttct tgtgcttcta tgatttgcat atgtctgctt caagctgcaa gtgttccccc  1440
   aaccggtttg catgtcttat ccacgctaag gatctgtgtt catgtgaaag taaggataga  1500
   tatatcctaa ttcgccacac cttggatgag ttatgggcac tggtcagagc tctagaaggg  1560
   gatcttgatg ccatagacct atgggcaagt aaatgtcgtg accagtatcc ttcacagcat  1620
   ccaagagcaa gagaatatgc ttacctcaag tctgccccat gtataaagag ccgtggttca  1680
   tcaaaagtac agcagcgaga acaaaacaat cttcagttag tgtctgaacg cttacaaagt  1740
   gatctaacct caaacaagga agttcagttg aaacaagatg gtgattcaga tgtaaatcgc  1800
   catggtcatg aaagcgaaag aaatcatgta catgggatca ctgataagtc agctgtcacg  1860
   gatgtaaaac ttggtgtggg aggtaagttt gatgagaaga agatttcggt tgaatctcaa  1920
   aacccacatt cagtttcaga tgtagggtgt agcgagctgg cgaagaaagt ggatggttgt  1980
   ttaggaggga aggaccaaaa tgctgcaacc aataggttaa gtctctctgt tgagcttttg  2040
   agttctggat ctctcgttgt taaaaagctg tggtgcagta aacaggccat atacccaaaa  2100
   gggttcaaga gccgtgttaa gttcttaagt gtgcttgacc caacaaacct aaccaactac  2160
   atctcagagg ttctggatgc agggcttctt ggtccattgt tcagggtctc agtagaagat    2220
   taccccactg agaatttctc gaatgtatct gctgaaaagt gctggcaaat ggtgacacaa    2280
   aggctcaaac tcgaaatcat caagaaatgt gatcaaccag ttagctcttt gacctcttta    2340
   caacctttgg agagtataaa tgggcttgaa atgtttggat ttctctcccc gcacgtaatt    2400
   aaggtggttg aggctcttga tccaaagcat caattggagg agtattggaa ccaaaaagca    2460
   gtgaaactgt ttggtgctga accaataaag gaaggagaaa aggatgatac agagaaagga    2520
   ggggcttcag atccctcttt ggaccgggac acaaggcttc tgcgtggact gttgaagaag    2580
   gcgacacctg aagaattagt aatgatgcat ggacttctgt gtggtgagac tcgcaacacc    2640
   gagctcaagg aagagctctc tactttggtt gataagatgg agataagtcc ttaa          2694
<210> 54
   <211> 897
   <212> PRT
   <213> Arabidopsis thaliana
<400> 54
   Met Ile Leu Leu His Gly Gln Asp Phe Asp Asp Pro Leu Gly Tyr Ile
   1               5                   10                  15
   Glu Lys Leu Arg Ser Lys Ala Glu Ser Tyr Gly Ile Cys Arg Ile Val
               20                  25                  30
   Pro Pro Val Ala Trp Arg Pro Pro Cys Pro Leu Lys Glu Lys Lys Ile
           35                  40                  45
   Trp Glu Asn Ser Lys Phe Pro Thr Arg Ile Gln Phe Ile Asp Leu Leu
       50                  55                  60
   Gln Asn Arg Glu Pro Ile Lys Lys Ser Thr Lys Thr Lys Lys Arg Lys
   65                  70                  75                  80
   Arg Arg Arg Ile Ser Lys Ile Gly Tyr Thr Arg Arg Lys Arg Asp Ser
                   85                  90                  95
   Gly Cys Asp Thr Ala Ser Ser Gly Ser Ser Asp Ser Glu Gly Lys Phe
               100                 105                 110
   Gly Phe Gln Thr Gly Pro Asp Phe Thr Leu Glu Glu Phe Gln Lys Tyr
           115                 120                 125
   Asp Glu Tyr Phe Lys Glu Cys Tyr Phe Gln Ser Glu Asp His Pro Gly
       130                 135                 140
   Ser Lys Ala Ser Glu Asn Lys Lys Phe Lys Pro Lys Val Lys Asp Leu
   145                 150                 155                 160
   Glu Gly Glu Tyr Trp Arg Ile Val Glu Gln Ala Thr Asp Glu Val Glu
                   165                 170                 175
   Val Tyr Tyr Gly Ala Asp Leu Glu Thr Lys Lys Phe Gly Ser Gly Phe
               180                 185                 190
   Pro Lys Tyr Lys Pro Gly Tyr Pro Ile Ser Glu Ala Asp Gln Tyr Ser
           195                 200                 205
   Gln Cys Gly Trp Asn Leu Asn Asn Leu Ser Arg Leu Pro Gly Ser Val
       210                 215                 220
   Leu Ala Phe Glu Ser Cys Asp Ile Ser Gly Val Ile Val Pro Trp Leu
   225                 230                 235                 240
   Tyr Val Gly Met Cys Phe Ser Thr Phe Cys Trp His Val Glu Asp His
                   245                 250                 255
   His Leu Tyr Ser Met Asn Tyr Leu His Thr Gly Asp Pro Lys Val Trp
               260                 265                 270
   Tyr Gly Ile Pro Gly Asn His Ala Glu Ser Phe Glu Asn Val Met Lys
           275                 280                 285
   Lys Arg Leu Pro Asp Leu Phe Glu Glu Gln Pro Asp Leu Leu His Gln
       290                 295                 300
   Leu Val Thr Gln Leu Ser Pro Arg Ile Leu Lys Glu Glu Gly Val Pro
   305 310 315 320
   Val Tyr Arg Ala Val Gln Arg Ser Gly Glu Phe Ile Leu Thr Phe Pro
   325 330 335
   Lys Ala Tyr His Ser Gly Phe Asn Cys Gly Phe Asn Cys Ala Glu Ala
   340 345 350
   Val Asn Val Ala Pro Val Asp Trp Leu Val His Gly Gln Asn Ala Val
   355 360 365
   Glu Gly Tyr Ser Lys Gln Arg Arg Lys Ser Ser Leu Ser His Asp Lys
   370 375 380
   Leu Leu Leu Gly Ala Ala Met Glu Ala Thr Tyr Cys Leu Trp Glu Leu
   385 390 395 400
   Ser Leu Ser Lys Lys Lys Thr Pro Val Ile Ala Arg Trp Lys Arg Val
   405 410 415
   Cys Ser Glu Asp Gly Leu Leu Thr Lys Ala Val Lys Lys Arg Val Gln
   420 425 430
   Met Glu Glu Glu Arg Leu Asn His Leu Gln Asp Gly Phe Ser Leu Arg
   435 440 445
   Lys Met Glu Gly Asp Phe Asp Asn Lys Arg Glu Arg Glu Cys Phe Leu
   450 455 460
   Cys Phe Tyr Asp Leu His Met Ser Ala Ser Ser Cys Lys Cys Ser Pro
   465 470 475 480
   Asn Arg Phe Ala Cys Leu Ile His Ala Lys Asp Leu Cys Ser Cys Glu
   485 490 495
   Ser Lys Asp Arg Tyr Ile Leu Ile Arg His Thr Leu Asp Glu Leu Trp
   500 505 510
   Ala Leu Val Arg Ala Leu Glu Gly Asp Leu Asp Ala Ile Asp Leu Trp
   515 520 525
   Ala Ser Lys Cys Arg Asp Gln Tyr Pro Ser Gln His Pro Arg Ala Arg
   530 535 540
   Glu Tyr Ala Tyr Leu Lys Ser Ala Pro Cys Ile Lys Ser Arg Gly Ser
   545 550 555 560
   Ser Lys Val Gln Gln Arg Glu Gln Asn Asn Leu Gln Leu Val Ser Glu
   565 570 575
   Arg Leu Gln Ser Asp Leu Thr Ser Asn Lys Glu Val Gln Leu Lys Gln
   580 585 590
   Asp Gly Asp Ser Asp Val Asn Arg His Gly His Glu Ser Glu Arg Asn
   595 600 605
   His Val His Gly Ile Thr Asp Lys Ser Ala Val Thr Asp Val Lys Leu
   610 615 620
   Gly Val Gly Gly Lys Phe Asp Glu Lys Lys Ile Ser Val Glu Ser Gln
   625 630 635 640
   Asn Pro His Ser Val Ser Asp Val Gly Cys Ser Glu Leu Ala Lys Lys
   645 650 655
   Val Asp Gly Cys Leu Gly Gly Lys Asp Gln Asn Ala Ala Thr Asn Arg
   660 665 670
   Leu Ser Leu Ser Val Glu Leu Leu Ser Ser Gly Ser Leu Val Val Lys
   675 680 685
   Lys Leu Trp Cys Ser Lys Gln Ala Ile Tyr Pro Lys Gly Phe Lys Ser
   690 695 700
   Arg Val Lys Phe Leu Ser Val Leu Asp Pro Thr Asn Leu Thr Asn Tyr
   705 710 715 720
   Ile Ser Glu Val Leu Asp Ala Gly Leu Leu Gly Pro Leu Phe Arg Val
   725 730 735
   Ser Val Glu Asp Tyr Pro Thr Glu Asn Phe Ser Asn Val Ser Ala Glu
               740                 745                 750
   Lys Cys Trp Gln Met Val Thr Gln Arg Leu Lys Leu Glu Ile Ile Lys
           755                 760                 765
   Lys Cys Asp Gln Pro Val Ser Ser Leu Thr Ser Leu Gln Pro Leu Glu
       770                 775                 780
   Ser Ile Asn Gly Leu Glu Met Phe Gly Phe Leu Ser Pro His Val Ile
   785                 790                 795                 800
   Lys Val Val Glu Ala Leu Asp Pro Lys His Gln Leu Glu Glu Tyr Trp
                   805                 810                 815
   Asn Gln Lys Ala Val Lys Leu Phe Gly Ala Glu Pro Ile Lys Glu Gly
               820                 825                 830
   Glu Lys Asp Asp Thr Glu Lys Gly Gly Ala Ser Asp Pro Ser Leu Asp
           835                 840                 845
   Arg Asp Thr Arg Leu Leu Arg Gly Leu Leu Lys Lys Ala Thr Pro Glu
       850                 855                 860
   Glu Leu Val Met Met His Gly Leu Leu Cys Gly Glu Thr Arg Asn Thr
   865                 870                 875                 880
   Glu Leu Lys Glu Glu Leu Ser Thr Leu Val Asp Lys Met Glu Ile Ser
                   885                 890                 895
   Pro
<210> 55
   <211> 4023
   <212> DNA
   <213> Arabidopsis thaliana
<400> 55
   atgggtaatg ttgaaattcc gaattggctt aaagctttgc cattggcacc tgtgtttaga     60
   cctacagaca ctgaatttgc agatcccatt gcttacatat cgaaaatcga aaaggaagct    120
   agtgcctttg ggatttgcaa gatcattcct cctttaccca agccatcgaa aaagtatgtt    180
   ttctacaact tgaacaagtc tcttttgaag tgtcccgaat tggtttcaga tgttgacatt    240
   tcgaaggtgt gtaaagagga tagagctgta ttcaccacta ggcagcaaga gttaggccaa    300
   actgtgaaaa aaaacaaagg agagaagggt aagagtaatt ctcaaaggag tggtgttaag    360
   caggtttggc aaagtggagg cgtttataca ttggatcagt ttgaagctaa gtcgaaagct    420
   ttctacaaaa cccaattagg aacggttaag gaactggcac cagttgtcat tgaggcattg    480
   ttctggaaag cggctttaga gaagcctata tatatagagt atgctaatga cgtgcctggc    540
   tctgctttcg gtgagccaga ggatcatttc aggcatttcc ggcagaggaa gaggaggggc    600
   aggggatttt atcagaggaa gacagaaaat aatgatccat caggtaagaa cggtgaaaag    660
   tcatcgcctg aggtagaaaa ggcacccctt gcttctacaa gtttatcatc tcaggattcg    720
   tccaagcaga agaacatgga tattgttgat gaaatggaag gtactgcagg ctggaagctc    780
   tccaacagtt cgtggaacct tcagatgatt gcacgttcac ctggttctgt tacacgtttc    840
   atgccagatg acattcctgg tgttacatct cccatggttt atatcggtat gttgttcagt    900
   tggtttgcct ggcacgttga ggatcatgag cttcacagta tgaattatct tcacactggc    960
   tcaccaaaga cttggtacgc tgtcccttgt gattatgcgc ttgactttga agaggttatc   1020
   cgcaaaaatt cttatggcag gaacattgac caactggctg ctctcaccca actaggcgaa   1080
   aagacaactc ttgtatcacc tgagatgata gttgcatctg gcattccctg ctgtaggttg   1140
   gtacaaaatc ctggtgaatt tgttgtgact tttccgaggt cttatcatgt aggattcagc   1200
   cacggtttta actgtgggga agccgctaat tttggaaccc cacagtggct caacgtagca   1260
   aaggaagctg ctgtgcgtcg ggcagccatg aattatctac ccatgctgtc ccatcagcag   1320
   ctgctatatc tcttgactat gtcctttgtt tcaagagtgc cacgatcatt gctaccaggt   1380
   ggtcgtagct cccgactaag agatcggcag agagaagaaa gagagttcct tgtgaaaaga   1440
   gcttttgtag aagatatatt gaacgaaaac aagaatttat ctgttcttct tcgagaacca   1500
   ggctctcgtt tggtgatgtg ggaccctgat ttacttccgc gtcatagcgc actggctctt   1560
   gcagctgctg gagttgctgg tgcttctgct gtttcacctc cagcagtggc aaaaaaagaa   1620
   cttgaggagg gtcattctga attgcagaat aaagaaaaga cttctctttt agaggaattg   1680
   agcttgttca tggagaagct gaatgatgta tactacgacg atgatgatgg tctgcttaac   1740
   gatttccaag ttgatactgg aaccttgcca tgtgtggctt gtggcgttct tggcttcccc   1800
   tttatgtctg tggtacagcc ttctgaaaaa gcattaaaag atctctcaga gagacaagga   1860
   gagacagatg ctcaggaaat tatgacactg tcatcagaaa agtctgactg cgaatggaaa   1920
   acgtcttcta gatatataag acctcgcatt ttctgcctcg aacacactat tgaacttcag   1980
   agactgctgc aatcaagagg tggattgaaa ttccttgtta tttgccataa agactttcaa   2040
   aaattcaagg cacatgcggc tatagtggca gaggaggtca aagtcccttt cagctatgat   2100
   gatgtcctgt tagagagtgc atctcaggaa gagttgagtc taattgatct tgcaattgaa   2160
   gatgaagaaa aatacgaaca cagtgtagac tggacctcag aacttggcat caatttacgg   2220
   tactgtgtga aagtgaggaa aaattcccct actaagaaaa ttcagcatgc actgtcacta   2280
   ggtggcttgt tctccgatac aagccagatg ctagatttta caactatcag atggctgcag   2340
   agaaaatcac gctcaaaagc taaacccagt tctacctcaa gtttcacacc ttgtgaacat   2400
   cttgaagtaa aagcagatgg aaaattaagg gataatttgg attctcagac cggaaaaaag   2460
   gaagaaaaga tcatccagta ctcgagaaag aaaaagttga atccaaagcc ttcagcagaa   2520
   caagttcagg agctagctac cctagctaaa tcaaaagatt ttgataagac atgtaaaaat   2580
   tttagcagta ggtcacatct ggatagtgca attcgttctg agatgaacag tgagattgga   2640
   gactctggaa gggtcattgg ggtatcattt tccataaatc cttgtagctc atctttcact   2700
   gtgggacatg ggcaagaaca tcccgagatc actgtcaagt ttggttcaga tttagatggg   2760
   aatgtcacaa atagtttgag tatggtgaat ggagactctg ctgacctaac cttaacctcc   2820
   atatccagag agcagcacca aggacactct atgaccagca ataataatgg ctccaactca   2880
   ggtagtcatg ttgtggcaag ccagaccata cttgtttcta caggtgataa tcatgacgga   2940
   ccaagaaaat tgtctggtga ttatgtctgc agtgatgtgt ctgtacgtgg tattcaggaa   3000
   gcggttgaaa tgagtgacca agagtttgga gaacctaggt ctactgtcac taatattgag   3060
   gatgaacagc aatcacagat tgtgaaacca acccaaagag aagctgtatt tggtgatcac   3120
   gaacaggtgg agggagcaga agctgtgtct accagagaaa acttgtgctc tgaaataatt   3180
   ctgcacactg agcattcttc agctcatgtg ggtatggaaa ttcctgacat aaacacggcc   3240
   agtgagaact tagttgttga catgacccat gatggtgaac ctttggaaag tagcgatata   3300
   ttaagttcga gcaatggtga tgaagcttct tcaaatggat tgcaagttct aaatgatgag   3360
   cttagcatgg agagtgaagt ttcaagctca gaaaacaccg aagttattga ggctcccaat   3420
   tctatgggag aagcaaagaa gaagcggaaa atagaatcag agtctgagac aaatgataat   3480
   ccagagagta gcattggttt cataaggagt ccttgtgaag ggttgaggtc aaggggcaag   3540
   aggaaagcaa catgtgaaac ttcattaaag cacactgaaa cgagtgatga agagaagaaa   3600
   cccattgcga aaaggctgaa gaaaacacca aaggcttgct cagggagtcg tcagcaagaa   3660
   gtccccacga caactcaccc caaccgttgt tacctagagg gatgcaagat gacttttgag   3720
   agtaaagcga aattacaaac tcacaagaga aaccgttgca ctcacgaagg gtgtggaaag   3780
   aaattcaggg ctcacaaata tctggtgctt catcaacgtg tgcataagga tgaaagacct   3840
   ttcgagtgct cttggaaagg atgttcaatg actttcaaat ggcaatgggc gaggacagaa   3900
   catttgcgtc tgcacacggg agagcgacca tacatatgca aggtcgatgg atgtggattg   3960
   tcgtttaggt ttgtatctga ctatagtcgc cacagacgta aaaccatgca ctatgtcaca   4020
   tag                                                                 4023
<210> 56
   <211> 1340
   <212> PRT
   <213> Arabidopsis thaliana
<400> 56
   Met Gly Asn Val Glu Ile Pro Asn Trp Leu Lys Ala Leu Pro Leu Ala
   1               5                   10                  15
   Pro Val Phe Arg Pro Thr Asp Thr Glu Phe Ala Asp Pro Ile Ala Tyr
               20                  25                  30
   Ile Ser Lys Ile Glu Lys Glu Ala Ser Ala Phe Gly Ile Cys Lys Ile
           35                  40                  45
   Ile Pro Pro Leu Pro Lys Pro Ser Lys Lys Tyr Val Phe Tyr Asn Leu
       50                  55                  60
   Asn Lys Ser Leu Leu Lys Cys Pro Glu Leu Val Ser Asp Val Asp Ile
   65                  70                  75                  80
   Ser Lys Val Cys Lys Glu Asp Arg Ala Val Phe Thr Thr Arg Gln Gln
                   85                  90                  95
   Glu Leu Gly Gln Thr Val Lys Lys Asn Lys Gly Glu Lys Gly Lys Ser
               100                 105                 110
   Asn Ser Gln Arg Ser Gly Val Lys Gln Val Trp Gln Ser Gly Gly Val
           115                 120                 125
   Tyr Thr Leu Asp Gln Phe Glu Ala Lys Ser Lys Ala Phe Tyr Lys Thr
       130                 135                 140
   Gln Leu Gly Thr Val Lys Glu Leu Ala Pro Val Val Ile Glu Ala Leu
   145                 150                 155                 160
   Phe Trp Lys Ala Ala Leu Glu Lys Pro Ile Tyr Ile Glu Tyr Ala Asn
                   165                 170                 175
   Asp Val Pro Gly Ser Ala Phe Gly Glu Pro Glu Asp His Phe Arg His
               180                 185                 190
   Phe Arg Gln Arg Lys Arg Arg Gly Arg Gly Phe Tyr Gln Arg Lys Thr
           195                 200                 205
   Glu Asn Asn Asp Pro Ser Gly Lys Asn Gly Glu Lys Ser Ser Pro Glu
       210                 215                 220
   Val Glu Lys Ala Pro Leu Ala Ser Thr Ser Leu Ser Ser Gln Asp Ser
   225                 230                 235                 240
   Ser Lys Gln Lys Asn Met Asp Ile Val Asp Glu Met Glu Gly Thr Ala
                   245                 250                 255
   Gly Trp Lys Leu Ser Asn Ser Ser Trp Asn Leu Gln Met Ile Ala Arg
               260                 265                 270
   Ser Pro Gly Ser Val Thr Arg Phe Met Pro Asp Asp Ile Pro Gly Val
           275                 280                 285
   Thr Ser Pro Met Val Tyr Ile Gly Met Leu Phe Ser Trp Phe Ala Trp
       290                 295                 300
   His Val Glu Asp His Glu Leu His Ser Met Asn Tyr Leu His Thr Gly
   305                 310                 315                 320
   Ser Pro Lys Thr Trp Tyr Ala Val Pro Cys Asp Tyr Ala Leu Asp Phe
                   325                 330                 335
   Glu Glu Val Ile Arg Lys Asn Ser Tyr Gly Arg Asn Ile Asp Gln Leu
               340                 345                 350
   Ala Ala Leu Thr Gln Leu Gly Glu Lys Thr Thr Leu Val Ser Pro Glu
           355                 360                 365
   Met Ile Val Ala Ser Gly Ile Pro Cys Cys Arg Leu Val Gln Asn Pro
       370                 375                 380
   Gly Glu Phe Val Val Thr Phe Pro Arg Ser Tyr His Val Gly Phe Ser
   385                 390                 395                 400
   His Gly Phe Asn Cys Gly Glu Ala Ala Asn Phe Gly Thr Pro Gln Trp
                   405                 410                 415
   Leu Asn Val Ala Lys Glu Ala Ala Val Arg Arg Ala Ala Met Asn Tyr
               420                 425                 430
   Leu Pro Met Leu Ser His Gln Gln Leu Leu Tyr Leu Leu Thr Met Ser
           435                 440                 445
   Phe Val Ser Arg Val Pro Arg Ser Leu Leu Pro Gly Gly Arg Ser Ser
       450                 455                 460
   Arg Leu Arg Asp Arg Gln Arg Glu Glu Arg Glu Phe Leu Val Lys Arg
   465                 470                 475                 480
   Ala Phe Val Glu Asp Ile Leu Asn Glu Asn Lys Asn Leu Ser Val Leu
                   485                 490                 495
   Leu Arg Glu Pro Gly Ser Arg Leu Val Met Trp Asp Pro Asp Leu Leu
               500                 505                 510
   Pro Arg His Ser Ala Leu Ala Leu Ala Ala Ala Gly Val Ala Gly Ala
           515                 520                 525
   Ser Ala Val Ser Pro Pro Ala Val Ala Lys Lys Glu Leu Glu Glu Gly
       530                 535                 540
   His Ser Glu Leu Gln Asn Lys Glu Lys Thr Ser Leu Leu Glu Glu Leu
   545                 550                 555                 560
   Ser Leu Phe Met Glu Lys Leu Asn Asp Val Tyr Tyr Asp Asp Asp Asp
                   565                 570                 575
   Gly Leu Leu Asn Asp Phe Gln Val Asp Thr Gly Thr Leu Pro Cys Val
               580                 585                 590
   Ala Cys Gly Val Leu Gly Phe Pro Phe Met Ser Val Val Gln Pro Ser
           595                 600                 605
   Glu Lys Ala Leu Lys Asp Leu Ser Glu Arg Gln Gly Glu Thr Asp Ala
       610                 615                 620
   Gln Glu Ile Met Thr Leu Ser Ser Glu Lys Ser Asp Cys Glu Trp Lys
   625                 630                 635                 640
   Thr Ser Ser Arg Tyr Ile Arg Pro Arg Ile Phe Cys Leu Glu His Thr
                   645                 650                 655
   Ile Glu Leu Gln Arg Leu Leu Gln Ser Arg Gly Gly Leu Lys Phe Leu
               660                 665                 670
   Val Ile Cys His Lys Asp Phe Gln Lys Phe Lys Ala His Ala Ala Ile
           675                 680                 685
   Val Ala Glu Glu Val Lys Val Pro Phe Ser Tyr Asp Asp Val Leu Leu
       690                 695                 700
   Glu Ser Ala Ser Gln Glu Glu Leu Ser Leu Ile Asp Leu Ala Ile Glu
   705                 710                 715                 720
   Asp Glu Glu Lys Tyr Glu His Ser Val Asp Trp Thr Ser Glu Leu Gly
                   725                 730                 735
   Ile Asn Leu Arg Tyr Cys Val Lys Val Arg Lys Asn Ser Pro Thr Lys
               740                 745                 750
   Lys Ile Gln His Ala Leu Ser Leu Gly Gly Leu Phe Ser Asp Thr Ser
           755                 760                 765
   Gln Met Leu Asp Phe Thr Thr Ile Arg Trp Leu Gln Arg Lys Ser Arg
       770                 775                 780
   Ser Lys Ala Lys Pro Ser Ser Thr Ser Ser Phe Thr Pro Cys Glu His
   785                 790                 795                 800
   Leu Glu Val Lys Ala Asp Gly Lys Leu Arg Asp Asn Leu Asp Ser Gln
                   805                 810                 815
   Thr Gly Lys Lys Glu Glu Lys Ile Ile Gln Tyr Ser Arg Lys Lys Lys
               820                 825                 830
   Leu Asn Pro Lys Pro Ser Ala Glu Gln Val Gln Glu Leu Ala Thr Leu
           835                 840                 845
   Ala Lys Ser Lys Asp Phe Asp Lys Thr Cys Lys Asn Phe Ser Ser Arg
       850                 855                 860
   Ser His Leu Asp Ser Ala Ile Arg Ser Glu Met Asn Ser Glu Ile Gly
   865                 870                 875                 880
   Asp Ser Gly Arg Val Ile Gly Val Ser Phe Ser Ile Asn Pro Cys Ser
                   885                 890                 895
   Ser Ser Phe Thr Val Gly His Gly Gln Glu His Pro Glu Ile Thr Val
               900                 905                 910
   Lys Phe Gly Ser Asp Leu Asp Gly Asn Val Thr Asn Ser Leu Ser Met
           915                 920                 925
   Val Asn Gly Asp Ser Ala Asp Leu Thr Leu Thr Ser Ile Ser Arg Glu
       930                 935                 940
   Gln His Gln Gly His Ser Met Thr Ser Asn Asn Asn Gly Ser Asn Ser
   945                 950                 955                 960
   Gly Ser His Val Val Ala Ser Gln Thr Ile Leu Val Ser Thr Gly Asp
                   965                 970                 975
   Asn His Asp Gly Pro Arg Lys Leu Ser Gly Asp Tyr Val Cys Ser Asp
               980                 985                 990
   Val Ser Val Arg Gly Ile Gln Glu  Ala Val Glu Met Ser  Asp Gln Glu
           995                 1000                1005
   Phe Gly  Glu Pro Arg Ser Thr  Val Thr Asn Ile Glu  Asp Glu Gln
       1010                1015                1020
   Gln Ser  Gln Ile Val Lys Pro  Thr Gln Arg Glu Ala  Val Phe Gly
       1025                1030                1035
   Asp His  Glu Gln Val Glu Gly  Ala Glu Ala Val Ser  Thr Arg Glu
       1040                1045                1050
   Asn Leu  Cys Ser Glu Ile Ile  Leu His Thr Glu His  Ser Ser Ala
       1055                1060                1065
   His Val  Gly Met Glu Ile Pro  Asp Ile Asn Thr Ala  Ser Glu Asn
       1070                1075                1080
   Leu Val  Val Asp Met Thr His  Asp Gly Glu Pro Leu  Glu Ser Ser
       1085                1090                1095
   Asp Ile  Leu Ser Ser Ser Asn  Gly Asp Glu Ala Ser  Ser Asn Gly
       1100                1105                1110
   Leu Gln  Val Leu Asn Asp Glu  Leu Ser Met Glu Ser  Glu Val Ser
       1115                1120                1125
   Ser Ser  Glu Asn Thr Glu Val  Ile Glu Ala Pro Asn  Ser Met Gly
       1130                1135                1140
   Glu Ala  Lys Lys Lys Arg Lys  Ile Glu Ser Glu Ser  Glu Thr Asn
       1145                1150                1155
   Asp Asn  Pro Glu Ser Ser Ile  Gly Phe Ile Arg Ser  Pro Cys Glu
       1160                1165                1170
   Gly Leu  Arg Ser Arg Gly Lys  Arg Lys Ala Thr Cys  Glu Thr Ser
       1175                1180                1185
   Leu Lys  His Thr Glu Thr Ser  Asp Glu Glu Lys Lys  Pro Ile Ala
       1190                1195                1200
   Lys Arg  Leu Lys Lys Thr Pro  Lys Ala Cys Ser Gly  Ser Arg Gln
       1205                1210                1215
   Gln Glu  Val Pro Thr Thr Thr  His Pro Asn Arg Cys  Tyr Leu Glu
       1220                1225                1230
   Gly Cys  Lys Met Thr Phe Glu  Ser Lys Ala Lys Leu  Gln Thr His
       1235                1240                1245
   Lys Arg  Asn Arg Cys Thr His  Glu Gly Cys Gly Lys  Lys Phe Arg
       1250                1255                1260
   Ala His  Lys Tyr Leu Val Leu  His Gln Arg Val His  Lys Asp Glu
       1265                1270                1275
   Arg Pro  Phe Glu Cys Ser Trp  Lys Gly Cys Ser Met  Thr Phe Lys
       1280                1285                1290
   Trp Gln  Trp Ala Arg Thr Glu  His Leu Arg Leu His  Thr Gly Glu
       1295                1300                1305
   Arg Pro  Tyr Ile Cys Lys Val  Asp Gly Cys Gly Leu  Ser Phe Arg
       1310                1315                1320
   Phe Val  Ser Asp Tyr Ser Arg  His Arg Arg Lys Thr  Met His Tyr
       1325                1330                1335
   Val Thr
       1340
<210> 57
   <211> 1509
   <212> DNA
   <213> Arabidopsis thaliana
<400> 57
   atgccaaagt gcaagaatct gttgctaacc tcgaagcgac gtaaatccaa atccaaaaga     60
   ctaaagctcc atcagcatga acctgagtct ctgtttccag agaaggaggt agaagaggaa    120
   gatgaagatg aaggaggctt caagctgaaa attgcagctc cttctcaaga gcatggagtt    180
   caacctctgg gaaacctata tttcaatcct ggtgcggtca atgtgcgaaa cactggatta    240
   ggtaatctcc agatactctc agatgagctc gttttggata ttctaggtct tcttggcgcc    300
   aatcatttgg gtgttttggc tactgtaact aagtcattct atatatttgc taatcatgag    360
   cctctctgga gaaatcttgt gttagaggag ttaaaaggag attttttgtt taatgggtcg    420
   tggagatcta cctatgtagc tgcttaccat ccgaaattca agtttgctgg agatggtgaa    480
   tcgaatttga agattataga tttctattct gattatctgt ttcagagctg gttatgtgcg    540
   aatcttgaaa tgaaaccgaa atggcttaga agagataata tcacacgggt gagaggcatt    600
   tctgttgaag acttcattac aaagtttgag gagccgaata agccagtttt gttggaggga    660
   tgtttggatg gttggcctgc tattgagaaa tggtcaagag attatctgac taaggtggtt    720
   ggtgatgtcg aattcgcggt aggtccagtg gaaatgaagc tcgagaagta ttttaggtat    780
   tctgatggag ctagggaaga aaggcctttg tacttgtttg atccaaagtt tgcagagaaa    840
   gttccggttt tggattcgga gtatgatgtt cctgtctatt tcagagagga cttgtttggt    900
   gttttaggaa acgagcgacc tgattataga tggatcataa ttggtccagc cggatcagga    960
   tcgtctttcc atattgatcc taactcaaca tcagcttgga atgcggtgat cacagggtcg   1020
   aagaaatggg ttttgttccc acctgatgtg gttcctccgg gtgtgcatcc aagtcctgat   1080
   ggagcagaag tggcctgtcc tgtttccata atagaatggt tcatgaactt ttatgatgat   1140
   actaaggatt gggagaagaa acctattgag tgtatatgca aagccgggga ggtgatgttt   1200
   gtacctaatg gatggtggca tctggtgatt aacctggagg aatcgattgc tattactcag   1260
   aattatgcta gcaggagtaa tttgttgaat gtgttagaat ttctgaagaa accaaatgct   1320
   aaagaacttg tctccgggac aactgacaga gagaatttgc atgacaaatt caagaaagcc   1380
   attgaagaag cttacccggg gactatccaa gagttagaaa agaaagcaga agaagcaaaa   1440
   agagccgaag aacaaagagt ttctttctgg gactctgcca aaactgatac tttcaagttt   1500
   tctttctaa                                                           1509
<210> 58
   <211> 502
   <212> PRT
   <213> Arabidopsis thaliana
<400> 58
   Met Pro Lys Cys Lys Asn Leu Leu Leu Thr Ser Lys Arg Arg Lys Ser
   1               5                   10                  15
   Lys Ser Lys Arg Leu Lys Leu His Gln His Glu Pro Glu Ser Leu Phe
               20                  25                  30
   Pro Glu Lys Glu Val Glu Glu Glu Asp Glu Asp Glu Gly Gly Phe Lys
           35                  40                  45
   Leu Lys Ile Ala Ala Pro Ser Gln Glu His Gly Val Gln Pro Leu Gly
       50                  55                  60
   Asn Leu Tyr Phe Asn Pro Gly Ala Val Asn Val Arg Asn Thr Gly Leu
   65                  70                  75                  80
   Gly Asn Leu Gln Ile Leu Ser Asp Glu Leu Val Leu Asp Ile Leu Gly
                   85                  90                  95
   Leu Leu Gly Ala Asn His Leu Gly Val Leu Ala Thr Val Thr Lys Ser
               100                 105                 110
   Phe Tyr Ile Phe Ala Asn His Glu Pro Leu Trp Arg Asn Leu Val Leu
           115                 120                 125
   Glu Glu Leu Lys Gly Asp Phe Leu Phe Asn Gly Ser Trp Arg Ser Thr
       130                 135                 140
   Tyr Val Ala Ala Tyr His Pro Lys Phe Lys Phe Ala Gly Asp Gly Glu
   145                 150                 155                 160
   Ser Asn Leu Lys Ile Ile Asp Phe Tyr Ser Asp Tyr Leu Phe Gln Ser
                   165                 170                 175
   Trp Leu Cys Ala Asn Leu Glu Met Lys Pro Lys Trp Leu Arg Arg Asp
               180                 185                 190
   Asn Ile Thr Arg Val Arg Gly Ile Ser Val Glu Asp Phe Ile Thr Lys
           195                 200                 205
   Phe Glu Glu Pro Asn Lys Pro Val Leu Leu Glu Gly Cys Leu Asp Gly
       210                 215                 220
   Trp Pro Ala Ile Glu Lys Trp Ser Arg Asp Tyr Leu Thr Lys Val Val
   225                 230                 235                 240
   Gly Asp Val Glu Phe Ala Val Gly Pro Val Glu Met Lys Leu Glu Lys
                   245                 250                 255
   Tyr Phe Arg Tyr Ser Asp Gly Ala Arg Glu Glu Arg Pro Leu Tyr Leu
               260                 265                 270
   Phe Asp Pro Lys Phe Ala Glu Lys Val Pro Val Leu Asp Ser Glu Tyr
           275                 280                 285
   Asp Val Pro Val Tyr Phe Arg Glu Asp Leu Phe Gly Val Leu Gly Asn
       290                 295                 300
   Glu Arg Pro Asp Tyr Arg Trp Ile Ile Ile Gly Pro Ala Gly Ser Gly
   305                 310                 315                 320
   Ser Ser Phe His Ile Asp Pro Asn Ser Thr Ser Ala Trp Asn Ala Val
                   325                 330                 335
   Ile Thr Gly Ser Lys Lys Trp Val Leu Phe Pro Pro Asp Val Val Pro
               340                 345                 350
   Pro Gly Val His Pro Ser Pro Asp Gly Ala Glu Val Ala Cys Pro Val
           355                 360                 365
   Ser Ile Ile Glu Trp Phe Met Asn Phe Tyr Asp Asp Thr Lys Asp Trp
       370                 375                 380
   Glu Lys Lys Pro Ile Glu Cys Ile Cys Lys Ala Gly Glu Val Met Phe
   385                 390                 395                 400
   Val Pro Asn Gly Trp Trp His Leu Val Ile Asn Leu Glu Glu Ser Ile
                   405                 410                 415
   Ala Ile Thr Gln Asn Tyr Ala Ser Arg Ser Asn Leu Leu Asn Val Leu
               420                 425                 430
   Glu Phe Leu Lys Lys Pro Asn Ala Lys Glu Leu Val Ser Gly Thr Thr
           435                 440                 445
   Asp Arg Glu Asn Leu His Asp Lys Phe Lys Lys Ala Ile Glu Glu Ala
       450                 455                 460
   Tyr Pro Gly Thr Ile Gln Glu Leu Glu Lys Lys Ala Glu Glu Ala Lys
   465                 470                 475                 480
   Arg Ala Glu Glu Gln Arg Val Ser Phe Trp Asp Ser Ala Lys Thr Asp
                   485                 490                 495
   Thr Phe Lys Phe Ser Phe
               500
<210> 59
   <211> 2124
   <212> DNA
   <213> Arabidopsis thaliana
<400> 59
   atgaaggaaa aagtggagaa gcttgagaca gatgatctaa aatggaccga gaggcttcca      60
   gaatgtcctg tctacagacc aacaaaggag gagtttgagg atcctctgac ttatttgcag     120
   aagattttcc cagaagcttc aaaatatggt atttgcaaga ttgtatctcc tttgacagca     180
   acagttcctg ctggcgctgt gttgatgaaa gagaaatcaa actttaagtt cactaccaga     240
   gtacaacccc ttcgacttgc tgagtgggat tctgatgata aagttacatt cttcatgagt     300
   gggaggacct atacatttcg cgactatgag aaaatggcga acaaggtatt tgcacgcaga     360
   tattgcagtg gtggctcttt gcccgactca ttcttggaga aagaattctg gaaggaaatt     420
   gcgtgtggca agactgaaac agttgagtat gcgtgtgatg tagatggtag cgcattttca     480
   tctgcaccag gtgacccact aggaagcagc aaatggaact tgaataaagt ttcaaggctt     540
   cctaagtcta ccctgcgcct tcttgaaaca tccattccgg gagtcacaga acccatgctt     600
   tacatcggaa tgcttttcag tatgtttgcc tggcatgttg aggaccatta tttgtacagc     660
   attaattacc aacattgtgg agcatcaaaa acttggtatg ggattccagg ttccgcagct     720
   ctaaaatttg aaaaggtggt taaagagtgt gtgtacaatg atgatattct atcaactaat     780
   ggagaagatg gtgcctttga cgtgcttctg gggaagacaa ctatattccc cccaaagact      840
   ctgttggatc ataatgtgcc agtgtataaa gctgtgcaaa aacctggaga atttgttgtc     900
   acattcccca gggcttatca tgctggtttc agccacggtt ttaattgtgg tgaggcagtg     960
   aactttgcga tgggtgattg gtttccattt ggagctattg ccagttgccg gtatgctcat    1020
   cttaaccgag taccgttgct tcctcatgaa gaattgattt gtaaagaggc aatgctcttg    1080
   aactcaagtt ccaaatccga gaatctggat cttacaccta cagaattgtc aggacaacga    1140
   agcatcaaga ctgcatttgt acacctgatt cgttttcttc atcttgctcg atggtctcta    1200
   atgaagtcag gattatgcac aggccttgtt tcgaatacat atggaaccat agtctgcagt    1260
   ttgtgtaaac gggattgcta tttggcattt atcaactgcg agtgttactc acaccccgtt    1320
   tgtctacgtc atgatgttaa aaagcttgac cttccatgtg ggacaacgca tactctttac    1380
   ttgagagaca acattgaaga catggaagct gctgcaatga agtttgagaa ggaagatgga    1440
   gtttcagatt tgattacaac tgatgaagat ttatataagt atccatcatc gattacactt    1500
   ccagctgcga aagaagacgg atatacaccg tattccacta tatactttga tttctatact    1560
   gaggtagaga tgacatctca tgaccagttg caatcgggaa atcccgtcat gagttatgag    1620
   gcaaacgctt catgtatctc ctcagttgct gatgattatg aatgctctga ttaccaggta    1680
   aatagacgag ctaactgtag cagcagtagt gattcaaagc tctcggaaga agtagcatgc    1740
   agcagtagca aaaaaactcg gttcttccct gtggttcaag atgaacaact agttgcggat    1800
   caggaaagtg atggatctga ttccgagtgt tttagagtca aacgccgttc ttcattgaag    1860
   tttgagaacc gaacagttgt tctagacaca agagagtctg accatcatca ggaacttaag    1920
   agactgaaga aatcgcatca tcacgaagga agatatagta gtagcagtag tgttagtagg    1980
   caagaagaag aagaagatga gttagtaatt tcaaacagga aagaaactca gcaacaaagt    2040
   gacgtgaaga tgcagaagaa gaggatcgag aatcattttg gtggctttaa acgtctgaaa    2100
   gtgaaagggc taataaagcc gtaa                                          2124
<210> 60
   <211> 707
   <212> PRT
   <213> Arabidopsis thaliana
<400> 60
   Met Lys Glu Lys Val Glu Lys Leu Glu Thr Asp Asp Leu Lys Trp Thr
   1               5                   10                  15
   Glu Arg Leu Pro Glu Cys Pro Val Tyr Arg Pro Thr Lys Glu Glu Phe
               20                  25                  30
   Glu Asp Pro Leu Thr Tyr Leu Gln Lys Ile Phe Pro Glu Ala Ser Lys
           35                  40                  45
   Tyr Gly Ile Cys Lys Ile Val Ser Pro Leu Thr Ala Thr Val Pro Ala
       50                  55                  60
   Gly Ala Val Leu Met Lys Glu Lys Ser Asn Phe Lys Phe Thr Thr Arg
   65                  70                  75                  80
   Val Gln Pro Leu Arg Leu Ala Glu Trp Asp Ser Asp Asp Lys Val Thr
                   85                  90                  95
   Phe Phe Met Ser Gly Arg Thr Tyr Thr Phe Arg Asp Tyr Glu Lys Met
               100                 105                 110
   Ala Asn Lys Val Phe Ala Arg Arg Tyr Cys Ser Gly Gly Ser Leu Pro
           115                 120                 125
   Asp Ser Phe Leu Glu Lys Glu Phe Trp Lys Glu Ile Ala Cys Gly Lys
       130                 135                 140
   Thr Glu Thr Val Glu Tyr Ala Cys Asp Val Asp Gly Ser Ala Phe Ser
   145                 150                 155                 160
   Ser Ala Pro Gly Asp Pro Leu Gly Ser Ser Lys Trp Asn Leu Asn Lys
                   165                 170                 175
   Val Ser Arg Leu Pro Lys Ser Thr Leu Arg Leu Leu Glu Thr Ser Ile
               180                 185                 190
   Pro Gly Val Thr Glu Pro Met Leu Tyr Ile Gly Met Leu Phe Ser Met
           195                 200                 205
   Phe Ala Trp His Val Glu Asp His Tyr Leu Tyr Ser Ile Asn Tyr Gln
       210                 215                 220
   His Cys Gly Ala Ser Lys Thr Trp Tyr Gly Ile Pro Gly Ser Ala Ala
   225                 230                 235                 240
   Leu Lys Phe Glu Lys Val Val Lys Glu Cys Val Tyr Asn Asp Asp Ile
                   245                 250                 255
   Leu Ser Thr Asn Gly Glu Asp Gly Ala Phe Asp Val Leu Leu Gly Lys
               260                 265                 270
   Thr Thr Ile Phe Pro Pro Lys Thr Leu Leu Asp His Asn Val Pro Val
           275                 280                 285
   Tyr Lys Ala Val Gln Lys Pro Gly Glu Phe Val Val Thr Phe Pro Arg
       290                 295                 300
   Ala Tyr His Ala Gly Phe Ser His Gly Phe Asn Cys Gly Glu Ala Val
   305                 310                 315                 320
   Asn Phe Ala Met Gly Asp Trp Phe Pro Phe Gly Ala Ile Ala Ser Cys
                   325                 330                 335
   Arg Tyr Ala His Leu Asn Arg Val Pro Leu Leu Pro His Glu Glu Leu
               340                 345                 350
   Ile Cys Lys Glu Ala Met Leu Leu Asn Ser Ser Ser Lys Ser Glu Asn
           355                 360                 365
   Leu Asp Leu Thr Pro Thr Glu Leu Ser Gly Gln Arg Ser Ile Lys Thr
       370                 375                 380
   Ala Phe Val His Leu Ile Arg Phe Leu His Leu Ala Arg Trp Ser Leu
   385                 390                 395                 400
   Met Lys Ser Gly Leu Cys Thr Gly Leu Val Ser Asn Thr Tyr Gly Thr
                   405                 410                 415
   Ile Val Cys Ser Leu Cys Lys Arg Asp Cys Tyr Leu Ala Phe Ile Asn
               420                 425                 430
   Cys Glu Cys Tyr Ser His Pro Val Cys Leu Arg His Asp Val Lys Lys
           435                 440                 445
   Leu Asp Leu Pro Cys Gly Thr Thr His Thr Leu Tyr Leu Arg Asp Asn
       450                 455                 460
   Ile Glu Asp Met Glu Ala Ala Ala Met Lys Phe Glu Lys Glu Asp Gly
   465                 470                 475                 480
   Val Ser Asp Leu Ile Thr Thr Asp Glu Asp Leu Tyr Lys Tyr Pro Ser
                   485                 490                 495
   Ser Ile Thr Leu Pro Ala Ala Lys Glu Asp Gly Tyr Thr Pro Tyr Ser
               500                 505                 510
   Thr Ile Tyr Phe Asp Phe Tyr Thr Glu Val Glu Met Thr Ser His Asp
           515                 520                 525
   Gln Leu Gln Ser Gly Asn Pro Val Met Ser Tyr Glu Ala Asn Ala Ser
       530                 535                 540
   Cys Ile Ser Ser Val Ala Asp Asp Tyr Glu Cys Ser Asp Tyr Gln Val
   545                 550                 555                 560
   Asn Arg Arg Ala Asn Cys Ser Ser Ser Ser Asp Ser Lys Leu Ser Glu
                   565                 570                 575
   Glu Val Ala Cys Ser Ser Ser Lys Lys Thr Arg Phe Phe Pro Val Val
               580                 585                 590
   Gln Asp Glu Gln Leu Val Ala Asp Gln Glu Ser Asp Gly Ser Asp Ser
           595                 600                 605
   Glu Cys Phe Arg Val Lys Arg Arg Ser Ser Leu Lys Phe Glu Asn Arg
       610                 615                 620
   Thr Val Val Leu Asp Thr Arg Glu Ser Asp His His Gln Glu Leu Lys
   625                 630                 635                 640
   Arg Leu Lys Lys Ser His His His Glu Gly Arg Tyr Ser Ser Ser Ser
                   645                 650                 655
   Ser Val Ser Arg Gln Glu Glu Glu Glu Asp Glu Leu Val Ile Ser Asn
               660                 665                 670
   Arg Lys Glu Thr Gln Gln Gln Ser Asp Val Lys Met Gln Lys Lys Arg
           675                 680                 685
   Ile Glu Asn His Phe Gly Gly Phe Lys Arg Leu Lys Val Lys Gly Leu
       690                 695                 700
   Ile Lys Pro
   705
<210> 61
   <211> 1389
   <212> DNA
   <213> Arabidopsis thaliana
<400> 61
   atgggaatac agattatagg tcaaattgag agaatcaatg gtaaagagct gagttacggc      60
   gattttgcag agagatactt agctaagaac cagccagtgg taatatccga tctcactgaa     120
   gattggcgag ctcgggaaga ttgggtcagt gagaatggaa accctaatct ccacgtattt     180
   gccactcact ttggaaaatc cagagttcag gttgcagatt gtgatacgag agaatttaca     240
   gatcagaaaa gattagaaat gtctgttact gaatttgtgg agcaatggac taataaggat     300
   tctattgagg aatctgtttt gtatctgaaa gattggcact ttgtgaagga gtatccagac     360
   tatacagctt accaaacacc gccgttgttt tctgatgatt ggcttaacgt gtatctagac     420
   aattaccaaa tgcatgagga tagagatagt ttccagaagt atgatcaaat aagctgctct     480
   gattatcggt ttgtttatat gggtgggaaa ggatcttgga cacctctaca tgctgatgta     540
   tttagatctt atagttggtc agctaatgtc tgtggtaaaa aaagatggct tttccttcct     600
   cctcctcaaa gtcatcttgt ttatgacagg tacatgaaaa attgcgttta tgacattttt     660
   gaagaggtga atgaaactaa attccctggt tttaagaaga ccacctggct tgagtgcatt     720
   caagaacccg gtgaaattat ctttgttccc agtggatggc atcatcaagt atataacttg     780
   gaagacacaa tatctattaa ccacaattgg ctcaacgcat acaacctatc ttgggtgtgg     840
   gatctactgt ggaaggacta caaggacact gaagagtcaa tagaagacat aagagacata     900
   tgtgatgatt ttgaagctat ctgccaacgt aatcttgctg ccaacacagg aatgaattta     960
   aatgatttct tcctcttcat gtcacggttc tctttgggga acatggttct actgcaatct    1020
   tactctgaca aacacaaaaa cctgaattcg tgttcattag caatggccca aaacctgtta    1080
   atgaatctct caactatact gaaagtcatg atgaagatga tatctgcagg cggtgtaact    1140
   gcagaggaag tgtatctgga cttgcgagaa acactggagg atccacagtt tctcagattt    1200
   gtcagagaca tgggaagaac ttatgcaagg attcacatgg aggaagagga tcagtttctt    1260
   tcttcgaaag aattattaca aaagctcagt ggtcttgcag gtccaaatat gcaaatatgt    1320
   tctccaaagg atcttgttga aatgataaac catcacaata ctttttcctc ccaaatctat    1380
   tttatttag                                                            1389
<210> 62
   <211> 462
   <212> PRT
   <213> Arabidopsis thaliana
<400> 62
   Met Gly Ile Gln Ile Ile Gly Gln Ile Glu Arg Ile Asn Gly Lys Glu
   1               5                   10                  15
   Leu Ser Tyr Gly Asp Phe Ala Glu Arg Tyr Leu Ala Lys Asn Gln Pro
               20                  25                  30
   Val Val Ile Ser Asp Leu Thr Glu Asp Trp Arg Ala Arg Glu Asp Trp
           35                  40                  45
   Val Ser Glu Asn Gly Asn Pro Asn Leu His Val Phe Ala Thr His Phe
       50                  55                  60
   Gly Lys Ser Arg Val Gln Val Ala Asp Cys Asp Thr Arg Glu Phe Thr
   65                  70                  75                  80
   Asp Gln Lys Arg Leu Glu Met Ser Val Thr Glu Phe Val Glu Gln Trp
                   85                  90                  95
   Thr Asn Lys Asp Ser Ile Glu Glu Ser Val Leu Tyr Leu Lys Asp Trp
               100                 105                 110
   His Phe Val Lys Glu Tyr Pro Asp Tyr Thr Ala Tyr Gln Thr Pro Pro
           115                 120                 125
   Leu Phe Ser Asp Asp Trp Leu Asn Val Tyr Leu Asp Asn Tyr Gln Met
       130                 135                 140
   His Glu Asp Arg Asp Ser Phe Gln Lys Tyr Asp Gln Ile Ser Cys Ser
   145                 150                 155                 160
   Asp Tyr Arg Phe Val Tyr Met Gly Gly Lys Gly Ser Trp Thr Pro Leu
                   165                 170                 175
   His Ala Asp Val Phe Arg Ser Tyr Ser Trp Ser Ala Asn Val Cys Gly
               180                 185                 190
   Lys Lys Arg Trp Leu Phe Leu Pro Pro Pro Gln Ser His Leu Val Tyr
           195                 200                 205
   Asp Arg Tyr Met Lys Asn Cys Val Tyr Asp Ile Phe Glu Glu Val Asn
       210                 215                 220
   Glu Thr Lys Phe Pro Gly Phe Lys Lys Thr Thr Trp Leu Glu Cys Ile
   225                 230                 235                 240
   Gln Glu Pro Gly Glu Ile Ile Phe Val Pro Ser Gly Trp His His Gln
                   245                 250                 255
   Val Tyr Asn Leu Glu Asp Thr Ile Ser Ile Asn His Asn Trp Leu Asn
               260                 265                 270
   Ala Tyr Asn Leu Ser Trp Val Trp Asp Leu Leu Trp Lys Asp Tyr Lys
           275                 280                 285
   Asp Thr Glu Glu Ser Ile Glu Asp Ile Arg Asp Ile Cys Asp Asp Phe
       290                 295                 300
   Glu Ala Ile Cys Gln Arg Asn Leu Ala Ala Asn Thr Gly Met Asn Leu
   305                 310                 315                 320
   Asn Asp Phe Phe Leu Phe Met Ser Arg Phe Ser Leu Gly Asn Met Val
                   325                 330                 335
   Leu Leu Gln Ser Tyr Ser Asp Lys His Lys Asn Leu Asn Ser Cys Ser
               340                 345                 350
   Leu Ala Met Ala Gln Asn Leu Leu Met Asn Leu Ser Thr Ile Leu Lys
           355                 360                 365
   Val Met Met Lys Met Ile Ser Ala Gly Gly Val Thr Ala Glu Glu Val
       370                 375                 380
   Tyr Leu Asp Leu Arg Glu Thr Leu Glu Asp Pro Gln Phe Leu Arg Phe
   385                 390                 395                 400
   Val Arg Asp Met Gly Arg Thr Tyr Ala Arg Ile His Met Glu Glu Glu
                   405                 410                 415
   Asp Gln Phe Leu Ser Ser Lys Glu Leu Leu Gln Lys Leu Ser Gly Leu
               420                 425                 430
   Ala Gly Pro Asn Met Gln Ile Cys Ser Pro Lys Asp Leu Val Glu Met
           435                 440                 445
   Ile Asn His His Asn Thr Phe Ser Ser Gln Ile Tyr Phe Ile
       450                 455                 460
<210> 63
   <211> 1188
   <212> DNA
   <213> Oryza sativa
<400> 63
   atgtcgatgc gggagtttgt cgaccactgg gctgcgagtt cgagcaatgg ggattctgat      60
   ggttccctgc tgtacctgaa agattggcat tttgtgaagg agtaccctgg ttatgttgca     120
   tacaccacac caacattctt tgccgatgat tggctcaaca tgtatcttga tagtcatcct     180
   atacatcgag attctgacat tgccaaccat acaaatgaaa taaactgtgc tgactatcgg     240
   tttgtttaca tgggaccaaa aggaacttgg actcctcttc atgctgatgt ttttaggtca     300
   tacagctggt cggcaaatgt atgcggcaga aaactatggc tctttctacc accatcacaa     360
   agtcattttg tatttgatag gaacctacga tcttcggtct ataacataaa tgatgatgtt     420
   tctgaaaagc agtttcctga attcaataat actaaatggc tagagtgcac tcaggagcag     480
   aatgaaatca tttttgtgcc tagtgggtgg tatcaccaag tccataacct ggaggatact     540
   atatcaataa accataattg gtttaatggc tacaaccttc attgggtgtg gaacttgctt     600
   catgaagatt acaaggtagc aaaagattat attgaagaca ttcgggacat atgtgacgat     660
   tttgagggac tatgtcaacg caacttagca gcaaatacag gcatgaactt ctatgacttc     720
   ttcgtcttca taacacgttt tgctttagcc aatatagttg agctttacca tattcagaat     780
   ccaaaagata ctgacttcat ttcagccgaa actgctaacc actttgtcta taatctcatg     840
   tcgatccgtg atgttgcatc aaaaatggta tctacagaag ctttcaatac cgagaatatt     900
   tgtaatatct cagaacaaaa ccggagtgct ttctctgata tcataaaaat attggaagaa     960
   gagagtttca gaaggttatt ggtggcgttg tcaaaggcat ataattacat agacagagga    1020
   caaaaagatt gtctcaaaat gaaggattca agtcagaagg gttgtttgtc agtaacctgc    1080
   ttaaaacctg actgtaatgt tgttggtgac attatttctt tcatgcgtga gattcatgga    1140
   cctatggatt tggtaacact aattgatagt gctctctctg atagatag                 1188
<210> 64
   <211> 395
   <212> PRT
   <213> Oryza sativa
<400> 64
   Met Ser Met Arg Glu Phe Val Asp His Trp Ala Ala Ser Ser Ser Asn
   1               5                   10                  15
   Gly Asp Ser Asp Gly Ser Leu Leu Tyr Leu Lys Asp Trp His Phe Val
               20                  25                  30
   Lys Glu Tyr Pro Gly Tyr Val Ala Tyr Thr Thr Pro Thr Phe Phe Ala
           35                  40                  45
   Asp Asp Trp Leu Asn Met Tyr Leu Asp Ser His Pro Ile His Arg Asp
       50                  55                  60
   Ser Asp Ile Ala Asn His Thr Asn Glu Ile Asn Cys Ala Asp Tyr Arg
   65                  70                  75                  80
   Phe Val Tyr Met Gly Pro Lys Gly Thr Trp Thr Pro Leu His Ala Asp
                   85                  90                  95
   Val Phe Arg Ser Tyr Ser Trp Ser Ala Asn Val Cys Gly Arg Lys Leu
               100                 105                 110
   Trp Leu Phe Leu Pro Pro Ser Gln Ser His Phe Val Phe Asp Arg Asn
           115                 120                 125
   Leu Arg Ser Ser Val Tyr Asn Ile Asn Asp Asp Val Ser Glu Lys Gln
       130                 135                 140
   Phe Pro Glu Phe Asn Asn Thr Lys Trp Leu Glu Cys Thr Gln Glu Gln
   145                 150                 155                 160
   Asn Glu Ile Ile Phe Val Pro Ser Gly Trp Tyr His Gln Val His Asn
                   165                 170                 175
   Leu Glu Asp Thr Ile Ser Ile Asn His Asn Trp Phe Asn Gly Tyr Asn
               180                 185                 190
   Leu His Trp Val Trp Asn Leu Leu His Glu Asp Tyr Lys Val Ala Lys
           195                 200                 205
   Asp Tyr Ile Glu Asp Ile Arg Asp Ile Cys Asp Asp Phe Glu Gly Leu
       210                 215                 220
   Cys Gln Arg Asn Leu Ala Ala Asn Thr Gly Met Asn Phe Tyr Asp Phe
   225                 230                 235                 240
   Phe Val Phe Ile Thr Arg Phe Ala Leu Ala Asn Ile Val Glu Leu Tyr
                   245                 250                 255
   His Ile Gln Asn Pro Lys Asp Thr Asp Phe Ile Ser Ala Glu Thr Ala
               260                 265                 270
   Asn His Phe Val Tyr Asn Leu Met Ser Ile Arg Asp Val Ala Ser Lys
           275                 280                 285
   Met Val Ser Thr Glu Ala Phe Asn Thr Glu Asn Ile Cys Asn Ile Ser
       290                 295                 300
   Glu Gln Asn Arg Ser Ala Phe Ser Asp Ile Ile Lys Ile Leu Glu Glu
   305                 310                 315                 320
   Glu Ser Phe Arg Arg Leu Leu Val Ala Leu Ser Lys Ala Tyr Asn Tyr
                   325                 330                 335
   Ile Asp Arg Gly Gln Lys Asp Cys Leu Lys Met Lys Asp Ser Ser Gln
               340                 345                 350
   Lys Gly Cys Leu Ser Val Thr Cys Leu Lys Pro Asp Cys Asn Val Val
           355                 360                 365
   Gly Asp Ile Ile Ser Phe Met Arg Glu Ile His Gly Pro Met Asp Leu
       370                 375                 380
   Val Thr Leu Ile Asp Ser Ala Leu Ser Asp Arg
   385                 390                 395
<210> 65
   <211> 3861
   <212> DNA
   <213> Oryza sativa
<400> 65
   atgaggccct cgccgcctcc cgccgccccg gcggcggaac cggtgccccc gtggctgaga 60
   tcgctgcccg tcgcgcccga gttccgcccc accgccgccg agttcgccga tcccgtctcc 120
   tacatcctca agatcgagcc cgccgcggcg ccgtacggca tctgcaaggt cgtgccgccg 180
   ttgcccccgc cgccgaagaa ggccaccttc tccaacctct cccgctcctt cgccgcgctc 240
   cacccggacg accgctcgcc gtccttcccc acccgccacc agcaggtcgg cctctgcccg 300
   cgccgcacgc gcccggggct caagcccgtg tggcggtcct cccaccgcta cacgctcccg 360
   cagttcgagt ccaaggccgg cgccacccgc aagtcgctcc tcgccggcct caacttccct 420
   gcctccaggc aactcacccc gctcgaccac gaggtgctct tctggcgcgc atcggccgac 480
   cgccccatcg tggtcgagta tggcagcgac atgtcgggct cgggattctc cccttgcgcc 540
   gcgcagccgc agccgccgcc gcagcagcaa ccgacggcgc gggcggcggc gcaccttggg 600
   gagacggcgt ggaacatgcg cggcgtggct aggagccccg gatcgctgct gcggttcatg 660
   ccggaggatg tccccggggt caccacgccg atgctctacg tcgggatgat gttcagttgg 720
   ttcgcgtggc acgtcgagga ccacgacctg cacagcctca actacatgca cctcggggcc 780
   gccaagacgt ggtacggcgt gccgcgcgac gccgcgctcg cgttcgagga cgtggtgcgc 840
   gaacatggct acggcgggga ggtgaacccc ctagaaacct tcgctacatt ggggcagaaa 900
   acaacagtga tgtcccctga agtgcttgtg gagtcgggta tcccctgctg cagattggtg 960
   cagaatgcag gggaatttgt ggtcactttt ccaggatctt atcactgcgg tttcagtcat 1020
   ggattcaact gtggggaagc atcaaatata gctactcctg aatggttgag aatagcaaaa 1080
   gaggcagcaa tccgaagagc ttcaatcaat cgtcctccca tggtttcgca ctatcaatta 1140
   ctttacgatc tcgcactgtc tatgcgtttt agggaaccat ccaatggtga aatggagaca 1200
   cggagctctc gaataaagga gaagaagaaa tgcgaagggg aacaactggt caagaagatg 1260
   ttcatccaaa atgttattga agataatgaa ctgcttagtc atcttctaaa tgacggatca 1320
   tcttgtatta ttcttccagc aaatgcccat gatggtcctg gtctttctac tttgcgctca 1380
   acagatcaat caaatatgaa ttccagaata tcacataacc tatgcagtag ggaagaagct 1440
   ccagaagcct caggatgctt atcgccgaac aggaatggtg ataccagaaa ttgtatttca 1500
   tcagatacgc ataacatgga aggtgataag ggagatataa tgagcgctac tggattgtta 1560
   gatcagggtc tattatcatg tgtcacttgt ggaattttaa gtttttcatg tgtggctgta 1620
   ctcaaaccaa gagattctac agcacgatac ttgatgtctg ctgattctaa ttcaattaat 1680
   aaccagcttt ctatttctgg agggagtatt ctggcagatg cgccaacaaa tgaaaggaat 1740
   ggtgtcattt ctcgtccata ttctgaacac tgttgcaacg aaataatggc cgatgatgca 1800
   gaaatcgata agaattctgc tcttgatctc ttggcatttg ctcatggggg tcaaccagat 1860
   cctgaagaag atccactgga gaaaatactg aagattgcac atggcattaa caaatcacaa 1920
   cctaatagtt caaataatgt tggttgtgtt ggaacaaagc tgtcctcaag tagcacagag 1980
   cgccaagaaa gaccatcttc ccagaacgct cattgtaatg gtagctcagt tatttcaaat 2040
   ggaccgaaag gtgttcgcac gagaaataag tatcaactta agatggtact ttctgaaggt 2100
   tttcaggcaa aggatatcta ttctgcgaag gaaaagaagg ttcaatcaga accatcaagc 2160
   tcaaaagggg atgttaagga gacaattgat gttagtggca cagagaacga tgtcggatgt 2220
   aaaagcacta caatctctgt cagtgaacac aggggttcta caaagaatat gtattcagtg 2280
   aaggaaaaga aggttcaatc gaaaccatca agcttaaagg ggactgttaa agagacagtt 2340
   gatgtcagtg gcacagagaa cgatgctaga tgtaaaagca ttacaatctc tgtcagtgaa 2400
   cacaggggtt ctacacctat gaccaatagt ttagctgcat caatcgtgaa acctgacaag 2460
   gattcatcaa gaatgcatgt attttgtctt gagcatgcta ttgaggttga gaagcaactg 2520
   catgctatag gtggttctaa tattatgctt atatgtcgtc cagaatatcc caaaatagaa 2580
   gcggaagcaa gattgttggg tgaagaaatg ggactggtat atgactggaa gggcattcat 2640
   ttcaaggagg ccaacatgga ggacaggcag aagatacagg aagttctgcg ggatgaggaa 2700
   gcaataccta caagcagtga ttgggctgta aaattgggta ttaaccttta ttatagtgca 2760
   aatctggcca aatctccttt atataacaaa caaatgccat acaacagagt catttatagg 2820
   gcatttggct gtgattctcc taatgactcg ccagtaatgt tcaatacttg cgaaaggaaa 2880
   caaagccacc agaagaaaat agtggttgct ggccggtggt gtgggaaagt atggatgtca 2940
   aaacaggtcc atccatactt ggctcacaga gttgaaagcc aggaagcgga agaagcagat    3000
   agaatctgtt cttatcattt tgatgagaag cacaaagctg agccagttgg gaattctagt    3060
   agagtggaag cctccaaaag aaagagtagc agtttgacag acgtaacaga atcaagcaat    3120
   agaagggggg aaatacctgg tgaggaaaca aacaccaaga ggcccaaaca ttctcaagag    3180
   aacaatctca gagctttgga aaccgctgct gaagttgtgg tgccctcacc agctggaaca    3240
   ggtctccgcg ttagctccag gattgccaac agggcaaaca agctaaaatc aaagatggaa    3300
   aaggaggatg tcccttctag ccgtccaaag tctaacataa aggagaaaag cagtcatgct    3360
   agcggtcaga aatcaaatgt tcaagaggcg aatgctaatt ctgctagcca tttgagagca    3420
   atgccaccaa aacagaaggc tgaagcagaa gcaaagaagc aaataagaac tccaaaacct    3480
   ccaaagcaag cagtggagta ctcgtgcgat attgagggtt gttctatgag ttttcgtaca    3540
   aagcgggact tgtctctgca caagagtgat atctgcccag tgaaaggctg cgggaagaag    3600
   ttcttctcgc acaagtatct gctgcagcat cgcaaggttc acacagatga ccgtccgcta    3660
   acatgcccat ggaagggctg caacatggca ttcaagtggc catgggcaag gactgaacat    3720
   ctcagggttc atacaggcga caggccctat gtttgccatg agccaggttg cgcacagaca    3780
   ttcaggtttg tctcagattt cagccgtcac aagcgcaaga caggtcattc tgtcaagaag    3840
   aagaagaaag caaagagttg a                                              3861
<210> 66
   <211> 1286
   <212> PRT
   <213> Oryza sativa
<400> 66
   Met Arg Pro Ser Pro Pro Pro Ala Ala Pro Ala Ala Glu Pro Val Pro
   1               5                   10                  15
   Pro Trp Leu Arg Ser Leu Pro Val Ala Pro Glu Phe Arg Pro Thr Ala
               20                  25                  30
   Ala Glu Phe Ala Asp Pro Val Ser Tyr Ile Leu Lys Ile Glu Pro Ala
           35                  40                  45
   Ala Ala Pro Tyr Gly Ile Cys Lys Val Val Pro Pro Leu Pro Pro Pro
       50                  55                  60
   Pro Lys Lys Ala Thr Phe Ser Asn Leu Ser Arg Ser Phe Ala Ala Leu
   65                  70                  75                  80
   His Pro Asp Asp Arg Ser Pro Ser Phe Pro Thr Arg His Gln Gln Val
                   85                  90                  95
   Gly Leu Cys Pro Arg Arg Thr Arg Pro Gly Leu Lys Pro Val Trp Arg
               100                 105                 110
   Ser Ser His Arg Tyr Thr Leu Pro Gln Phe Glu Ser Lys Ala Gly Ala
           115                 120                 125
   Thr Arg Lys Ser Leu Leu Ala Gly Leu Asn Phe Pro Ala Ser Arg Gln
       130                 135                 140
   Leu Thr Pro Leu Asp His Glu Val Leu Phe Trp Arg Ala Ser Ala Asp
   145                 150                 155                 160
   Arg Pro Ile Val Val Glu Tyr Gly Ser Asp Met Ser Gly Ser Gly Phe
                   165                 170                 175
   Ser Pro Cys Ala Ala Gln Pro Gln Pro Pro Pro Gln Gln Gln Pro Thr
               180                 185                 190
   Ala Arg Ala Ala Ala His Leu Gly Glu Thr Ala Trp Asn Met Arg Gly
           195                 200                 205
   Val Ala Arg Ser Pro Gly Ser Leu Leu Arg Phe Met Pro Glu Asp Val
       210                 215                 220
   Pro Gly Val Thr Thr Pro Met Leu Tyr Val Gly Met Met Phe Ser Trp
   225                 230                 235                 240
   Phe Ala Trp His Val Glu Asp His Asp Leu His Ser Leu Asn Tyr Met
                   245                 250                 255
   His Leu Gly Ala Ala Lys Thr Trp Tyr Gly Val Pro Arg Asp Ala Ala
               260                 265                 270
   Leu Ala Phe Glu Asp Val Val Arg Glu His Gly Tyr Gly Gly Glu Val
           275                 280                 285
   Asn Pro Leu Glu Thr Phe Ala Thr Leu Gly Gln Lys Thr Thr Val Met
       290                 295                 300
   Ser Pro Glu Val Leu Val Glu Ser Gly Ile Pro Cys Cys Arg Leu Val
   305                 310                 315                 320
   Gln Asn Ala Gly Glu Phe Val Val Thr Phe Pro Gly Ser Tyr His Cys
                   325                 330                 335
   Gly Phe Ser His Gly Phe Asn Cys Gly Glu Ala Ser Asn Ile Ala Thr
               340                 345                 350
   Pro Glu Trp Leu Arg Ile Ala Lys Glu Ala Ala Ile Arg Arg Ala Ser
           355                 360                 365
   Ile Asn Arg Pro Pro Met Val Ser His Tyr Gln Leu Leu Tyr Asp Leu
       370                 375                 380
   Ala Leu Ser Met Arg Phe Arg Glu Pro Ser Asn Gly Glu Met Glu Thr
   385                 390                 395                 400
   Arg Ser Ser Arg Ile Lys Glu Lys Lys Lys Cys Glu Gly Glu Gln Leu
                   405                 410                 415
   Val Lys Lys Met Phe Ile Gln Asn Val Ile Glu Asp Asn Glu Leu Leu
               420                 425                 430
   Ser His Leu Leu Asn Asp Gly Ser Ser Cys Ile Ile Leu Pro Ala Asn
           435                 440                 445
   Ala His Asp Gly Pro Gly Leu Ser Thr Leu Arg Ser Thr Asp Gln Ser
       450                 455                 460
   Asn Met Asn Ser Arg Ile Ser His Asn Leu Cys Ser Arg Glu Glu Ala
   465                 470                 475                 480
   Pro Glu Ala Ser Gly Cys Leu Ser Pro Asn Arg Asn Gly Asp Thr Arg
                   485                 490                 495
   Asn Cys Ile Ser Ser Asp Thr His Asn Met Glu Gly Asp Lys Gly Asp
               500                 505                 510
   Ile Met Ser Ala Thr Gly Leu Leu Asp Gln Gly Leu Leu Ser Cys Val
           515                 520                 525
   Thr Cys Gly Ile Leu Ser Phe Ser Cys Val Ala Val Leu Lys Pro Arg
       530                 535                 540
   Asp Ser Thr Ala Arg Tyr Leu Met Ser Ala Asp Ser Asn Ser Ile Asn
   545                 550                 555                 560
   Asn Gln Leu Ser Ile Ser Gly Gly Ser Ile Leu Ala Asp Ala Pro Thr
                   565                 570                 575
   Asn Glu Arg Asn Gly Val Ile Ser Arg Pro Tyr Ser Glu His Cys Cys
               580                 585                 590
   Asn Glu Ile Met Ala Asp Asp Ala Glu Ile Asp Lys Asn Ser Ala Leu
           595                 600                 605
   Asp Leu Leu Ala Phe Ala His Gly Gly Gln Pro Asp Pro Glu Glu Asp
       610                 615                 620
   Pro Leu Glu Lys Ile Leu Lys Ile Ala His Gly Ile Asn Lys Ser Gln
   625                 630                 635                 640
   Pro Asn Ser Ser Asn Asn Val Gly Cys Val Gly Thr Lys Leu Ser Ser
                   645                 650                 655
   Ser Ser Thr Glu Arg Gln Glu Arg Pro Ser Ser Gln Asn Ala His Cys
               660                 665                 670
   Asn Gly Ser Ser Val Ile Ser Asn Gly Pro Lys Gly Val Arg Thr Arg
           675                 680                 685
   Asn Lys Tyr Gln Leu Lys Met Val Leu Ser Glu Gly Phe Gln Ala Lys
       690                 695                 700
   Asp Ile Tyr Ser Ala Lys Glu Lys Lys Val Gln Ser Glu Pro Ser Ser
   705                 710                 715                 720
   Ser Lys Gly Asp Val Lys Glu Thr Ile Asp Val Ser Gly Thr Glu Asn
                   725                 730                 735
   Asp Val Gly Cys Lys Ser Thr Thr Ile Ser Val Ser Glu His Arg Gly
               740                 745                 750
   Ser Thr Lys Asn Met Tyr Ser Val Lys Glu Lys Lys Val Gln Ser Lys
           755                 760                 765
   Pro Ser Ser Leu Lys Gly Thr Val Lys Glu Thr Val Asp Val Ser Gly
       770                 775                 780
   Thr Glu Asn Asp Ala Arg Cys Lys Ser Ile Thr Ile Ser Val Ser Glu
   785                 790                 795                 800
   His Arg Gly Ser Thr Pro Met Thr Asn Ser Leu Ala Ala Ser Ile Val
                   805                 810                 815
   Lys Pro Asp Lys Asp Ser Ser Arg Met His Val Phe Cys Leu Glu His
               820                 825                 830
   Ala Ile Glu Val Glu Lys Gln Leu His Ala Ile Gly Gly Ser Asn Ile
           835                 840                 845
   Met Leu Ile Cys Arg Pro Glu Tyr Pro Lys Ile Glu Ala Glu Ala Arg
       850                 855                 860
   Leu Leu Gly Glu Glu Met Gly Leu Val Tyr Asp Trp Lys Gly Ile His
   865                 870                 875                 880
   Phe Lys Glu Ala Asn Met Glu Asp Arg Gln Lys Ile Gln Glu Val Leu
                   885                 890                 895
   Arg Asp Glu Glu Ala Ile Pro Thr Ser Ser Asp Trp Ala Val Lys Leu
               900                 905                 910
   Gly Ile Asn Leu Tyr Tyr Ser Ala Asn Leu Ala Lys Ser Pro Leu Tyr
           915                 920                 925
   Asn Lys Gln Met Pro Tyr Asn Arg Val Ile Tyr Arg Ala Phe Gly Cys
       930                 935                 940
   Asp Ser Pro Asn Asp Ser Pro Val Met Phe Asn Thr Cys Glu Arg Lys
   945                 950                 955                 960
   Gln Ser His Gln Lys Lys Ile Val Val Ala Gly Arg Trp Cys Gly Lys
                   965                 970                 975
   Val Trp Met Ser Lys Gln Val His Pro Tyr Leu Ala His Arg Val Glu
               980                 985                 990
   Ser Gln Glu Ala Glu Glu Ala Asp  Arg Ile Cys Ser Tyr  His Phe Asp
           995                 1000                1005
   Glu Lys  His Lys Ala Glu Pro  Val Gly Asn Ser Ser  Arg Val Glu
       1010                1015                1020
   Ala Ser  Lys Arg Lys Ser Ser  Ser Leu Thr Asp Val  Thr Glu Ser
       1025                1030                1035
   Ser Asn  Arg Arg Gly Glu Ile  Pro Gly Glu Glu Thr  Asn Thr Lys
       1040                1045                1050
   Arg Pro  Lys His Ser Gln Glu  Asn Asn Leu Arg Ala  Leu Glu Thr
       1055                1060                1065
   Ala Ala  Glu Val Val Val Pro  Ser Pro Ala Gly Thr  Gly Leu Arg
       1070                1075                1080
   Val Ser  Ser Arg Ile Ala Asn  Arg Ala Asn Lys Leu  Lys Ser Lys
       1085                1090                1095
   Met Glu  Lys Glu Asp Val Pro  Ser Ser Arg Pro Lys  Ser Asn Ile
           1100                1105                1110
   Lys Glu  Lys Ser Ser His Ala  Ser Gly Gln Lys Ser  Asn Val Gln
       1115                1120                1125
   Glu Ala  Asn Ala Asn Ser Ala  Ser His Leu Arg Ala  Met Pro Pro
       1130                1135                1140
   Lys Gln  Lys Ala Glu Ala Glu  Ala Lys Lys Gln Ile  Arg Thr Pro
       1145                1150                1155
   Lys Pro  Pro Lys Gln Ala Val  Glu Tyr Ser Cys Asp  Ile Glu Gly
       1160                1165                1170
   Cys Ser  Met Ser Phe Arg Thr  Lys Arg Asp Leu Ser  Leu His Lys
       1175                1180                1185
   Ser Asp  Ile Cys Pro Val Lys  Gly Cys Gly Lys Lys  Phe Phe Ser
       1190                1195                1200
   His Lys  Tyr Leu Leu Gln His  Arg Lys Val His Thr  Asp Asp Arg
       1205                1210                1215
   Pro Leu  Thr Cys Pro Trp Lys  Gly Cys Asn Met Ala  Phe Lys Trp
       1220                1225                1230
   Pro Trp  Ala Arg Thr Glu His  Leu Arg Val His Thr  Gly Asp Arg
       1235                1240                1245
   Pro Tyr  Val Cys His Glu Pro  Gly Cys Ala Gln Thr  Phe Arg Phe
       1250                1255                1260
   Val Ser  Asp Phe Ser Arg His  Lys Arg Lys Thr Gly  His Ser Val
       1265                1270                1275
   Lys Lys  Lys Lys Lys Ala Lys  Ser
       1280                1285

<210> 67
   <211> 2994
   <212> DNA
   <213> Oryza sativa
<400> 67
   atgccgccca agaggaagcg cggtggcggg aggccgcgca aggccccgga ggacgccgcc      60
   gccaaggaaa acggtgagaa gacaaacaag gaggaggaaa cgcaggcttc tcctgaggag     120
   aatggcgcag ggcagactca ggcctcgaga accgcaagaa agcgcaggaa gggtcctgtt     180
   gctgatccct cttcgactga acttccaccc aggaagctca gggacaggcg gaacgttcca     240
   gcggtagact acaaggagaa taagcacaca aaaaagatgg atggaacttc aaccatgtgc     300
   catcagtgcc aaaggaagga cagtggaaga gttgtgaggt gccggaatgg tgctgaaaaa     360
   aacaggaggc acagatattg tgtcaagtgc atcaaacgct ggtacccgca tctaacagag     420
   gacgattttg agaactgctg tccagtttgt cacaataact gcaattgtaa gacttgtctg     480
   cggacaaatg taataaataa gggtgacaag gagtttgccg atggtaagaa caaaattaaa     540
   tactctctgc gtattgcacg ctttttgctc ccttggttga aacaacttca ccaagagcag     600
   atgctagaga aaagtgttga ggccacaatt aaagggatcg atgtaactga cttggaggtt     660
   cctcaagctc agtttaataa tgatgagcgg atttactgtg ataactgcag aacatccatt     720
   gttgacttcc acagaagttg taaaagtggt cactatgatc tctgcctcag ctgctgccag     780
   gagttgcgtc aaggtcttac tactggtact gttgtcactt gtgacacagc tgttgatgta     840
   cctgaaatag aaggcaagga aggtttgcaa gagggaagta gtcatagcag tgctgtaggt     900
   caaggggctt ctgatcaaca gaatgacagg ttgataggta gtgcagctcc ttcagaggat     960
   tgcactccta gtttgatatg gagggcaaaa agcaacggaa gcataccatg cccacctaat    1020
   gcaggtggtt gcggggattg tcttctcgaa cttaggtgtt tgtttaagga gaactttatt    1080
   tctgatttac tggataaagt caattcagtg gtcaataagg aaacagagca ggagttggga    1140
   ggctctagat gttcctgctt cactgaatcc ggtgaagtga acaatgaaac atcacggaaa    1200
   tcagcttgca gagaggactc caatgataac tacatatatt gtccaactgc tagagaagtt    1260
   caaagtggag ctttagatca ttttcagcag cattggttga atggtcagcc agtcattgtt    1320
   cgtgacgtgc ttgagctaac ttctggactg agctgggagc caatggttat gtggcgggcc   1380
   ttacgggaaa agagagacaa gaaagagcat gaacggctct cagttatagc tcttgattgt   1440
   ctgacatggt ttgagtttat gtaccaccag gatgtgttgg tggtgccagt ttcatatctg   1500
   gggttcaaca gcaccattga aactacttta tatttcaaac tggttgatat aaatattcac   1560
   atgttttttg aggggtattc ccgtggagct gttggttcgg aggatttgcc tgtgctactt   1620
   aagcttaaag attggccaca acatagttcc tttgaggagc gcctgccgcg gcatggtgct   1680
   gagttcatgt ctgcactacc atttcgtgag tatacagatc caaaatctgg tccccttaat   1740
   ctggcagtga agcttccaaa acatgttaaa aagccagatc ttggtccaaa gacatacatt   1800
   gcttatggtg tcgcccaaga gttgggaata ggtgattctg ttacgaagat ccattgcgac   1860
   atgtctgatg cggttaatat tctcatgcat actgatgagg tagaactcaa agctgaaagg   1920
   attacagcaa tagagaaaaa gaaagagagc ttacgtaaag atggcaagaa tcttcatgtt   1980
   ctgcgaccag accatgacga tgatacatca atagctctca gtgaatcaac tgaagtgcca   2040
   agatctcgag gacttgaaaa tggctcaagc attaagcagc cagcaccaaa tgttgctgta   2100
   atggaccagg gaggtgttca tacagatatg gtagctgatg aagctgaggg aaacttgagt   2160
   ctgtctaatg ggcaatcacc caatcaaagt gatgcacata atatggatat tactttctcc   2220
   aagggggaaa cagaccattc tatctgcaca attaatgggg gagaggagat gggcaatggc   2280
   tttgggaggg aagataaatg taaatcttct catggtgtag gatcatctga atcttctgat   2340
   tgtcaaagac gcagtaggcg acgtgatgct tgctcttcca gtgcaacagg ggaaataaac   2400
   gaaaccagca tggagactaa caaatttacc atatctattg agcccaaaga tgatcaccca   2460
   tttgttgagg gaaatcagac agagggtggt gcattgtggg atatcttccg acgtgaagat   2520
   gtcagcaaat tgcatgatta tctgatgaag catgcagaag agtttagaca ttataattat   2580
   gaaacagtca aacaggtttc tcatcctatt cacgatcaat gcttttatct aacaaatgag   2640
   cacaagagaa agctcaagga agaacatgga attgagcctt ggacatttga acagaaactt   2700
   ggtgaggctg tgtttatccc agcaggatgt ccccaccaag tcagaaattt gaagtcatgt   2760
   ataaaggttg cacttgactt tgtttctccg gaaaatgtgc aagagtgcat caggctgaca   2820
   gaagaatttc gcttgcttcc gaagggtcat agggtgaatg aagataaact agaggttaag   2880
   aagatagctc tatatgcact tgatcaagcc attgacgata tcacaggcaa aagttgcaat   2940
   gaaaggacaa aggatgaagg ggaagaggag gcgtctgccc caagtgttag ttaa         2994
<210> 68
   <211> 997
   <212> PRT
   <213> Oryza sativa
<400> 68
   Met Pro Pro Lys Arg Lys Arg Gly Gly Gly Arg Pro Arg Lys Ala Pro
   1               5                   10                  15
   Glu Asp Ala Ala Ala Lys Glu Asn Gly Glu Lys Thr Asn Lys Glu Glu
               20                  25                  30
   Glu Thr Gln Ala Ser Pro Glu Glu Asn Gly Ala Gly Gln Thr Gln Ala
           35                  40                  45
   Ser Arg Thr Ala Arg Lys Arg Arg Lys Gly Pro Val Ala Asp Pro Ser
       50                  55                  60
   Ser Thr Glu Leu Pro Pro Arg Lys Leu Arg Asp Arg Arg Asn Val Pro
   65                  70                  75                  80
   Ala Val Asp Tyr Lys Glu Asn Lys His Thr Lys Lys Met Asp Gly Thr
                   85                  90                  95
   Ser Thr Met Cys His Gln Cys Gln Arg Lys Asp Ser Gly Arg Val Val
               100                 105                 110
   Arg Cys Arg Asn Gly Ala Glu Lys Asn Arg Arg His Arg Tyr Cys Val
           115                 120                 125
   Lys Cys Ile Lys Arg Trp Tyr Pro His Leu Thr Glu Asp Asp Phe Glu
       130                 135                 140
   Asn Cys Cys Pro Val Cys His Asn Asn Cys Asn Cys Lys Thr Cys Leu
   145                 150                 155                 160
   Arg Thr Asn Val Ile Asn Lys Gly Asp Lys Glu Phe Ala Asp Gly Lys
                   165                 170                 175
   Asn Lys Ile Lys Tyr Ser Leu Arg Ile Ala Arg Phe Leu Leu Pro Trp
               180                 185                 190
   Leu Lys Gln Leu His Gln Glu Gln Met Leu Glu Lys Ser Val Glu Ala
           195                 200                 205
   Thr Ile Lys Gly Ile Asp Val Thr Asp Leu Glu Val Pro Gln Ala Gln
       210                 215                 220
   Phe Asn Asn Asp Glu Arg Ile Tyr Cys Asp Asn Cys Arg Thr Ser Ile
   225                 230                 235                 240
   Val Asp Phe His Arg Ser Cys Lys Ser Gly His Tyr Asp Leu Cys Leu
                   245                 250                 255
   Ser Cys Cys Gln Glu Leu Arg Gln Gly Leu Thr Thr Gly Thr Val Val
               260                 265                 270
   Thr Cys Asp Thr Ala Val Asp Val Pro Glu Ile Glu Gly Lys Glu Gly
           275                 280                 285
   Leu Gln Glu Gly Ser Ser His Ser Ser Ala Val Gly Gln Gly Ala Ser
       290                 295                 300
   Asp Gln Gln Asn Asp Arg Leu Ile Gly Ser Ala Ala Pro Ser Glu Asp
   305                 310                 315                 320
   Cys Thr Pro Ser Leu Ile Trp Arg Ala Lys Ser Asn Gly Ser Ile Pro
                   325                 330                 335
   Cys Pro Pro Asn Ala Gly Gly Cys Gly Asp Cys Leu Leu Glu Leu Arg
               340                 345                 350
   Cys Leu Phe Lys Glu Asn Phe Ile Ser Asp Leu Leu Asp Lys Val Asn
           355                 360                 365
   Ser Val Val Asn Lys Glu Thr Glu Gln Glu Leu Gly Gly Ser Arg Cys
       370                 375                 380
   Ser Cys Phe Thr Glu Ser Gly Glu Val Asn Asn Glu Thr Ser Arg Lys
   385                 390                 395                 400
   Ser Ala Cys Arg Glu Asp Ser Asn Asp Asn Tyr Ile Tyr Cys Pro Thr
                   405                 410                 415
   Ala Arg Glu Val Gln Ser Gly Ala Leu Asp His Phe Gln Gln His Trp
               420                 425                 430
   Leu Asn Gly Gln Pro Val Ile Val Arg Asp Val Leu Glu Leu Thr Ser
           435                 440                 445
   Gly Leu Ser Trp Glu Pro Met Val Met Trp Arg Ala Leu Arg Glu Lys
       450                 455                 460
   Arg Asp Lys Lys Glu His Glu Arg Leu Ser Val Ile Ala Leu Asp Cys
   465                 470                 475                 480
   Leu Thr Trp Phe Glu Phe Met Tyr His Gln Asp Val Leu Val Val Pro
                   485                 490                 495
   Val Ser Tyr Leu Gly Phe Asn Ser Thr Ile Glu Thr Thr Leu Tyr Phe
               500                 505                 510
   Lys Leu Val Asp Ile Asn Ile His Met Phe Phe Glu Gly Tyr Ser Arg
           515                 520                 525
   Gly Ala Val Gly Ser Glu Asp Leu Pro Val Leu Leu Lys Leu Lys Asp
       530                 535                 540
   Trp Pro Gln His Ser Ser Phe Glu Glu Arg Leu Pro Arg His Gly Ala
   545                 550                 555                 560
   Glu Phe Met Ser Ala Leu Pro Phe Arg Glu Tyr Thr Asp Pro Lys Ser
                   565                 570                 575
   Gly Pro Leu Asn Leu Ala Val Lys Leu Pro Lys His Val Lys Lys Pro
                   580                 585                 590
   Asp Leu Gly Pro Lys Thr Tyr Ile Ala Tyr Gly Val Ala Gln Glu Leu
               595                 600                 605
   Gly Ile Gly Asp Ser Val Thr Lys Ile His Cys Asp Met Ser Asp Ala
       610                 615                 620
   Val Asn Ile Leu Met His Thr Asp Glu Val Glu Leu Lys Ala Glu Arg
   625                 630                 635                 640
   Ile Thr Ala Ile Glu Lys Lys Lys Glu Ser Leu Arg Lys Asp Gly Lys
                   645                 650                 655
   Asn Leu His Val Leu Arg Pro Asp His Asp Asp Asp Thr Ser Ile Ala
               660                 665                 670
   Leu Ser Glu Ser Thr Glu Val Pro Arg Ser Arg Gly Leu Glu Asn Gly
           675                 680                 685
   Ser Ser Ile Lys Gln Pro Ala Pro Asn Val Ala Val Met Asp Gln Gly
       690                 695                 700
   Gly Val His Thr Asp Met Val Ala Asp Glu Ala Glu Gly Asn Leu Ser
   705                 710                 715                 720
   Leu Ser Asn Gly Gln Ser Pro Asn Gln Ser Asp Ala His Asn Met Asp
                   725                 730                 735
   Ile Thr Phe Ser Lys Gly Glu Thr Asp His Ser Ile Cys Thr Ile Asn
               740                 745                 750
   Gly Gly Glu Glu Met Gly Asn Gly Phe Gly Arg Glu Asp Lys Cys Lys
           755                 760                 765
   Ser Ser His Gly Val Gly Ser Ser Glu Ser Ser Asp Cys Gln Arg Arg
       770                 775                 780
   Ser Arg Arg Arg Asp Ala Cys Ser Ser Ser Ala Thr Gly Glu Ile Asn
   785                 790                 795                 800
   Glu Thr Ser Met Glu Thr Asn Lys Phe Thr Ile Ser Ile Glu Pro Lys
                   805                 810                 815
   Asp Asp His Pro Phe Val Glu Gly Asn Gln Thr Glu Gly Gly Ala Leu
               820                 825                 830
   Trp Asp Ile Phe Arg Arg Glu Asp Val Ser Lys Leu His Asp Tyr Leu
           835                 840                 845
   Met Lys His Ala Glu Glu Phe Arg His Tyr Asn Tyr Glu Thr Val Lys
       850                 855                 860
   Gln Val Ser His Pro Ile His Asp Gln Cys Phe Tyr Leu Thr Asn Glu
   865                 870                 875                 880
   His Lys Arg Lys Leu Lys Glu Glu His Gly Ile Glu Pro Trp Thr Phe
                   885                 890                 895
   Glu Gln Lys Leu Gly Glu Ala Val Phe Ile Pro Ala Gly Cys Pro His
               900                 905                 910
   Gln Val Arg Asn Leu Lys Ser Cys Ile Lys Val Ala Leu Asp Phe Val
           915                 920                 925
   Ser Pro Glu Asn Val Gln Glu Cys Ile Arg Leu Thr Glu Glu Phe Arg
       930                 935                 940
   Leu Leu Pro Lys Gly His Arg Val Asn Glu Asp Lys Leu Glu Val Lys
   945                 950                 955                 960
   Lys Ile Ala Leu Tyr Ala Leu Asp Gln Ala Ile Asp Asp Ile Thr Gly
                   965                 970                 975
   Lys Ser Cys Asn Glu Arg Thr Lys Asp Glu Gly Glu Glu Glu Ala Ser
               980                 985                 990
   Ala Pro Ser Val Ser
           995
<210> 69
   <211> 2988
   <212> DNA
   <213> Oryza sativa
<400> 69
<210> 70
   <211> 995
   <212> PRT
   <213> Oryza sativa
<400> 70
   Met Ala Ala Glu Glu Val Glu Ala Ala Ala Ile Asp Gly Ser Gly Glu
   1               5                   10                  15
   Glu Ala Lys Arg Lys Ser Gly Lys Gln Arg Gly Ser Gly Ala Lys Gly
               20                  25                  30
   Arg Arg Arg Asn Gly Asp Arg Ala Phe Arg Pro Pro Ala Met Arg Pro
           35                  40                  45
   Glu Glu Glu Gly Arg Gly Val Ala Thr Arg Pro Gly Ala Leu Arg Glu
       50                  55                  60
   Arg Lys Pro Pro Pro Asn Ala Phe Asn Ala Pro Asp Asp Asp Glu Asp
   65                  70                  75                  80
   Val Glu Lys Thr Asp Gln Leu Leu Glu Pro Leu Asn Lys Pro Lys Arg
                   85                  90                  95
   Arg Asp Ala Gly Lys Lys Arg Gly Pro Arg Lys Lys Lys Val Asp Gln
               100                 105                 110
   Glu Asn Ile Lys Thr His Arg His Asn Ala Asn Ala Val Lys Gly Lys
           115                 120                 125
   Met Leu Val Asn Asp Lys Val Ser Lys Thr Glu Lys Lys Arg Lys Arg
       130                 135                 140
   Gly Asp Thr Gly Ala Ala Glu Asn Asn Gly Lys Gly Lys Lys Met Leu
   145                 150                 155                 160
   Thr Gly Glu Asn Ala Leu Met Cys His Gln Cys Gln Arg Asn Asp Lys
                   165                 170                 175
   Gly Arg Val Val Trp Cys Lys Thr Cys Asn Asn Lys Arg Phe Cys Val
               180                 185                 190
   Pro Cys Ile Asn Gln Trp Tyr Pro Asp Leu Pro Glu Asn Glu Phe Ala
           195                 200                 205
   Ala Lys Cys Pro Tyr Cys Arg Lys Asn Cys Asn Cys Lys Ala Cys Leu
       210                 215                 220
   Arg Met Arg Gly Val Glu Glu Pro Pro Arg Lys Glu Ile Ser Lys Glu
   225                 230                 235                 240
   Asn Gln Ile Arg Tyr Ala Cys His Val Leu Arg Leu Leu Arg Pro Trp
                   245                 250                 255
   Leu Ile Glu Leu Arg Gln Glu Gln Met Ala Glu Lys Glu Leu Glu Ala
               260                 265                 270
   Lys Ile Gln Gly Val Ser Val Asp Gln Ile Lys Val Glu Gln Ala Val
           275                 280                 285
   Cys Asp Leu Asp Glu Arg Val Tyr Cys Asn Arg Cys Ser Thr Ser Ile
       290                 295                 300
   Val Asp Phe His Arg Ser Cys Lys His Cys Phe Tyr Asp Leu Cys Leu
   305                 310                 315                 320
   Thr Cys Cys Gln Glu Leu Arg Lys Gly Glu Ile Pro Gly Gly Glu Glu
                   325                 330                 335
   Val Glu Ile Leu Asp Pro Glu Glu Arg Asp Lys Asp Tyr Ala Phe Gly
               340                 345                 350
   Lys Ile Leu Ser Asp Gly Glu Asn Gln Arg Asp Ser Leu Lys Cys Arg
           355                 360                 365
   Ser Asp Thr Gln Asn Ser Glu Ser Asn Lys Gly Met Ala Ser Asp Glu
       370                 375                 380
   Asn Gln Lys Lys Ala Leu Leu Leu Trp Lys Ala Asn Ser Asn Gly Ser
   385                 390                 395                 400
   Ile Pro Cys Pro Arg Lys Glu Lys Glu Asp Cys Ser Phe Ser Ser Leu
                   405                 410                 415
   Asp Leu Lys Cys Leu Phe Pro Glu Lys Leu Leu Pro Glu Leu Glu Asp
               420                 425                 430
   Arg Ser Glu Lys Val Phe Trp Ser Glu Thr Phe Ala Lys Glu Leu Gly
           435                 440                 445
   Arg Thr Ser Glu Leu Cys Pro Cys Phe Asp His Ser Gly Lys Ile Arg
       450                 455                 460
   Ser Asp Ser Lys Lys Leu Arg Gln Ala Ala Asn Arg Glu Asp Ser Ser
   465                 470                 475                 480
   Asp Asn Tyr Leu Tyr Cys Pro Val Ala Thr Asp Ile Gln Asp Ala Asp
                   485                 490                 495
   Leu Leu His Phe Gln Met His Trp Ala Lys Gly Glu Pro Val Val Val
               500                 505                 510
   Ser Asp Thr Leu Lys Leu Thr Ser Gly Leu Ser Trp Glu Pro Met Val
           515                 520                 525
   Met Trp Arg Ala Val Arg Glu Arg Thr Lys Gly Lys Ala Glu Asp Glu
       530                 535                 540
   Gln Phe Ala Val Arg Ala Val Asp Cys Leu Asp Trp Cys Glu Val Glu
   545                 550                 555                 560
   Ile Asn Ile His Met Phe Phe Met Gly Tyr Thr Arg Gly Arg Thr His
                   565                 570                 575
   Pro Arg Thr Tyr Trp Pro Glu Met Leu Lys Leu Lys Asp Trp Pro Pro
               580                 585                 590
   Ser Ser Ser Phe Asp Gln Arg Leu Pro Arg His Gly Ala Glu Phe Ile
           595                 600                 605
   Ser Ala Leu Pro Phe Pro Glu Tyr Thr Asp Pro Arg Tyr Gly Pro Leu
       610                 615                 620
   Asn Leu Ala Val Lys Leu Pro Gly Gly Val Leu Lys Pro Asp Leu Gly
   625                 630                 635                 640
   Pro Lys Thr Tyr Ile Ala Tyr Gly Phe Ser Glu Glu Leu Gly Arg Gly
                   645                 650                 655
   Asp Ser Val Thr Lys Leu His Cys Asp Met Ser Asp Ala Val Asn Ile
               660                 665                 670
   Leu Thr His Thr Ala Glu Val Pro Cys Glu Thr Tyr Asp Ala Val Gln
           675                 680                 685
   Ile Lys Asn Thr Gln Lys Lys Met Lys Met Gln Asp Asp Met Glu Ile
       690                 695                 700
   Tyr Gly Met Ile Glu Ser Gly Ser Glu Leu Lys Pro Ser Ala Cys Pro
   705                 710                 715                 720
   Val Glu Leu Gly Asn Lys Ala Val Gly Glu Ala Pro Lys Ala Ser Cys
                   725                 730                 735
   Ser Lys Glu Asn Val His Thr Leu Lys Asp Lys Ser Asn Gly Leu Asp
               740                 745                 750
   Ile Asn Ala Ser Pro Pro Asp Asp Ala Gly Gly Asp Ala Arg Asp Glu
           755                 760                 765
   Ala Leu Ser Tyr Glu Ser Val Val His Ser Asp Val Ala Gln Cys Pro
       770                 775                 780
   Asn His Asn His Glu Thr Asn Asn Ser Asp Asp Ala Arg Ile Gly Ala
   785                 790                 795                 800
   Gln Arg Cys Gln Lys Lys Ala Lys Gly Arg Pro Pro Lys Thr Gly Ser
                   805                 810                 815
   Gly Val Ser Glu His Gln Glu Ser Gly Gly Ala Leu Trp Asp Ile Phe
               820                 825                 830
   Arg Arg Glu Asp Ser Glu Lys Leu Gln Asp Phe Leu Arg Lys His Ala
           835                 840                 845
   Pro Glu Phe Arg His Ile His Cys Asn Pro Val Lys Gln Val Ile His
       850                 855                 860
   Pro Ile His Asp Gln Ala Phe Tyr Leu Thr Ala Glu His Lys Arg Lys
   865                 870                 875                 880
   Leu Lys Glu Glu Tyr Gly Val Glu Pro Trp Thr Phe Glu Gln Lys Leu
                   885                 890                 895
   Gly Glu Ala Val Leu Ile Pro Ala Gly Cys Pro His Gln Val Arg Asn
               900                 905                 910
   Leu Lys Ser Cys Ile Lys Val Ala Leu Asp Phe Val Ser Pro Glu Asn
           915                 920                 925
   Val Gly Glu Cys Val Arg Leu Thr Lys Glu Phe Arg Arg Leu Pro Ser
       930                 935                 940
   Ser His Arg Ala Lys Glu Asp Lys Leu Glu Ile Lys Lys Met Ala Phe
   945                 950                 955                 960
   His Ala Leu Asn Glu Val Leu Asn Phe Leu Asp Pro Pro Ser Ser Glu
                   965                 970                 975
   Gly Ser Lys Glu Ala Ala Glu Lys Pro Arg Arg Gly Arg Gly Arg Pro
               980                 985                 990
   Arg Lys His
           995
<210> 71
   <211> 2607
   <212> DNA
   <213> Oryza sativa
<400> 71
   atgttagtca atgataaggt ttcgaagact gaaaagaagc gtaagagagg agacacagga      60
   gcggcagaaa ataatgggaa agggaagaaa atgttgacag gtgaaaacgc cttaatgtgt     120
   caccaatgcc agagaaatga caaagggaga gtggtctggt gcaagacttg caacaataag     180
   cgcttttgtg tgccatgcat taatcaatgg tatcctgatt tgcctgaaaa tgaatttgct     240
   gcaaaatgcc cttattgtcg caagaattgt aattgcaagg catgcctgcg aatgagaggt     300
   gtcgaagagc agcctccaag aaaggaaatt tctaaagaaa atcagattcg ttatgcctgt     360
   catgtgttac gcttgttgcg tccttggctg atagaactgc gacaggagca gatggcagag     420
   aaggaattag aggctaagat acaaggtgtt tcagttgatc aaataaaggt ggaacaagct     480
   gtgtgtgatc tagatgagcg tgtctattgc aatagatgca gcacttctat agttgatttt     540
   catagaagct gcaagcactg tttctatgac ttgtgcttga cctgctgcca ggagcttcgc     600
   aaaggtgaga tccctggagg agaagaggtg gaaattttgg atcctgaaga aagagacaag     660
   gattatgctt ttggtaagat tctgtcagat ggtgagaacc aaagggactc tttgaagtgt      720
   cgcagcgaca cccaaaacag tgagtcaaac aaaggcatgg cttctgacga aaaccaaaag     780
   aaggctctgt tactttggaa agcaaacagt aatggtagca taccttgccc acgaaaggaa     840
   aaggaagact gcagtttttc atctctagat ctgaaatgct tgtttccaga gaagttgctc     900
   cctgaattag aagatagatc tgagaaggtc ttctggagtg aaacatttgc aaaagaacta     960
   ggtagaacaa gtgagctgtg cccttgtttt gatcactcag gcaagataag aagtgacagc    1020
   aagaaattac gtcaggctgc aaatagggag gactcgagtg ataactactt atactgtccg    1080
   gtggccactg atatccagga cgctgacctg ttgcactttc agatgcattg ggcaaagggc    1140
   gaaccagttg ttgtttcaga cactcttaag ctaacttctg gtcttagctg ggagccgatg    1200
   gttatgtggc gggcagtacg agaacgaacc aaagggaagg cagaagatga gcagtttgct   1260
   gtgagggcag tagattgcct cgactggtgt gaggtggaaa ttaacataca catgtttttt   1320
   atgggatata caaggggcag aactcatccc aggacctact ggcctgagat gcttaagcta   1380
   aaggactggc ccccttctag ctcatttgat cagcgattgc ctcgccatgg tgctgaattt   1440
   atcagtgcat tgccatttcc ggagtatact gatccacgat atggtccgct aaatcttgca   1500
   gtgaagcttc ctggtggtgt cctgaagcca gatcttgggc caaaaactta cattgcctat   1560
   ggattttctg aagagttggg caggggtgac tctgtaacca aacttcactg tgacatgtct   1620
   gatgcagtaa acatcttaac acacacagcg gaagtgccct gtgagactta tgatgctgtt   1680
   cagattaaaa acacgcagaa aaagatgaaa atgcaagatg acatggaaat atatgggatg   1740
   atagaatcag gcagtgaact caagccatca gcatgcccag ttgaattggg gaacaaagct   1800
   gttggtgaag caccaaaggc ctcatgcagc aaagaaaatg ttcatacctt gaaagacaag   1860
   tctaatggtt tggatataaa cgcttcacca cctgatgatg ctggaggtga tgccagggat   1920
   gaagcattgt cctatgaatc tgtggtacat agtgatgtag ctcagtgtcc aaaccataac   1980
   catgagacca ataattctga tgatgcacgt attggagctc agagatgtca aaagaaagct   2040
   aaaggtcgtc ctccgaagac aggttcaggg gtatcagagc accaggaaag tggtggtgct   2100
   ttgtgggata ttttccgaag agaggactct gagaaattac aagacttcct taggaagcat   2160
   gcgccagaat ttcggcacat ccactgcaat ccagtgaaac aggttattca tccaattcat   2220
   gatcaagctt tctatttaac tgcggagcat aaaagaaagc tgaaggaaga atatggtgtt   2280
   gaaccttgga cctttgaaca aaagcttggc gaagcagttt tgattcctgc cggatgtcct   2340
   caccaagtca ggaatttgaa gtcctgcatc aaggttgcac tggactttgt ttcccccgag   2400
   aatgttggtg agtgtgttag gctgaccaag gaatttagac gacttccatc ttcccacagg   2460
   gctaaagaag ataagctaga gatcaagaag atggctttcc atgctctgaa tgaagttcta   2520
   aactttctgg atcctccttc ctcagaaggg tcgaaagaag cggcggagaa gcctcgaagg   2580
   gggcgtggtc gcccaagaaa acactag                                      2607
<210> 72
   <211> 868
   <212> PRT
   <213> Oryza sativa
<400> 72
   Met Leu Val Asn Asp Lys Val Ser Lys Thr Glu Lys Lys Arg Lys Arg
   1               5                   10                  15
   Gly Asp Thr Gly Ala Ala Glu Asn Asn Gly Lys Gly Lys Lys Met Leu
               20                  25                  30
   Thr Gly Glu Asn Ala Leu Met Cys His Gln Cys Gln Arg Asn Asp Lys
           35                  40                  45
   Gly Arg Val Val Trp Cys Lys Thr Cys Asn Asn Lys Arg Phe Cys Val
       50                  55                  60
   Pro Cys Ile Asn Gln Trp Tyr Pro Asp Leu Pro Glu Asn Glu Phe Ala
   65                  70                  75                  80
   Ala Lys Cys Pro Tyr Cys Arg Lys Asn Cys Asn Cys Lys Ala Cys Leu
                   85                  90                  95
   Arg Met Arg Gly Val Glu Glu Gln Pro Pro Arg Lys Glu Ile Ser Lys
               100                 105                 110
   Glu Asn Gln Ile Arg Tyr Ala Cys His Val Leu Arg Leu Leu Arg Pro
           115                 120                 125
   Trp Leu Ile Glu Leu Arg Gln Glu Gln Met Ala Glu Lys Glu Leu Glu
       130                 135                 140
   Ala Lys Ile Gln Gly Val Ser Val Asp Gln Ile Lys Val Glu Gln Ala
   145                 150                 155                 160
   Val Cys Asp Leu Asp Glu Arg Val Tyr Cys Asn Arg Cys Ser Thr Ser
                   165                 170                 175
   Ile Val Asp Phe His Arg Ser Cys Lys His Cys Phe Tyr Asp Leu Cys
                   180                     185                     190
   Leu Thr Cys Cys Gln Glu Leu Arg Lys Gly Glu Ile Pro Gly Gly Glu
           195                     200                     205
   Glu Val Glu Ile Leu Asp Pro Glu Glu Arg Asp Lys Asp Tyr Ala Phe
       210                     215                     220
   Gly Lys Ile Leu Ser Asp Gly Glu Asn Gln Arg Asp Ser Leu Lys Cys
   225                     230                     235                     240
   Arg Ser Asp Thr Gln Asn Ser Glu Ser Asn Lys Gly Met Ala Ser Asp
                   245                     250                     255
   Glu Asn Gln Lys Lys Ala Leu Leu Leu Trp Lys Ala Asn Ser Asn Gly
               260                     265                     270
   Ser Ile Pro Cys Pro Arg Lys Glu Lys Glu Asp Cys Ser Phe Ser Ser
           275                     280                     285
   Leu Asp Leu Lys Cys Leu Phe Pro Glu Lys Leu Leu Pro Glu Leu Glu
       290                     295                     300
   Asp Arg Ser Glu Lys Val Phe Trp Ser Glu Thr Phe Ala Lys Glu Leu
   305                     310                     315                     320
   Gly Arg Thr Ser Glu Leu Cys Pro Cys Phe Asp His Ser Gly Lys Ile
                   325                     330                     335
   Arg Ser Asp Ser Lys Lys Leu Arg Gln Ala Ala Asn Arg Glu Asp Ser
               340                     345                     350
   Ser Asp Asn Tyr Leu Tyr Cys Pro Val Ala Thr Asp Ile Gln Asp Ala
           355                     360                     365
   Asp Leu Leu His Phe Gln Met His Trp Ala Lys Gly Glu Pro Val Val
       370                     375                     380
   Val Ser Asp Thr Leu Lys Leu Thr Ser Gly Leu Ser Trp Glu Pro Met
   385                     390                     395                     400
   Val Met Trp Arg Ala Val Arg Glu Arg Thr Lys Gly Lys Ala Glu Asp
                   405                     410                     415
   Glu Gln Phe Ala Val Arg Ala Val Asp Cys Leu Asp Trp Cys Glu Val
               420                     425                     430
   Glu Ile Asn Ile His Met Phe Phe Met Gly Tyr Thr Arg Gly Arg Thr
           435                     440                     445
   His Pro Arg Thr Tyr Trp Pro Glu Met Leu Lys Leu Lys Asp Trp Pro
       450                     455                     460
   Pro Ser Ser Ser Phe Asp Gln Arg Leu Pro Arg His Gly Ala Glu Phe
   465                     470                     475                     480
   Ile Ser Ala Leu Pro Phe Pro Glu Tyr Thr Asp Pro Arg Tyr Gly Pro
                   485                     490                     495
   Leu Asn Leu Ala Val Lys Leu Pro Gly Gly Val Leu Lys Pro Asp Leu
               500                     505                     510
   Gly Pro Lys Thr Tyr Ile Ala Tyr Gly Phe Ser Glu Glu Leu Gly Arg
           515                     520                     525
   Gly Asp Ser Val Thr Lys Leu His Cys Asp Met Ser Asp Ala Val Asn
       530                     535                     540
   Ile Leu Thr His Thr Ala Glu Val Pro Cys Glu Thr Tyr Asp Ala Val
   545                     550                     555                     560
   Gln Ile Lys Asn Thr Gln Lys Lys Met Lys Met Gln Asp Asp Met Glu
                   565                     570                     575
   Ile Tyr Gly Met Ile Glu Ser Gly Ser Glu Leu Lys Pro Ser Ala Cys
               580                     585                     590
   Pro Val Glu Leu Gly Asn Lys Ala Val Gly Glu Ala Pro Lys Ala Ser
           595                     600                     605
   Cys Ser Lys Glu Asn Val His Thr Leu Lys Asp Lys Ser Asn Gly Leu
                   610                 615                 620
   Asp Ile Asn Ala Ser Pro Pro Asp Asp Ala Gly Gly Asp Ala Arg Asp
   625                 630                 635                 640
   Glu Ala Leu Ser Tyr Glu Ser Val Val His Ser Asp Val Ala Gln Cys
                   645                 650                 655
   Pro Asn His Asn His Glu Thr Asn Asn Ser Asp Asp Ala Arg Ile Gly
               660                 665                 670
   Ala Gln Arg Cys Gln Lys Lys Ala Lys Gly Arg Pro Pro Lys Thr Gly
           675                 680                 685
   Ser Gly Val Ser Glu His Gln Glu Ser Gly Gly Ala Leu Trp Asp Ile
       690                 695                 700
   Phe Arg Arg Glu Asp Ser Glu Lys Leu Gln Asp Phe Leu Arg Lys His
   705                 710                 715                 720
   Ala Pro Glu Phe Arg His Ile His Cys Asn Pro Val Lys Gln Val Ile
                   725                 730                 735
   His Pro Ile His Asp Gln Ala Phe Tyr Leu Thr Ala Glu His Lys Arg
               740                 745                 750
   Lys Leu Lys Glu Glu Tyr Gly Val Glu Pro Trp Thr Phe Glu Gln Lys
           755                 760                 765
   Leu Gly Glu Ala Val Leu Ile Pro Ala Gly Cys Pro His Gln Val Arg
       770                 775                 780
   Asn Leu Lys Ser Cys Ile Lys Val Ala Leu Asp Phe Val Ser Pro Glu
   785                 790                 795                 800
   Asn Val Gly Glu Cys Val Arg Leu Thr Lys Glu Phe Arg Arg Leu Pro
                   805                 810                 815
   Ser Ser His Arg Ala Lys Glu Asp Lys Leu Glu Ile Lys Lys Met Ala
               820                 825                 830
   Phe His Ala Leu Asn Glu Val Leu Asn Phe Leu Asp Pro Pro Ser Ser
           835                 840                 845
   Glu Gly Ser Lys Glu Ala Ala Glu Lys Pro Arg Arg Gly Arg Gly Arg
       850                 855                 860
   Pro Arg Lys His
   865
<210> 73
   <211> 1062
   <212> DNA
   <213> Oryza sativa
<400> 73
   atgccgcccc agcctccgcc ggcggcgtcg gcgtcggcca gtgccccgga tcccgccgtc      60
   ccggcttggc tcagggggct cccccgcgcg cccgagtacc gcccgacgga gtccgagttc    120
   gccgacccca tcgccttcct ctcccgcgtg gagcgcgagg ccgccgccta cggcatctgc     180
   aaggtcatcc cgccccaccc gcgcccttcc cgccgcttcg tcttcgccca cctcaaccgc     240
   tccctcgtct cctcctgcga cgcccccgcg ccctctcccg ccgccgcctc cgactcgtcc     300
   attcctccat cctcctcctc gccgccgccg gtctccgccg cggtgttcac gacacggcac     360
   caggagctcg gcaacccgcg gcgtggacgc ccgacgccgc aggtgcttaa gcaggtgtgg     420
   cagagtgggg agcggtacac gctcgaccag tttgagtcca agtcccgcgc cttctcaaag     480
   acgcacctcg ctggactcca cgaaccgact gcgctcgcgg tggaatcact cttctggaag     540
   gcgtcggcgg accgtcctat ctatattgag tacgccaatg atgtccctgg ctctgggttt     600
   gctgcacccg tgcagttgca acgcaagaag aagcagaaaa gagagactgc cccgatggac     660
   gaatgggaga agagttcagg ctggaggctg tcaaatagcc catggaacct gcaagcaatt     720
   gcgcgggctc ctggttcgct cacacggttt atgcctgacg atgttcctgg ggtgacttct     780
   ccaatggttt acattggcat gctgttcagc tggtttgcgt ggcatgtgga ggatcatgat     840
   ctgcatagtc tcaacttcct ccacactggc gcacctaaga cgtggtatgc ggttcctggt     900
   gatagggctg ttgagcttga ggaagttatc cgtgtacatg gctatggagg caacactgat     960
   cggatcgcgt cactagcagt gcttggtgag aaaacaacac taatgtcccc agaggtcctt    1020
   atagacaatg attggtgcaa tatcctggtg agtttgtggt ga                       1062
<210> 74
   <211> 353
   <212> PRT
   <213> Oryza sativa
<400> 74
   Met Pro Pro Gln Pro Pro Pro Ala Ala Ser Ala Ser Ala Ser Ala Pro
   1               5                   10                  15
   Asp Pro Ala Val Pro Ala Trp Leu Arg Gly Leu Pro Arg Ala Pro Glu
               20                  25                  30
   Tyr Arg Pro Thr Glu Ser Glu Phe Ala Asp Pro Ile Ala Phe Leu Ser
           35                  40                  45
   Arg Val Glu Arg Glu Ala Ala Ala Tyr Gly Ile Cys Lys Val Ile Pro
       50                  55                  60
   Pro His Pro Arg Pro Ser Arg Arg Phe Val Phe Ala His Leu Asn Arg
   65                  70                  75                  80
   Ser Leu Val Ser Ser Cys Asp Ala Pro Ala Pro Ser Pro Ala Ala Ala
                   85                  90                  95
   Ser Asp Ser Ser Ile Pro Pro Ser Ser Ser Ser Pro Pro Pro Val Ser
               100                 105                 110
   Ala Ala Val Phe Thr Thr Arg His Gln Glu Leu Gly Asn Pro Arg Arg
           115                 120                 125
   Gly Arg Pro Thr Pro Gln Val Leu Lys Gln Val Trp Gln Ser Gly Glu
       130                 135                 140
   Arg Tyr Thr Leu Asp Gln Phe Glu Ser Lys Ser Arg Ala Phe Ser Lys
   145                 150                 155                 160
   Thr His Leu Ala Gly Leu His Glu Pro Thr Ala Leu Ala Val Glu Ser
                   165                 170                 175
   Leu Phe Trp Lys Ala Ser Ala Asp Arg Pro Ile Tyr Ile Glu Tyr Ala
               180                 185                 190
   Asn Asp Val Pro Gly Ser Gly Phe Ala Ala Pro Val Gln Leu Gln Arg
           195                 200                 205
   Lys Lys Lys Gln Lys Arg Glu Thr Ala Pro Met Asp Glu Trp Glu Lys
       210                 215                 220
   Ser Ser Gly Trp Arg Leu Ser Asn Ser Pro Trp Asn Leu Gln Ala Ile
   225                 230                 235                 240
   Ala Arg Ala Pro Gly Ser Leu Thr Arg Phe Met Pro Asp Asp Val Pro
                   245                 250                 255
   Gly Val Thr Ser Pro Met Val Tyr Ile Gly Met Leu Phe Ser Trp Phe
               260                 265                 270
   Ala Trp His Val Glu Asp His Asp Leu His Ser Leu Asn Phe Leu His
           275                 280                 285
   Thr Gly Ala Pro Lys Thr Trp Tyr Ala Val Pro Gly Asp Arg Ala Val
       290                 295                 300
   Glu Leu Glu Glu Val Ile Arg Val His Gly Tyr Gly Gly Asn Thr Asp
   305                 310                 315                 320
   Arg Ile Ala Ser Leu Ala Val Leu Gly Glu Lys Thr Thr Leu Met Ser
                   325                 330                 335
   Pro Glu Val Leu Ile Asp Asn Asp Trp Cys Asn Ile Leu Val Ser Leu
                   340                 345                 350
   Trp
<210> 75
   <211> 2784
   <212> DNA
   <213> Oryza sativa
<400> 75
   atggaggcgg cggcggcggc ggccgccgtg ccggaggagc tgcggtgcaa gcgctccgac     60
   ggcaagcagt ggcgatgcag cgcgccctcc atgcccgaca agaccgtctg cgagaagcac    120
   tacgtccagg ccaagaagcg cgccgcctcc tccgcgctcc gggcctccct ccgcaggtcc    180
   tccgcctccg cctccgccgc gcggggcacg accccgcctg cgcggatggc ggtcgcgagg    240
   cccatctacg gcagggtcgc gggggagccg gtgtacgtgg cggagccggc gctgccgccg    300
   ccgccgccgc cgccacggag gaggcagccg gtccacgggc tgcccatggg caatgctgcc    360
   ggcgcgcgca ccgcggcgga gctggtcggg aggggctcgg cggggctggt tgcctgcagc    420
   tcggcggcgg gggcggcagc ggccgcaacc tgccatcagt gccggagggt cgctaatacc    480
   atttgttgta caagctgcga cagaaggggg tactgcacca actgcatctc gagatggtac    540
   tctgatatcc caattgatga tgttcgaaag gtttgtccag catgtcgtgg catttgtaac    600
   tgcagagttt gcttactagg agataatgta ataaaggcaa gggttcagga gatatctgct    660
   gtagataagt tggaatatct tcatagcatt ctggcatctg tccttccagt attaaagcag    720
   atttattctg atcaatgttt cgaaattggt gttgatacaa aagcttatgg acttaggaca    780
   gatataatca gggcgaaggt caatcctgat gagcaaatgt gctgtgactt ctgcaaagtg    840
   ccagtatttg attatcaccg acactgccca aggtgtttgt atgacttatg tcttgactgt    900
   tgtcgagaca taaggcgatc taggaccagt gttgcaagag gagaatatgc tgaaggtcgt    960
   gtggtagata gaagtaaaga tacttcgaat aaaagagcaa gaatggaacc atctgcagaa   1020
   agtgcaaatg ataagtcagt cccacaacga agggacataa aaaatattga cattagatct   1080
   ttattcccta catggagagt caataatgat ggaagcatta cttgtgggcc tcatgaggct   1140
   ggtggttgtg gctcctcgaa gttagtgtta aggcgaatat tcaaaataaa ctggattagt   1200
   aagcttgtaa aaaattctga ggaaatggtc aatggttgta aagtacatgt tcttgagaat   1260
   ggatgctcat cctgcaatga tggcagaaca ttagagttaa ctggtcatcg taactttggt   1320
   gtctcaacat gttcaaataa tggtggtatt gatcggttct gtgtgttctc tcctgtatta   1380
   gaggacttga aatctgaagg tattattcat tttcgtaaac actggataaa aggagaacct   1440
   gtagttatca ggaatgcgtt tgagccttct ctgtcatcaa gctgggaccc actaaatatt   1500
   tggcgaggaa tccaggaaat catggatgaa gaagtggatg acgatgtcat tgtcaaagct   1560
   gtggattgct caaaccaagc agaggtggat attgagctga aacagtttat caaaggttat   1620
   tcagatggtc acaaggggga agatggggaa ttgatgatgc tgaaattgaa agagtggccc   1680
   ccacccagtg tattggagga gtttctgcta tgccaaagac cagaatttat tgtcaatttt   1740
   ccattagttg attttataca ttccaggtgg gggctcctaa atctttctgc taaattgccc   1800
   ccagacaccc tacaacctga agttggcttg aagctgttaa ttgcatatgg aagacatcaa   1860
   gaagctggta aaggtgattc agtgacaaat ctaatgatta acatggctga tgtggtgcat   1920
   atgctaatgc atacagccaa agggcatgac gtatgtccaa agaggctaca acctgagcga   1980
   tctgaaaaga ttgccaatgg aatgacaatg catgtaaatg ctcatgcacc tgttcaaaat   2040
   ttgaatgtgg atatggggga acaatcacct gaccatgtaa gctcaaagtt tgatgaaaga   2100
   gcgcatgcgt ctgccttgcg attacaagag aagtcttcag atgccaaact taattgtggt   2160
   ttcgaaggct cttcaactga gttatcttgc tcgtcacatt cagaggaacc aaaagttaat   2220
   ggttcagaaa gaagtcaggc tggttctgtt tgggatgtgt tccgcaggca ggatatttca   2280
   aagctgaatg aatatttaac tgctaactgg gaagaactgg cagctagtag tcaggttaag   2340
   aatcctattt atgaacaatc tatatatctc aacaagtatc ataaaaggat actgaaggat   2400
   caatatggaa ttgaaccctg gacattccaa caacatatcg gtgaggctgt atttgttcct   2460
   gctggctgcc cattccaagt gaaaaatctc cagtctacgg ttcaattggc tcttgatttc   2520
   ctgtcaccgg aaagtttggg ggagtcggcc cgaatggccc aggagattcg ctgcctacca   2580
   aatgatcatg atgcaaaact gaagatgctc gagattggaa aaatctcttt atatgcagct   2640
   agttctgctg tcagagaaat tcagagaata accctcgatc ccaagtttaa tctagacctt   2700
   aaattcgagg atcagaatct aactcaggcg gtttctgaga acttggctag agtcaccaaa   2760
   caacggaatg taccatgcag ctga                                          2784
<210> 76
   <211> 927
   <212> PRT
   <213> Oryza sativa
<400> 76
   Met Glu Ala Ala Ala Ala Ala Ala Ala Val Pro Glu Glu Leu Arg Cys
   1               5                   10                  15
   Lys Arg Ser Asp Gly Lys Gln Trp Arg Cys Ser Ala Pro Ser Met Pro
               20                  25                  30
   Asp Lys Thr Val Cys Glu Lys His Tyr Val Gln Ala Lys Lys Arg Ala
           35                  40                  45
   Ala Ser Ser Ala Leu Arg Ala Ser Leu Arg Arg Ser Ser Ala Ser Ala
       50                  55                  60
   Ser Ala Ala Arg Gly Thr Thr Pro Pro Ala Arg Met Ala Val Ala Arg
   65                  70                  75                  80
   Pro Ile Tyr Gly Arg Val Ala Gly Glu Pro Val Tyr Val Ala Glu Pro
                   85                  90                  95
   Ala Leu Pro Pro Pro Pro Pro Pro Pro Arg Arg Arg Gln Pro Val His
               100                 105                 110
   Gly Leu Pro Met Gly Asn Ala Ala Gly Ala Arg Thr Ala Ala Glu Leu
           115                 120                 125
   Val Gly Arg Gly Ser Ala Gly Leu Val Ala Cys Ser Ser Ala Ala Gly
       130                 135                 140
   Ala Ala Ala Ala Ala Thr Cys His Gln Cys Arg Arg Val Ala Asn Thr
   145                 150                 155                 160
   Ile Cys Cys Thr Ser Cys Asp Arg Arg Gly Tyr Cys Thr Asn Cys Ile
                   165                 170                 175
   Ser Arg Trp Tyr Ser Asp Ile Pro Ile Asp Asp Val Arg Lys Val Cys
               180                 185                 190
   Pro Ala Cys Arg Gly Ile Cys Asn Cys Arg Val Cys Leu Leu Gly Asp
           195                 200                 205
   Asn Val Ile Lys Ala Arg Val Gln Glu Ile Ser Ala Val Asp Lys Leu
       210                 215                 220
   Glu Tyr Leu His Ser Ile Leu Ala Ser Val Leu Pro Val Leu Lys Gln
   225                 230                 235                 240
   Ile Tyr Ser Asp Gln Cys Phe Glu Ile Gly Val Asp Thr Lys Ala Tyr
                   245                 250                 255
   Gly Leu Arg Thr Asp Ile Ile Arg Ala Lys Val Asn Pro Asp Glu Gln
               260                 265                 270
   Met Cys Cys Asp Phe Cys Lys Val Pro Val Phe Asp Tyr His Arg His
           275                 280                 285
   Cys Pro Arg Cys Leu Tyr Asp Leu Cys Leu Asp Cys Cys Arg Asp Ile
       290                 295                 300
   Arg Arg Ser Arg Thr Ser Val Ala Arg Gly Glu Tyr Ala Glu Gly Arg
   305                 310                 315                 320
   Val Val Asp Arg Ser Lys Asp Thr Ser Asn Lys Arg Ala Arg Met Glu
                   325                 330                 335
   Pro Ser Ala Glu Ser Ala Asn Asp Lys Ser Val Pro Gln Arg Arg Asp
               340                 345                 350
   Ile Lys Asn Ile Asp Ile Arg Ser Leu Phe Pro Thr Trp Arg Val Asn
           355                 360                 365
   Asn Asp Gly Ser Ile Thr Cys Gly Pro His Glu Ala Gly Gly Cys Gly
       370                 375                 380
   Ser Ser Lys Leu Val Leu Arg Arg Ile Phe Lys Ile Asn Trp Ile Ser
   385                 390                 395                 400
   Lys Leu Val Lys Asn Ser Glu Glu Met Val Asn Gly Cys Lys Val His
                   405                 410                 415
   Val Leu Glu Asn Gly Cys Ser Ser Cys Asn Asp Gly Arg Thr Leu Glu
               420                 425                 430
   Leu Thr Gly His Arg Asn Phe Gly Val Ser Thr Cys Ser Asn Asn Gly
           435                 440                 445
   Gly Ile Asp Arg Phe Cys Val Phe Ser Pro Val Leu Glu Asp Leu Lys
       450                 455                 460
   Ser Glu Gly Ile Ile His Phe Arg Lys His Trp Ile Lys Gly Glu Pro
   465                 470                 475                 480
   Val Val Ile Arg Asn Ala Phe Glu Pro Ser Leu Ser Ser Ser Trp Asp
                   485                 490                 495
   Pro Leu Asn Ile Trp Arg Gly Ile Gln Glu Ile Met Asp Glu Glu Val
               500                 505                 510
   Asp Asp Asp Val Ile Val Lys Ala Val Asp Cys Ser Asn Gln Ala Glu
           515                 520                 525
   Val Asp Ile Glu Leu Lys Gln Phe Ile Lys Gly Tyr Ser Asp Gly His
       530                 535                 540
   Lys Gly Glu Asp Gly Glu Leu Met Met Leu Lys Leu Lys Glu Trp Pro
   545                 550                 555                 560
   Pro Pro Ser Val Leu Glu Glu Phe Leu Leu Cys Gln Arg Pro Glu Phe
                   565                 570                 575
   Ile Val Asn Phe Pro Leu Val Asp Phe Ile His Ser Arg Trp Gly Leu
               580                 585                 590
   Leu Asn Leu Ser Ala Lys Leu Pro Pro Asp Thr Leu Gln Pro Glu Val
           595                 600                 605
   Gly Leu Lys Leu Leu Ile Ala Tyr Gly Arg His Gln Glu Ala Gly Lys
       610                 615                 620
   Gly Asp Ser Val Thr Asn Leu Met Ile Asn Met Ala Asp Val Val His
   625                 630                 635                 640
   Met Leu Met His Thr Ala Lys Gly His Asp Val Cys Pro Lys Arg Leu
                   645                 650                 655
   Gln Pro Glu Arg Ser Glu Lys Ile Ala Asn Gly Met Thr Met His Val
               660                 665                 670
   Asn Ala His Ala Pro Val Gln Asn Leu Asn Val Asp Met Gly Glu Gln
           675                 680                 685
   Ser Pro Asp His Val Ser Ser Lys Phe Asp Glu Arg Ala His Ala Ser
       690                 695                 700
   Ala Leu Arg Leu Gln Glu Lys Ser Ser Asp Ala Lys Leu Asn Cys Gly
   705                 710                 715                 720
   Phe Glu Gly Ser Ser Thr Glu Leu Ser Cys Ser Ser His Ser Glu Glu
                   725                 730                 735
   Pro Lys Val Asn Gly Ser Glu Arg Ser Gln Ala Gly Ser Val Trp Asp
               740                 745                 750
   Val Phe Arg Arg Gln Asp Ile Ser Lys Leu Asn Glu Tyr Leu Thr Ala
           755                 760                 765
   Asn Trp Glu Glu Leu Ala Ala Ser Ser Gln Val Lys Asn Pro Ile Tyr
       770                 775                 780
   Glu Gln Ser Ile Tyr Leu Asn Lys Tyr His Lys Arg Ile Leu Lys Asp
   785                 790                 795                 800
   Gln Tyr Gly Ile Glu Pro Trp Thr Phe Gln Gln His Ile Gly Glu Ala
                   805                 810                 815
   Val Phe Val Pro Ala Gly Cys Pro Phe Gln Val Lys Asn Leu Gln Ser
               820                 825                 830
   Thr Val Gln Leu Ala Leu Asp Phe Leu Ser Pro Glu Ser Leu Gly Glu
           835                 840                 845
   Ser Ala Arg Met Ala Gln Glu Ile Arg Cys Leu Pro Asn Asp His Asp
       850                 855                 860
   Ala Lys Leu Lys Met Leu Glu Ile Gly Lys Ile Ser Leu Tyr Ala Ala
   865                 870                 875                 880
   Ser Ser Ala Val Arg Glu Ile Gln Arg Ile Thr Leu Asp Pro Lys Phe
                   885                 890                 895
   Asn Leu Asp Leu Lys Phe Glu Asp Gln Asn Leu Thr Gln Ala Val Ser
               900                 905                 910
   Glu Asn Leu Ala Arg Val Thr Lys Gln Arg Asn Val Pro Cys Ser
           915                 920                 925
<210> 77
   <211> 2862
   <212> DNA
   <213> Oryza sativa
<400> 77
   atggcggcgg ggaacggccg gatggaggcg gcgctgggct gcctcgcggc gctccccgac     60
   gaggtgctct gcgccgtcgt cgacctcctc ccgcccaccg acgtcggccg cctcgcctgc    120
   gtcagcagtg tcatgtacat actttgcaat gaggagcctc tctggatgag caagtgtctt    180
   tcagttgggg gtcttcttgt gtatagaggt tcttggaaga aaacagcatt gtctagactt    240
   aatctttgtt cagaaaatga tgagatttac cagaagcctc gccatttgga tgggttcaat    300
   tccatgcact tatacaggag atggtacaga tgttttacta atttgagtag cttttccttt    360
   gataatgggc acgttgaaag gaaagatgac ctttctctag accaatttcg cgctcagtat    420
   gatggaaaat gtccagtttt gcttactaaa ctggctgaaa cctggccagc aaggactaaa    480
   tggacagcgc agcaactgac acatgattat ggtgaagttc cctttaggat atctcagaga    540
   agccctcaaa agataaaaat gaaactaaaa gattatgttt tttacatgga actccagcat    600
   gacgaagatc cactttacat atttgatgat aagtttggag aatcagcacc tacactattg    660
   gaagattaca gtgtccctca tctatttcaa gaagatttct ttgaaatcat ggattacgac    720
   caacgaccag ctttcagatg gcttattatt ggaccagaga gatcaggtgc ttcttggcat    780
   gttgatccag ggttgaccag tgcctggaat actcttcttt gtggccgaaa aaggtgggca    840
   atgtaccctc ctggaagagt accaggtggt gtcacagtac atgtcagtga tgaagatggt    900
   gatgttgaca ttgaaactcc tacatctttg cagtggtggc tagatatcta cccaaatctt    960
   gctgagcatg agaaaccact ggaatgcaca caattaccag gagagaccat atttgttcct   1020
   agtgggtggt ggcattgtgt tttgaacctt gacatgacaa ttgctgtcac gcaaaatttt   1080
   gtcaaccaat caaattttaa gcatgtatgt ttggacatgg cacctggtta ctgtcacaaa   1140
   ggagtttgcc gtgctggctt acttgctgct ccagacaaat ctattagaga tattgaaaat   1200
   cttcctagta taacgagtag attgaaccac tctgacatgg cctgtaagga aaaaagactg   1260
   aaaagttcag agcctataag aacttcaaat aatgcaaatc agtgttctgc atttgagttc   1320
   tcagatgttc atgaaaactt gggggaccaa gttttttcgt atgatataga tttcttatcc   1380
   caattccttg agaaagaaaa ggatcactat tcttctgtct ggagccctac taattcaatt   1440
   ggccagagag aagcaagaga atggctacgt aggctatggg ttcttaaacc tgaattgaga   1500
   gaactaatat ggaagggtgc atgtctagca attaatgtag acaagtggta ttcatgctta   1560
   gaggaaataa gtgcatgcca tagtttacca ccaccttctg aagatgagaa gcttcctgtt   1620
   ggcacaggta gcaacccagt cttcattgtt tctggcaatg tgatcaaaat ttatgctgaa   1680
   ggagggttgg gttattctat acatggtttg ggcacagagc ttgagttcta tgatcttctg   1740
   caaaaacttg gctcgccatt gatcaaccat gtccctgaga tcattgcaag tggctttctt   1800
   gtgtacctgg atggtgtcta caagacagtt ccatgggatg gaaacggaat accagatgtt   1860
   ctagctaaat actactcttt ggaggtgtct tatgcaaacg gctcttttcc tcttggatta   1920
   tggagcaagc aactgtttgg attgagtaat tcaactgatg ctccagacag accaatttgt   1980
   ccttacatgg ttaccagaaa atgcaaaggg gatatttttg ctcgcatacg tgataaattg   2040
   accaagactg atgttttgaa tcttgcatca tccttgggag ttcaaatgcg aaatattcat   2100
   caattacccc ttccacatgt ggaacacata tccaaatctg ggaacgaaga tatcaaagca   2160
   aaggaaaatt caatttctga tgtcactcat gttccgcctg aatggaaaca agtagtttct   2220
   actctagaca ggagaaagaa aagtataaag aagcatctaa gtaactgggg tggttcaatt   2280
   ccacaggttc taattgagaa ggctgaagaa tatctccctg acgacatccg ctttcttatc   2340
   aagtttgtta aggacgatga tggtgattca gtctatgtgg taccttcttg gatacattca   2400
   gatataatgg atgataacat tctcattgag gggaccacag aaccaggaac ttccactgat   2460
   tgcattgccg ttgaagatct gaacaaaatg gatgcaattc atatcattga tttcagtgat   2520
   ctgtccattg gggatcctct atgtgactta attccactgc acttggatgt attccgtggt   2580
   gatattgatc ttctcaggca gtttttacga agctatcagc ttccttttct gagagcagaa   2640
   tcaaataaag atatatacaa gtcaatacaa aattctaaat tcagcagggc atcgtatcgt   2700
   gcgatgtgct actgcatact tcacgaggac aacgtcctgg gagccatatt tagcctgtgg   2760
   aaggatctgg gcaccgcgac gtcatgggaa gatgttgaac acttggtttg gggagagctg   2820
   aatcaatacc agcagtcatg cagcgtgggc gaaattaact ga                      2862
<210> 78
   <211> 953
   <212> PRT
   <213> Oryza sativa
<400> 78
   Met Ala Ala Gly Asn Gly Arg Met Glu Ala Ala Leu Gly Cys Leu Ala
   1               5                   10                  15
   Ala Leu Pro Asp Glu Val Leu Cys Ala Val Val Asp Leu Leu Pro Pro
               20                  25                  30
   Thr Asp Val Gly Arg Leu Ala Cys Val Ser Ser Val Met Tyr Ile Leu
           35                  40                  45
   Cys Asn Glu Glu Pro Leu Trp Met Ser Lys Cys Leu Ser Val Gly Gly
       50                  55                  60
   Leu Leu Val Tyr Arg Gly Ser Trp Lys Lys Thr Ala Leu Ser Arg Leu
   65                  70                  75                  80
   Asn Leu Cys Ser Glu Asn Asp Glu Ile Tyr Gln Lys Pro Arg His Phe
                   85                  90                  95
   Asp Gly Phe Asn Ser Met His Leu Tyr Arg Arg Trp Tyr Arg Cys Phe
               100                 105                 110
   Thr Asn Leu Ser Ser Phe Ser Phe Asp Asn Gly His Val Glu Arg Lys
           115                 120                 125
   Asp Asp Leu Ser Leu Asp Gln Phe Arg Ala Gln Tyr Asp Gly Lys Cys
       130                 135                 140
   Pro Val Leu Leu Thr Lys Leu Ala Glu Thr Trp Pro Ala Arg Thr Lys
   145                 150                 155                 160
   Trp Thr Ala Gln Gln Leu Thr His Asp Tyr Gly Glu Val Pro Phe Arg
                   165                 170                 175
   Ile Ser Gln Arg Ser Pro Gln Lys Ile Lys Met Lys Leu Lys Asp Tyr
               180                 185                 190
   Val Phe Tyr Met Glu Leu Gln His Asp Glu Asp Pro Leu Tyr Ile Phe
           195                 200                 205
   Asp Asp Lys Phe Gly Glu Ser Ala Pro Thr Leu Leu Glu Asp Tyr Ser
       210                 215                 220
   Val Pro His Leu Phe Gln Glu Asp Phe Phe Glu Ile Met Asp Tyr Asp
   225 230 235 240
   Gln Arg Pro Ala Phe Arg Trp Leu Ile Ile Gly Pro Glu Arg Ser Gly
   245 250 255
   Ala Ser Trp His Val Asp Pro Gly Leu Thr Ser Ala Trp Asn Thr Leu
   260 265 270
   Leu Cys Gly Arg Lys Arg Trp Ala Met Tyr Pro Pro Gly Arg Val Pro
   275 280 285
   Gly Gly Val Thr Val His Val Ser Asp Glu Asp Gly Asp Val Asp Ile
   290 295 300
   Glu Thr Pro Thr Ser Leu Gln Trp Trp Leu Asp Ile Tyr Pro Asn Leu
   305 310 315 320
   Ala Glu His Glu Lys Pro Leu Glu Cys Thr Gln Leu Pro Gly Glu Thr
   325 330 335
   Ile Phe Val Pro Ser Gly Trp Trp His Cys Val Leu Asn Leu Asp Met
   340 345 350
   Thr Ile Ala Val Thr Gln Asn Phe Val Asn Gln Ser Asn Phe Lys His
   355 360 365
   Val Cys Leu Asp Met Ala Pro Gly Tyr Cys His Lys Gly Val Cys Arg
   370 375 380
   Ala Gly Leu Leu Ala Ala Pro Asp Lys Ser Ile Arg Asp Ile Glu Asn
   385 390 395 400
   Leu Pro Ser Ile Thr Ser Arg Leu Asn His Ser Asp Met Ala Cys Lys
   405 410 415
   Glu Lys Arg Leu Lys Ser Ser Glu Pro Ile Arg Thr Ser Asn Asn Ala
   420 425 430
   Asn Gln Cys Ser Ala Phe Glu Phe Ser Asp Val His Glu Asn Leu Gly
   435 440 445
   Asp Gln Val Phe Ser Tyr Asp Ile Asp Phe Leu Ser Gln Phe Leu Glu
   450 455 460
   Lys Glu Lys Asp His Tyr Ser Ser Val Trp Ser Pro Thr Asn Ser Ile
   465 470 475 480
   Gly Gln Arg Glu Ala Arg Glu Trp Leu Arg Arg Leu Trp Val Leu Lys
   485 490 495
   Pro Glu Leu Arg Glu Leu Ile Trp Lys Gly Ala Cys Leu Ala Ile Asn
   500 505 510
   Val Asp Lys Trp Tyr Ser Cys Leu Glu Glu Ile Ser Ala Cys His Ser
   515 520 525
   Leu Pro Pro Pro Ser Glu Asp Glu Lys Leu Pro Val Gly Thr Gly Ser
   530 535 540
   Asn Pro Val Phe Ile Val Ser Gly Asn Val Ile Lys Ile Tyr Ala Glu
   545 550 555 560
   Gly Gly Leu Gly Tyr Ser Ile His Gly Leu Gly Thr Glu Leu Glu Phe
   565 570 575
   Tyr Asp Leu Leu Gln Lys Leu Gly Ser Pro Leu Ile Asn His Val Pro
   580 585 590
   Glu Ile Ile Ala Ser Gly Phe Leu Val Tyr Leu Asp Gly Val Tyr Lys
   595 600 605
   Thr Val Pro Trp Asp Gly Asn Gly Ile Pro Asp Val Leu Ala Lys Tyr
   610 615 620
   Tyr Ser Leu Glu Val Ser Tyr Ala Asn Gly Ser Phe Pro Leu Gly Leu
   625 630 635 640
   Trp Ser Lys Gln Leu Phe Gly Leu Ser Asn Ser Thr Asp Ala Pro Asp
   645 650 655
   Arg Pro Ile Cys Pro Tyr Met Val Thr Arg Lys Cys Lys Gly Asp Ile
               660                 665                 670
   Phe Ala Arg Ile Arg Asp Lys Leu Thr Lys Thr Asp Val Leu Asn Leu
           675                 680                 685
   Ala Ser Ser Leu Gly Val Gln Met Arg Asn Ile His Gln Leu Pro Leu
       690                 695                 700
   Pro His Val Glu His Ile Ser Lys Ser Gly Asn Glu Asp Ile Lys Ala
   705                 710                 715                 720
   Lys Glu Asn Ser Ile Ser Asp Val Thr His Val Pro Pro Glu Trp Lys
                   725                 730                 735
   Gln Val Val Ser Thr Leu Asp Arg Arg Lys Lys Ser Ile Lys Lys His
               740                 745                 750
   Leu Ser Asn Trp Gly Gly Ser Ile Pro Gln Val Leu Ile Glu Lys Ala
           755                 760                 765
   Glu Glu Tyr Leu Pro Asp Asp Ile Arg Phe Leu Ile Lys Phe Val Lys
       770                 775                 780
   Asp Asp Asp Gly Asp Ser Val Tyr Val Val Pro Ser Trp Ile His Ser
   785                 790                 795                 800
   Asp Ile Met Asp Asp Asn Ile Leu Ile Glu Gly Thr Thr Glu Pro Gly
                   805                 810                 815
   Thr Ser Thr Asp Cys Ile Ala Val Glu Asp Leu Asn Lys Met Asp Ala
               820                 825                 830
   Ile His Ile Ile Asp Phe Ser Asp Leu Ser Ile Gly Asp Pro Leu Cys
           835                 840                 845
   Asp Leu Ile Pro Leu His Leu Asp Val Phe Arg Gly Asp Ile Asp Leu
       850                 855                 860
   Leu Arg Gln Phe Leu Arg Ser Tyr Gln Leu Pro Phe Leu Arg Ala Glu
   865                 870                 875                 880
   Ser Asn Lys Asp Ile Tyr Lys Ser Ile Gln Asn Ser Lys Phe Ser Arg
                   885                 890                 895
   Ala Ser Tyr Arg Ala Met Cys Tyr Cys Ile Leu His Glu Asp Asn Val
               900                 905                 910
   Leu Gly Ala Ile Phe Ser Leu Trp Lys Asp Leu Gly Thr Ala Thr Ser
           915                 920                 925
   Trp Glu Asp Val Glu His Leu Val Trp Gly Glu Leu Asn Gln Tyr Gln
       930                 935                 940
   Gln Ser Cys Ser Val Gly Glu Ile Asn
   945                 950
<210> 79
   <211> 3171
   <212> DNA
   <213> Oryza sativa
<400> 79
   atggagatgg aggaggcggt agacggcaag cacccgaagc gtgggagggg caggccgagg     60
   ggacgacggg gcaggggcag gggcaggggg aggggcggcc gctccctcgc gtcgccggcg    120
   gcggggcccg gagaccaagg gccgcggcgg cggcgcgggg tcgtcccggc ggcggcggcg    180
   gcggcggggg gaagggcgct gagggagagg aggccggcgc ctggtgccta ccgcgagagc    240
   ggagctgata atgacgatga tggcggcggc gacgatgagc acgacgagca gaatgatgat    300
   ggtgctgaaa aatcggataa tcaagttgtg gattctctta atgaacccaa cagaagtaat    360
   acagggaaga agcggggaag accaaagaaa gtgaaagcag agcaggagga cagtaaccaa    420
   ctctccaacg ggaaacacct tggcgaaaac aatgggaatg atgaagcgat aatgatgaag    480
   ccttcaaaag aatcgaagaa aagaggtgca ggaaagaaac aagaggaaga agagaataat    540
   accatatcca ttgaggatga aatgtgtgat gcgaacaata agaaggggaa gaagatgctc    600
   actggggaaa atgctttgat gtgccaccag tgtcaaagaa atgacaaagg gagggtgatt    660
   tggtgtaaat catgcaacaa taagcgcttc tgtgagccat gcatgaaacg atggtaccca    720
   ggtttgtcag aagttgattt tgctgcaaaa tgcccatatt gtagaaagaa ctgtaattgc    780
   aaggcatgcc taagaatgat aggagttgaa aagccccctg aaaagaagat ttctgaggaa    840
   aatcaacgac gttatgcttt tcgtattgta gacttgttgc ttccttggtt gaaagaactt    900
   caacaggagc agatgaagga gaaggaatta gagggtagat tacaaggtgt ttctatggat    960
   gaagtaaagt tggaacaagc tgattgtgat atggatgagc gtgtttattg tgataggtgc   1020
   aaaacttcga tagttgattt tcatagaagc tgcaaggctt gttcgtatga cttgtgcctt   1080
   gcatgctgct gggagcttcg caaaggtgag attccaggag gagaagaggc aaagagtgtg   1140
   cagtgggaag agagaggtca gaagtatgtt tttggtaata tttccaagga tgagaagaaa   1200
   agggtatctt cgaagaggca catggagacc cccagtactg aaacttgcaa tgacatggct   1260
   gttgctgggg acccaaataa ccctttgtta ctgtggaaag ctaatagtga tggcagtata   1320
   ccttgtccac caaaggaaat agggggctgc ggtgcttcat ctttggtact cagatgctta   1380
   ttaccagaaa ttatgctttc tgagttagaa cacagggcta acaaagttat taagagagaa   1440
   gcatttgata aagcaataaa cgaaacaagt gatcagtgcc cttgctttta tcacacaagc   1500
   aagataagaa caaatgctac tcgagaggca gcaaacagaa agggctcaag tgataactac   1560
   ttgtactgtc cagatgccaa taatatccag gaggatgacc tgtcgcactt tcagatgcat   1620
   tggtcaaaag gtgaaccggt tattgtttct gatgctcttc ggttaacatc tggtctgagc   1680
   tgggagccat tggttatgtg gcgggcattg cgagagaaga aaaccaatgg tgatgttgaa   1740
   gatgagcact ttgctgttaa ggcagtggat tgccttgatt ggaatgaggt ggaaattaac   1800
   atacacatgt tctttatggg gtatatgaga ggtagaagac atccgatgac cttttggcct   1860
   gagatgctta agctaaaaga ttggccacca tctagtatgt ttgaccagag gttacctcgc   1920
   catggtgctg agtttataac tgcattgcca tttcccgagt atactgatcc acgatatggc   1980
   ccgctaaatc ttgctgtcag gcttcctgct ggtgtactga agcctgatct tgggccaaaa   2040
   acttatattg cttatggatg ttatgaagag ttaggcaggg gtgactctgt gaccaagctt   2100
   cattgcgaca tgtctgatgc ggtaaatatc ttgatgcaca cagctgaagt gtcctatgac   2160
   actgaacagc ttgacaagat agcaaaaatt aaaatgaaaa tgagagaaca agatcttcat   2220
   gaactgtttg gggtttcaga atcaggcgcc aagggtaaag ctgatgatga agcatcgaaa   2280
   atctcatgta acatggaaaa caaacacacc tctaaccaaa gtactaaggg tttggacatt   2340
   aatgctttac cacctgatga ttctggaagt gatattgggg ataaaccatc attttgtcaa   2400
   tctgaggtag aaagtgaatt aacacaatgt tcaaaacaca atcacgaggt taatagttct   2460
   gtcaagatgc atgctggagc tcattgtact tcagacaacc aaggatacat tgacaggagt   2520
   ggattcaaac gcaaagattc agactgttca gaccaacaga aaactggtgg cgctttgtgg   2580
   gatattttcc gaagagaaga ttctgagaag ttacaagatt atcttcggaa gcatgcctca   2640
   gaatttcggc acatacactg caatccagtg aaaaatgttt ctcacccaat tcatgaccag   2700
   actttctatt taactgtgga gcataagaga aagctcaagg aagaacacgg tgttgaacca   2760
   tggacattcg agcagaagct aggcgatgca gttttcattc ctgctggatg tccacaccaa   2820
   gtgagaaatt taaagtcttg catcaaggtt gctctagact ttgtttcccc tgaaaatgtt   2880
   ggtgagtgtg ttaagctgac cggagagttt agacgtcttc catctgatca cagggctaaa   2940
   gaagataaac tagagattaa gaagattgct ctcaatgctc ttaaagaagt cgtaaatttc   3000
   ttagatcctt taccaaaagg gtcaaagaac agggatgaag tggtagaagt gaccaaacca   3060
   aaaaggaaat acggtaaccg aagaggtgac ctgaagagtg ggaagaccca acccattgat   3120
   gaatccatag aagaaaggaa gcctaaaaag cgagggagat ctaaacggtg a            3171
<210> 80
   <211> 1056
   <212> PRT
   <213> Oryza sativa
<400> 80
   Met Glu Met Glu Glu Ala Val Asp Gly Lys His Pro Lys Arg Gly Arg
   1               5                   10                  15
   Gly Arg Pro Arg Gly Arg Arg Gly Arg Gly Arg Gly Arg Gly Arg Gly
               20                      25                      30
   Gly Arg Ser Leu Ala Ser Pro Ala Ala Gly Pro Gly Asp Gln Gly Pro
           35                      40                      45
   Arg Arg Arg Arg Gly Val Val Pro Ala Ala Ala Ala Ala Ala Gly Gly
       50                      55                      60
   Arg Ala Leu Arg Glu Arg Arg Pro Ala Pro Gly Ala Tyr Arg Glu Ser
   65                      70                      75                      80
   Gly Ala Asp Asn Asp Asp Asp Gly Gly Gly Asp Asp Glu His Asp Glu
                   85                      90                      95
   Gln Asn Asp Asp Gly Ala Glu Lys Ser Asp Asn Gln Val Val Asp Ser
               100                     105                     110
   Leu Asn Glu Pro Asn Arg Ser Asn Thr Gly Lys Lys Arg Gly Arg Pro
           115                     120                     125
   Lys Lys Val Lys Ala Glu Gln Glu Asp Ser Asn Gln Leu Ser Asn Gly
       130                     135                     140
   Lys His Leu Gly Glu Asn Asn Gly Asn Asp Glu Ala Ile Met Met Lys
   145                     150                     155                     160
   Pro Ser Lys Glu Ser Lys Lys Arg Gly Ala Gly Lys Lys Gln Glu Glu
                   165                     170                     175
   Glu Glu Asn Asn Thr Ile Ser Ile Glu Asp Glu Met Cys Asp Ala Asn
               180                     185                     190
   Asn Lys Lys Gly Lys Lys Met Leu Thr Gly Glu Asn Ala Leu Met Cys
           195                     200                     205
   His Gln Cys Gln Arg Asn Asp Lys Gly Arg Val Ile Trp Cys Lys Ser
       210                     215                     220
   Cys Asn Asn Lys Arg Phe Cys Glu Pro Cys Met Lys Arg Trp Tyr Pro
   225                     230                     235                     240
   Gly Leu Ser Glu Val Asp Phe Ala Ala Lys Cys Pro Tyr Cys Arg Lys
                   245                     250                     255
   Asn Cys Asn Cys Lys Ala Cys Leu Arg Met Ile Gly Val Glu Lys Pro
               260                     265                     270
   Pro Glu Lys Lys Ile Ser Glu Glu Asn Gln Arg Arg Tyr Ala Phe Arg
           275                     280                     285
   Ile Val Asp Leu Leu Leu Pro Trp Leu Lys Glu Leu Gln Gln Glu Gln
       290                     295                     300
   Met Lys Glu Lys Glu Leu Glu Gly Arg Leu Gln Gly Val Ser Met Asp
   305                     310                     315                     320
   Glu Val Lys Leu Glu Gln Ala Asp Cys Asp Met Asp Glu Arg Val Tyr
                   325                     330                     335
   Cys Asp Arg Cys Lys Thr Ser Ile Val Asp Phe His Arg Ser Cys Lys
               340                     345                     350
   Ala Cys Ser Tyr Asp Leu Cys Leu Ala Cys Cys Trp Glu Leu Arg Lys
           355                     360                     365
   Gly Glu Ile Pro Gly Gly Glu Glu Ala Lys Ser Val Gln Trp Glu Glu
       370                     375                     380
   Arg Gly Gln Lys Tyr Val Phe Gly Asn Ile Ser Lys Asp Glu Lys Lys
   385                     390                     395                     400
   Arg Val Ser Ser Lys Arg His Met Glu Thr Pro Ser Thr Glu Thr Cys
                   405                     410                     415
   Asn Asp Met Ala Val Ala Gly Asp Pro Asn Asn Pro Leu Leu Leu Trp
               420                     425                     430
   Lys Ala Asn Ser Asp Gly Ser Ile Pro Cys Pro Pro Lys Glu Ile Gly
           435                     440                     445
   Gly Cys Gly Ala Ser Ser Leu Val Leu Arg Cys Leu Leu Pro Glu Ile
       450                 455                 460
   Met Leu Ser Glu Leu Glu His Arg Ala Asn Lys Val Ile Lys Arg Glu
   465                 470                 475                 480
   Ala Phe Asp Lys Ala Ile Asn Glu Thr Ser Asp Gln Cys Pro Cys Phe
                   485                 490                 495
   Tyr His Thr Ser Lys Ile Arg Thr Asn Ala Thr Arg Glu Ala Ala Asn
               500                 505                 510
   Arg Lys Gly Ser Ser Asp Asn Tyr Leu Tyr Cys Pro Asp Ala Asn Asn
           515                 520                 525
   Ile Gln Glu Asp Asp Leu Ser His Phe Gln Met His Trp Ser Lys Gly
       530                 535                 540
   Glu Pro Val Ile Val Ser Asp Ala Leu Arg Leu Thr Ser Gly Leu Ser
   545                 550                 555                 560
   Trp Glu Pro Leu Val Met Trp Arg Ala Leu Arg Glu Lys Lys Thr Asn
                   565                 570                 575
   Gly Asp Val Glu Asp Glu His Phe Ala Val Lys Ala Val Asp Cys Leu
               580                 585                 590
   Asp Trp Asn Glu Val Glu Ile Asn Ile His Met Phe Phe Met Gly Tyr
           595                 600                 605
   Met Arg Gly Arg Arg His Pro Met Thr Phe Trp Pro Glu Met Leu Lys
       610                 615                 620
   Leu Lys Asp Trp Pro Pro Ser Ser Met Phe Asp Gln Arg Leu Pro Arg
   625                 630                 635                 640
   His Gly Ala Glu Phe Ile Thr Ala Leu Pro Phe Pro Glu Tyr Thr Asp
                   645                 650                 655
   Pro Arg Tyr Gly Pro Leu Asn Leu Ala Val Arg Leu Pro Ala Gly Val
               660                 665                 670
   Leu Lys Pro Asp Leu Gly Pro Lys Thr Tyr Ile Ala Tyr Gly Cys Tyr
           675                 680                 685
   Glu Glu Leu Gly Arg Gly Asp Ser Val Thr Lys Leu His Cys Asp Met
       690                 695                 700
   Ser Asp Ala Val Asn Ile Leu Met His Thr Ala Glu Val Ser Tyr Asp
   705                 710                 715                 720
   Thr Glu Gln Leu Asp Lys Ile Ala Lys Ile Lys Met Lys Met Arg Glu
                   725                 730                 735
   Gln Asp Leu His Glu Leu Phe Gly Val Ser Glu Ser Gly Ala Lys Gly
               740                 745                 750
   Lys Ala Asp Asp Glu Ala Ser Lys Ile Ser Cys Asn Met Glu Asn Lys
           755                 760                 765
   His Thr Ser Asn Gln Ser Thr Lys Gly Leu Asp Ile Asn Ala Leu Pro
       770                 775                 780
   Pro Asp Asp Ser Gly Ser Asp Ile Gly Asp Lys Pro Ser Phe Cys Gln
   785                 790                 795                 800
   Ser Glu Val Glu Ser Glu Leu Thr Gln Cys Ser Lys His Asn His Glu
                   805                 810                 815
   Val Asn Ser Ser Val Lys Met His Ala Gly Ala His Cys Thr Ser Asp
               820                 825                 830
   Asn Gln Gly Tyr Ile Asp Arg Ser Gly Phe Lys Arg Lys Asp Ser Asp
           835                 840                 845
   Cys Ser Asp Gln Gln Lys Thr Gly Gly Ala Leu Trp Asp Ile Phe Arg
       850                 855                 860
   Arg Glu Asp Ser Glu Lys Leu Gln Asp Tyr Leu Arg Lys His Ala Ser
   865                 870                 875                 880
   Glu Phe Arg His Ile His Cys Asn Pro Val Lys Asn Val Ser His Pro
                       885                     890                     895
   Ile His Asp Gln Thr Phe Tyr Leu Thr Val Glu His Lys Arg Lys Leu
                   900                     905                     910
   Lys Glu Glu His Gly Val Glu Pro Trp Thr Phe Glu Gln Lys Leu Gly
               915                     920                     925
   Asp Ala Val Phe Ile Pro Ala Gly Cys Pro His Gln Val Arg Asn Leu
       930                     935                     940
   Lys Ser Cys Ile Lys Val Ala Leu Asp Phe Val Ser Pro Glu Asn Val
   945                     950                     955                     960
   Gly Glu Cys Val Lys Leu Thr Gly Glu Phe Arg Arg Leu Pro Ser Asp
                       965                     970                     975
   His Arg Ala Lys Glu Asp Lys Leu Glu Ile Lys Lys Ile Ala Leu Asn
                   980                     985                     990
   Ala Leu Lys Glu Val Val Asn Phe  Leu Asp Pro Leu Pro  Lys Gly Ser
               995                    1000                    1005
   Lys Asn  Arg Asp Glu Val Val  Glu Val Thr Lys Pro  Lys Arg Lys
       1010                    1015                    1020
   Tyr Gly  Asn Arg Arg Gly Asp  Leu Lys Ser Gly Glu  Asp Gln Pro
       1025                    1030                    1035
   Ile Asp  Glu Ser Ile Glu Glu  Arg Lys Pro Lys Lys  Arg Gly Arg
       1040                    1045                    1050
   Ser Lys  Arg
       1055
<210> 81
   <211> 3159
   <212> DNA
   <213> Oryza sativa
<400> 81
   atggagatgg aggaggcggt agacggcaag cacccgaagc gtgggagggg caggccgagg     60
   ggacgacggg gcaggggcag gggcaggggg aggggcggcc gctccctcgc gtcgccggcg    120
   gcggggcccg gagaccaagg gccgcggcgg cggcgcgggg tcgtcccggc ggcggcggcg    180
   gcggcggggg gaagggcgct gaggagaggg aggccggcgc ctggtgccta ccgcgagagc    240
   ggagctgata atgacgatga tggcggcggc gacgatgagc acgacgagca gaatgatgat    300
   ggtgctgaaa aatcggataa tcaagttgtg gattctctta atgaacccaa cagaagtaat    360
   acagggaaga agcggggaag accaaagaaa gtgaaagcag agcaggagga cagtaaccaa    420
   ctctccaacg ggaaacacct tggcgaaaac aatgggaatg atgaagcgat aatgatgaag    480
   ccttcaaaag aatcgaagaa aagaggtgca ggaaagaaac aagaggaaga agagaataat    540
   accatatcca ttgaggatga aatgtgtgat gcgaacaata agaaggggaa gaagatgctc    600
   actggggaaa atgctttgat gtgccaccag tgtcaaagaa atgacaaagg gagggtgatt    660
   tggtgtaaat catgcaacaa taagcgcttc tgtgagccat gcatgaaacg atggtaccca    720
   ggtttgtcag aagttgattt tgctgcaaaa tgcccatatt gtagaaagaa ctgtaattgc    780
   aaggcatgcc taagaatgat aggagttgaa aagccccctg aaaagaagat ttctgaggaa    840
   aatcaacgac gttatgcttt tcgtattgta gacttgttgc ttccttggtt gaaagaactt    900
   caacaggagc agatgaagga gaaggaatta gagggtagat tacaaggtgt ttctatggat    960
   gaagtaaagt tggaacaagc tgattgtgat atggatgagc gtgtttattg tgataggtgc   1020
   aaaacttcga tagttgattt tcatagaagc tgcaaggctt gttcgtatga cttgtgcctt   1080
   gcatgctgct gggagcttcg caaaggtgag attccaggag gagaagaggc aaagagtgtg   1140
   cagtgggaag agagaggtca gaagtatgtt tttggtaata tttccaagga tgagaagaaa   1200
   agggtatctt cgaagaggca catggagacc cccagtactg aaacttgcaa tgacatggct   1260
   gttgctgggg acccaaataa ccctttgtta ctgtggaaag ctaatagtga tggcagtata   1320
   ccttgtccac caaaggaaat agggggctgc ggtgcttcat ctttggtact cagatgctta   1380
   ttaccagaaa ttatgctttc tgagttagaa cacagggcta acaaagttat taagagagaa   1440
   gcatttgata aagcaataaa cgaaacaagt gatcagtgcc cttgctttta tcacacaagc   1500
   aagataagaa caaatgctac tcgagaggca gcaaacagaa agggctcaag tgataactac   1560
   ttgtactgtc cagatgccaa taatatccag gaggatgacc tgtcgcactt tcagatgcat   1620
   tggtcaaaag gtgaaccggt tattgtttct gatgctcttc ggttaacatc tggtctgagc   1680
   tgggagccat tggttatgtg gcgggcattg cgagagaaga aaaccaatgg tgatgttgaa   1740
   gatgagcact ttgctgttaa ggcagtggat tgccttgatt ggaatgaggt ggaaattaac   1800
   atacacatgt tctttatggg gtatatgaga ggtagaagac atccgatgac cttttggcct   1860
   gagatgctta agctaaaaga ttggccacca tctagtatgt ttgaccagag gttacctcgc   1920
   catggtgctg agtttataac tgcattgcca tttcccgagt atactgatcc acgatatggc   1980
   ccgctaaatc ttgctgtcag gcttcctgct ggtgtactga agcctgatct tgggccaaaa   2040
   acttatattg cttatggatg ttatgaagag ttaggcaggg gtgactctgt gaccaagctt   2100
   cattgcgaca tgtctgatgc ggtaaatatc ttgatgcaca cagctgaagt gtcctatgac   2160
   actgaacagc ttgacaagat agcaaaaatt aaaatgaaaa tgagagaaca agatcttcat   2220
   gaactgtttg ggtttcagaa atcaggcgcc aagggtaaag ctgatgatga agcatcgaaa   2280
   atctcatgta acatggaaaa caaacacacc tctaaccaaa gtactaaggg tttggacatt   2340
   aatgctttac cacctgatga ttctggaagt gatattgggg ataaaccatc attttgtcaa   2400
   tctgaggtag aaagtgaatt aacacaatgt tcaaaacaca atcacgaggt taatagttct   2460
   gtcaagatgc atgctggagc tcattgtact tcagacaacc aaggatacat tgacaggagt   2520
   ggattcaaac gcaaagattc agactgttca gaccaacaga aaactggtgg cgctttgtgg   2580
   gatattttcc gaagagaaga ttctgagaag ttacaagatt atcttcggaa gcatgcctca   2640
   gaatttcggc acatacactg caatccagtg aaaaatgttt ctcacccaat tcatgaccag   2700
   actttctatt taactgtgga gcataagaga aagctcaagg aagaacacgg tgttgaacca   2760
   tggacattcg agcagaagct aggcgatgca gttttcattc ctgctggatg tccacaccaa   2820
   gtgagaaatt taaagtcttg catcaaggtt gctctagact ttgtttcccc tgaaaatgtt   2880
   ggtgagtgtg ttaagctgac cggagagttt agacgtcttc catctgatca cagggctaaa   2940
   gaagataaac tagagattaa gaagattgct ctcaatgctc ttaaagaagt cgtaaatttc   3000
   ttagatcctt taccaaaagg ggatgaagtg gtagaagtga ccaaaccaaa aaggaaatac   3060
   ggtaaccgaa gaggtgacct gaagagtggg gaagaccaac ccattgatga atccatagaa   3120
   gaaaggaagc ctaaaaagcg agggagatct aaacggtga                          3159
<210> 82
   <211> 1052
   <212> PRT
   <213> Oryza sativa
<400> 82
   Met Glu Met Glu Glu Ala Val Asp Gly Lys His Pro Lys Arg Gly Arg
   1               5                   10                  15
   Gly Arg Pro Arg Gly Arg Arg Gly Arg Gly Arg Gly Arg Gly Arg Gly
               20                  25                  30
   Gly Arg Ser Leu Ala Ser Pro Ala Ala Gly Pro Gly Asp Gln Gly Pro
           35                  40                  45
   Arg Arg Arg Arg Gly Val Val Pro Ala Ala Ala Ala Ala Ala Gly Gly
       50                  55                  60
   Arg Ala Leu Arg Glu Arg Arg Pro Ala Pro Gly Ala Tyr Arg Glu Ser
   65                  70                  75                  80
   Gly Ala Asp Asn Asp Asp Asp Gly Gly Gly Asp Asp Glu His Asp Glu
                   85                  90                  95
   Gln Asn Asp Asp Gly Ala Glu Lys Ser Asp Asn Gln Val Val Asp Ser
               100                 105                 110
   Leu Asn Glu Pro Asn Arg Ser Asn Thr Gly Lys Lys Arg Gly Arg Pro
           115                 120                 125
   Lys Lys Val Lys Ala Glu Gln Glu Asp Ser Asn Gln Leu Ser Asn Gly
       130                 135                 140
   Lys His Leu Gly Glu Asn Asn Gly Asn Asp Glu Ala Ile Met Met Lys
   145                 150                 155                 160
   Pro Ser Lys Glu Ser Lys Lys Arg Gly Ala Gly Lys Lys Gln Glu Glu
                   165                 170                 175
   Glu Glu Asn Asn Thr Ile Ser Ile Glu Asp Glu Met Cys Asp Ala Asn
               180                 185                 190
   Asn Lys Lys Gly Lys Lys Met Leu Thr Gly Glu Asn Ala Leu Met Cys
           195                 200                 205
   His Gln Cys Gln Arg Asn Asp Lys Gly Arg Val Ile Trp Cys Lys Ser
       210                 215                 220
   Cys Asn Asn Lys Arg Phe Cys Glu Pro Cys Met Lys Arg Trp Tyr Pro
   225                 230                 235                 240
   Gly Leu Ser Glu Val Asp Phe Ala Ala Lys Cys Pro Tyr Cys Arg Lys
                   245                 250                 255
   Asn Cys Asn Cys Lys Ala Cys Leu Arg Met Ile Gly Val Glu Lys Pro
               260                 265                 270
   Pro Glu Lys Lys Ile Ser Glu Glu Asn Gln Arg Arg Tyr Ala Phe Arg
           275                 280                 285
   Ile Val Asp Leu Leu Leu Pro Trp Leu Lys Glu Leu Gln Gln Glu Gln
       290                 295                 300
   Met Lys Glu Lys Glu Leu Glu Gly Arg Leu Gln Gly Val Ser Met Asp
   305                 310                 315                 320
   Glu Val Lys Leu Glu Gln Ala Asp Cys Asp Met Asp Glu Arg Val Tyr
                   325                 330                 335
   Cys Asp Arg Cys Lys Thr Ser Ile Val Asp Phe His Arg Ser Cys Lys
               340                 345                 350
   Ala Cys Ser Tyr Asp Leu Cys Leu Ala Cys Cys Trp Glu Leu Arg Lys
           355                 360                 365
   Gly Glu Ile Pro Gly Gly Glu Glu Ala Lys Ser Val Gln Trp Glu Glu
       370                 375                 380
   Arg Gly Gln Lys Tyr Val Phe Gly Asn Ile Ser Lys Asp Glu Lys Lys
   385                 390                 395                 400
   Arg Val Ser Ser Lys Arg His Met Glu Thr Pro Ser Thr Glu Thr Cys
                   405                 410                 415
   Asn Asp Met Ala Val Ala Gly Asp Pro Asn Asn Pro Leu Leu Leu Trp
               420                 425                 430
   Lys Ala Asn Ser Asp Gly Ser Ile Pro Cys Pro Pro Lys Glu Ile Gly
           435                 440                 445
   Gly Cys Gly Ala Ser Ser Leu Val Leu Arg Cys Leu Leu Pro Glu Ile
       450                 455                 460
   Met Leu Ser Glu Leu Glu His Arg Ala Asn Lys Val Ile Lys Arg Glu
   465                 470                 475                 480
   Ala Phe Asp Lys Ala Ile Asn Glu Thr Ser Asp Gln Cys Pro Cys Phe
                   485                 490                 495
   Tyr His Thr Ser Lys Ile Arg Thr Asn Ala Thr Arg Glu Ala Ala Asn
               500                 505                 510
   Arg Lys Gly Ser Ser Asp Asn Tyr Leu Tyr Cys Pro Asp Ala Asn Asn
           515                 520                 525
   Ile Gln Glu Asp Asp Leu Ser His Phe Gln Met His Trp Ser Lys Gly
       530                 535                 540
   Glu Pro Val Ile Val Ser Asp Ala Leu Arg Leu Thr Ser Gly Leu Ser
   545                 550                 555                 560
   Trp Glu Pro Leu Val Met Trp Arg Ala Leu Arg Glu Lys Lys Thr Asn
                   565                 570                 575
   Gly Asp Val Glu Asp Glu His Phe Ala Val Lys Ala Val Asp Cys Leu
               580                 585                 590
   Asp Trp Asn Glu Val Glu Ile Asn Ile His Met Phe Phe Met Gly Tyr
           595                 600                 605
   Met Arg Gly Arg Arg His Pro Met Thr Phe Trp Pro Glu Met Leu Lys
       610                 615                 620
   Leu Lys Asp Trp Pro Pro Ser Ser Met Phe Asp Gln Arg Leu Pro Arg
   625                 630                 635                 640
   His Gly Ala Glu Phe Ile Thr Ala Leu Pro Phe Pro Glu Tyr Thr Asp
                   645                 650                 655
   Pro Arg Tyr Gly Pro Leu Asn Leu Ala Val Arg Leu Pro Ala Gly Val
               660                 665                 670
   Leu Lys Pro Asp Leu Gly Pro Lys Thr Tyr Ile Ala Tyr Gly Cys Tyr
           675                 680                 685
   Glu Glu Leu Gly Arg Gly Asp Ser Val Thr Lys Leu His Cys Asp Met
       690                 695                 700
   Ser Asp Ala Val Asn Ile Leu Met His Thr Ala Glu Val Ser Tyr Asp
   705                 710                 715                 720
   Thr Glu Gln Leu Asp Lys Ile Ala Lys Ile Lys Met Lys Met Arg Glu
                   725                 730                 735
   Gln Asp Leu His Glu Leu Phe Gly Val Ser Glu Ser Gly Ala Lys Gly
               740                 745                 750
   Lys Ala Asp Asp Glu Ala Ser Lys Ile Ser Cys Asn Met Glu Asn Lys
           755                 760                 765
   His Thr Ser Asn Gln Ser Thr Lys Gly Leu Asp Ile Asn Ala Leu Pro
       770                 775                 780
   Pro Asp Asp Ser Gly Ser Asp Ile Gly Asp Lys Pro Ser Phe Cys Gln
   785                 790                 795                 800
   Ser Glu Val Glu Ser Glu Leu Thr Gln Cys Ser Lys His Asn His Glu
                   805                 810                 815
   Val Asn Ser Ser Val Lys Met His Ala Gly Ala His Cys Thr Ser Asp
               820                 825                 830
   Asn Gln Gly Tyr Ile Asp Arg Ser Gly Phe Lys Arg Lys Asp Ser Asp
           835                 840                 845
   Cys Ser Asp Gln Gln Lys Thr Gly Gly Ala Leu Trp Asp Ile Phe Arg
       850                 855                 860
   Arg Glu Asp Ser Glu Lys Leu Gln Asp Tyr Leu Arg Lys His Ala Ser
   865                 870                 875                 880
   Glu Phe Arg His Ile His Cys Asn Pro Val Lys Asn Val Ser His Pro
                   885                 890                 895
   Ile His Asp Gln Thr Phe Tyr Leu Thr Val Glu His Lys Arg Lys Leu
               900                 905                 910
   Lys Glu Glu His Gly Val Glu Pro Trp Thr Phe Glu Gln Lys Leu Gly
           915                 920                 925
   Asp Ala Val Phe Ile Pro Ala Gly Cys Pro His Gln Val Arg Asn Leu
       930                 935                 940
   Lys Ser Cys Ile Lys Val Ala Leu Asp Phe Val Ser Pro Glu Asn Val
   945                 950                 955                 960
   Gly Glu Cys Val Lys Leu Thr Gly Glu Phe Arg Arg Leu Pro Ser Asp
                   965                 970                 975
   His Arg Ala Lys Glu Asp Lys Leu Glu Ile Lys Lys Ile Ala Leu Asn
               980                 985                 990
   Ala Leu Lys Glu Val Val Asn Phe  Leu Asp Pro Leu Pro  Lys Gly Asp
           995                 1000                 1005
   Glu Val  Val Glu Val Thr Lys  Pro Lys Arg Lys Tyr  Gly Asn Arg
       1010                 1015                 1020
   Arg Gly  Asp Leu Lys Ser Gly  Glu Asp Gln Pro Ile  Asp Glu Ser
       1025                 1030                 1035
   Ile Glu  Glu Arg Lys Pro Lys  Lys Arg Gly Arg Ser  Lys Arg
       1040                 1045                 1050

<210> 83
   <211> 3717
   <212> DNA
   <213> Oryza sativa
<400> 83
   atgatggggg ttaccaccac gctcaacgag gacactgaac cctctattcc acctggattt     60
   ggaccttttg ctacccttcc gttatgggga atccacaatg atgccaaacc tgctgttact    120
   cattctactc ctgttcaagc attgcaaagc attagaaaag acagcgaaga atgccaaccc    180
   agtgcggctg tgtctcggag tgatacacct tgcagcactt ccggaaccca gacatgcaga    240
   aaatcactgc gtaacagacc cccaatagac tatagccgct ttgaacatat atcggatgaa    300
   gattctgatg tcgaaatagt ggaaaaggat gtaagttcaa cgagacgcag acaacagcta    360
   ccgaaaggag tacttcgagg atgtgcagaa tgcagtgact gtcaaaaggt tatcgcaaaa    420
   tggaatccag ctggtgcacg caggcctgtt cttgatgagg ctcctgtttt ctatccaaca    480
   gaggaggaat ttgaagacac tctaaaatac attgagagta tacggccaat ggcggaacca    540
   tatggtattt gccgtattgt cccaccatct tcttggaagc ctccatgcct tcttaaagat    600
   aaaagcatat gggaaggatc aaaattctct actcgggtac aaaaggttga caagctccaa    660
   aaccgtaaat catcaaaaaa gggcagaaga ggtggaatga tgaagaggag aaagcttgca    720
   gagtcagagg agaacagtgc cactgctcac actcagacag ggatgcagca aagtccagag    780
   agatttggat ttgaacctgg gccagagttc acgttacaga catttcagaa atatgcagat    840
   gatttcagta agcagtactt taggaaagat acatcgatgg attcagtacc atcagtggaa    900
   gatattgaag gtgagtactg gcgcatcgtt gaggttccca cagaagagat agaggtgata    960
   tatggtgctg atctggagac tggaactttc ggcagtggtt ttccaaaatt atctcctgag   1020
   acaaaatctg atgctgagga taaatatgca caatctggtt ggaatctaaa taacttgcct   1080
   agactacaag gttcagttct ttctttcgag ggcggtgaca tttctggtgt tctagtgcct   1140
   tgggtgtatg ttggcatgtg tttttcatca ttctgctggc atgttgaaga ccatcattta   1200
   tactcactaa actacatgca ttggggtgcc ccaaagttgt ggtatggagt tccaggaaag   1260
   gatgctgtga atttggaatc tgcaatgagg aaacatctac ctgaattatt tgaggagcaa   1320
   cctgatttgc tacacaacct tgttacccag ttttcaccat cgctgctgaa atctgaagga   1380
   gtacatgtat accgttgtgt tcagcatgag ggcgagtttg tcttgacatt cccaagggcg   1440
   taccatgctg gtttcaattg tggcttcaat tgtgccgaag ctgttaatgt ggctcctatt   1500
   gattggttac cgattggaca taatgctgta gagctttatc gtgagcaagc taggaaaata   1560
   accatttctc atgataagtt gttgttgggg gctgcaagag aagcaataag agctcagtgg   1620
   gatatcctat tcctcaagag gaatactgct gataatatga ggtggaagag tatatgcgga   1680
   gctgatagca ctatattcaa ggctcttaag gcacgaattg agacagagtt ggtgcaaagg   1740
   aaaactctag gtgttccagc tcaatcaagg aaaatggatg ctgaattcga ttccattgat   1800
   agggaatgtg ccttgtgcta ctatgattta catctttctg cttctggctg tccatgctgc   1860
   ccagagaaat atgcttgcct tgtacatgca aagcaacttt gctcatgtga ctgggacaaa   1920
   aggtttttcc tattccgcta tgatgtcaat gagctaaata tcttagctga tgctttaggg   1980
   gggaaattaa gtgccattca tagatggggc gtctctgatc ttggattaag tttgagttca   2040
   tgtgtcaaac gagaaaaggt ccaagattcc aagactgttc gcagattaac tgatggtcca   2100
   agaaggtctt acatgtcaca ggcatcagca gtatccttgg tttcttcttc tacttccaat   2160
   gaacagaaag atgaaggaaa taagatcatg aagatagcta gcccacagac aaataatgtg   2220
   tgcccttctg tcgagcaaag gaaatcagag aatatttcac cattgaagga gccatgtgta   2280
   aggaatgagt tgtcatgtac aacaaattct gatagtaacg gattgcaata taatggagga   2340
   cttggaggcc ataaaggatc tgcaccaggc ttgccagttt cttctagccc atcattttct   2400
   tccaacgttg caacaaggcc cattagtact tcaagtgtat ccatgaaaat tgtgcaaggc   2460
   ttggtggcat ctaaaagttg tatacaagct tcctctcgaa ctggagacag tagatcattg   2520
   cttggtgagc atcataacag atcaccggca atgattcatg atggaaccaa catgaagtcc   2580
   agtttggaaa gctcaaacaa ttcttgcagg ttgattgcat ctgactataa tgcaactccg   2640
   tgtcattcat ccaaggatca ggtattagta acaccaggga ctaatgcctc agtagtgact   2700
   ctgaaagata gcagccaggt ccatagtgcg tcaagtcagc agtttgtcag aactggccca   2760
   tggacacaaa gtgcttctca tgaagcatca tcacctagta cctctgcttt gaagccttct   2820
   ttagatcccc ctgccatgaa aaatctgtat gggggtttta ctcaaggcag tgcccatcct    2880
   ggacctccaa gtttcagtaa tcagcaacca aatgatgggc gtcttcaaag aacatctgaa   2940
   tctctaccag gtgtggaagc tagagctagg ggacatccaa ctgtcacggc acagcctgca   3000
   ctagaaattc acagcaggaa tggaggtgca cagaagggtc ctcgcatagc caatgttgtg   3060
   catcgtttca agtgctctgt tgaacctctc gaaattggtg ttgtgctatc agggaggctg   3120
   tggtcttcaa gccaagcaat cttcccgaaa gggtttagaa gcagagtgaa atacttcagc   3180
   attgtggatc caatccaaat ggcatactac atatcggaaa tactggatgc tgggatgcag   3240
   gggcctctgt ttatggtaaa attagagaac tgtccaggtg aagttttcat taacttatct   3300
   ccaaccaagt gttggaacat ggtccgtgaa aggctgaaca tggaaataag gaggcaactt   3360
   aatatgggaa aatcaaatct tcctacattg cagcctccag gatcagttga tggtcttgaa   3420
   atgtttggtt tattatcacc accaatagtt caggcaattt gggcgcggga cagagatcac   3480
   atctgtacag agtactggag atcaaggccc catgttctca ttgaggatcc aaacaatcgg   3540
   catatgttat ctcagggtcc acctctcctt gccctgaggg gtctcatcca aagggctaac   3600
   cgggatgaat tgcaagtcct gcggagtttg atgacgaaca gcaacaattt ggatgatagc   3660
   tccaggcaac aggccgcgca cattatcgaa gaggagattg cgaagcaatt gtgctga      3717
<210> 84
   <211> 1238
   <212> PRT
   <213> Oryza sativa
<400> 84
   Met Met Gly Val Thr Thr Thr Leu Asn Glu Asp Thr Glu Pro Ser Ile
   1               5                   10                  15
   Pro Pro Gly Phe Gly Pro Phe Ala Thr Leu Pro Leu Trp Gly Ile His
               20                  25                  30
   Asn Asp Ala Lys Pro Ala Val Thr His Ser Thr Pro Val Gln Ala Leu
           35                  40                  45
   Gln Ser Ile Arg Lys Asp Ser Glu Glu Cys Gln Pro Ser Ala Ala Val
       50                  55                  60
   Ser Arg Ser Asp Thr Pro Cys Ser Thr Ser Gly Thr Gln Thr Cys Arg
   65                  70                  75                  80
   Lys Ser Leu Arg Asn Arg Pro Pro Ile Asp Tyr Ser Arg Phe Glu His
                   85                  90                  95
   Ile Ser Asp Glu Asp Ser Asp Val Glu Ile Val Glu Lys Asp Val Ser
               100                 105                 110
   Ser Thr Arg Arg Arg Gln Gln Leu Pro Lys Gly Val Leu Arg Gly Cys
           115                 120                 125
   Ala Glu Cys Ser Asp Cys Gln Lys Val Ile Ala Lys Trp Asn Pro Ala
       130                 135                 140
   Gly Ala Arg Arg Pro Val Leu Asp Glu Ala Pro Val Phe Tyr Pro Thr
   145                 150                 155                 160
   Glu Glu Glu Phe Glu Asp Thr Leu Lys Tyr Ile Glu Ser Ile Arg Pro
                   165                 170                 175
   Met Ala Glu Pro Tyr Gly Ile Cys Arg Ile Val Pro Pro Ser Ser Trp
               180                 185                 190
   Lys Pro Pro Cys Leu Leu Lys Asp Lys Ser Ile Trp Glu Gly Ser Lys
           195                 200                 205
   Phe Ser Thr Arg Val Gln Lys Val Asp Lys Leu Gln Asn Arg Lys Ser
   210 215 220
   Ser Lys Lys Gly Arg Arg Gly Gly Met Met Lys Arg Arg Lys Leu Ala
   225 230 235 240
   Glu Ser Glu Glu Asn Ser Ala Thr Ala His Thr Gln Thr Gly Met Gln
   245 250 255
   Gln Ser Pro Glu Arg Phe Gly Phe Glu Pro Gly Pro Glu Phe Thr Leu
   260 265 270
   Gln Thr Phe Gln Lys Tyr Ala Asp Asp Phe Ser Lys Gln Tyr Phe Arg
   275 280 285
   Lys Asp Thr Ser Met Asp Ser Val Pro Ser Val Glu Asp Ile Glu Gly
   290 295 300
   Glu Tyr Trp Arg Ile Val Glu Val Pro Thr Glu Glu Ile Glu Val Ile
   305 310 315 320
   Tyr Gly Ala Asp Leu Glu Thr Gly Thr Phe Gly Ser Gly Phe Pro Lys
   325 330 335
   Leu Ser Pro Glu Thr Lys Ser Asp Ala Glu Asp Lys Tyr Ala Gln Ser
   340 345 350
   Gly Trp Asn Leu Asn Asn Leu Pro Arg Leu Gln Gly Ser Val Leu Ser
   355 360 365
   Phe Glu Gly Gly Asp Ile Ser Gly Val Leu Val Pro Trp Val Tyr Val
   370 375 380
   Gly Met Cys Phe Ser Ser Phe Cys Trp His Val Glu Asp His His Leu
   385 390 395 400
   Tyr Ser Leu Asn Tyr Met His Trp Gly Ala Pro Lys Leu Trp Tyr Gly
   405 410 415
   Val Pro Gly Lys Asp Ala Val Asn Leu Glu Ser Ala Met Arg Lys His
   420 425 430
   Leu Pro Glu Leu Phe Glu Glu Gln Pro Asp Leu Leu His Asn Leu Val
   435 440 445
   Thr Gln Phe Ser Pro Ser Leu Leu Lys Ser Glu Gly Val His Val Tyr
   450 455 460
   Arg Cys Val Gln His Glu Gly Glu Phe Val Leu Thr Phe Pro Arg Ala
   465 470 475 480
   Tyr His Ala Gly Phe Asn Cys Gly Phe Asn Cys Ala Glu Ala Val Asn
   485 490 495
   Val Ala Pro Ile Asp Trp Leu Pro Ile Gly His Asn Ala Val Glu Leu
   500 505 510
   Tyr Arg Glu Gln Ala Arg Lys Ile Thr Ile Ser His Asp Lys Leu Leu
   515 520 525
   Leu Gly Ala Ala Arg Glu Ala Ile Arg Ala Gln Trp Asp Ile Leu Phe
   530 535 540
   Leu Lys Arg Asn Thr Ala Asp Asn Met Arg Trp Lys Ser Ile Cys Gly
   545 550 555 560
   Ala Asp Ser Thr Ile Phe Lys Ala Leu Lys Ala Arg Ile Glu Thr Glu
   565 570 575
   Leu Val Gln Arg Lys Thr Leu Gly Val Pro Ala Gln Ser Arg Lys Met
   580 585 590
   Asp Ala Glu Phe Asp Ser Ile Asp Arg Glu Cys Ala Leu Cys Tyr Tyr
   595 600 605
   Asp Leu His Leu Ser Ala Ser Gly Cys Pro Cys Cys Pro Glu Lys Tyr
   610 615 620
   Ala Cys Leu Val His Ala Lys Gln Leu Cys Ser Cys Asp Trp Asp Lys
   625 630 635 640
   Arg Phe Phe Leu Phe Arg Tyr Asp Val Asn Glu Leu Asn Ile Leu Ala
                   645                 650                 655
   Asp Ala Leu Gly Gly Lys Leu Ser Ala Ile His Arg Trp Gly Val Ser
               660                 665                 670
   Asp Leu Gly Leu Ser Leu Ser Ser Cys Val Lys Arg Glu Lys Val Gln
           675                 680                 685
   Asp Ser Lys Thr Val Arg Arg Leu Thr Asp Gly Pro Arg Arg Ser Tyr
       690                 695                 700
   Met Ser Gln Ala Ser Ala Val Ser Leu Val Ser Ser Ser Thr Ser Asn
   705                 710                 715                 720
   Glu Gln Lys Asp Glu Gly Asn Lys Ile Met Lys Ile Ala Ser Pro Gln
                   725                 730                 735
   Thr Asn Asn Val Cys Pro Ser Val Glu Gln Arg Lys Ser Glu Asn Ile
               740                 745                 750
   Ser Pro Leu Lys Glu Pro Cys Val Arg Asn Glu Leu Ser Cys Thr Thr
           755                 760                 765
   Asn Ser Asp Ser Asn Gly Leu Gln Tyr Asn Gly Gly Leu Gly Gly His
       770                 775                 780
   Lys Gly Ser Ala Pro Gly Leu Pro Val Ser Ser Ser Pro Ser Phe Ser
   785                 790                 795                 800
   Ser Asn Val Ala Thr Arg Pro Ile Ser Thr Ser Ser Val Ser Met Lys
                   805                 810                 815
   Ile Val Gln Gly Leu Val Ala Ser Lys Ser Cys Ile Gln Ala Ser Ser
               820                 825                 830
   Arg Thr Gly Asp Ser Arg Ser Leu Leu Gly Glu His His Asn Arg Ser
           835                 840                 845
   Pro Ala Met Ile His Asp Gly Thr Asn Met Lys Ser Ser Leu Glu Ser
       850                 855                 860
   Ser Asn Asn Ser Cys Arg Leu Ile Ala Ser Asp Tyr Asn Ala Thr Pro
   865                 870                 875                 880
   Cys His Ser Ser Lys Asp Gln Val Leu Val Thr Pro Gly Thr Asn Ala
                   885                 890                 895
   Ser Val Val Thr Leu Lys Asp Ser Ser Gln Val His Ser Ala Ser Ser
               900                 905                 910
   Gln Gln Phe Val Arg Thr Gly Pro Trp Thr Gln Ser Ala Ser His Glu
           915                 920                 925
   Ala Ser Ser Pro Ser Thr Ser Ala Leu Lys Pro Ser Leu Asp Pro Pro
       930                 935                 940
   Ala Met Lys Asn Leu Tyr Gly Gly Phe Thr Gln Gly Ser Ala His Pro
   945                 950                 955                 960
   Gly Pro Pro Ser Phe Ser Asn Gln Gln Pro Asn Asp Gly Arg Leu Gln
                   965                 970                 975
   Arg Thr Ser Glu Ser Leu Pro Gly Val Glu Ala Arg Ala Arg Gly His
               980                 985                 990
   Pro Thr Val Thr Ala Gln Pro Ala  Leu Glu Ile His Ser  Arg Asn Gly
           995                 1000                1005
   Gly Ala  Gln Lys Gly Pro Arg  Ile Ala Asn Val Val  His Arg Phe
       1010                1015                1020
   Lys Cys  Ser Val Glu Pro Leu  Glu Ile Gly Val Val  Leu Ser Gly
       1025                1030                1035
   Arg Leu  Trp Ser Ser Ser Gln  Ala Ile Phe Pro Lys  Gly Phe Arg
       1040                1045                1050
   Ser Arg  Val Lys Tyr Phe Ser  Ile Val Asp Pro Ile  Gln Met Ala
       1055                1060                1065
   Tyr Tyr  Ile Ser Glu Ile Leu  Asp Ala Gly Met Gln  Gly Pro Leu
       1070                 1075                 1080
   Phe Met  Val Lys Leu Glu Asn  Cys Pro Gly Glu Val  Phe Ile Asn
       1085                 1090                 1095
   Leu Ser  Pro Thr Lys Cys Trp  Asn Met Val Arg Glu  Arg Leu Asn
       1100                 1105                 1110
   Met Glu  Ile Arg Arg Gln Leu  Asn Met Gly Lys Ser  Asn Leu Pro
       1115                 1120                 1125
   Thr Leu  Gln Pro Pro Gly Ser  Val Asp Gly Leu Glu  Met Phe Gly
       1130                 1135                 1140
   Leu Leu  Ser Pro Pro Ile Val  Gln Ala Ile Trp Ala  Arg Asp Arg
       1145                 1150                 1155
   Asp His  Ile Cys Thr Glu Tyr  Trp Arg Ser Arg Pro  His Val Leu
       1160                 1165                 1170
   Ile Glu  Asp Pro Asn Asn Arg  His Met Leu Ser Gln  Gly Pro Pro
       1175                 1180                 1185
   Leu Leu  Ala Leu Arg Gly Leu  Ile Gln Arg Ala Asn  Arg Asp Glu
       1190                 1195                 1200
   Leu Gln  Val Leu Arg Ser Leu  Met Thr Asn Ser Asn  Asn Leu Asp
       1205                 1210                 1215
   Asp Ser  Ser Arg Gln Gln Ala  Ala His Ile Ile Glu  Glu Glu Ile
       1220                 1225                 1230
   Ala Lys  Gln Leu Cys
       1235
<210> 85
   <211> 2916
   <212> DNA
   <213> Oryza sativa
<400> 85
   atggtttcct cccgcgaccc cggcgaggag gccagcgcgc cgccgccccc gcccccgcgc     60
   cgcggcgaga agcggcgaat gcgcggccgc accccgtcgc cggagccggc ctccgcgccg    120
   caggatctct gcccatcagg agcttgcggg gacaatgttg ctggagctac aactacaaat    180
   ggaaagtggc atccacatga atcgtacaga cctgaaattg atgatgcccc tgttttcact    240
   ccaacggaag aggagtttaa agatccaatt agatatatta cgagcattcg tccccaagca    300
   gaaaagtatg gaatttgtcg tattgttcca ccatcttctt ggcgaccgcc ttgttctctg    360
   aaggagaaga acttctggga atgtacagag ttcaataccc gtgttcaaca agttgacaag    420
   cttcaaaacc gggaacccac aaagaaaaaa tcacaacctc gagttcagaa gaagaggaag    480
   aggagaaaga gactgagatt tgggatgact cacaggcgtc ctagtgcaaa tacatcagaa    540
   gactgcgcag atgcagacga gaagtttggc tttcaatctg gctcagattt cacactagat    600
   gagtttcaga aatatgcaga tgagtttaag cagcagtatt ttggaataaa gggaagtgac    660
   gaaatccctc tttctgaaat taaaaagaag aaaaaaaatt ggcaaccatc ggtcgatgaa    720
   atagagggag aatattggcg gatagttgta tgccccactg acgaagttga ggtggattat    780
   ggtgctgatt tggacacttc aatgttcagt agtggattct ctaaattatc ttcagattca    840
   aatagacgag atccatatgg tttatcttgt tggaatttga acaatcttcc acgtattcct    900
   gggtctgtac tgtcatttga aactgaggat atatctggcg tcgtagtccc ttggctttat    960
   gtagggatgt gcttctcatc attctgttgg cacgtggaag atcatttcct ttattctatg   1020
   aattacatgc attttggtga accaaaagta tggtatggtg ttcctggtgc tgatgcagtg   1080
   aagctggaag aagctatgag aaagaactta ccaagattgt ttgaagaaca gcctgatctc   1140
   ctacatgagc tggttacgca attatctcct tctgttctta aatcagaagg agttcctgtt   1200
   tatcgtgttg ttcagaatcc aggcgagttt gttctaacgc taccgcgagc ttaccattct   1260
   gggttcaact gtggcttcaa ctgtgcggag gcagtaaatg tcgcacctgt ggattggctg   1320
   cctcacggac aatgtgctgt tgagctctac agggagcagc ggcgcaagac atccatatca   1380
   catgacaaat tattactaaa aactgcaaat gaagctgtca gacagctttg gatgaacctt   1440
   agcgactgca aaagtgaaca aggagtatac agatggcagg atacttgcgg aaaggacgga   1500
   atgctgacaa gtgcaattaa gacaagggtt aaaatggaga aggcagcacg gggagggaat   1560
   atggcactgc gatataagaa aatggatggg gattatgatt cagctgaccg ggaatgcttt   1620
   tcatgttttt atgatctcca tttgtcagct gtcagctgcc aatgctcccc aaatcgtttt   1680
   gcttgcttaa accatgcaaa cattctatgt tcatgtgaaa tggacagaaa aaccgcgttg   1740
   ttgcggtata ccatagagga gctccatact cttgttgcag ctctagaggg tgatccaact   1800
   gcggtctacc agtggggaca gaatgattta ggtttagtct gcccatctgg ttctactcag   1860
   tacaagaaga tggacttggg tgaaaacacg gaatttccgg attcagcaac caacgtcaat   1920
   catggctgca gcttaggaag tcaagatcaa tatcactatg accccgcaaa gccagcagga   1980
   taccagcaag agaagggaat ccagattgct tcagaaaaac atgataagaa caagatggtt   2040
   gtcaatcttg agtctccagc aacagctagt aatccaagca ggtcaaagtc tgactgcagt   2100
   ggctcactgt ccttgaatca ttcatctgag ttaccatctt caagaattca aacaggaaat   2160
   tctacgctag cttccattac cacagagaaa ctgtttggtg ttgacattaa atccaattta   2220
   gcacagtctt ctgatggcca agttagtcaa ttggccaagc cttcctcgag ccaaactgat   2280
   gaagtctcta agccagcaat agctaagtat acggttgagc tgctagacag tggaacaatg   2340
   atgattggta aaaagtggtg caatcagcaa gctatattcc ccaaaggatt taagagtcga   2400
   gttacatttc atagtgtact agatccaaca aggacatgct gctacatctc cgaagttctt   2460
   gatgctgggc ttcttggacc attgtttagg gtgactgtcg aaggtcttcc agaagtttcg   2520
   tttactcaca catcaccaat gcaatgttgg gacagtgtaa gagacagagt aaatgaagaa   2580
   atagcaaaac aaataagttt tggaaaatct ggccttcctg atttctatcc ctgcaattct   2640
   ttgaatggac ttgaaatgtt tgggttctta tcctccccta taattaagga aatcgaggct   2700
   ctagatccct gtcaccaatg cttggactat tggttgtcaa gggtttcttc tgttggaact   2760
   gaactcccct cggaatctgt gatggcagca atggttaatg actccactaa ccccccaata   2820
   aagttgctcg ggattgagat taaccggagg gaatcagaac aatcaagtag cttcaataat   2880
   tcctgtgtga ggaggtcaca cttggcaggt tgctga                             2916
<210> 86
   <211> 971
   <212> PRT
   <213> Oryza sativa
<400> 86
   Met Val Ser Ser Arg Asp Pro Gly Glu Glu Ala Ser Ala Pro Pro Pro
   1               5                   10                  15
   Pro Pro Pro Arg Arg Gly Glu Lys Arg Arg Met Arg Gly Arg Thr Pro
               20                  25                  30
   Ser Pro Glu Pro Ala Ser Ala Pro Gln Asp Leu Cys Pro Ser Gly Ala
           35                  40                  45
   Cys Gly Asp Asn Val Ala Gly Ala Thr Thr Thr Asn Gly Lys Trp His
       50                  55                  60
   Pro His Glu Ser Tyr Arg Pro Glu Ile Asp Asp Ala Pro Val Phe Thr
   65                  70                  75                  80
   Pro Thr Glu Glu Glu Phe Lys Asp Pro Ile Arg Tyr Ile Thr Ser Ile
                   85                  90                  95
   Arg Pro Gln Ala Glu Lys Tyr Gly Ile Cys Arg Ile Val Pro Pro Ser
               100                 105                 110
   Ser Trp Arg Pro Pro Cys Ser Leu Lys Glu Lys Asn Phe Trp Glu Cys
           115                 120                 125
   Thr Glu Phe Asn Thr Arg Val Gln Gln Val Asp Lys Leu Gln Asn Arg
       130                 135                 140
   Glu Pro Thr Lys Lys Lys Ser Gln Pro Arg Val Gln Lys Lys Arg Lys
   145                 150                 155                 160
   Arg Arg Lys Arg Leu Arg Phe Gly Met Thr His Arg Arg Pro Ser Ala
                   165                 170                 175
   Asn Thr Ser Glu Asp Cys Ala Asp Ala Asp Glu Lys Phe Gly Phe Gln
               180                 185                 190
   Ser Gly Ser Asp Phe Thr Leu Asp Glu Phe Gln Lys Tyr Ala Asp Glu
           195                 200                 205
   Phe Lys Gln Gln Tyr Phe Gly Ile Lys Gly Ser Asp Glu Ile Pro Leu
       210                 215                 220
   Ser Glu Ile Lys Lys Lys Lys Lys Asn Trp Gln Pro Ser Val Asp Glu
   225                 230                 235                 240
   Ile Glu Gly Glu Tyr Trp Arg Ile Val Val Cys Pro Thr Asp Glu Val
                   245                 250                 255
   Glu Val Asp Tyr Gly Ala Asp Leu Asp Thr Ser Met Phe Ser Ser Gly
               260                 265                 270
   Phe Ser Lys Leu Ser Ser Asp Ser Asn Arg Arg Asp Pro Tyr Gly Leu
           275                 280                 285
   Ser Cys Trp Asn Leu Asn Asn Leu Pro Arg Ile Pro Gly Ser Val Leu
       290                 295                 300
   Ser Phe Glu Thr Glu Asp Ile Ser Gly Val Val Val Pro Trp Leu Tyr
   305                 310                 315                 320
   Val Gly Met Cys Phe Ser Ser Phe Cys Trp His Val Glu Asp His Phe
                   325                 330                 335
   Leu Tyr Ser Met Asn Tyr Met His Phe Gly Glu Pro Lys Val Trp Tyr
               340                 345                 350
   Gly Val Pro Gly Ala Asp Ala Val Lys Leu Glu Glu Ala Met Arg Lys
           355                 360                 365
   Asn Leu Pro Arg Leu Phe Glu Glu Gln Pro Asp Leu Leu His Glu Leu
       370                 375                 380
   Val Thr Gln Leu Ser Pro Ser Val Leu Lys Ser Glu Gly Val Pro Val
   385                 390                 395                 400
   Tyr Arg Val Val Gln Asn Pro Gly Glu Phe Val Leu Thr Leu Pro Arg
                   405                 410                 415
   Ala Tyr His Ser Gly Phe Asn Cys Gly Phe Asn Cys Ala Glu Ala Val
               420                 425                 430
   Asn Val Ala Pro Val Asp Trp Leu Pro His Gly Gln Cys Ala Val Glu
           435                 440                 445
   Leu Tyr Arg Glu Gln Arg Arg Lys Thr Ser Ile Ser His Asp Lys Leu
       450                 455                 460
   Leu Leu Lys Thr Ala Asn Glu Ala Val Arg Gln Leu Trp Met Asn Leu
   465                 470                 475                 480
   Ser Asp Cys Lys Ser Glu Gln Gly Val Tyr Arg Trp Gln Asp Thr Cys
                   485                 490                 495
   Gly Lys Asp Gly Met Leu Thr Ser Ala Ile Lys Thr Arg Val Lys Met
               500                 505                 510
   Glu Lys Ala Ala Arg Gly Gly Asn Met Ala Leu Arg Tyr Lys Lys Met
           515                 520                 525
   Asp Gly Asp Tyr Asp Ser Ala Asp Arg Glu Cys Phe Ser Cys Phe Tyr
       530                 535                 540
   Asp Leu His Leu Ser Ala Val Ser Cys Gln Cys Ser Pro Asn Arg Phe
   545                 550                 555                 560
   Ala Cys Leu Asn His Ala Asn Ile Leu Cys Ser Cys Glu Met Asp Arg
                   565                 570                 575
   Lys Thr Ala Leu Leu Arg Tyr Thr Ile Glu Glu Leu His Thr Leu Val
               580                 585                 590
   Ala Ala Leu Glu Gly Asp Pro Thr Ala Val Tyr Gln Trp Gly Gln Asn
               595                 600                 605
   Asp Leu Gly Leu Val Cys Pro Ser Gly Ser Thr Gln Tyr Lys Lys Met
       610                 615                 620
   Asp Leu Gly Glu Asn Thr Glu Phe Pro Asp Ser Ala Thr Asn Val Asn
   625                 630                 635                 640
   His Gly Cys Ser Leu Gly Ser Gln Asp Gln Tyr His Tyr Asp Pro Ala
                   645                 650                 655
   Lys Pro Ala Gly Tyr Gln Gln Glu Lys Gly Ile Gln Ile Ala Ser Glu
               660                 665                 670
   Lys His Asp Lys Asn Lys Met Val Val Asn Leu Glu Ser Pro Ala Thr
           675                 680                 685
   Ala Ser Asn Pro Ser Arg Ser Lys Ser Asp Cys Ser Gly Ser Leu Ser
       690                 695                 700
   Leu Asn His Ser Ser Glu Leu Pro Ser Ser Arg Ile Gln Thr Gly Asn
   705                 710                 715                 720
   Ser Thr Leu Ala Ser Ile Thr Thr Glu Lys Leu Phe Gly Val Asp Ile
                   725                 730                 735
   Lys Ser Asn Leu Ala Gln Ser Ser Asp Gly Gln Val Ser Gln Leu Ala
               740                 745                 750
   Lys Pro Ser Ser Ser Gln Thr Asp Glu Val Ser Lys Pro Ala Ile Ala
           755                 760                 765
   Lys Tyr Thr Val Glu Leu Leu Asp Ser Gly Thr Met Met Ile Gly Lys
       770                 775                 780
   Lys Trp Cys Asn Gln Gln Ala Ile Phe Pro Lys Gly Phe Lys Ser Arg
   785                 790                 795                 800
   Val Thr Phe His Ser Val Leu Asp Pro Thr Arg Thr Cys Cys Tyr Ile
                   805                 810                 815
   Ser Glu Val Leu Asp Ala Gly Leu Leu Gly Pro Leu Phe Arg Val Thr
               820                 825                 830
   Val Glu Gly Leu Pro Glu Val Ser Phe Thr His Thr Ser Pro Met Gln
           835                 840                 845
   Cys Trp Asp Ser Val Arg Asp Arg Val Asn Glu Glu Ile Ala Lys Gln
       850                 855                 860
   Ile Ser Phe Gly Lys Ser Gly Leu Pro Asp Phe Leu Ser Cys Asn Ser
   865                 870                 875                 880
   Leu Asn Gly Leu Glu Met Phe Gly Phe Leu Ser Ser Pro Ile Ile Lys
                   885                 890                 895
   Glu Ile Glu Ala Leu Asp Pro Cys His Gln Cys Leu Asp Tyr Trp Leu
               900                 905                 910
   Ser Arg Val Ser Ser Val Gly Thr Glu Leu Pro Ser Glu Ser Val Met
           915                 920                 925
   Ala Ala Met Val Asn Asp Ser Thr Asn Pro Pro Ile Lys Leu Leu Gly
       930                 935                 940
   Ile Glu Ile Asn Arg Arg Glu Ser Glu Gln Ser Ser Ser Phe Asn Asn
   945                 950                 955                 960
   Ser Cys Val Arg Arg Ser His Leu Ala Gly Cys
                   965                 970
<210> 87
   <211> 1140
   <212> DNA
   <213> Oryza sativa
<400> 87
   atgtcatgtt tgagttggga gccaccagat atgtggtcta aagtacatgg caccggcact     60
   agtcctgaga tgaaaaacgt gaaggctatt gattgtttat cttgctgtga ggtcgagata    120
   tgcactcaag acttcttcaa tgggtattat gaaggtcgga tgtatcaaaa tctatggcct    180
   gaaatgctta aattgaagga ctggcctaca tcaaatcatt ttgaagagct tttgccttcc    240
   catggagtta aatacatgaa ttctttacct tttcaaccat acacaaactt gaagtctggt    300
   ttgctgaatg tctcaacctt gcttcctgat gacatcttaa agcttgacat gggcccaaaa    360
   tcatatatag cttatggcta tgcacaggaa cttggtagag gagattctgt tacaaagctt    420
   cactgcgatt tgtctgatgc agttaacgtt ttgatgcata ctgctgaagt tgacccttcc    480
   gaggaacaaa tagatgcaat aaaaagtttg aaaagaagac atacagcgca aaatgaaaag    540
   gagtgttctg ggaatgcaga cggaaattat acatctccta aaatctgtgg ggatgcaaat    600
   gagttgtcct gtcctataaa cagtgagacc aacaagggag gtgctttatg ggatattttt    660
   aggagggaag atgttccaaa actgaaattg tatctcgaca agcattctaa ggaatttcgc    720
   catatatact gttctgcagt tcaaaaggta tgtaaccctg tacatgatga aacattttat    780
   ctaacagaag aacacaagag aaaactcaag gaggagcatg gaattgagcc ttggacattt    840
   gtacaaaaac ttggggaggc agtattcatt cctgctgggt gtcctcatca agtacggaat    900
   cttaagtcct gcaccaagat tgccttggat tttgtatcac ccgagaatgt taaggagtgt    960
   ctcagcttaa ccgaggactt ccgaagactt cctaagaacc acagggccaa agaagacaaa   1020
   ttagagctag gtgtggttca aaatggaccc aagcccacat accctcgaga aactattcta   1080
   aactctcaat gggacatctt gttgtttgca tatcatccaa atagttatga caaagtttaa   1140
<210> 88
   <211> 379
   <212> PRT
   <213> Oryza sativa
<400> 88
   Met Ser Cys Leu Ser Trp Glu Pro Pro Asp Met Trp Ser Lys Val His
   1               5                   10                  15
   Gly Thr Gly Thr Ser Pro Glu Met Lys Asn Val Lys Ala Ile Asp Cys
               20                  25                  30
   Leu Ser Cys Cys Glu Val Glu Ile Cys Thr Gln Asp Phe Phe Asn Gly
           35                  40                  45
   Tyr Tyr Glu Gly Arg Met Tyr Gln Asn Leu Trp Pro Glu Met Leu Lys
       50                  55                  60
   Leu Lys Asp Trp Pro Thr Ser Asn His Phe Glu Glu Leu Leu Pro Ser
   65                  70                  75                  80
   His Gly Val Lys Tyr Met Asn Ser Leu Pro Phe Gln Pro Tyr Thr Asn
                   85                  90                  95
   Leu Lys Ser Gly Leu Leu Asn Val Ser Thr Leu Leu Pro Asp Asp Ile
               100                 105                 110
   Leu Lys Leu Asp Met Gly Pro Lys Ser Tyr Ile Ala Tyr Gly Tyr Ala
           115                 120                 125
   Gln Glu Leu Gly Arg Gly Asp Ser Val Thr Lys Leu His Cys Asp Leu
       130                 135                 140
   Ser Asp Ala Val Asn Val Leu Met His Thr Ala Glu Val Asp Pro Ser
   145                 150                 155                 160
   Glu Glu Gln Ile Asp Ala Ile Lys Ser Leu Lys Arg Arg His Thr Ala
                   165                 170                 175
   Gln Asn Glu Lys Glu Cys Ser Gly Asn Ala Asp Gly Asn Tyr Thr Ser
               180                 185                 190
   Pro Lys Ile Cys Gly Asp Ala Asn Glu Leu Ser Cys Pro Ile Asn Ser
           195                 200                 205
   Glu Thr Asn Lys Gly Gly Ala Leu Trp Asp Ile Phe Arg Arg Glu Asp
       210                 215                 220
   Val Pro Lys Leu Lys Leu Tyr Leu Asp Lys His Ser Lys Glu Phe Arg
   225                 230                 235                 240
   His Ile Tyr Cys Ser Ala Val Gln Lys Val Cys Asn Pro Val His Asp
                   245                 250                 255
   Glu Thr Phe Tyr Leu Thr Glu Glu His Lys Arg Lys Leu Lys Glu Glu
               260                 265                 270
   His Gly Ile Glu Pro Trp Thr Phe Val Gln Lys Leu Gly Glu Ala Val
           275                 280                 285
   Phe Ile Pro Ala Gly Cys Pro His Gln Val Arg Asn Leu Lys Ser Cys
       290                 295                 300
   Thr Lys Ile Ala Leu Asp Phe Val Ser Pro Glu Asn Val Lys Glu Cys
   305                 310                 315                 320
   Leu Ser Leu Thr Glu Asp Phe Arg Arg Leu Pro Lys Asn His Arg Ala
                   325                 330                 335
   Lys Glu Asp Lys Leu Glu Leu Gly Val Val Gln Asn Gly Pro Lys Pro
               340                 345                 350
   Thr Tyr Pro Arg Glu Thr Ile Leu Asn Ser Gln Trp Asp Ile Leu Leu
           355                 360                 365
   Phe Ala Tyr His Pro Asn Ser Tyr Asp Lys Val
       370                 375
<210> 89
   <211> 2577
   <212> DNA
   <213> Oryza sativa
<400> 89
   atgcaacagg tggagggcag gaactgtctt cctgcggagg tcaggattgg cctcgagacg      60
   ctcaagaggc gccggcttga gaggatgcgt ttgactgctc agaacaatgc cggcgacggt     120
   cctccggtgc ccgcaaggag cggtggggat gcgctaagga ctcccgcaaa ctgcggggtc     180
   aggttgcatg ctaacaatgg cacagctcta cctagcagaa ccacccagaa caaggaccct     240
   tttgcaaagc gcagggtgga caagtttgat atgtctagcc tagaatggat tgacaagatc     300
   gaagaatgcc ctgtgtacta tcctaccaag gaggagttcg aggatcccat tggttatata     360
   cagaagattg cacctgtggc ttcgaaatac ggaatttgca aaatcgtatc tccagtaagc     420
   gcttctgttc ctgctggtgt cgtgttgatg aaggaacagc ctggtttcaa gttcatgacc     480
   agggttcagc cgcttcgcct cgccaaatgg gctgaagatg acacggtcac tttcttcatg     540
   agcgaaagaa agtacacttt ccgggattat gagaaaatgg ccaacaaggt gttcgccaag     600
   aaatactcaa gtgctagttg tctcccagct aagtacgtgg aggaggaatt ctggcgcgaa     660
   attgcttttg gtaaaatgga ttttgttgaa tatgcctgtg atgttgatgg tagtgctttc     720
   tcctcttctc ctcatgatca acttgggaaa agcaactgga acttgaagaa tttttcacgg     780
   ctttccaatt ctgtgcttag acttctgcag acaccaattc caggagtaac agatccaatg     840
   ctttatatcg ggatgctctt cagcatgttt gcttggcatg tggaagatca ttatttgtac     900
   agcatcaatt accatcattg tggggcattt aagacatggt atggcatacc gggtgatgct     960
   gctcctgggt ttgaaaaggt ggctagccag tttgtataca acaaggatat tttggttggt    1020
   gaaggagagg atgcagcatt tgatgttctc ttggggaaga caacaatgtt ccccccaaat    1080
   gtcttgttag accacaacgt tcctgtttat aaagctgtgc aaaaacctgg ggagtttgtc    1140
   attactttcc ctcgttccta ccacgcgggt ttcagccacg gcttcaattg tggcgaggct    1200
   gtcaactttg ctatcagtga ctggtttcct ctgggttctg tggccagcag acgctacgcg    1260
   cttctgaaca gaacaccctt gcttgcacac gaggagttac tttgccgttc tgcagtgctt    1320
   ctgtcccaca aactgttaaa cagcgaccca aaatccctca ataaatctga gcatccacat    1380
   tcacagcgtt gtttgaagtc ttgctttgtg cagttgatgc gattccagag aaacacacgt    1440
   ggcctacttg ctaaaatggg ctctcagata cattataagc caaaaacata cccgaatctc    1500
   tcatgtagca tgtgtcggcg tgattgctac attacacatg tgttgtgtgg atgcaacttt    1560
   gacccagtct gtcttcatca cgaacaagaa ctccggagct gcccttgtaa atctaaccag    1620
   gttgtctacg ttagggagga catacaggag ctagaagctc tatcaagaaa atttgagaag   1680
   gatatttgct tggataagga aataagtggt tttgactcat acaagcaggc cgaaaagaat   1740
   gagccatttt ttgagataac tcggaacctc aggaacactg aagtaaattt gatagaggat   1800
   gccttctcag gagcaactgc tgctgatgct gcaaagagtt ctcctgcaac gtcaacactg   1860
   acatcttttg cacaacatga tgtgcctgtt cttgctgaag caattgtctg tgctaatcaa   1920
   gccgaccaat tatactccac caccgagcaa accatcagct cacctttagt caaaggaact   1980
   gatgctgtgg gtgcaaattc atccagcatg gctgatgcta ataacggaac tggttcttgt   2040
   aatgcttcag ctgtggaata cagtggaaat tcagattctg aatctgaaat atttcgagtc   2100
   aagcgcaggt ctggcgtatc agtaaagcct gcatctgatg ccaagacatc aaacttgtct   2160
   gatcaacagg ttctcaggcg gttgaagaag gtgcgccctg aaatacaaca gcacaataag   2220
   cgaccagaag actatggtca ctgttcagtt ccctcaggtc gtatgagtat gaagaatttg   2280
   aattcatcct cctcatgtgg tgaagaacac tggaggatga agcggcggca gttggagact   2340
   cagcaggatg agagcagtta ttctgcaaag cagaagtcgt actcgtatcc atccaccagc   2400
   tattctttcc gaggagagtt tgtggaaatg agtagagatg ctgctgcaga agtccgacca   2460
   aagcgactga aaatccggct accttcttct agcacgaaca gagtggttga gcagggcagt   2520
   tcagggcaaa gatttacaag ggatgacaag tcgcttggtt gttggcctgc aatttag      2577
<210> 90
   <211> 858
   <212> PRT
   <213> Oryza sativa
<400> 90
   Met Gln Gln Val Glu Gly Arg Asn Cys Leu Pro Ala Glu Val Arg Ile
   1               5                   10                  15
   Gly Leu Glu Thr Leu Lys Arg Arg Arg Leu Glu Arg Met Arg Leu Thr
               20                  25                  30
   Ala Gln Asn Asn Ala Gly Asp Gly Pro Pro Val Pro Ala Arg Ser Gly
           35                  40                  45
   Gly Asp Ala Leu Arg Thr Pro Ala Asn Cys Gly Val Arg Leu His Ala
       50                  55                  60
   Asn Asn Gly Thr Ala Leu Pro Ser Arg Thr Thr Gln Asn Lys Asp Pro
   65                  70                  75                  80
   Phe Ala Lys Arg Arg Val Asp Lys Phe Asp Met Ser Ser Leu Glu Trp
                   85                  90                  95
   Ile Asp Lys Ile Glu Glu Cys Pro Val Tyr Tyr Pro Thr Lys Glu Glu
               100                 105                 110
   Phe Glu Asp Pro Ile Gly Tyr Ile Gln Lys Ile Ala Pro Val Ala Ser
           115                 120                 125
   Lys Tyr Gly Ile Cys Lys Ile Val Ser Pro Val Ser Ala Ser Val Pro
       130                 135                 140
   Ala Gly Val Val Leu Met Lys Glu Gln Pro Gly Phe Lys Phe Met Thr
   145                 150                 155                 160
   Arg Val Gln Pro Leu Arg Leu Ala Lys Trp Ala Glu Asp Asp Thr Val
                   165                 170                 175
   Thr Phe Phe Met Ser Glu Arg Lys Tyr Thr Phe Arg Asp Tyr Glu Lys
               180                 185                 190
   Met Ala Asn Lys Val Phe Ala Lys Lys Tyr Ser Ser Ala Ser Cys Leu
           195                 200                 205
   Pro Ala Lys Tyr Val Glu Glu Glu Phe Trp Arg Glu Ile Ala Phe Gly
       210                 215                 220
   Lys Met Asp Phe Val Glu Tyr Ala Cys Asp Val Asp Gly Ser Ala Phe
   225                 230                 235                 240
   Ser Ser Ser Pro His Asp Gln Leu Gly Lys Ser Asn Trp Asn Leu Lys
                       245                 250                 255
   Asn Phe Ser Arg Leu Ser Asn Ser Val Leu Arg Leu Leu Gln Thr Pro
               260                 265                 270
   Ile Pro Gly Val Thr Asp Pro Met Leu Tyr Ile Gly Met Leu Phe Ser
           275                 280                 285
   Met Phe Ala Trp His Val Glu Asp His Tyr Leu Tyr Ser Ile Asn Tyr
       290                 295                 300
   His His Cys Gly Ala Phe Lys Thr Trp Tyr Gly Ile Pro Gly Asp Ala
   305                 310                 315                 320
   Ala Pro Gly Phe Glu Lys Val Ala Ser Gln Phe Val Tyr Asn Lys Asp
                   325                 330                 335
   Ile Leu Val Gly Glu Gly Glu Asp Ala Ala Phe Asp Val Leu Leu Gly
               340                 345                 350
   Lys Thr Thr Met Phe Pro Pro Asn Val Leu Leu Asp His Asn Val Pro
           355                 360                 365
   Val Tyr Lys Ala Val Gln Lys Pro Gly Glu Phe Val Ile Thr Phe Pro
       370                 375                 380
   Arg Ser Tyr His Ala Gly Phe Ser His Gly Phe Asn Cys Gly Glu Ala
   385                 390                 395                 400
   Val Asn Phe Ala Ile Ser Asp Trp Phe Pro Leu Gly Ser Val Ala Ser
                   405                 410                 415
   Arg Arg Tyr Ala Leu Leu Asn Arg Thr Pro Leu Leu Ala His Glu Glu
               420                 425                 430
   Leu Leu Cys Arg Ser Ala Val Leu Leu Ser His Lys Leu Leu Asn Ser
           435                 440                 445
   Asp Pro Lys Ser Leu Asn Lys Ser Glu His Pro His Ser Gln Arg Cys
       450                 455                 460
   Leu Lys Ser Cys Phe Val Gln Leu Met Arg Phe Gln Arg Asn Thr Arg
   465                 470                 475                 480
   Gly Leu Leu Ala Lys Met Gly Ser Gln Ile His Tyr Lys Pro Lys Thr
                   485                 490                 495
   Tyr Pro Asn Leu Ser Cys Ser Met Cys Arg Arg Asp Cys Tyr Ile Thr
               500                 505                 510
   His Val Leu Cys Gly Cys Asn Phe Asp Pro Val Cys Leu His His Glu
           515                 520                 525
   Gln Glu Leu Arg Ser Cys Pro Cys Lys Ser Asn Gln Val Val Tyr Val
       530                 535                 540
   Arg Glu Asp Ile Gln Glu Leu Glu Ala Leu Ser Arg Lys Phe Glu Lys
   545                 550                 555                 560
   Asp Ile Cys Leu Asp Lys Glu Ile Ser Gly Phe Asp Ser Tyr Lys Gln
                   565                 570                 575
   Ala Glu Lys Asn Glu Pro Phe Phe Glu Ile Thr Arg Asn Leu Arg Asn
               580                 585                 590
   Thr Glu Val Asn Leu Ile Glu Asp Ala Phe Ser Gly Ala Thr Ala Ala
           595                 600                 605
   Asp Ala Ala Lys Ser Ser Pro Ala Thr Ser Thr Leu Thr Ser Phe Ala
       610                 615                 620
   Gln His Asp Val Pro Val Leu Ala Glu Ala Ile Val Cys Ala Asn Gln
   625                 630                 635                 640
   Ala Asp Gln Leu Tyr Ser Thr Thr Glu Gln Thr Ile Ser Ser Pro Leu
                   645                 650                 655
   Val Lys Gly Thr Asp Ala Val Gly Ala Asn Ser Ser Ser Met Ala Asp
               660                 665                 670
   Ala Asn Asn Gly Thr Gly Ser Cys Asn Ala Ser Ala Val Glu Tyr Ser
           675                 680                 685
   Gly Asn Ser Asp Ser Glu Ser Glu Ile Phe Arg Val Lys Arg Arg Ser
       690                 695                 700
   Gly Val Ser Val Lys Pro Ala Ser Asp Ala Lys Thr Ser Asn Leu Ser
   705                 710                 715                 720
   Asp Gln Gln Val Leu Arg Arg Leu Lys Lys Val Arg Pro Glu Ile Gln
                   725                 730                 735
   Gln His Asn Lys Arg Pro Glu Asp Tyr Gly His Cys Ser Val Pro Ser
               740                 745                 750
   Gly Arg Met Ser Met Lys Asn Leu Asn Ser Ser Ser Ser Cys Gly Glu
           755                 760                 765
   Glu His Trp Arg Met Lys Arg Arg Gln Leu Glu Thr Gln Gln Asp Glu
       770                 775                 780
   Ser Ser Tyr Ser Ala Lys Gln Lys Ser Tyr Ser Tyr Pro Ser Thr Ser
   785                 790                 795                 800
   Tyr Ser Phe Arg Gly Glu Phe Val Glu Met Ser Arg Asp Ala Ala Ala
                   805                 810                 815
   Glu Val Arg Pro Lys Arg Leu Lys Ile Arg Leu Pro Ser Ser Ser Thr
               820                 825                 830
   Asn Arg Val Val Glu Gln Gly Ser Ser Gly Gln Arg Phe Thr Arg Asp
           835                 840                 845
   Asp Lys Ser Leu Gly Cys Trp Pro Ala Ile
       850                 855
<210> 91
   <211> 1566
   <212> DNA
   <213> Oryza sativa
<400> 91
   atgccgagcg ccttccactc cctcctcctc cccgccattc gcaaccccaa acctagccgt     60
   cgccgcggcc gcggccgcgg cggcagcaaa cgccccaaga agaccaccaa atccaagaac    120
   cgcctcgccg acgccgccgc cggagacgcc accgccttcc acctgaagac ctccgcgcgc    180
   gccggtccgg ggggtgccgg gagcggccgg cgaggcgatg ggggatgcct cgtgcagccg    240
   ctcggcaacc tcctcctcct cggcggcggc ggcaacctcc gcgacgcggg gctcggcgcg    300
   ctccgcccgc tccccgacga cgtcctcctc gacgtgctcg gcctgctcgc ggcgcgcgac    360
   ctcgctaggc tctccgcggc gtcgagggcg ctctacgtcg tcgcctccca cgacccgctc    420
   tggcgggcgc tcgtcctcga cgagctcggc ggggacttcg ccttctcggg ctcgtggcgc    480
   gccacctaca tcgccgccgc gtcgggcggc cgcgcccacc tccccccgcg gggcctagag    540
   atcagggggt tctactccga ctacctcttc cagagctggc tctgcgccaa catggagatg    600
   cggccggagt ggctccaccg ggacaccatc gatcgccgcc gcggcatgtc cgtcgagcaa    660
   ttcgtctccg aattcgagga gcccaatagg ccggtgcttc tggaaggctg cctcgagagc    720
   tggccagcat tgcagaagtg gaccagggag cacttgctga aggtctcggc cgggaaggag    780
   ttcgccgtcg ggccggtgag catgacgctg gataggtacc tccagtatgc cgacaatgtg    840
   caggaggaga ggccattgta cctgttcgat gccaagttca ccgagaaggt gccggagatg    900
   gggagggact atgaggtgcc ggcgtacttc cgagaggacc tattcggggt gcttggggag    960
   gaaaggccgg accaccgatg ggttatcatt gggccggcag gttcagggtc gtcgtttcat   1020
   gttgatccaa actcgacatc ggcgtggaat gctgtgatca agggagccaa gaagtgggtg   1080
   atgttcccac cggaggtggt gccgccagga gttcatccaa gtgcggatgg agcagaagtc   1140
   actagccctg tatctatcat ggaatggttc atgaattttt atggggcatg taagacctgg   1200
   gaaaagaggc ctgttgagtg tatatgccgg gctggagagg tggtttttgt gcctaatgga   1260
   tggtggcatt tggttatcaa tctggaggaa tctattgcga ttactcagaa ttatgtgagc   1320
   aggaggaatc tgttgaatgt tcttgacttt ctcaagaggc ccaatgcaag tgaacttgta   1380
   tcagggacta cagacagggt aaacctgcat gacaagttcc gcaatgccat cgatatgact   1440
   tatcctggga tgattaaaca gcttgaactt gaagctcagc agaaggctgc tgctcgcaag   1500
   aagaaagtct cgttctggga atctgcggta gatgccaata ctggaggatt caagttctca   1560
   ttctga                                                              1566
<210> 92
   <211> 521
   <212> PRT
   <213> Oryza sativa
<400> 92
   Met Pro Ser Ala Phe His Ser Leu Leu Leu Pro Ala Ile Arg Asn Pro
   1               5                   10                  15
   Lys Pro Ser Arg Arg Arg Gly Arg Gly Arg Gly Gly Ser Lys Arg Pro
               20                  25                  30
   Lys Lys Thr Thr Lys Ser Lys Asn Arg Leu Ala Asp Ala Ala Ala Gly
           35                  40                  45
   Asp Ala Thr Ala Phe His Leu Lys Thr Ser Ala Arg Ala Gly Pro Gly
       50                  55                  60
   Gly Ala Gly Ser Gly Arg Arg Gly Asp Gly Gly Cys Leu Val Gln Pro
   65                  70                  75                  80
   Leu Gly Asn Leu Leu Leu Leu Gly Gly Gly Gly Asn Leu Arg Asp Ala
                   85                  90                  95
   Gly Leu Gly Ala Leu Arg Pro Leu Pro Asp Asp Val Leu Leu Asp Val
               100                 105                 110
   Leu Gly Leu Leu Ala Ala Arg Asp Leu Ala Arg Leu Ser Ala Ala Ser
           115                 120                 125
   Arg Ala Leu Tyr Val Val Ala Ser His Asp Pro Leu Trp Arg Ala Leu
       130                 135                 140
   Val Leu Asp Glu Leu Gly Gly Asp Phe Ala Phe Ser Gly Ser Trp Arg
   145                 150                 155                 160
   Ala Thr Tyr Ile Ala Ala Ala Ser Gly Gly Arg Ala His Leu Pro Pro
                   165                 170                 175
   Arg Gly Leu Glu Ile Arg Gly Phe Tyr Ser Asp Tyr Leu Phe Gln Ser
               180                 185                 190
   Trp Leu Cys Ala Asn Met Glu Met Arg Pro Glu Trp Leu His Arg Asp
           195                 200                 205
   Thr Ile Asp Arg Arg Arg Gly Met Ser Val Glu Gln Phe Val Ser Glu
       210                 215                 220
   Phe Glu Glu Pro Asn Arg Pro Val Leu Leu Glu Gly Cys Leu Glu Ser
   225                 230                 235                 240
   Trp Pro Ala Leu Gln Lys Trp Thr Arg Glu His Leu Leu Lys Val Ser
                   245                 250                 255
   Ala Gly Lys Glu Phe Ala Val Gly Pro Val Ser Met Thr Leu Asp Arg
               260                 265                 270
   Tyr Leu Gln Tyr Ala Asp Asn Val Gln Glu Glu Arg Pro Leu Tyr Leu
           275                 280                 285
   Phe Asp Ala Lys Phe Thr Glu Lys Val Pro Glu Met Gly Arg Asp Tyr
       290                 295                 300
   Glu Val Pro Ala Tyr Phe Arg Glu Asp Leu Phe Gly Val Leu Gly Glu
   305                 310                 315                 320
   Glu Arg Pro Asp His Arg Trp Val Ile Ile Gly Pro Ala Gly Ser Gly
                   325                 330                 335
   Ser Ser Phe His Val Asp Pro Asn Ser Thr Ser Ala Trp Asn Ala Val
               340                 345                 350
   Ile Lys Gly Ala Lys Lys Trp Val Met Phe Pro Pro Glu Val Val Pro
           355                 360                 365
   Pro Gly Val His Pro Ser Ala Asp Gly Ala Glu Val Thr Ser Pro Val
       370                 375                 380
   Ser Ile Met Glu Trp Phe Met Asn Phe Tyr Gly Ala Cys Lys Thr Trp
   385                 390                 395                 400
   Glu Lys Arg Pro Val Glu Cys Ile Cys Arg Ala Gly Glu Val Val Phe
                   405                 410                 415
   Val Pro Asn Gly Trp Trp His Leu Val Ile Asn Leu Glu Glu Ser Ile
               420                 425                 430
   Ala Ile Thr Gln Asn Tyr Val Ser Arg Arg Asn Leu Leu Asn Val Leu
           435                 440                 445
   Asp Phe Leu Lys Arg Pro Asn Ala Ser Glu Leu Val Ser Gly Thr Thr
       450                 455                 460
   Asp Arg Val Asn Leu His Asp Lys Phe Arg Asn Ala Ile Asp Met Thr
   465                 470                 475                 480
   Tyr Pro Gly Met Ile Lys Gln Leu Glu Leu Glu Ala Gln Gln Lys Ala
                   485                 490                 495
   Ala Ala Arg Lys Lys Lys Val Ser Phe Trp Glu Ser Ala Val Asp Ala
               500                 505                 510
   Asn Thr Gly Gly Phe Lys Phe Ser Phe
           515                 520
<210> 93
   <211> 432
   <212> DNA
   <213> Oryza sativa
<400> 93
   atggtggcag acagcgcggt cggcgtgcac ggcagtgcgg ccgacagtgg gtcggtgcgg      60
   tcggcgcgga cggcggcaga gagggacgcg gaggcggagg ctacgcggga cacaaaggca     120
   gcggcggtgg agccgccaga gtggctgcag accctgcccg tggcgccgga gtaccacccg     180
   acgctggtgg agttcgccga ccccatcgcc tacatcctca ggatcaagcc cgaggcgtcc     240
   cgcaacggca tttgcaagat ccacgtcggc ctctccacca agaaccgccg tgccgccagc     300
   cgccgagtgt gggagagcgg ggagcgctac accctggagg cgttctgcgc caaggtgccc     360
   gagttcgagc cctcgaggca cgccgcactg cccaagaacc ccacccacct ccagctcaag     420
   gccctcttct ag                                                         432
<210> 94
   <211> 143
   <212> PRT
   <213> Oryza sativa
<400> 94
   Met Val Ala Asp Ser Ala Val Gly Val His Gly Ser Ala Ala Asp Ser
   1               5                   10                  15
   Gly Ser Val Arg Ser Ala Arg Thr Ala Ala Glu Arg Asp Ala Glu Ala
               20                  25                  30
   Glu Ala Thr Arg Asp Thr Lys Ala Ala Ala Val Glu Pro Pro Glu Trp
           35                  40                  45
   Leu Gln Thr Leu Pro Val Ala Pro Glu Tyr His Pro Thr Leu Val Glu
       50                  55                  60
   Phe Ala Asp Pro Ile Ala Tyr Ile Leu Arg Ile Lys Pro Glu Ala Ser
   65                  70                  75                  80
   Arg Asn Gly Ile Cys Lys Ile Gln Val Gly Leu Ser Thr Lys Asn Arg
                   85                  90                  95
   Arg Ala Ala Ser Arg Arg Val Trp Glu Ser Gly Glu Arg Tyr Thr Leu
               100                 105                 110
   Glu Ala Phe Cys Ala Lys Val Pro Glu Phe Glu Pro Ser Arg His Ala
           115                 120                 125
   Ala Leu Pro Lys Asn Pro Thr His Leu Gln Leu Lys Ala Leu Phe
       130                 135                 140
<210> 95
   <211> 4101
   <212> DNA
   <213> Oryza sativa
<400> 95
   atgtcgctgc agccgccggc ggtggagccg ccggagtggc tgcggacgct gcccgtggcg     60
   ccggagtacc acccgacgct ggcggagttc gccgacccca tcgcctacat cctcaggatc    120
   gagccggagg cgtcccgcta cggcatctgc aagatcgtgc ccccgctccc gcggcccccc    180
   gaggacgaca ccttccgccg cctccaggcc gccttcgccg ccgcggcctc ctccaatggc    240
   gacccctccc cgaccttccc cacgcggctc cagcaggtcg gcctctccgc caggaaccgc    300
   cgcgccgcca gccgccgggt gtgggagagc ggggagcgct acaccctgga ggcgttccgc    360
   gccaaggcgg ccgagttcga gcccccgagg cacgccgcgc cgcccaggaa ccccacccac    420
   ctccagctcg aggccctctt ctgggccgcc tgcgcctcca ggcccttcag cgtcgagtac    480
   ggcaacgaca tgcccggctc cggcttcgcc tcccccgacg agctccccga cgccgccaat    540
   gccaccgacg tcggggagac ggagtggaac atgcgggtgg cgccccgcgc ccgggggtcg    600
   ctgctccgcg ccatggcccg cgacgtggcg ggcgtcacca ccccgatgct gtacgtggcc    660
   atgctctaca gctggttcgc ctggcacgtg gaggaccacg agctccacag cctcaacttc    720
   ctccacttcg gcaaggccaa gacctggtac ggcgtccccc gcgacgccat gctcgccttc    780
   gaggagacgg tgcgcgtcca cggctacgcc gacgacctca acgccatcat ggcctttcaa    840
   acgttaaatg agaagacaac tgtcttatct cctgaggtgc ttctttctgc tggtgttccc    900
   tgctgcagat tggttcaaaa agcgggagaa tttgttatca cattccctgg agcttatcat    960
   tcaggcttta gccatggatt taattgtggg gaagcatcaa atattgcaac tccccattgg   1020
   ttacaagtgg ctaaagaagc tgcaatccgg agggcttcaa ctaattgtgg tccgatggtg   1080
   tctcattatc agctgcttta tgagctagca ctctcattgc gtccaaggga gcctaagaat   1140
   ttctattctg ttccaagaag ctcacgttta agggacaaga ataagaatga aggtgatata   1200
   atggtcaaag aaaattttgt tggaagtgta accgaaaaca acaatttgct tagcgccctt   1260
   ctggataaga attcttgtat aattgttcca aacgctgatt tcttcgttcc atcctttcct   1320
   gttgcactgg aatctgaagt tactgttaaa caaaggttta cagctggtcc ctgcagtatt   1380
   agccagcaag gagcagaaaa tatggctgct gatcatgtgg ctgtagacaa ggttacagag   1440
   attcaggaca tgagtggatc cttatatcct tgtgaaacca gtcttgtggg ctgtagcaat   1500
   agaaaacttt atgaaacaaa atatggccaa cgggatgctg ctgctttgtg cttatctact   1560
   tcagagattc agagcagagg aattgataca gcaagatcac atccagcagg cgggatctta   1620
   gatcaaggac ggttgccatg tgtacagtgc ggaatactaa gctttgcatg tgtagccatc   1680
   attcaaccta gagaagcagc agtacagttt atcatgtcta aagagtgcat ctcatcgagt   1740
   gcaaaacaag gaggaattgg tgcatctgat gatacctcaa actggattga ccagagtcat   1800
   gagattagtc ctccaccagg tccagcatct ggaacagatg ataatgtaaa gcatgccgta   1860
   agtttggccc atgtctctga tcggtgtaga gaactatatg ccagcaatac agatggatgc   1920
   acctctgctc ttgggcttct agcttctgca tatgactcat cagactctga tgatgaaaca   1980
   actgaggatg tttcaaaaca tagcaagaaa aatgattcag taaaccaaag cacggatcct   2040
   caaatcttgg aaacatcagc tagctgttcc agtacagttc agtgtcaaaa gacaaattca   2100
   cacttgcatg aagaggaatg tgaggcaaga gccacatcat tgatgaaacc tgtaagccat   2160
   aatagtaggc ccattagtca gtctaacagg gatacagata tcgaccattt tatagaactg   2220
   ggaaagtcag gaacacagtg ttcaggttat cttgatttgg ttgatgatct aactacatca   2280
   gttttaaaat cctcttcaga tacttgtgta agtgcagcta aagcctcaat ggatccagat   2340
   gttttgacca tgctcaggta caataaagac tcttgcagaa tgcatgtgtt ttgtcttgaa   2400
   catgctttgg aaacatggac acagcttcaa caaatcggtg gtgctaatat tatgcttttg   2460
   tgccatccag aataccctag agcagagtca gcagcaaaag tcatagcaga agaacttggt   2520
   atcaagcatg attggaaaga tatcactttc aaagaagcaa ctgaagagga cgttaaaaag   2580
   attcagttgg ctttacagga tgaagatgct gaacccactg gcagtgactg ggcagtcaaa   2640
   atgggtatca acatatatta cagtgccaaa cagagcaaat caccacttta cagcaagcag   2700
   ataccataca attccatcat ttataaggca tttggccaag aaaacccaga cagtttgaca   2760
   gattatggat gtcaaaagtc aggctcaaca aagaaaaagg tggctgggtg gtggtgtggg   2820
   aaagtttgga tgtcaaatca agttcatcca cttctagctc gtgagcgtga agaacagaac   2880
   agtagcgtag tgtacggcaa agcaatgttc actactattt cccatggcaa agtacaagat   2940
   gaagcgtcaa caagatgcaa tacaagcaac cgaaccccat caagaagaac atcaagaaga   3000
   aaaaaggggg tatctgctga gaaatctaaa ccaaaaaaca agagatccac tgcttctgat   3060
   gaagctagta tgctttgcag tggccttgga atgaactctg gagttatcca tgaccaaact   3120
   gaaaactctg atgattatga caaacatggc aatggagatg aaattgagga aggaacaaat   3180
   cctcagaaat atcaacaacg taaattgcaa aacgtgacca ggaaatcaag ttctaagaag   3240
   cggaaggatg agaaaagaac agatagtttt catgaactat atgacgagga taatggtgtg   3300
   gattattggc ttaacatggg tagtggagat gatgccacac taggtaactc tcgacaacaa   3360
   agtcctgatc cagtgaaagt gaaatctgga ggcaaattgc aaggtaagag aaaatctagc   3420
   aagtacaagt ccaatgatga tttgttgaat gaagaaaata agttacaaaa gatgaacaaa   3480
   aaatcaagct ctaagaagca aaagaatgat aagataaata gacaacttca agaagatcaa   3540
   accgaagatg accatatgga ccacttagtt gacgtagcgg ttgcagatga agtcacacta   3600
   gacaatgagg ataaaattac agaagacaaa attgatgacg tgaaagtgaa atctagagga   3660
   aaatcgcaaa atggtaagag aaaaggcagc aaacatcagg ctaccgatgg tttgcgcgct   3720
   ggaaataaag tggccaaatt tccgtgtgat atagaaggat gtgacatgag cttcagtacc   3780
   cagcaggact tgttattgca taagcgtgac atttgtcctg tcaaaggatg caagaagaag   3840
   ttcttctgcc acaagtactt gcttcagcac cggaaggtgc atatagatga aaggccactt   3900
   aagtgtacat ggaaggggtg taagaaggca ttcaagtggc catgggcgag gacggagcac   3960
   atgagagtcc atacgggggt aagaccttac gagtgccagg aacctggctg tggtcagaca   4020
   ttccgattcg tttcagattt cagccgccac aagagaaaga ctggccattc ctctgataag   4080
   agaagaaaga acagtacata a                                              4101
<210> 96
   <211> 1366
   <212> PRT
   <213> Oryza sativa
<400> 96
   Met Ser Leu Gln Pro Pro Ala Val Glu Pro Pro Glu Trp Leu Arg Thr
   1               5                   10                  15
   Leu Pro Val Ala Pro Glu Tyr His Pro Thr Leu Ala Glu Phe Ala Asp
               20                  25                  30
   Pro Ile Ala Tyr Ile Leu Arg Ile Glu Pro Glu Ala Ser Arg Tyr Gly
           35                  40                  45
   Ile Cys Lys Ile Val Pro Pro Leu Pro Arg Pro Pro Glu Asp Asp Thr
       50                  55                  60
   Phe Arg Arg Leu Gln Ala Ala Phe Ala Ala Ala Ala Ser Ser Asn Gly
   65                  70                  75                  80
   Asp Pro Ser Pro Thr Phe Pro Thr Arg Leu Gln Gln Val Gly Leu Ser
                   85                  90                  95
   Ala Arg Asn Arg Arg Ala Ala Ser Arg Arg Val Trp Glu Ser Gly Glu
               100                 105                 110
   Arg Tyr Thr Leu Glu Ala Phe Arg Ala Lys Ala Ala Glu Phe Glu Pro
           115                 120                 125
   Pro Arg His Ala Ala Pro Pro Arg Asn Pro Thr His Leu Gln Leu Glu
       130                 135                 140
   Ala Leu Phe Trp Ala Ala Cys Ala Ser Arg Pro Phe Ser Val Glu Tyr
   145                 150                 155                 160
   Gly Asn Asp Met Pro Gly Ser Gly Phe Ala Ser Pro Asp Glu Leu Pro
                   165                 170                 175
   Asp Ala Ala Asn Ala Thr Asp Val Gly Glu Thr Glu Trp Asn Met Arg
               180                 185                 190
   Val Ala Pro Arg Ala Arg Gly Ser Leu Leu Arg Ala Met Ala Arg Asp
           195                 200                 205
   Val Ala Gly Val Thr Thr Pro Met Leu Tyr Val Ala Met Leu Tyr Ser
       210                 215                 220
   Trp Phe Ala Trp His Val Glu Asp His Glu Leu His Ser Leu Asn Phe
   225                 230                 235                 240
   Leu His Phe Gly Lys Ala Lys Thr Trp Tyr Gly Val Pro Arg Asp Ala
                   245                 250                 255
   Met Leu Ala Phe Glu Glu Thr Val Arg Val His Gly Tyr Ala Asp Asp
               260                 265                 270
   Leu Asn Ala Ile Met Ala Phe Gln Thr Leu Asn Glu Lys Thr Thr Val
           275                 280                 285
   Leu Ser Pro Glu Val Leu Leu Ser Ala Gly Val Pro Cys Cys Arg Leu
       290                 295                 300
   Val Gln Lys Ala Gly Glu Phe Val Ile Thr Phe Pro Gly Ala Tyr His
   305                 310                 315                 320
   Ser Gly Phe Ser His Gly Phe Asn Cys Gly Glu Ala Ser Asn Ile Ala
                   325                 330                 335
   Thr Pro His Trp Leu Gln Val Ala Lys Glu Ala Ala Ile Arg Arg Ala
               340                 345                 350
   Ser Thr Asn Cys Gly Pro Met Val Ser His Tyr Gln Leu Leu Tyr Glu
           355                 360                 365
   Leu Ala Leu Ser Leu Arg Pro Arg Glu Pro Lys Asn Phe Tyr Ser Val
       370                 375                 380
   Pro Arg Ser Ser Arg Leu Arg Asp Lys Asn Lys Asn Glu Gly Asp Ile
   385                 390                 395                 400
   Met Val Lys Glu Asn Phe Val Gly Ser Val Thr Glu Asn Asn Asn Leu
                   405                 410                 415
   Leu Ser Ala Leu Leu Asp Lys Asn Ser Cys Ile Ile Val Pro Asn Ala
               420                 425                 430
   Asp Phe Phe Val Pro Ser Phe Pro Val Ala Leu Glu Ser Glu Val Thr
           435                 440                 445
   Val Lys Gln Arg Phe Thr Ala Gly Pro Cys Ser Ile Ser Gln Gln Gly
       450                 455                 460
   Ala Glu Asn Met Ala Ala Asp His Val Ala Val Asp Lys Val Thr Glu
   465                 470                 475                 480
   Ile Gln Asp Met Ser Gly Ser Leu Tyr Pro Cys Glu Thr Ser Leu Val
                   485                 490                 495
   Gly Cys Ser Asn Arg Lys Leu Tyr Glu Thr Lys Tyr Gly Gln Arg Asp
               500                 505                 510
   Ala Ala Ala Leu Cys Leu Ser Thr Ser Glu Ile Gln Ser Arg Gly Ile
           515                 520                 525
   Asp Thr Ala Arg Ser His Pro Ala Gly Gly Ile Leu Asp Gln Gly Arg
       530                 535                 540
   Leu Pro Cys Val Gln Cys Gly Ile Leu Ser Phe Ala Cys Val Ala Ile
   545                 550                 555                 560
   Ile Gln Pro Arg Glu Ala Ala Val Gln Phe Ile Met Ser Lys Glu Cys
                   565                 570                 575
   Ile Ser Ser Ser Ala Lys Gln Gly Gly Ile Gly Ala Ser Asp Asp Thr
               580                 585                 590
   Ser Asn Trp Ile Asp Gln Ser His Glu Ile Ser Pro Pro Pro Gly Pro
           595                 600                 605
   Ala Ser Gly Thr Asp Asp Asn Val Lys His Ala Val Ser Leu Ala His
       610                 615                 620
   Val Ser Asp Arg Cys Arg Glu Leu Tyr Ala Ser Asn Thr Asp Gly Cys
   625                 630                 635                 640
   Thr Ser Ala Leu Gly Leu Leu Ala Ser Ala Tyr Asp Ser Ser Asp Ser
                   645                 650                 655
   Asp Asp Glu Thr Thr Glu Asp Val Ser Lys His Ser Lys Lys Asn Asp
               660                 665                 670
   Ser Val Asn Gln Ser Thr Asp Pro Gln Ile Leu Glu Thr Ser Ala Ser
           675                 680                 685
   Cys Ser Ser Thr Val Gln Cys Gln Lys Thr Asn Ser His Leu His Glu
       690                 695                 700
   Glu Glu Cys Glu Ala Arg Ala Thr Ser Leu Met Lys Pro Val Ser His
   705                 710                 715                 720
   Asn Ser Arg Pro Ile Ser Gln Ser Asn Arg Asp Thr Asp Ile Asp His
                   725                 730                 735
   Phe Ile Glu Leu Gly Lys Ser Gly Thr Gln Cys Ser Gly Tyr Leu Asp
               740                 745                 750
   Leu Val Asp Asp Leu Thr Thr Ser Val Leu Lys Ser Ser Ser Asp Thr
           755                 760                 765
   Cys Val Ser Ala Ala Lys Ala Ser Met Asp Pro Asp Val Leu Thr Met
       770                 775                 780
   Leu Arg Tyr Asn Lys Asp Ser Cys Arg Met His Val Phe Cys Leu Glu
   785                 790                 795                 800
   His Ala Leu Glu Thr Trp Thr Gln Leu Gln Gln Ile Gly Gly Ala Asn
                   805                 810                 815
   Ile Met Leu Leu Cys His Pro Glu Tyr Pro Arg Ala Glu Ser Ala Ala
               820                 825                 830
   Lys Val Ile Ala Glu Glu Leu Gly Ile Lys His Asp Trp Lys Asp Ile
           835                 840                 845
   Thr Phe Lys Glu Ala Thr Glu Glu Asp Val Lys Lys Ile Gln Leu Ala
       850                 855                 860
   Leu Gln Asp Glu Asp Ala Glu Pro Thr Gly Ser Asp Trp Ala Val Lys
   865                 870                 875                 880
   Met Gly Ile Asn Ile Tyr Tyr Ser Ala Lys Gln Ser Lys Ser Pro Leu
                   885                 890                 895
   Tyr Ser Lys Gln Ile Pro Tyr Asn Ser Ile Ile Tyr Lys Ala Phe Gly
               900                 905                 910
   Gln Glu Asn Pro Asp Ser Leu Thr Asp Tyr Gly Cys Gln Lys Ser Gly
           915                 920                 925
   Ser Thr Lys Lys Lys Val Ala Gly Trp Trp Cys Gly Lys Val Trp Met
       930                 935                 940
   Ser Asn Gln Val His Pro Leu Leu Ala Arg Glu Arg Glu Glu Gln Asn
   945                 950                 955                 960
   Ser Ser Val Val Tyr Gly Lys Ala Met Phe Thr Thr Ile Ser His Gly
                   965                 970                 975
   Lys Val Gln Asp Glu Ala Ser Thr Arg Cys Asn Thr Ser Asn Arg Thr
               980                 985                 990
   Pro Ser Arg Arg Thr Ser Arg Arg  Lys Lys Gly Val Ser  Ala Glu Lys
           995                 1000                1005
   Ser Lys  Pro Lys Asn Lys Arg  Ser Thr Ala Ser Asp  Glu Ala Ser
       1010                1015                1020
   Met Leu  Cys Ser Gly Leu Gly  Met Asn Ser Gly Val  Ile His Asp
       1025                1030                1035
   Gln Thr  Glu Asn Ser Asp Asp  Tyr Asp Lys His Gly  Asn Gly Asp
       1040                1045                1050
   Glu Ile  Glu Glu Gly Thr Asn  Pro Gln Lys Tyr Gln  Gln Arg Lys
       1055                1060                1065
   Leu Gln  Asn Val Thr Arg Lys  Ser Ser Ser Lys Lys  Arg Lys Asp
       1070                1075                1080
   Glu Lys  Arg Thr Asp Ser Phe  His Glu Leu Tyr Asp  Glu Asp Asn
       1085                1090                1095
   Gly Val  Asp Tyr Trp Leu Asn  Met Gly Ser Gly Asp  Asp Ala Thr
       1100                1105                1110
   Leu Gly  Asn Ser Arg Gln Gln  Ser Pro Asp Pro Val  Lys Val Lys
       1115                1120                1125
   Ser Gly  Gly Lys Leu Gln Gly  Lys Arg Lys Ser Ser  Lys Tyr Lys
       1130                1135                1140
   Ser Asn  Asp Asp Leu Leu Asn  Glu Glu Asn Lys Leu  Gln Lys Met
       1145                1150                1155
   Asn Lys  Lys Ser Ser Ser Lys  Lys Gln Lys Asn Asp  Lys Ile Asn
       1160                1165                1170
   Arg Gln  Leu Gln Glu Asp Gln  Thr Glu Asp Asp His  Met Asp His
       1175                1180                1185
   Leu Val  Asp Val Ala Val Ala  Asp Glu Val Thr Leu  Asp Asn Glu
       1190                1195                1200
   Asp Lys  Ile Thr Glu Asp Lys  Ile Asp Asp Val Lys  Val Lys Ser
       1205                1210                1215
   Arg Gly  Lys Ser Gln Asn Gly  Lys Arg Lys Gly Ser  Lys His Gln
       1220                1225                1230
   Ala Thr  Asp Gly Leu Arg Ala  Gly Asn Lys Val Ala  Lys Phe Pro
       1235                1240                1245
   Cys Asp  Ile Glu Gly Cys Asp  Met Ser Phe Ser Thr  Gln Gln Asp
       1250                1255                1260
   Leu Leu  Leu His Lys Arg Asp  Ile Cys Pro Val Lys  Gly Cys Lys
       1265                1270                1275
   Lys Lys  Phe Phe Cys His Lys  Tyr Leu Leu Gln His  Arg Lys Val
       1280                1285                1290
   His Ile  Asp Glu Arg Pro Leu  Lys Cys Thr Trp Lys  Gly Cys Lys
       1295                1300                1305
   Lys Ala  Phe Lys Trp Pro Trp  Ala Arg Thr Glu His  Met Arg Val
       1310                1315                1320
   His Thr  Gly Val Arg Pro Tyr  Glu Cys Gln Glu Pro  Gly Cys Gly
       1325                1330                1335
   Gln Thr  Phe Arg Phe Val Ser  Asp Phe Ser Arg His  Lys Arg Lys
       1340                1345                1350
   Thr Gly  His Ser Ser Asp Lys  Arg Arg Lys Asn Ser  Thr
       1355                1360                1365
<210> 97
   <211> 1242
   <212> DNA
   <213> Glycine max
<400> 97
   atgttgacga caactgtatc cggcggcgac cctccttctc gcggttttga tacgccgacg      60
   ctggaccggg aagcggcggc gctgctccac gcgatctccg agcatggcgg gtacgcgtac     120
   gtgagcatgg cggtgctggc ttccggcggc gacattcgcg cggcggaggc ggcgtgggag     180
   atggcgtggg agcagctgca ctcgggtccg tggcactcgg tgctgccggt gtggcgcgac     240
   gcttactcca tggcgtgcct ccacgtggcg cgccaccact acggcaacgg tgagttcttg     300
   gacgcactta gggttttgga tttgggaatc atcatgggag gcacgctcct ccgcaaggat     360
   ttggactccg ccatcgagaa aatgtcggaa caaacgagga agaccgttag ggtttctgat     420
   ttggggaact ccgagcaccg actcgtcgat cgcgaatttg atatggcaga ggtgctccaa     480
   cttttacctg tgaagtctct ttcaacgaaa cttgtggtga agaaatcggc gctgtccttg     540
   gagaaattcc tgaaggatca ttacctgtct ggctgcccgg ttattatcag tgattgtatg     600
   tctcactggc cagccaagat gaaatggaat gacgaagatt acttgctgag agttgccgga     660
   gaccgtacag ttccagttga ggttgggaaa aactatttat gtactgagtg gaagcaagag     720
   ctaattactt tttcagagtt tcttcagcgg ataaagtctg atagctgttc tcctggtggt     780
   cctacatatc ttgctcagca tccattattt gatcagataa atgagcttcg gaaagatatc     840
   tttattcctg actattgttt tactggtgga ggggagctac gatctctcaa tgcttggttt     900
   ggtccagcag gaacagtaac accgttacac catgatccac atcataacat actagctcag     960
   gttgttggaa agaaatacat taggctatac tcttcgtctt tatctgagga actttccccc    1020
   cactctggta ccatgctcca caactccagc caggttgatt tagatgatat ggatgaaaag    1080
   aagtttccga aggtgcaaga cttggaattt gtagactgta ttttagagga aggcgaaatg    1140
   ttatatatcc cgccaaaatg gtggcactat gtgcggtctt tgactaccag tttttcggtt    1200
   agcttttggt ggagtgaggg tgaaagttcg gatgcatcct aa                       1242
<210> 98
   <211> 413
   <212> PRT
   <213> Glycine max
<400> 98
   Met Leu Thr Thr Thr Val Ser Gly Gly Asp Pro Pro Ser Arg Gly Phe
   1               5                   10                  15
   Asp Thr Pro Thr Leu Asp Arg Glu Ala Ala Ala Leu Leu His Ala Ile
               20                  25                  30
   Ser Glu His Gly Gly Tyr Ala Tyr Val Ser Met Ala Val Leu Ala Ser
           35                  40                  45
   Gly Gly Asp Ile Arg Ala Ala Glu Ala Ala Trp Glu Met Ala Trp Glu
       50                  55                  60
   Gln Leu His Ser Gly Pro Trp His Ser Val Leu Pro Val Trp Arg Asp
   65                  70                  75                  80
   Ala Tyr Ser Met Ala Cys Leu His Val Ala Arg His His Tyr Gly Asn
                   85                  90                  95
   Gly Glu Phe Leu Asp Ala Leu Arg Val Leu Asp Leu Gly Ile Ile Met
               100                 105                 110
   Gly Gly Thr Leu Leu Arg Lys Asp Leu Asp Ser Ala Ile Glu Lys Met
           115                 120                 125
   Ser Glu Gln Thr Arg Lys Thr Val Arg Val Ser Asp Leu Gly Asn Ser
       130                 135                 140
   Glu His Arg Leu Val Asp Arg Glu Phe Asp Met Ala Glu Val Leu Gln
   145                 150                 155                 160
   Leu Leu Pro Val Lys Ser Leu Ser Thr Lys Leu Val Val Lys Lys Ser
                   165                 170                 175
   Ala Leu Ser Leu Glu Lys Phe Leu Lys Asp His Tyr Leu Ser Gly Cys
               180                 185                 190
   Pro Val Ile Ile Ser Asp Cys Met Ser His Trp Pro Ala Lys Met Lys
           195                 200                 205
   Trp Asn Asp Glu Asp Tyr Leu Leu Arg Val Ala Gly Asp Arg Thr Val
       210                 215                 220
   Pro Val Glu Val Gly Lys Asn Tyr Leu Cys Thr Glu Trp Lys Gln Glu
   225                 230                 235                 240
   Leu Ile Thr Phe Ser Glu Phe Leu Gln Arg Ile Lys Ser Asp Ser Cys
                   245                 250                 255
   Ser Pro Gly Gly Pro Thr Tyr Leu Ala Gln His Pro Leu Phe Asp Gln
               260                 265                 270
   Ile Asn Glu Leu Arg Lys Asp Ile Phe Ile Pro Asp Tyr Cys Phe Thr
           275                 280                 285
   Gly Gly Gly Glu Leu Arg Ser Leu Asn Ala Trp Phe Gly Pro Ala Gly
       290                 295                 300
   Thr Val Thr Pro Leu His His Asp Pro His His Asn Ile Leu Ala Gln
   305                 310                 315                 320
   Val Val Gly Lys Lys Tyr Ile Arg Leu Tyr Ser Ser Ser Leu Ser Glu
                   325                 330                 335
   Glu Leu Ser Pro His Ser Gly Thr Met Leu His Asn Ser Ser Gln Val
               340                 345                 350
   Asp Leu Asp Asp Met Asp Glu Lys Lys Phe Pro Lys Val Gln Asp Leu
           355                 360                 365
   Glu Phe Val Asp Cys Ile Leu Glu Glu Gly Glu Met Leu Tyr Ile Pro
       370                 375                 380
   Pro Lys Trp Trp His Tyr Val Arg Ser Leu Thr Thr Ser Phe Ser Val
   385                 390                 395                 400
   Ser Phe Trp Trp Ser Glu Gly Glu Ser Ser Asp Ala Ser
                   405                 410